# EUROPEAN PATENT APPLICATION

(11) **EP 4 624 471 A1**
(43) Date of publication of application: **01.10.2025**
(21) Application number: 23894031.6
(22) Date of filing: 24.11.2023
(51) Int. Cl.: C07D 487/04, C07D 471/04

(54) **PKMYT1 INHIBITOR, PREPARATION METHOD THEREFOR, AND PHARMACEUTICAL COMPOSITION AND USE THEREOF**

(30) Priority: 25.11.2022 CN 202211488114; 09.05.2023 CN 202310519621; 04.09.2023 CN 202311134285; 09.11.2023 CN 202311494322
(71) Applicant: Shanghai Qilu Pharmaceutical Research and Development Centre Ltd., Shanghai 201203 (CN)
(72) Inventor: CHEN, Jianping, Shanghai 201203 (CN); FU, Jiasheng, Shanghai 201203 (CN); SUN, Daqing, Shanghai 201203 (CN); ZHANG, Yuxing, Shanghai 201203 (CN); WANG, Rongyan, Shanghai 201203 (CN); SUN, Weimei, Shanghai 201203 (CN); QIN, Hua, Shanghai 201203 (CN); SHI, Guqin, Shanghai 201203 (CN); BAO, Jinxiao, Shanghai 201203 (CN); LEI, Guangyue, Shanghai 201203 (CN); TAO, Weikang, Shanghai 201203 (CN)
(74) Representative: Dai, Simin
(86) International application number: PCT/CN2023/134116
(87) International publication number: WO 2024/109942

(57) **Abstract**

The present invention belongs to the field of medicine. Disclosed are a PKMYT1 inhibitor, a preparation method therefor, and a pharmaceutical composition and the use thereof. Provided in the present invention is a compound as represented by the below general formula (I), a pharmaceutically acceptable salt thereof, or an isomer thereof. The PKMYT1 inhibitor of the present invention can be used for treating neoplastic diseases harboring CCNE1 amplification or FBXW7 inactivating mutations.

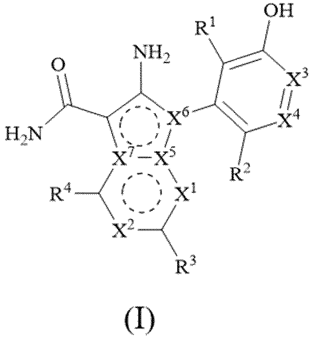

## Description

The present application claims the right of the priorities of Chinese patent application 2022114881146 filed on November 25, 2022, Chinese patent application 202310519621X filed on May 9, 2023, Chinese patent application 2023111342853 filed on September 4, 2023, and Chinese patent application 2023114943221 filed on November 9, 2023. The contents of the above Chinese patent applications are incorporated herein by reference in their entireties.

### TECHNICAL FIELD

The present disclosure belongs to the field of medicine, and specifically relates to a PKMYT1 inhibitor compound, a pharmaceutically acceptable salt thereof, an isomer thereof, a preparation method therefor, a pharmaceutical composition thereof, and a medical use thereof.

### BACKGROUND

Cyclin E is a cyclin of cyclin-dependent kinase 2 (CDK2). It binds to CDK2 in the G1 phase to form an activated CDK2-Cyclin E complex, which promotes the transition of the cell cycle from the G1 phase to the S phase at the initiation of DNA replication. The Cyclin E1 (CCNE1) gene encodes the main protein of Cyclin E, and thus plays an important role in regulating the transition from the G1 phase to the S phase of the cell cycle. Some studies suggest that overexpression of CCNE1 protein can lead to an increase in the expression level of Cyclin E protein encoded by the CCNE1 gene, thereby enhancing the activity of the CDK2-Cyclin E complex, triggering premature cell cycle transition, increasing DNA replication pressure and genomic instability. The amplification of CCNE1 is common in many tumor types, especially in highly malignant gynecological and digestive tract cancers, such as high-grade serous ovarian cancer (HGSOC), uterine cancer and gastric esophageal cancer, and is associated with cytotoxicity and resistance to targeted therapies.

CCNE1 itself is not considered a druggable target. Current research is treatment with multi-target CDK inhibitors by acting on its downstream cell cycle protein CDK2. Therefore, the lack of treatment options for CCNE1-amplified tumors has become a key unmet need in the development of new therapies for these types of tumors. In order to study the therapeutic targets of CCNE1-amplified tumors, the Mount Sinai Hospital in Toronto, Canada, the University of Toronto, and Repare Therapeutics in the United States collaborated to find that CCNE1 amplification and PKMYT1 inhibition constitute a synthetic lethal pair. This result was published in the journal Nature on April 20, 2022. They performed a genome-scale CRISPR-Cas9-based synthetic lethality screen in a CCNE1-amplified cell model. The results of the study showed that PKMYT1 is essential in CCNE1-amplified cells, but not in other healthy cells with normal levels of CCNE1. Therefore, PKMYT1 is a synthetic lethal gene of CCNE1, and PKMYT1 inhibitors can be used to treat CCNE1-amplified tumor diseases. Furthermore, the protein encoded by the FBXW7 gene is the target protein recognition component of the cullin-RING ubiquitin ligase. The FBXW7 protein targets and binds to CCNE1 through the ubiquitin-dependent protein degradation pathway. Therefore, FBXW7 inactivating mutations will lead to increased CCNE1 levels. PKMYT1 inhibitors can also be used to treat tumor diseases with FBXW7 inactivating mutation.

PKMYT1 kinase, also known as MYT1, whose full name is "membrane-associated tyrosine- and threonine-specific cdc2-inhibitory kinase", is a member of the WEE family kinase. It inactivates the CDK1-Cyclin B complex by phosphorylating the threonine 14 site of CDK1 kinase during cell cycle transition, negatively regulates the cell cycle checkpoint from G2 to M, and has an important impact on tumor cell proliferation, migration, and xenograft tumor formation.

Based on the discovery of the synthetic lethal relationship between CCNE1 and PKMYT1, the research team of Mount Sinai Hospital in Toronto developed a selective PKMYT1 inhibitor drug RP-6306, which showed single-agent activity and persistent tumor regression when combined with gemcitabine in CCNE1 amplification model. RP-6306 treatment leads to the selective unplanned activation of CDK1 in CCNE1 overexpressing cells and promotes the early mitosis in cells with DNA synthesis. CCNE1 overexpression disrupts the stability of CDK1 at least in part through early activation of the MMB-FOXM1 mitotic transcriptional program. They concluded that PKMYT1 inhibition is a promising strategy for treating CCNE1-amplified cancers.

Currently, RP-6306 is still in the clinical stage and there are few drug options. Therefore, it is still of great clinical significance to continue to develop selective PKMYT1 inhibitors, enrich the types of drugs, and increase drug accessibility.

### SUMMARY

The problem to be solved by the present disclosure is to provide a PKMYT1 inhibitor with a novel structure in order to solve one or more shortcomings of existing PKMYT1 inhibitors, such as limited number of PKMYT1 inhibitors, low drug accessibility, low PKMYT1 enzyme inhibitory activity, low HCC1569 cell inhibitory activity, short half-life, fast clearance rate, poor metabolic stability, poor anti-tumor activity, and poor solubility. The PKMYT1 inhibitor of the present disclosure has one or more advantages such as high PKMYT1 enzyme inhibitory activity, high HCC1569 cell inhibitory activity, long half-life, slow clearance rate, good metabolic stability, good anti-tumor activity, and good solubility, and can be used to treat tumor diseases with CCNE1 amplification or FBXW7 inactivating mutation.

The present disclosure mainly solves the above technical problems through the following technical solutions.

In a first aspect, the present disclosure provides a compound represented by the following general formula (I), a pharmaceutically acceptable salt thereof, or an isomer thereof, 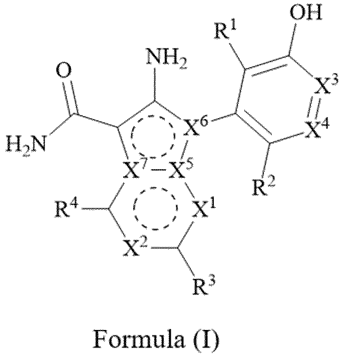 wherein
X¹ is N or CR⁵;
X² is N or CR⁶;
X³ is N or CR⁷;
X⁴ is N or CR⁸;
X⁵ is N or C;
X⁶ is N or C;
X⁷ is N or C;
provided that when X² is CR⁶, X⁵ is C, X⁶ is N, and X⁷ is C, then X³ and X⁴ are not simultaneously CH;
R¹ and R² are each independently halogen, C₁₋₄ alkyl, C₁₋₄ alkoxy, hydroxy-C₁₋₄ alkyl, or deuterated C₁₋₄ alkyl;
R³ is -H, -CN, -OH, -N(R^{a})(R^{b}), halogen, C₁₋₄ alkyl, halo-C₁₋₄ alkyl, C₁₋₄ alkoxy, C₂₋₄ alkenyl, C₂₋₄ alkynyl, -L-R^{c}, phenyl, C₃₋₆ cycloalkyl, C₃₋₆ cycloalkenyl, 3- to 6-membered heterocyclyl, 3- to 6-membered heterocycloalkenyl, deuterated C₁₋₄ alkyl, or 5- to 6-membered heteroaryl; wherein the phenyl, C₃₋₆ cycloalkyl, C₃₋₆ cycloalkenyl, 3- to 6-membered heterocyclyl, 3- to 6-membered heterocycloalkenyl, and 5- to 6-membered heteroaryl are optionally substituted by one or more R^{3a}, and the R^{3a} is halogen, C₁₋₄ alkyl, or C₁₋₄ alkoxy;
L is C₂₋₄ alkynylene, C₁₋₄ alkylene, or C₂₋₄ alkenylene;
R^{c} is C₁₋₄ alkyl, C₃₋₆ cycloalkyl, or 4- to 6-membered heterocyclyl, and the C₁₋₄ alkyl, C₃₋₆ cycloalkyl, and 4- to 6-membered heterocyclyl are optionally substituted by one or more R^{ca}; the R^{ca} is halogen or -OH;
R^{a} and R^{b} are each independently -H, C₁₋₄ alkyl, phenyl, p-methoxybenzyl, C₁₋₄ alkyl-C(O)-, C₃₋₆ cycloalkyl, or halo-C₁₋₄ alkyl, or R^{a} and R^{b} together with the nitrogen atom to which they are attached form a 4- to 6-membered heterocyclyl ring, and the 4- to 6-membered heterocyclyl is optionally substituted by halogen;
R⁴ is -H, halogen, C₁₋₄ alkyl, C₃₋₆ cycloalkyl, C₁₋₄ alkoxy, or halo-C₁₋₄ alkyl;
R⁵, R⁷, and R⁸ are each independently -H, -OH, -CN, -N(R^{d})(R^{e}), halogen, C₁₋₄ alkyl, C₁₋₄ alkoxy, or halo-C₁₋₄ alkyl;
R⁶ is -H, -OH, -CN, -N(R^{d})(R^{e}), halogen, C₁₋₄ alkyl, C₁₋₄ alkoxy, -COOH, halo-C₁₋₄ alkyl, halo-C₁₋₄ alkoxy, C₂₋₄ alkenyl, C₂₋₄ alkynyl, C₁₋₄ alkyl-S(O)₂-, hydroxy-C₁₋₄ alkyl, C₃₋₆ cycloalkyl, C₅₋₆ cycloalkenyl, C₃₋₆ cycloalkyl-C₁₋₄ alkyl, C₃₋₆ cycloalkyl-O-, 3- to 6-membered heterocyclyl, 3- to 6-membered heterocycloalkenyl, phenyl, 5- to 6-membered heteroaryl, or - CD₃; wherein the C₁₋₄ alkyl, C₃₋₆ cycloalkyl, C₅₋₆ cycloalkenyl, C₃₋₆ cycloalkyl-C₁₋₄ alkyl, C₃₋₆ cycloalkyl-O-, 3- to 6-membered heterocyclyl, 3- to 6-membered heterocycloalkenyl, phenyl, and 5- to 6-membered heteroaryl are optionally substituted by one or more R^{6a}, and the R^{6a} is halogen, C₁₋₄ alkyl, halo-C₁₋₄ alkyl, or C₁₋₄ alkoxy;
R^{d} and R^{e} are each independently -H, C₁₋₄ alkyl, -S(O)₂-N(R^{f})(R^{g}), -C(O)-aryl, -C(O)-NR^{f}-aryl, or -(C=S)-NR^{f}-aryl;
R^{f} and R^{g} are each independently -H or C₁₋₄ alkyl;
or, R² and R⁸ together with the atom to which they are attached form a C₅₋₆ cycloalkenyl ring;
and/or, R³ and R⁶ together with the atom to which they are attached form a phenyl, 5-to 6-membered heterocycloalkenyl, C₅₋₆ cycloalkenyl, or 5- to 6-membered heteroaryl ring;
and/or, R⁴ and R⁶ together with the atom to which they are attached form a phenyl, 5-to 6-membered heterocycloalkenyl, C₅₋₆ cycloalkenyl, or 5- to 6-membered heteroaryl ring;
the phenyl, 5- to 6-membered heteroaryl, 5- to 6-membered heterocycloalkenyl, and C₅₋₆ cycloalkenyl are optionally further substituted by one or more substituents selected from halogen, C₁₋₄ alkyl, halo-C₁₋₄ alkyl, C₁₋₄ alkoxy, and C₁₋₆ alkyl-C(O)-.

In another aspect, the present disclosure provides a compound represented by the following general formula (I), a pharmaceutically acceptable salt thereof, or an isomer thereof, 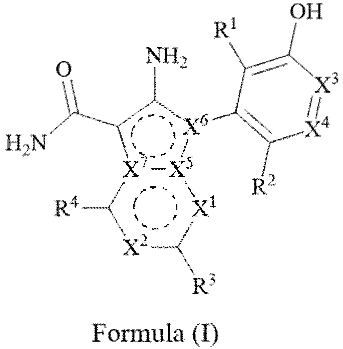 wherein
X¹ is N or CR⁵;
X² is N or CR⁶;
X³ is N or CR⁷;
X⁴ is N or CR⁸;
X⁵ is N or C;
X⁶ is N or C;
X⁷ is N or C;
provided that when X² is CR⁶, X⁵ is C, X⁶ is N, and X⁷ is C, then X³ and X⁴ are not simultaneously CH;
R¹ and R² are each independently halogen, C₁₋₄ alkyl, C₁₋₄ alkoxy, hydroxy-C₁₋₄ alkyl, or deuterated C₁₋₄ alkyl;
R³ is -H, -CN, -OH, -N(R^{a})(R^{b}), halogen, C₁₋₄ alkyl, halo-C₁₋₄ alkyl, C₁₋₄ alkoxy, C₂₋₄ alkenyl, C₂₋₄ alkynyl, -L-R^{c}, phenyl, C₃₋₆ cycloalkyl, C₃₋₆ cycloalkenyl, 3- to 6-membered heterocyclyl, 3- to 6-membered heterocycloalkenyl, deuterated C₁₋₄ alkyl, or 5- to 6-membered heteroaryl; wherein the phenyl, C₃₋₆ cycloalkyl, C₃₋₆ cycloalkenyl, 3- to 6-membered heterocyclyl, 3- to 6-membered heterocycloalkenyl, and 5- to 6-membered heteroaryl are optionally substituted by one or more R^{3a}, and the R^{3a} is halogen, C₁₋₄ alkyl, or C₁₋₄ alkoxy;
L is C₂₋₄ alkynylene, C₁₋₄ alkylene, or C₂₋₄ alkenylene;
R^{c} is C₁₋₄ alkyl, C₃₋₆ cycloalkyl, or 4- to 6-membered heterocyclyl, and the C₁₋₄ alkyl, C₃₋₆ cycloalkyl, and 4- to 6-membered heterocyclyl are optionally substituted by one or more R^{ca}; the R^{ca} is halogen or -OH;
R^{a} and R^{b} are each independently -H, C₁₋₄ alkyl, phenyl, p-methoxybenzyl, C₁₋₄ alkyl-C(O)-, C₃₋₆ cycloalkyl, or halo-C₁₋₄ alkyl, or R^{a} and R^{b} together with the nitrogen atom to which they are attached form a 4- to 6-membered heterocyclyl ring, and the 4- to 6-membered heterocyclyl is optionally substituted by halogen;
R⁴ is -H, halogen, C₁₋₄ alkyl, C₃₋₆ cycloalkyl, C₁₋₄ alkoxy, or halo-C₁₋₄ alkyl;
R⁵, R⁷, and R⁸ are each independently -H, -OH, -CN, -N(R^{d})(R^{e}), halogen, C₁₋₄ alkyl, C₁₋₄ alkoxy, or halo-C₁₋₄ alkyl;
R⁶ is -H, -OH, -CN, -N(R^{d})(R^{e}), halogen, C₁₋₄ alkyl, C₁₋₄ alkoxy, -COOH, halo-C₁₋₄ alkyl, halo-C₁₋₄ alkoxy, C₂₋₄ alkenyl, C₂₋₄ alkynyl, C₁₋₄ alkyl-S(O)₂-, hydroxy-C₁₋₄ alkyl, C₃₋₆ cycloalkyl, C₅₋₆ cycloalkenyl, C₃₋₆ cycloalkyl-C₁₋₄ alkyl, C₃₋₆ cycloalkyl-O-, 3- to 6-membered heterocyclyl, 3- to 6-membered heterocycloalkenyl, phenyl, 5- to 6-membered heteroaryl, or - CD₃; wherein the C₁₋₄ alkyl, C₃₋₆ cycloalkyl, C₅₋₆ cycloalkenyl, C₃₋₆ cycloalkyl-C₁₋₄ alkyl, C₃₋₆ cycloalkyl-O-, 3- to 6-membered heterocyclyl, 3- to 6-membered heterocycloalkenyl, phenyl, and 5- to 6-membered heteroaryl are optionally substituted by one or more R^{6a}, and the R^{6a} is halogen, C₁₋₄ alkyl, halo-C₁₋₄ alkyl, or C₁₋₄ alkoxy;
R^{d} and R^{e} are each independently -H, C₁₋₄ alkyl, -S(O)₂-N(R^{f})(R^{g}), -C(O)-aryl, -C(O)-NR^{f}-aryl, or -(C=S)-NR^{f}-aryl;
R^{f} and R^{g} are each independently -H or C₁₋₄ alkyl;
or, R² and R⁸ together with the atom to which they are attached form a C₅₋₆ cycloalkenyl ring;
and/or, R³ and R⁶ together with the atom to which they are attached form a phenyl, 5-to 6-membered heterocycloalkenyl, C₅₋₆ cycloalkenyl, or 5- to 6-membered heteroaryl ring;
and/or, R⁴ and R⁶ together with the atom to which they are attached form a phenyl, 5-to 6-membered heterocycloalkenyl, C₅₋₆ cycloalkenyl, or 5- to 6-membered heteroaryl ring;
the phenyl, 5- to 6-membered heteroaryl, 5- to 6-membered heterocycloalkenyl, and C₅₋₆ cycloalkenyl are optionally further substituted by one or more substituents selected from halogen, C₁₋₄ alkyl, halo-C₁₋₄ alkyl, and C₁₋₄ alkoxy.

In some embodiments,
X¹ is N or CR⁵;
X² is N or CR⁶;
X³ is N or CR⁷;
X⁴ is N or CR⁸;
X⁵ is N or C;
X⁶ is N or C;
X⁷ is N or C;
provided that when X² is CR⁶, X⁵ is C, X⁶ is N, and X⁷ is C, then X³ and X⁴ are not simultaneously CH;
R¹ and R² are each independently halogen, C₁₋₄ alkyl, C₁₋₄ alkoxy, or hydroxy-C₁₋₄ alkyl;
R³ is -H, -CN, -OH, -N(R^{a})(R^{b}), halogen, C₁₋₄ alkyl, halo-C₁₋₄ alkyl, C₁₋₄ alkoxy, C₂₋₄ alkenyl, C₂₋₄ alkynyl, -L-R^{c}, phenyl, C₃₋₆ cycloalkyl, C₃₋₆ cycloalkenyl, 3- to 6-membered heterocyclyl, or 3- to 6-membered heterocycloalkenyl; wherein the phenyl, C₃₋₆ cycloalkyl, C₃₋₆ cycloalkenyl, 3- to 6-membered heterocyclyl, and 3- to 6-membered heterocycloalkenyl are optionally substituted by one or more R^{3a}, and the R^{3a} is halogen, C₁₋₄ alkyl, or, C₁₋₄ alkoxy;
L is C₂₋₄ alkynylene, C₁₋₄ alkylene, or C₂₋₄ alkenylene;
R^{c} is C₁₋₄ alkyl, C₃₋₆ cycloalkyl, or 4- to 6-membered heterocyclyl, and the C₁₋₄ alkyl, C₃₋₆ cycloalkyl, and 4- to 6-membered heterocyclyl are optionally substituted by one or more R^{ca}; the R^{ca} is halogen or -OH;
R^{a} and R^{b} are each independently -H, C₁₋₄ alkyl, phenyl, p-methoxybenzyl, C₁₋₄ alkyl-C(O)-, C₃₋₆ cycloalkyl, or halo-C₁₋₄ alkyl, or R^{a} and R^{b} together with the nitrogen atom to which they are attached form a 4- to 6-membered heterocyclyl ring, and the 4- to 6-membered heterocyclyl is optionally substituted by halogen;
R⁴ is -H, halogen, C₁₋₄ alkyl, or C₃₋₆ cycloalkyl;
R⁵, R⁷, and R⁸ are each independently -H, -OH, -CN, -N(R^{d})(R^{e}), halogen, C₁₋₄ alkyl, C₁₋₄ alkoxy, or halo-C₁₋₄ alkyl;
R⁶ is -H, -OH, -CN, -N(R^{d})(R^{e}), halogen, C₁₋₄ alkyl, C₁₋₄ alkoxy, -COOH, halo-C₁₋₄ alkyl, halo-C₁₋₄ alkoxy, C₂₋₄ alkenyl, C₂₋₄ alkynyl, C₁₋₄ alkyl-S(O)₂-, hydroxy-C₁₋₄ alkyl, C₃₋₆ cycloalkyl, C₅₋₆ cycloalkenyl, C₃₋₆ cycloalkyl-C₁₋₄ alkyl, C₃₋₆ cycloalkyl-O-, 3- to 6-membered heterocyclyl, 3- to 6-membered heterocycloalkenyl, phenyl, or 5- to 6-membered heteroaryl; wherein the C₁₋₄ alkyl, C₃₋₆ cycloalkyl, C₅₋₆ cycloalkenyl, C₃₋₆ cycloalkyl-C₁₋₄ alkyl, C₃₋₆ cycloalkyl-O-, 3- to 6-membered heterocyclyl, 3- to 6-membered heterocycloalkenyl, phenyl, and 5- to 6-membered heteroaryl are optionally substituted by one or more R^{6a}, and the R^{6a} is halogen, C₁₋₄ alkyl, halo-C₁₋₄ alkyl, or C₁₋₄ alkoxy;
R^{d} and R^{e} are each independently -H, C₁₋₄ alkyl, -S(O)₂-N(R^{f})(R^{g}), -C(O)-aryl, -C(O)-NR^{f}-aryl, or -(C=S)-NR^{f}-aryl;
R^{f} and R^{g} are each independently -H or C₁₋₄ alkyl;
or, R² and R⁸ together with the atom to which they are attached form a C₅₋₆ cycloalkenyl ring;
and/or, R³ and R⁶ together with the atom to which they are attached form a phenyl, 5-to 6-membered heterocycloalkenyl, or C₅₋₆ cycloalkenyl ring;
and/or, R⁴ and R⁶ together with the atom to which they are attached form a phenyl, 5-to 6-membered heterocycloalkenyl, or C₅₋₆ cycloalkenyl ring.

In a preferred embodiment of formula (I), the compound, the pharmaceutically acceptable salt thereof, or the isomer thereof, 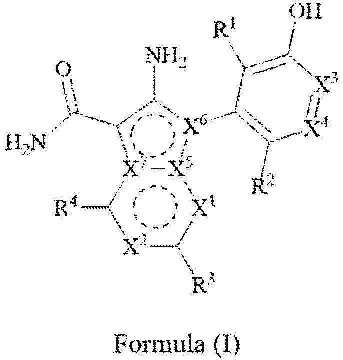 wherein
X¹ is N or CR⁵;
X² is N or CR⁶;
X³ is N or CR⁷;
X⁴ is N or CR⁸;
X⁵ is N or C;
X⁶ is N or C;
X⁷ is N or C;
provided that when X² is CR⁶, X⁵ is C, X⁶ is N, and X⁷ is C, then X³ and X⁴ are not simultaneously CH;
R¹ and R² are each independently C₁₋₄ alkyl;
R³ is -H, -CN, -N(R^{a})(R^{b}), halogen, C₁₋₄ alkyl, halo-C₁₋₄ alkyl, C₁₋₄ alkoxy, C₂₋₄ alkenyl, C₂₋₄ alkynyl, -L-R^{c}, phenyl, C₃₋₆ cycloalkyl, C₃₋₆ cycloalkenyl, 3- to 6-membered heterocyclyl, or 3- to 6-membered heterocycloalkenyl;
L is C₂₋₄ alkynylene;
R^{c} is C₁₋₄ alkyl, C₃₋₆ cycloalkyl, or 4- to 6-membered heterocyclyl, and the C₁₋₄ alkyl, C₃₋₆ cycloalkyl, and 4- to 6-membered heterocyclyl are optionally substituted by one or more R^{ca}; the R^{ca} is halogen or -OH;
R^{a} and R^{b} are each independently -H, C₁₋₄ alkyl, phenyl, or *p*-methoxybenzyl, or R^{a} and R^{b} together with the nitrogen atom to which they are attached form a 4- to 6-membered heterocyclyl ring, and the 4- to 6-membered heterocyclyl is optionally substituted by halogen;
R⁴ is -H, halogen, or C₁₋₄ alkyl;
R⁵, R⁶, R⁷, and R⁸ are each independently -H, -OH, -CN, -N(R^{d})(R^{e}), halogen, C₁₋₄ alkyl, or C₁₋₄ alkoxy;
R^{d} and R^{e} are each independently -H or C₁₋₄ alkyl;
or R² and R⁸ together with the atom to which they are attached form a C₅₋₆ cycloalkenyl ring.

In another aspect, the present disclosure provides a compound represented by the following general formula (I), 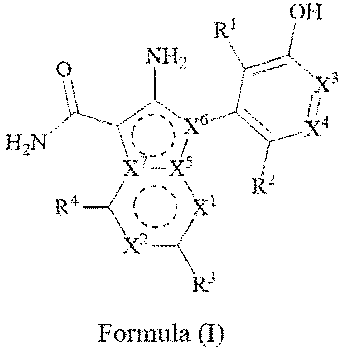 wherein
X¹ is N or CR⁵;
X² is N or CR⁶;
X³ is N or CR⁷;
X⁴ is N or CR⁸;
X⁵ is N or C;
X⁶ is N or C;
X⁷ is N or C;
provided that when X² is CR⁶, X⁵ is C, X⁶ is N, and X⁷ is C, then X³ and X⁴ are not simultaneously CH;
R¹ and R² are each independently C₁₋₄ alkyl;
R³ is -H, -CN, -N(R^{a})(R^{b}), halogen, C₁₋₄ alkyl, halo-C₁₋₄ alkyl, C₁₋₄ alkoxy, C₁₋₄ alkenyl, C₁₋₄ alkynyl, -L-R^{c}, phenyl, C₃₋₆ cycloalkyl, C₃₋₆ cycloalkenyl, 3- to 6-membered heterocyclyl, or 3- to 6-membered heterocycloalkenyl;
L is C₁₋₄ alkynylene;
R^{c} is C₁₋₄ alkyl, C₃₋₆ cycloalkyl, or 4- to 6-membered heterocyclyl, and the C₁₋₄ alkyl, C₃₋₆ cycloalkyl, and 4- to 6-membered heterocyclyl are optionally substituted by halogen or - OH;
R^{a} and R^{b} are each independently -H, C₁₋₄ alkyl, phenyl, or *p*-methoxybenzyl, or R^{a} and R^{b} together with the nitrogen atom to which they are attached form a 4- to 6-membered heterocyclyl ring, and the 4- to 6-membered heterocyclyl is optionally substituted by halogen;
R⁴ is -H, halogen, or C₁₋₄ alkyl;
R⁵, R⁶, R⁷, and R⁸ are each independently -H, -OH, -CN, -N(R^{d})(R^{e}), halogen, C₁₋₄ alkyl, or C₁₋₄ alkoxy;
R^{d} and R^{e} are each independently -H or C₁₋₄ alkyl;
or, R² and R⁸ together with the atom to which they are attached form a 5- to 6-membered cycloalkenyl ring.

In a preferred embodiment of formula (I), X¹ is CR⁵, X² is N, X⁵ is C, X⁶ is N, and X⁷ is C.

In a preferred embodiment of formula (I), X¹ and X² are both N, X⁵ is C, X⁶ is N, and X⁷ is C.

In a preferred embodiment of formula (I), R¹ and R² are each independently -CH₃, - CH₂CH₃, -CH(CH₃)₂, -Cl, -F, -Br, -OCH₃, -CH₂OH, or -CD₃.

In a preferred embodiment of formula (I), R¹ and R² are each independently -CH₃, - CH₂CH₃, or -CH(CH₃)₂.

In a preferred embodiment of formula (I), R¹ and R² are each independently -Cl, -F, - Br, -OCH₃, or -CH₂OH.

In a preferred embodiment of formula (I), R¹ and R² are both -CH₃.

In a preferred embodiment of formula (I), R¹ and R² are each independently -CD₃.

In a preferred embodiment of formula (I), R¹ and R² are both -CH₃ or -CD₃.

In a preferred embodiment of formula (I), R³ is -H, -CN, -NH₂, -F, -Br, -Cl, -CH₃, - CH₂CH₃, -CH₂CH(CH₃)₂, -CF₃, -OCH₃, -OCH₂CH₃, -CH=CH₂, -N(CH₃)₂, -N(CH₂CH₃)₂, - NHPMB, phenyl, cyclopropyl, cyclobutyl, cyclopentyl, cyclohexyl, 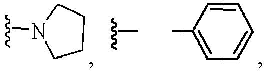 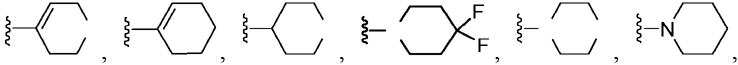 ethynyl, -C=C-R^{c}, - OH, -CH(CH₃)₂, -CH₂CH₂CH₃, -CH₂CH(CH₃)₂, -OCH₃, -CH₂F, -CHF₂, -CH=CH₂, - CH=C(CH₃)₂, -CH=CHCH₃, -C(CH₃)=CH₂, -NHCH₃, -NHCH₂CH₃, -NHC(O)CH₃, 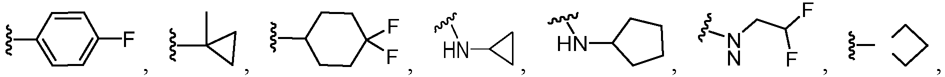 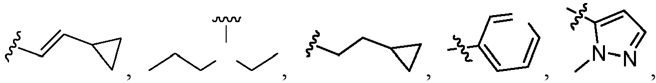 or -CD₃; R^{c} is the following group optionally substituted by one or more R^{ca}: methyl, ethyl, cyclopropyl, cyclobutyl, isopropyl, oxetanyl, or azetidinyl; the R^{ca} is -F, -Cl, or -OH.

In a preferred embodiment of formula (I), R³ is -H, -CN, -NH₂, -F, -Br, -Cl, -CH₃, - CH₂CH₃, -CH₂CH(CH₃)₂, -CF₃, -OCH₃, -OCH₂CH₃, -CH=CH₂, -N(CH₃)₂, -N(CH₂CH₃)₂, - NHPMB, phenyl, cyclopropyl, cyclobutyl, cyclopentyl, cyclohexyl, 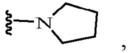 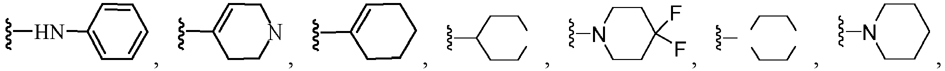 ethynyl, or -C=C-R^{c}, R^{c} is the following group optionally substituted by one or more R^{ca}: methyl, ethyl, cyclopropyl, cyclobutyl, isopropyl, oxetanyl, or azetidinyl; the R^{ca} is -F, -Cl, or -OH.

In a preferred embodiment of formula (I), R³ is -OH, -CH(CH₃)₂, -CH₂CH₂CH₃, - CH₂CH(CH₃)₂, -OCH₃, -CH₂F, -CHF₂, -CH=CH₂, -CH=C(CH₃)₂, -CH=CHCH₃, -C(CH₃)=CH₂, -NHCH₃, -NHCH₂CH₃, -NHC(O)CH₃,

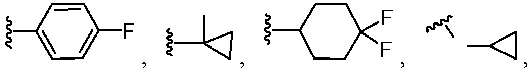

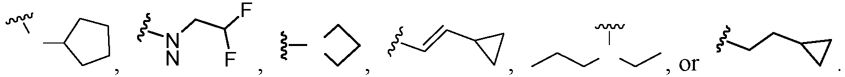

In a preferred embodiment of formula (I), R³ is 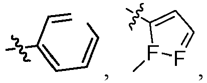 or -CD₃.

In a preferred embodiment of formula (I), -C≡C-R^{c} is -C=C-CH₃,

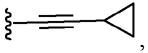

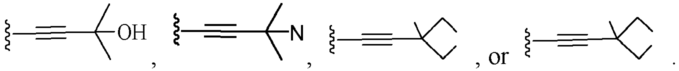

In a preferred embodiment of formula (I), R⁴ is -H, -Cl, -F, -Br, -CH₃, -CH₂CH₃, cyclopropyl, methoxy, or -CH₂CF₃.

In a preferred embodiment of formula (I), R⁴ is -H, -Cl, -F, -Br, -CH₃, or -CH₂CH₃.

In a preferred embodiment of formula (I), R⁴ is cyclopropyl.

In a preferred embodiment of formula (I), R⁴ is -H.

In a preferred embodiment of formula (I), R⁴ is methoxy or -CH₂CF₃.

In a preferred embodiment of formula (I), wherein R⁵ is -H, -Cl, -F, -CH₃, -CN, -CF₃, or -OCH₃.

In a preferred embodiment of formula (I), wherein R⁵ is -H, -Cl, -F, -CH₃, or -OCH₃.

In a preferred embodiment of formula (I), wherein R⁷ and R⁸ are each independently -H, -OH, -CN, -NH₂, -F, -Br, -Cl, -CH₃, -CH₂CH₃, -OCH₃, or -OCH₂CH₃.

In a preferred embodiment of formula (I), R⁷ and R⁸ are both H.

In a preferred embodiment of formula (I), R⁷ and R⁸ are both F.

In a preferred embodiment of formula (I), the compound, the pharmaceutically acceptable salt thereof, or the isomer thereof has any one of the following structures: 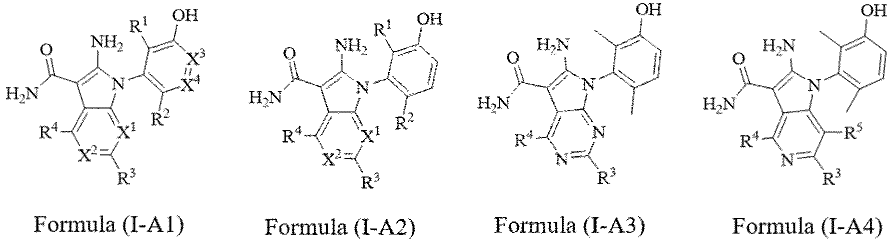 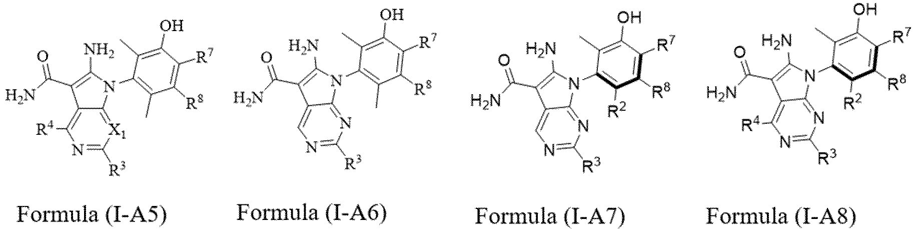 wherein X¹, X², R¹, R², R³, R⁴, R⁵, R⁷, and R⁸ are as defined in any of the above embodiments.

In another preferred embodiment of formula (I), X¹ is N; X² is CR⁶; X³ is N or CR⁷; X⁵ is C; X⁶ is N; X⁷ is C, and X³ and X⁴ are not simultaneously CH.

In another preferred embodiment of formula (I), X¹ is N; X² is CR⁶; X⁵ is C; X⁶ is N; X⁷ is C; X³ is N or CR⁷; X⁴ is N or CR⁸, and X³ and X⁴ are not simultaneously CH.

In a preferred embodiment of formula (I), R¹ and R² are each independently -CH₃, - CH₂CH₃, -CH(CH₃)₂, -Cl, -F, -Br, -OCH₃, -CH₂OH, or -CD₃; preferably, R¹ and R² are both - CH₃ or -CD₃;
R³ is -CH₃, -NH₂, -OH, -F, -Cl, -Br, -CH(CH₃)₂, -CH₂CH₃, -CH₂CH₂CH₃, - CH₂CH(CH₃)₂, -OCH₃, -CH₂F, -CHF₂, -CF₃, -CH=CH₂, -CH=C(CH₃)₂, -CH=CHCH₃, - C(CH₃)=CH₂, -NHCH₃, -NHCH₂CH₃, -NHC(O)CH₃, 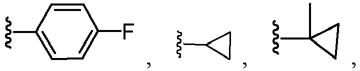 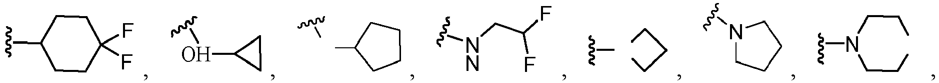 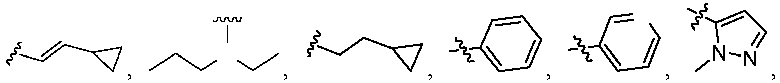 or -CD₃;
R⁴ is -H, -Cl, -F, -Br, -CH₃, -CH₂CH₃, cyclopropyl, or -CH₂CF₃;
R⁶ is -CH₃, -H, -OH, -Cl, -F, -Br, -COOH, -CN, -CH₂CH₃, -CH(CH₃)₂, -CH₂CH(CH₃)₂, -CF₃, -CHF₂, -CH₂F, -CF₂CH₃, -CF(CH₃)₂, -OCH₃, -OCH(CH₃), -OCF₃, -OCH₂CF₃, -N(CH₃)₂, -CH=CH₂, -C(CH₃)=CH₂, -C≡CH, -S(O)₂CH₃, 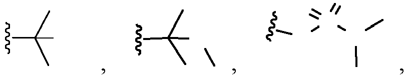 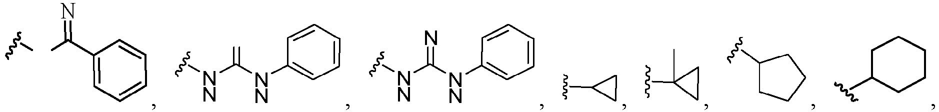 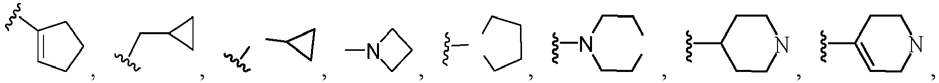 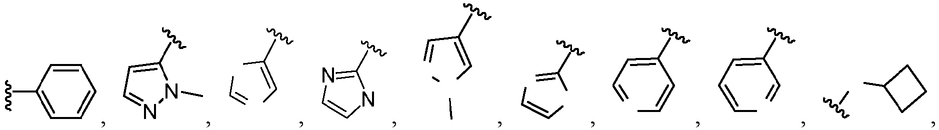 -CH₂CH₂CH=CH₂, -OCH(CH₃)₂, -CH₂OH, -CH₂CF₃, or -CD₃;
X³ is N or CR⁷; X⁴ is N or CR⁸;
R⁷ and R⁸ are each independently -H, -OH, -CN, -NH₂, -CH₃, -CH₂CH₃, -Cl, -F, -Br, -OCH₃, or -OCH₂CH₃; R⁷ and R⁸ are not simultaneously H;
or, R² and R⁸ together with the atom to which they are attached form a C₅₋₆ cycloalkenyl ring.

In a preferred embodiment of formula (I), R¹ and R² are each independently -CH₃, - CH₂CH₃, or -CH(CH₃)₂; preferably, R¹ and R² are both -CH₃;
R³ is -CH₃;
R⁴ is -H;
R⁶ is -CH₃;
X³ is N or CR⁷; X⁴ is N or CR⁸, and X³ and X⁴ are not simultaneously CH;
R⁷ and R⁸ are each independently -H, -OH, -CN, -NH₂, -CH₃, -CH₂CH₃, -Cl, -F, - OCH₃, or -OCH₂CH₃;
R⁷ and R⁸ are not simultaneously H;
or, R² and R⁸ together with the atom to which they are attached form a C₅₋₆ cycloalkenyl ring.

In a preferred embodiment of formula (I), X³ is CR⁷, and X⁴ is CR⁸; R⁷ and R⁸ are each independently -H, -OH, -CN, -NH₂, -CH₃, -CH₂CH₃, -Cl, -F, -OCH₃, or -OCH₂CH₃; R⁷ and R⁸ are not simultaneously H.

In a preferred embodiment of formula (I), R¹ and R² are each independently -Cl, -F, - Br, -OCH₃, or -CH₂OH.

In a preferred embodiment of formula (I), R¹ and R² are each independently -CD₃.

In a preferred embodiment of formula (I), R³ is -NH₂, -OH, -F, -Cl, -Br, -CH(CH₃)₂, - CH₂CH₃, -CH₂CH₂CH₃, -CH₂CH(CH₃)₂, -OCH₃, -CH₂F, -CHF₂, -CF₃, -CH=CH₂, - CH=C(CH₃)₂, -CH=CHCH₃, -C(CH₃)=CH₂, -NHCH₃, -NHCH₂CH₃, -NHC(O)CH₃,

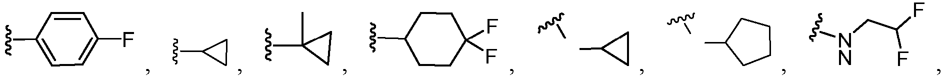

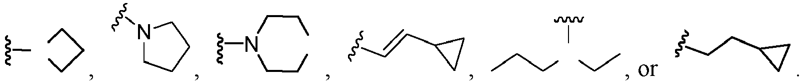

In a preferred embodiment of formula (I), R³ is 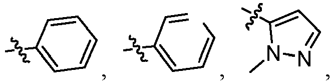 or - CD₃.

In a preferred embodiment of formula (I), R⁴ is -Cl, -F, -Br, -CH₃, -CH₂CH₃, or cyclopropyl.

In a preferred embodiment of formula (I), R⁴ is -CH₂CF₃.

In a preferred embodiment of formula (I), R⁶ is -H, -OH, -Cl, -F, -Br, -COOH, -CN, - CH₂CH₃, -CH(CH₃)₂, -CH₂CH(CH₃)₂, -CF₃, -CHF₂, -CH₂F, -CF₂CH₃, -CF(CH₃)₂, -OCH₃, - OCH(CH₃), -OCF₃, -OCH₂CF₃, -N(CH₃)₂, -CH=CH₂, -C(CH₃)=CH₂, -C≡CH, -S(O)₂CH₃,

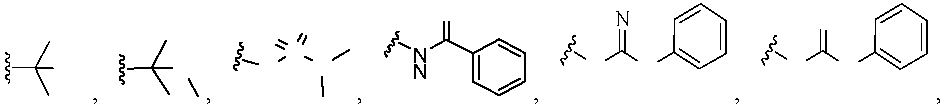

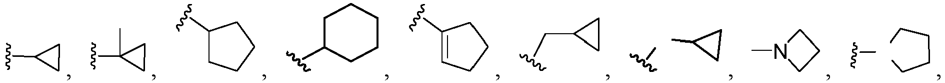

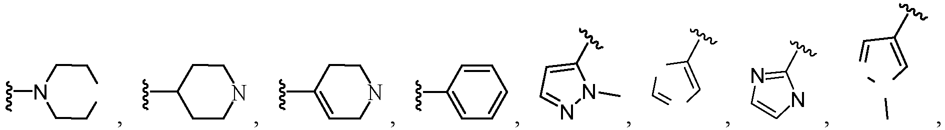

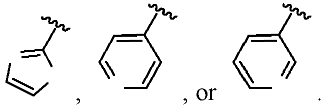

In a preferred embodiment of formula (I), R⁶ is 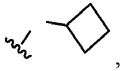 -CH₂CH₂CH=CH₂, - OCH(CH₃)₂, -CH₂OH, -CH₂CF₃, or -CD₃.

In a preferred embodiment of formula (I), R⁷ and R⁸ are each independently -Br.

In a preferred embodiment of formula (I), R⁷ and R⁸ are each independently -H or -F, and at least one of R⁷ or R⁸ is -F.

In a preferred embodiment of formula (I), X³ is CR⁷, and X⁴ is CR⁸;
R¹ and R² are C₁₋₄ alkyl or deuterated C₁₋₄ alkyl; preferably, R¹ and R² are -CH₃ or - CD₃;
R³ is C₁₋₄ alkyl; preferably, R³ is -CH₃ or -CH₂CH₃;
R⁴ is -H;
R⁶ is halogen or halo-C₁₋₄ alkyl; preferably, R⁶ is -Cl, -Br, or -CF₃;
R⁷ and R⁸ are each independently -H or halogen, and R⁷ and R⁸ are not simultaneously H; preferably, R⁷ and R⁸ are each independently -H or F, and R⁷ and R⁸ are not simultaneously H.

In a preferred embodiment of formula (I), X¹ is N; X² is CR⁶; X³ is CR⁷; X⁴ is CR⁸; X⁵ is C; X⁶ is N; X⁷ is C;
R¹ and R² are each independently C₁₋₄ alkyl, preferably -CH₃;
R³ is C₁₋₄ alkyl, preferably -CH₃;
R⁴ is -H;
R⁷ is halogen, preferably F; R⁸ is -H;
R⁶ is halogen or halo-C₁₋₄ alkyl, preferably Br or -CF₃, more preferably -CF₃.

In a preferred embodiment of formula (I), R¹ and R² are each independently -CH₃, - CH₂CH₃, -CH(CH₃)₂, -Cl, -F, -Br, -OCH₃, -CH₂OH, or -CD₃; preferably, R¹ and R² are both - CH₃ or -CD₃;
R³ and R⁶ together with the atom to which they are attached form the following group: 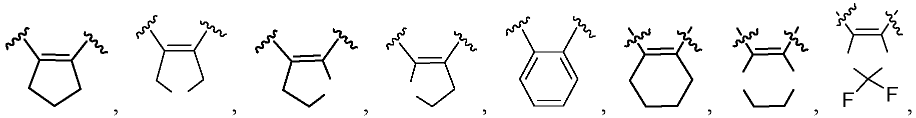 or 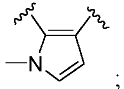
R⁴ is -H, -Cl, -F, -Br, -CH₃, -CH₂CH₃, or cyclopropyl;
X³ is N or CR⁷; X⁴ is N or CR⁸;
R⁷ and R⁸ are each independently -H, -OH, -CN, -NH₂, -CH₃, -CH₂CH₃, -Cl, -F, -Br, -OCH₃, or -OCH₂CH₃.

In a preferred embodiment of formula (I), wherein R¹ and R² are each independently -CH₃, -CH₂CH₃, -CH(CH₃)₂, -Cl, -F, -Br, -OCH₃, or -CH₂OH; preferably, R¹ and R² are both - CH₃;
R³ and R⁶ together with the atom to which they are attached form the following group: 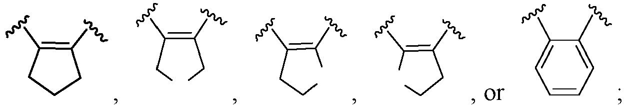
R⁴ is -H, -Cl, -F, -Br, -CH₃, -CH₂CH₃, or cyclopropyl;
X³ is N or CR⁷; X⁴ is N or CR⁸;
R⁷ and R⁸ are each independently -H, -OH, -CN, -NH₂, -CH₃, -CH₂CH₃, -Cl, -F, -Br, -OCH₃, or -OCH₂CH₃.

In a preferred embodiment of formula (I), R¹ and R² are each independently -CH₃, - CH₂CH₃, -CH(CH₃)₂, -Cl, -F, -Br, -OCH₃, -CH₂OH, or -CD₃; preferably, R¹ and R² are both - CH₃ or -CD₃;
R³ is -H, -CH₃, or -CH₂CH₃;
R⁴ and R⁶ together with the atom to which they are attached form the following group: 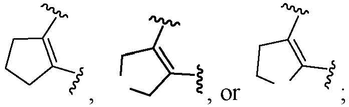
X³ is N or CR⁷; X⁴ is N or CR⁸;
R⁷ and R⁸ are each independently -H, -OH, -CN, -NH₂, -CH₃, -CH₂CH₃, -Cl, -F, -Br, -OCH₃, or -OCH₂CH₃.

In a preferred embodiment of formula (I), wherein R¹ and R² are each independently -CH₃, -CH₂CH₃, -CH(CH₃)₂, -Cl, -F, -Br, -OCH₃, or -CH₂OH; preferably, R¹ and R² are both -CH₃;
R³ is -H, -CH₃, or -CH₂CH₃;
R⁴ and R⁶ together with the atom to which they are attached form the following group: 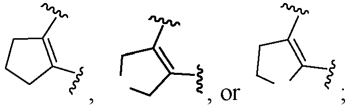
X³ is N or CR⁷; X⁴ is N or CR⁸;
R⁷ and R⁸ are each independently -H, -OH, -CN, -NH₂, -CH₃, -CH₂CH₃, -Cl, -F, -Br, -OCH₃, or -OCH₂CH₃.

In some embodiments, R¹ and R² are both -CD₃.

In some embodiments, R³ and R⁶ together with the atom to which they are attached form the following group:

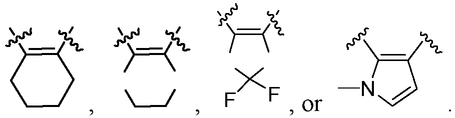

In some embodiments, R⁴ and R⁶ together with the atom to which they are attached form

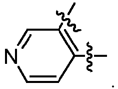

In a preferred embodiment of formula (I), wherein X³ is CR⁷; X⁴ is CR⁸; X⁶ is N; X⁵ and X⁷ are C; X¹ is CH; X² is CR⁶;
R¹ and R² are each independently -CH₃, -CH₂CH₃, -CH(CH₃)₂, -Cl, -F, -Br, -OCH₃, - CH₂OH, or -CD₃; preferably, R¹ and R² are both -CH₃ or -CD₃;
R³ is -CH₃ or -CH₂CH₃;
R⁴ and R⁶ together with the atom to which they are attached form the following group: 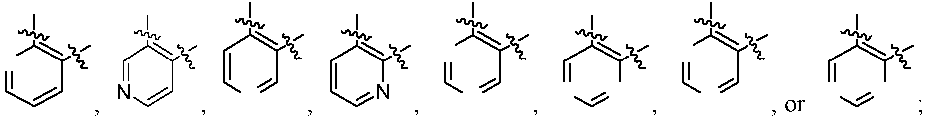
R⁷ and R⁸ are each independently -H, -OH, -CN, -NH₂, -CH₃, -CH₂CH₃, -Cl, -F, -Br, -OCH₃, or -OCH₂CH₃, preferably, R⁷ and R⁸ are each independently -H or -F.

In a preferred embodiment of formula (I), the compound, the pharmaceutically acceptable salt thereof, or the isomer thereof has any one of the following structures: 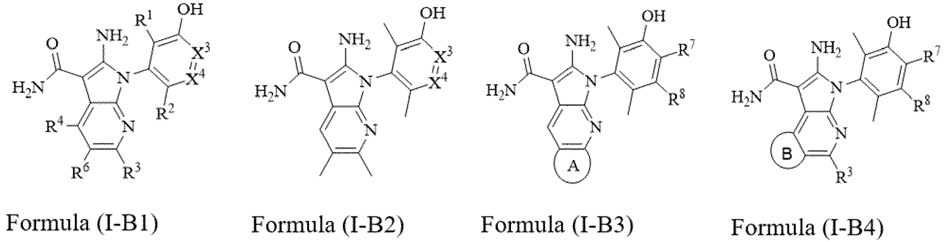 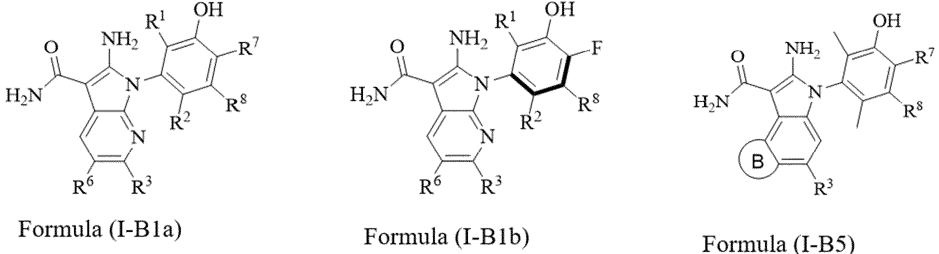 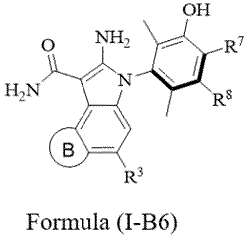
ring A and ring B are each independently phenyl, 5- to 6-membered heterocycloalkenyl, or C₅₋₆ cycloalkenyl;
wherein R¹, R², R³, R⁴, R⁶, X³, X⁴, R⁷, and R⁸ are as defined in any of the above embodiments.

In some embodiments, ring A and ring B are each independently phenyl, 5- to 6-membered heterocycloalkenyl, C₅₋₆ cycloalkenyl, or 5- to 6-membered heteroaryl, and the phenyl, 5- to 6-membered heterocycloalkenyl, C₅₋₆ cycloalkenyl, and 5- to 6-membered heteroaryl are optionally further substituted by one or more substituents selected from halogen, CN, C₁₋₆ alkyl, halo-C₁₋₆ alkyl, hydroxy-C₁₋₆ alkyl, and C₁₋₆ alkoxy.

In a preferred embodiment of formula (I), the compound, the pharmaceutically acceptable salt thereof, or the isomer thereof has any one of the following structures: 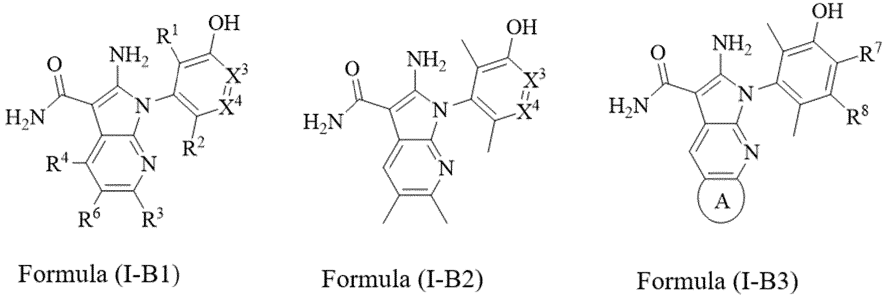 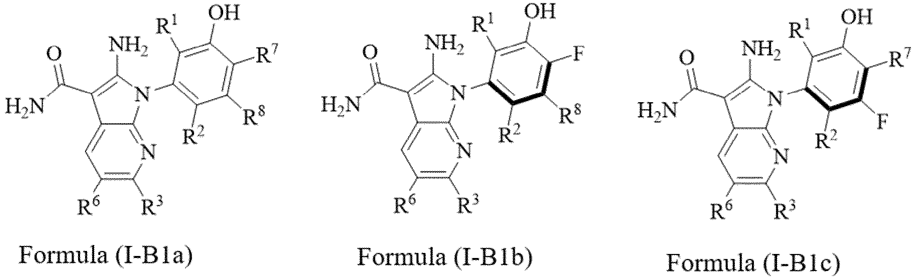 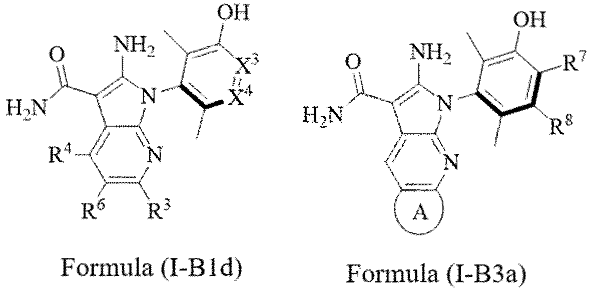
each ring A is independently phenyl, 5- to 6-membered heterocycloalkenyl, or C₅₋₆ cycloalkenyl;
wherein R¹, R², R³, R⁴, R⁶, X³, X⁴, R⁷, and R⁸ are as defined in any of the above embodiments.

In a preferred embodiment of formula (I), ring A is phenyl, 5- to 6-membered heterocycloalkenyl, C₅₋₆ cycloalkenyl, or 5- to 6-membered heteroaryl, and the phenyl, 5- to 6-membered heterocycloalkenyl, C₅₋₆ cycloalkenyl, and 5- to 6-membered heteroaryl are optionally further substituted by one or more substituents selected from halogen, CN, C₁₋₆ alkyl, halo-C₁₋₆ alkyl, hydroxy-C₁₋₆ alkyl, C₁₋₆ alkoxy, and C₁₋₆ alkyl-C(O)-.

In another aspect, the present disclosure provides a compound represented by the following general formula (II), a pharmaceutically acceptable salt thereof, or an isomer thereof, 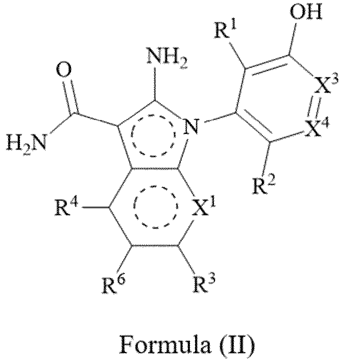
X¹ is N or CR⁵;
X³ is N or CR⁷;
X⁴ is N or CR⁸;
R¹ and R² are each independently halogen, C₁₋₄ alkyl, C₁₋₄ alkoxy, hydroxy-C₁₋₄ alkyl, or deuterated C₁₋₄ alkyl;
R³ is -CN, -OH, -N(R^{a})(R^{b}), halogen, C₁₋₄ alkyl, halo-C₁₋₄ alkyl, C₁₋₄ alkoxy, C₂₋₄ alkenyl, C₂₋₄ alkynyl, -L-R^{c}, phenyl, C₃₋₆ cycloalkyl, C₃₋₆ cycloalkenyl, 3- to 6-membered heterocyclyl, 3- to 6-membered heterocycloalkenyl, deuterated C₁₋₄ alkyl, or 5- to 6-membered heteroaryl; wherein the phenyl, C₃₋₆ cycloalkyl, C₃₋₆ cycloalkenyl, 3- to 6-membered heterocyclyl, 3- to 6-membered heterocycloalkenyl, and 5- to 6-membered heteroaryl are optionally substituted by one or more R^{3a}, and the R^{3a} is halogen, C₁₋₄ alkyl, or C₁₋₄ alkoxy;
L is C₂₋₄ alkynylene, C₁₋₄ alkylene, or C₂₋₄ alkenylene;
R^{c} is C₁₋₄ alkyl, C₃₋₆ cycloalkyl, or 4- to 6-membered heterocyclyl, and the C₁₋₄ alkyl, C₃₋₆ cycloalkyl, and 4- to 6-membered heterocyclyl are optionally substituted by one or more R^{ca}; the R^{ca} is halogen or -OH;
R^{a} and R^{b} are each independently -H, C₁₋₄ alkyl, phenyl, p-methoxybenzyl, C₁₋₄ alkyl-C(O)-, C₃₋₆ cycloalkyl, or halo-C₁₋₄ alkyl, or R^{a} and R^{b} together with the nitrogen atom to which they are attached form a 4- to 6-membered heterocyclyl ring, and the 4- to 6-membered heterocyclyl is optionally substituted by halogen;
R⁴ and R⁶ together with the atom to which they are attached form a phenyl, 5- to 6-membered heterocycloalkenyl, C₅₋₆ cycloalkenyl, or 5- to 6-membered heteroaryl ring; the phenyl, 5- to 6-membered heteroaryl, 5- to 6-membered heterocycloalkenyl, and C₅₋₆ cycloalkenyl are optionally further substituted by one or more substituents selected from halogen, CN, C₁₋₆ alkyl, halo-C₁₋₆ alkyl, hydroxy-C₁₋₆ alkyl, C₁₋₆ alkoxy, and C₁₋₆ alkyl-C(O)-;
R⁵, R⁷, and R⁸ are each independently -H, -OH, -CN, -N(R^{d})(R^{e}), halogen, C₁₋₄ alkyl, C₁₋₄ alkoxy, or halo-C₁₋₄ alkyl.

In a preferred embodiment of formula (II), R¹ and R² are each independently -CH₃, - CH₂CH₃, -CH(CH₃)₂, -Cl, -F, -Br, -OCH₃, -CH₂OH, or -CD₃;
R³ is -CH₃ or -CH₂CH₃;
R⁴ and R⁶ together with the atom to which they are attached form the following group: 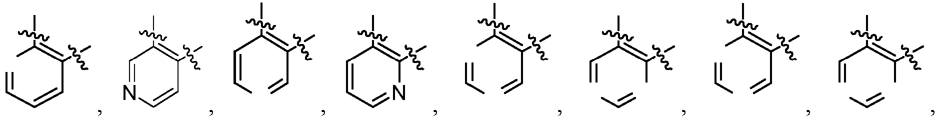 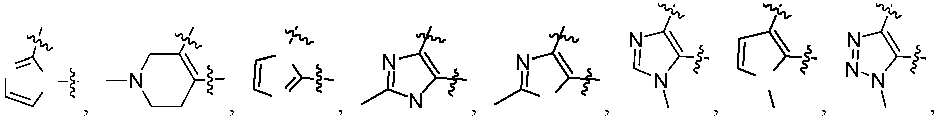 or 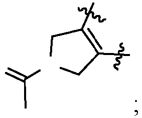
R⁵ is -H, -CN, halo-C₁₋₃ alkyl, or halogen;
R⁷ and R⁸ are each independently -H, -OH, -CN, -NH₂, -CH₃, -CH₂CH₃, -Cl, -F, -Br, -OCH₃, or -OCH₂CH₃.

In a preferred embodiment of formula (II), X¹ is CR⁵;
R¹ and R² are both -CH₃ or -CD₃;
R³ is -CH₃ or -CH₂CH₃;
R⁴ and R⁶ together with the atom to which they are attached form the following group: 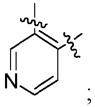
*R⁵* is -H or -F;
R⁷ and R⁸ are each independently -H or -F.

In a preferred embodiment of formula (II), X¹ is CR⁵; X³ is CR⁷; X⁴ is CR⁸;
R¹ and R² are each independently C₁₋₄ alkyl, preferably -CH₃;
R³ is C₁₋₄ alkyl, preferably -CH₃;
R⁴ and R⁶ together with the atom to which they are attached form a 5- to 6-membered heteroaryl ring, preferably a pyridyl ring, more preferably 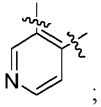
R⁵ is -H or halogen, preferably -H or -F, more preferably -H;
R⁷ and R⁸ are each independently -H.

In a preferred embodiment of formula (II), the compound, the pharmaceutically acceptable salt thereof, or the isomer thereof has any one of the following structures: 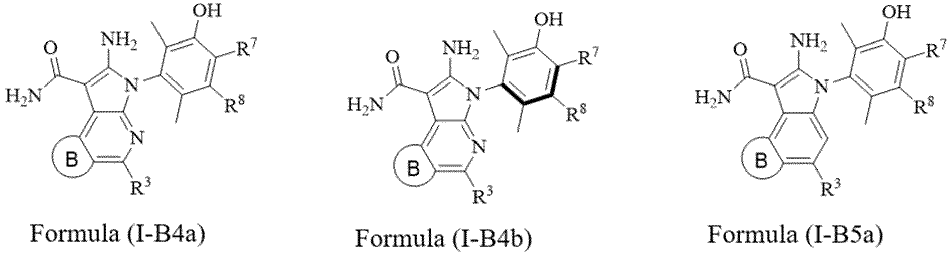 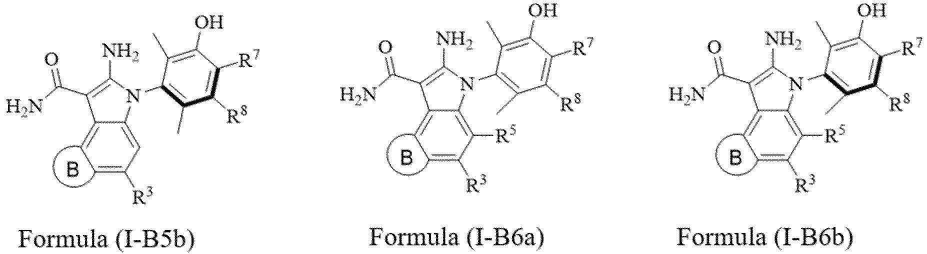
each ring B is independently phenyl, 5- to 6-membered heterocycloalkenyl, C₅₋₆ cycloalkenyl, or 5- to 6-membered heteroaryl, and the phenyl, 5- to 6-membered heterocycloalkenyl, C₅₋₆ cycloalkenyl, and 5- to 6-membered heteroaryl are optionally further substituted by one or more substituents selected from halogen, CN, C₁₋₆ alkyl, halo-C₁₋₆ alkyl, hydroxy-C₁₋₆ alkyl, and C₁₋₆ alkoxy;
wherein R³, R⁴, R⁵, R⁶, X³, X⁴, R⁷, and R⁸ are as defined in any of the above embodiments.

In another preferred embodiment of formula (I), wherein X¹ is N, X⁵ is N, X² is CR⁶, X⁶ is C, and X⁷ is C.

In another preferred embodiment of formula (I), wherein X¹ is N, X² is CR⁶, X⁵ is C, X⁶ is C, and X⁷ is N.

In a preferred embodiment of formula (I), R¹ and R² are each independently -CH₃, - CH₂CH₃, or -CH(CH₃)₂; preferably, R¹ and R² are both -CH₃;
R³ is -CH₃;
R⁴ is -H;
R⁶ is -CH₃;
X³ is N or CR⁷; X⁴ is N or CR⁸;
R⁷ and R⁸ are each independently -H, -OH, -CN, -NH₂, -CH₃, -CH₂CH₃, -Cl, -F, - OCH₃, or -OCH₂CH₃;
or, R² and R⁸ together with the atom to which they are attached form a C₅₋₆ cycloalkenyl ring.

In a preferred embodiment of formula (I), the compound, the pharmaceutically acceptable salt thereof, or the isomer thereof has any one of the following structures: 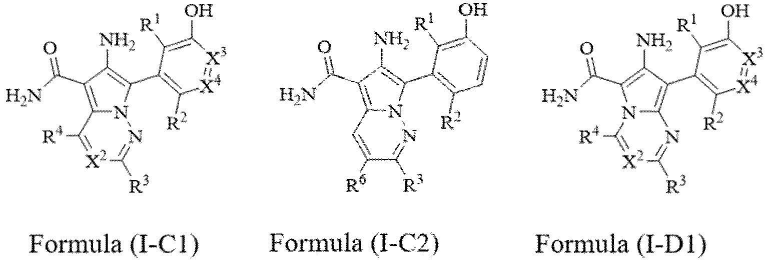 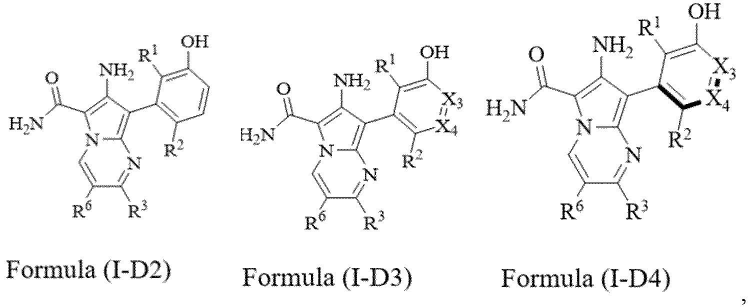 wherein R¹, R², R³, R⁴, R⁶, X³, and X⁴ are as defined in formula (I).

In a preferred embodiment of formula (I), the compound, the pharmaceutically acceptable salt thereof, or the isomer thereof has a structure represented by formula (I-E1): 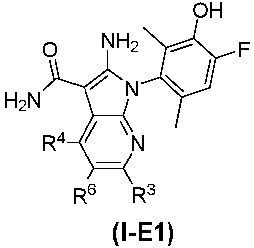
wherein R³ is H, -CH₃, or -CD₃;
R⁶ is -Br, -Cl, -CH₃, -CD₃-, (CH₃)₂CH-, (CH₃)₂CHCH₂-, CF₃, CF₃CH₂-, OH(CH₃)₂C-, CH₃-S(O)₂-, cyclopropyl, 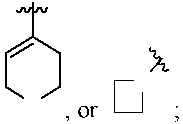
R⁴ is H; or, R⁴ and R⁶ together with the C atom to which they are attached form

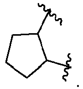

In a preferred embodiment of formula (I-E1), R³ is -CH₃ or -CD₃.

In a preferred embodiment of formula (I-E1), R⁶ is -Br, -Cl, -CD₃, -CF₃, CF₃CH₂-, or CH₃-S(O)₂-.

In a preferred embodiment of formula (I-E1), R⁴ is H.

In a preferred embodiment of formula (I), the compound, the pharmaceutically acceptable salt thereof, or the isomer thereof has a structure represented by formula (I-E2): 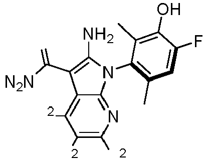
wherein R³ is -H, -CH₃, or -CD₃;
R⁶ is -Br, -Cl, -CH₃, -CD₃-, (CH₃)₂CH-, (CH₃)₂CHCH₂-, CF₃, CF₃CH₂-, OH(CH₃)₂C-, CH₃-S(O)₂-, cyclopropyl, 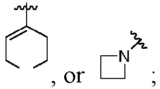
R⁴ is H; or, R⁴ and R⁶ together with the C atom to which they are attached form

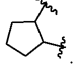

In a preferred embodiment of formula (I-E2), R³ is H, -CH₃, or -CD₃.

In a preferred embodiment of formula (I-E2), R⁶ is -Br, -Cl, -CF₃, or cyclopropyl.

In a preferred embodiment of formula (I-E2), R⁴ is H.

In a preferred embodiment of formula (I), the compound, the pharmaceutically acceptable salt thereof, or the isomer thereof has a structure represented by formula (I-F 1): 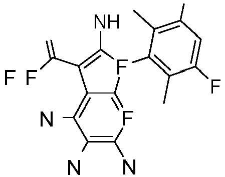
wherein R³ is -H, Cl, -CH₃, CH₃CH₂-, -CD₃, -CH=CH₂, or 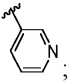
R⁶ is -Cl, -Br, -F, -CH₃, CH₃CH₂-, (CH₃)₂CH-, -CF₃, CH₂=C(CH₃)-, -CH=CH₂, CH₃O-, 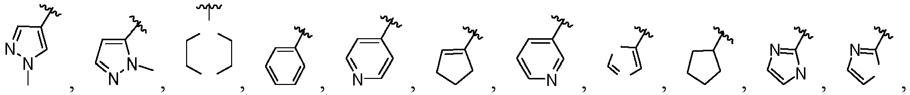 or cyclopropyl;
R⁴ is H;
or, R⁴ and R⁶ together with the C atom to which they are attached form 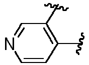 or 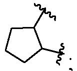
or, R³ and R⁶ together with the C atom to which they are attached form 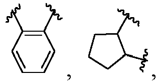 or

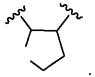

In a preferred embodiment of formula (I-F1), R³ is -H, -CH₃, CH₃CH₂-, or -CH=CH₂.

In a preferred embodiment of formula (I-F1), R⁶ is -Cl, -Br, -F, -CH₃, CH₃CH₂-, (CH₃)₂CH-, -CF₃, CH₂=C(CH₃)-, -CH=CH₂, CH₃O-, 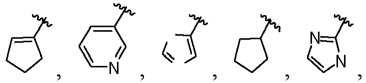 or cyclopropyl.

In a preferred embodiment of formula (I-F1), R³ and R⁶ together with the C atom to which they are attached form

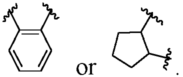

In a preferred embodiment of formula (I-F1), R⁴ and R⁶ together with the C atom to which they are attached form

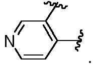

In a preferred embodiment of formula (I), the compound, the pharmaceutically acceptable salt thereof, or the isomer thereof has a structure represented by formula (I-F2): 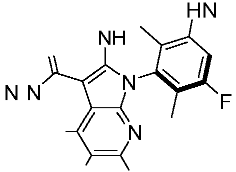
wherein R³ is -H, Cl, -CH₃, CH₃CH₂-, -CD₃, -CH=CH₂, or 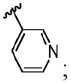
R⁶ is -Cl, -Br, -F, -CH₃, CH₃CH₂-, (CH₃)₂CH-, -CF₃, CH₂=C(CH₃)-, -CH=CH₂, CH₃O-, 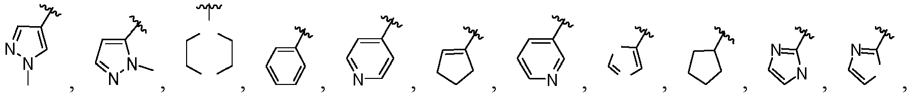 or cyclopropyl;
R⁴ is H;
or, R⁴ and R⁶ together with the C atom to which they are attached form 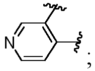
or, R³ and R⁶ together with the C atom to which they are attached form 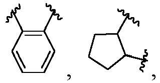 or

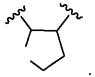

In a preferred embodiment of formula (I-F2), R³ is -H, -CH₃, CH₃CH₂-, or -CH=CH₂.

In a preferred embodiment of formula (I-F2), R⁶ is -Cl, -Br, -F, -CH₃, CH₃CH₂-, (CH₃)₂CH-, -CF₃, CH₂=C(CH₃)-, -CH=CH₂, CH₃O-, 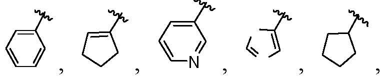 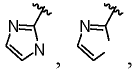 or cyclopropyl.

In a preferred embodiment of formula (I-F2), R³ and R⁶ together with the C atom to which they are attached form

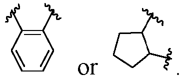

In a preferred embodiment of formula (I-F1), R⁴ and R⁶ together with the C atom to which they are attached form

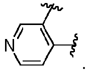

In a preferred embodiment of formula (I), the compound, the pharmaceutically acceptable salt thereof, or the isomer thereof has a structure represented by formula (I-G1): 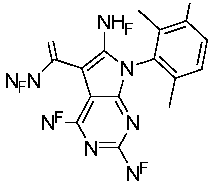
wherein R³ is CH₃-, CH₃CH₂CH₂-, (CH₃)₂CHCH₂-, CH₃CH=CH-, 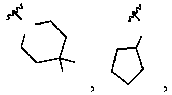 or 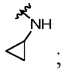
R⁴ is H, CH₃-, or cyclopropyl.

In a preferred embodiment of formula (I-G1), R³ is CH₃CH₂CH₂-, (CH₃)₂CHCH₂-, CH₃CH=CH-,

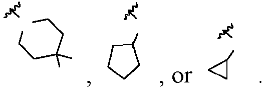

In a preferred embodiment of formula (I-G1), R⁴ is H.

In a preferred embodiment of formula (I), the compound, the pharmaceutically acceptable salt thereof, or the isomer thereof has a structure represented by formula (I-G2): 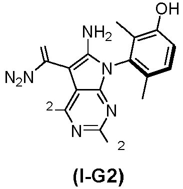
wherein R³ is CH₃-, (CH₃)₂CHCH₂-, 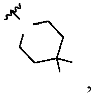 CH₃CH₂CH₂-, 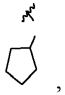 CH₃CH=CH-, or 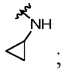
R⁴ is H, CH₃-, or cyclopropyl.

In a preferred embodiment of formula (I-G2), R⁴ is H.

In a preferred embodiment of formula (I), the compound, the pharmaceutically acceptable salt thereof, or the isomer thereof has a structure represented by formula (I-H1): 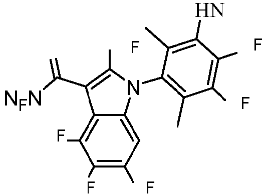
R³ is -CH₃;
R⁷ is H or F;
R⁸ is H or F;
R⁴ and R⁶ together with the C atom to which they are attached form

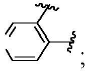

In a preferred embodiment of formula (I-H1), R⁷ is H; R⁸ is H.

In a preferred embodiment of formula (I-H1), R⁷ is H; R⁸ is F.

In a preferred embodiment of formula (I-H1), R⁷ is F; R⁸ is H.

In a preferred embodiment of formula (I), the compound, the pharmaceutically acceptable salt thereof, or the isomer thereof has a structure represented by formula (I-H2): 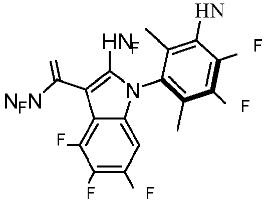
R³ is -CH₃;
R⁷ is H or F;
R⁸ is H or F;
R⁴ and R⁶ together with the C atom to which they are attached form

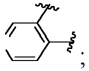

In a preferred embodiment of formula (I-H1), R⁷ is H; R⁸ is H.

In a preferred embodiment of formula (I-H1), R⁷ is H; R⁸ is F.

In a preferred embodiment of formula (I-H1), R⁷ is F; R⁸ is H.

Any substituent and any optional group in the technical solution described in the present disclosure can be combined with each other to form a new complete technical solution. The formed new technical solution has the same or similar technical effect as the solution recorded in the present disclosure and is included in the scope of the present disclosure.

In a preferred embodiment of formula (I), the compound, the pharmaceutically acceptable salt thereof, or the isomer thereof is provided, and the compound is selected from any one of the following structures:

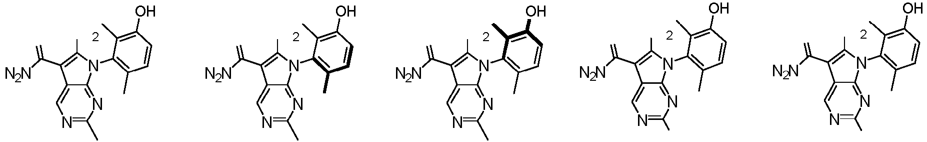

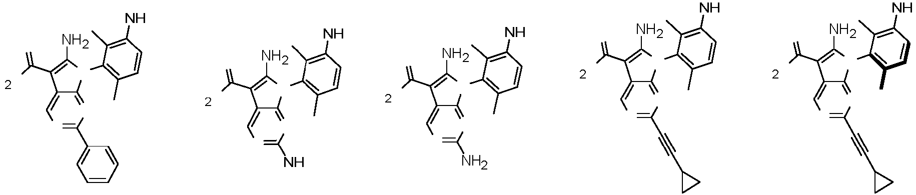

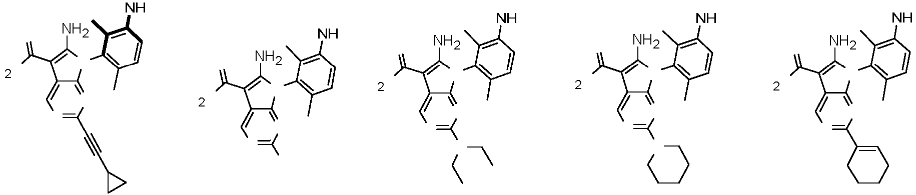

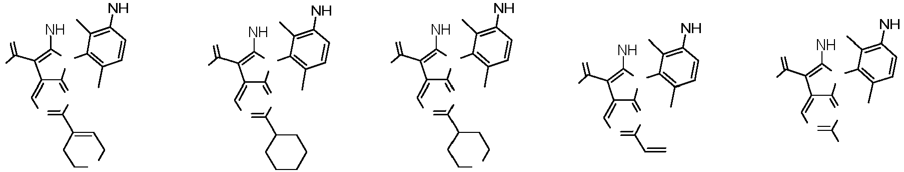

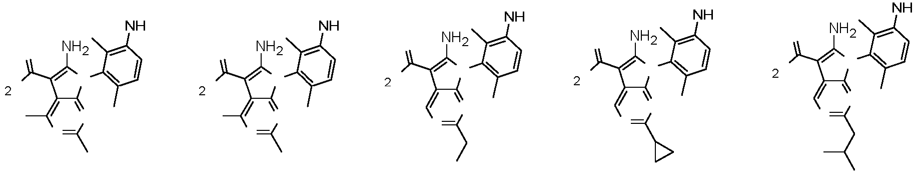

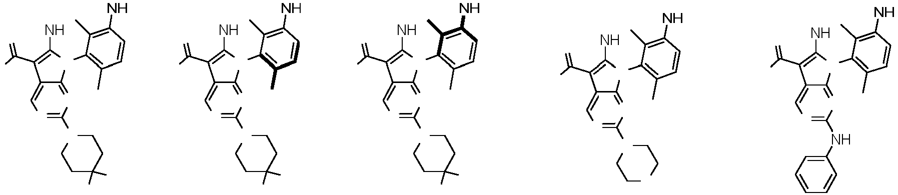

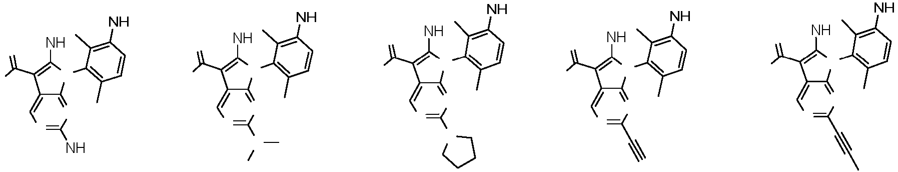

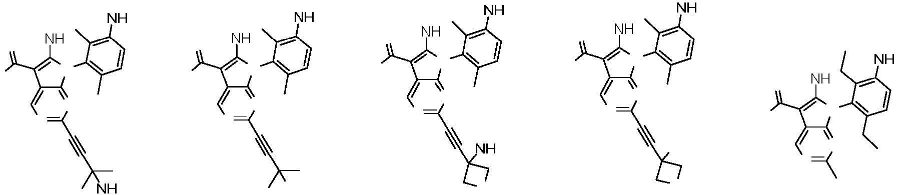

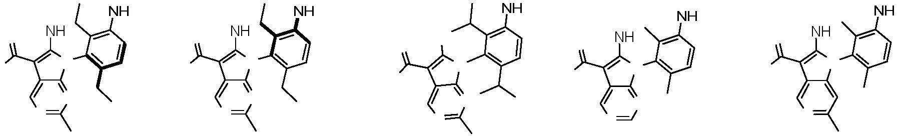

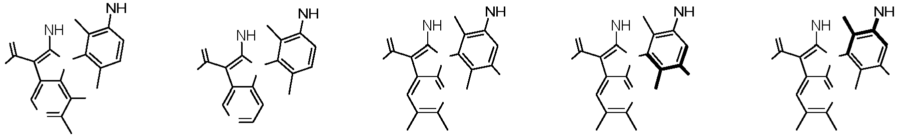

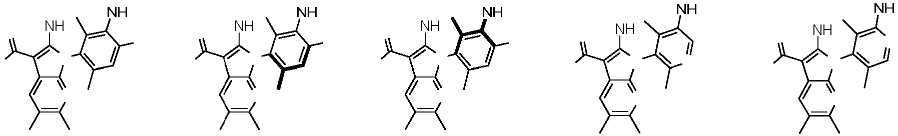

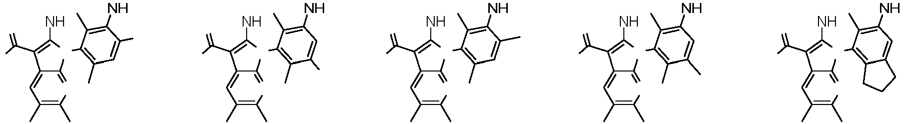

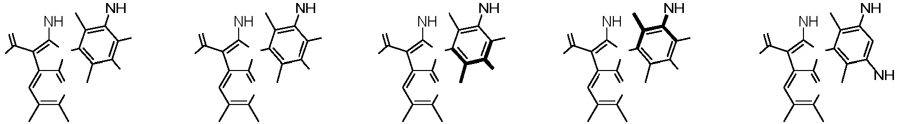

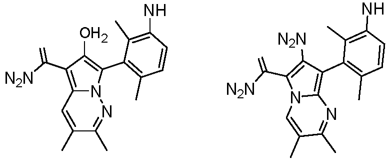

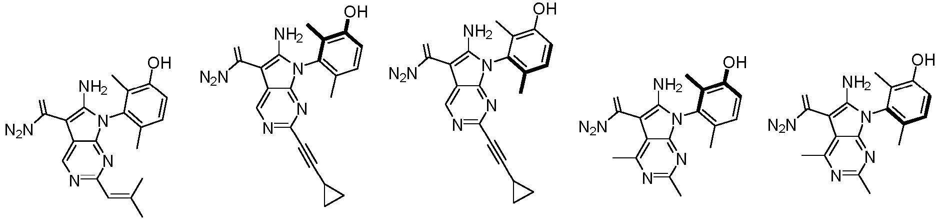

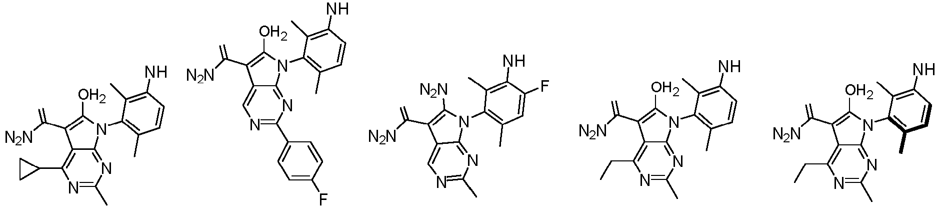

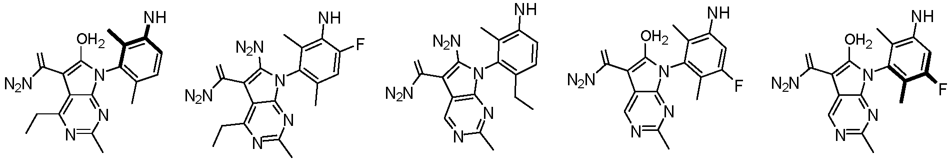

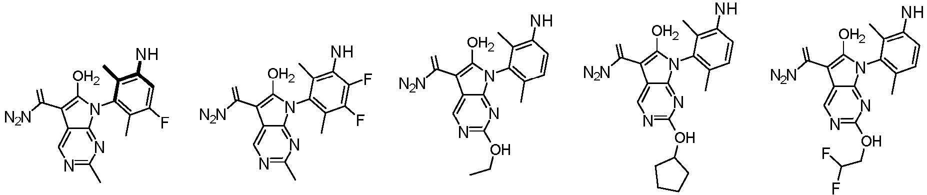

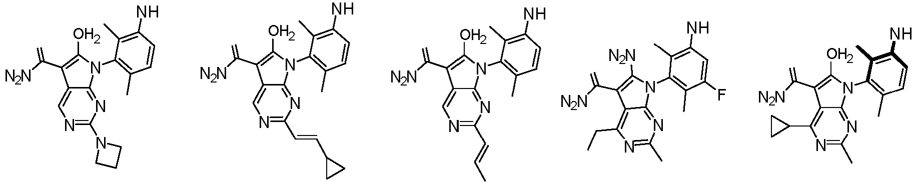

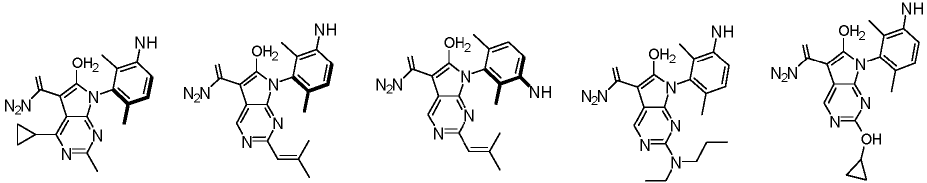

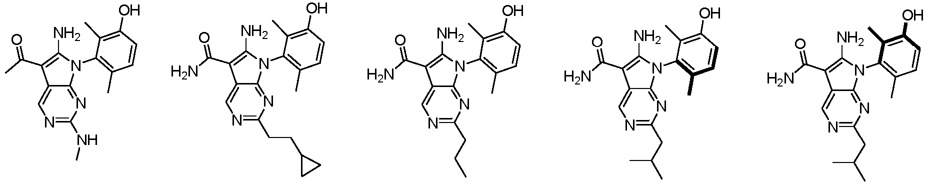

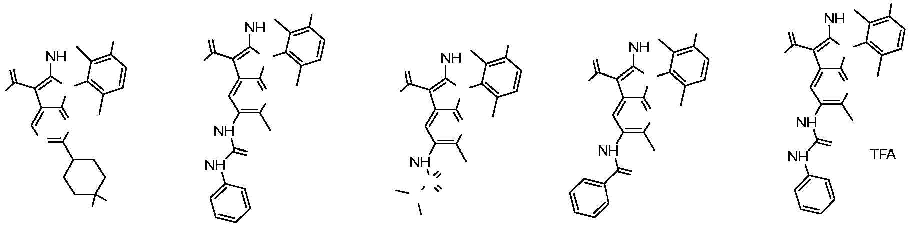

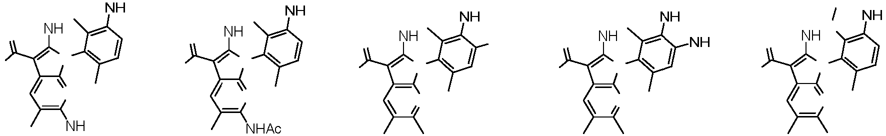

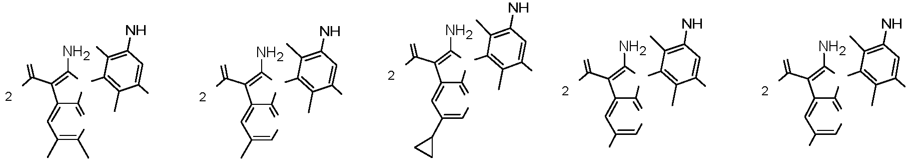

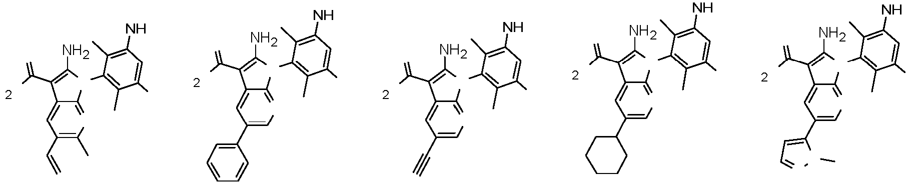

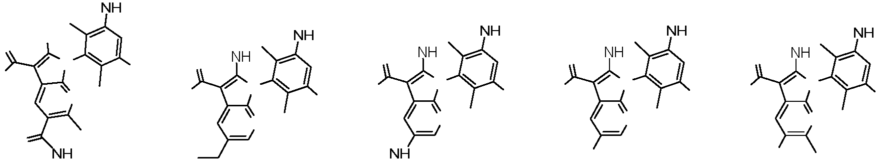

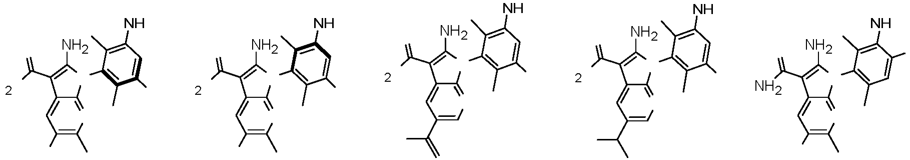

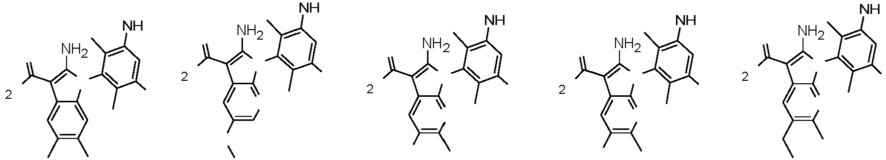

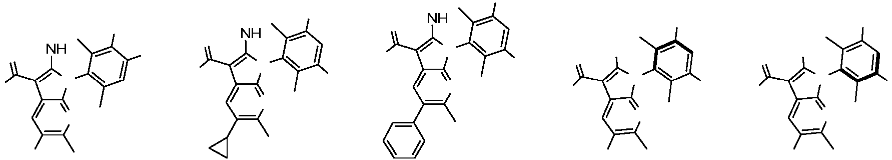

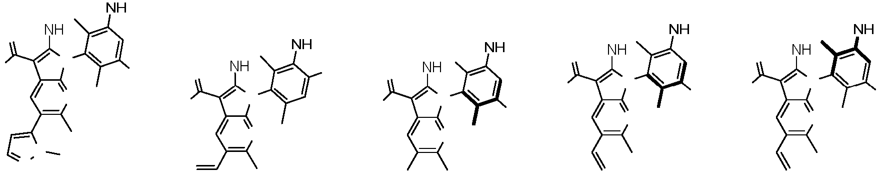

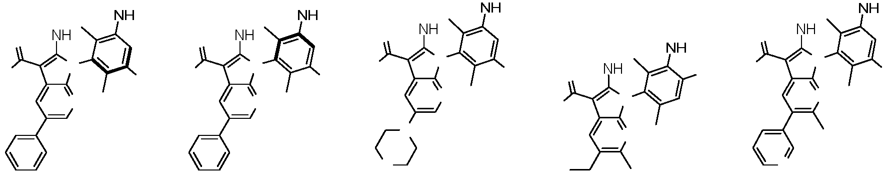

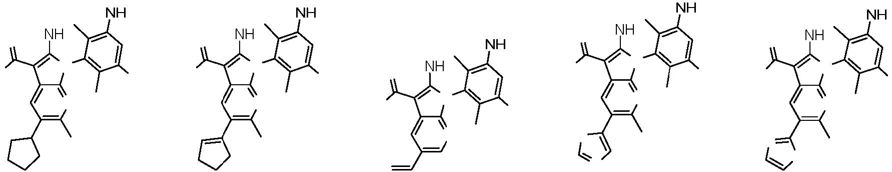

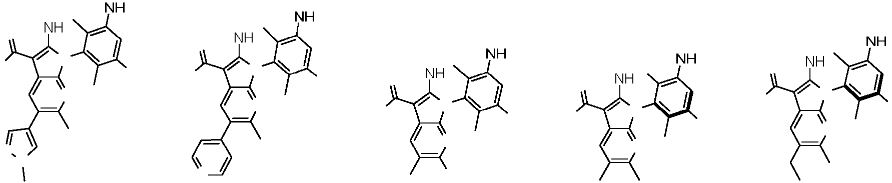

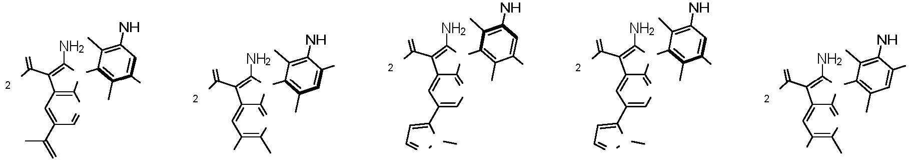

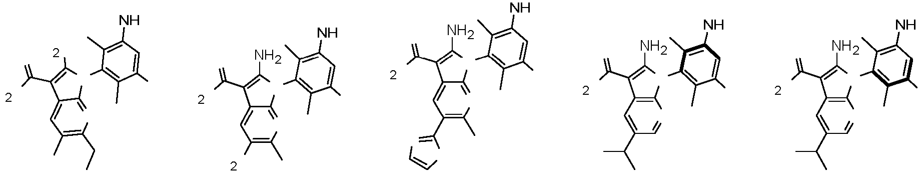

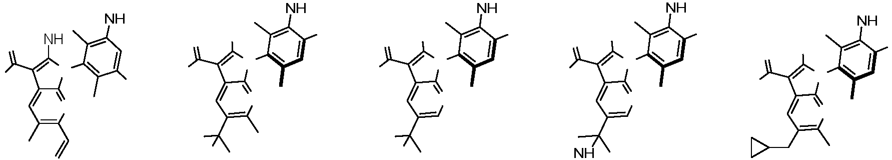

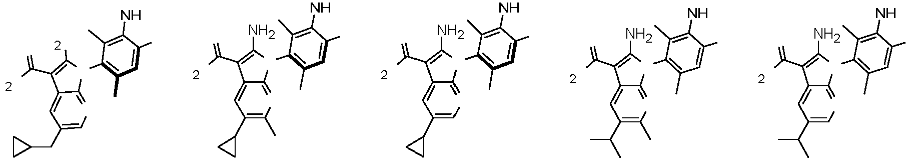

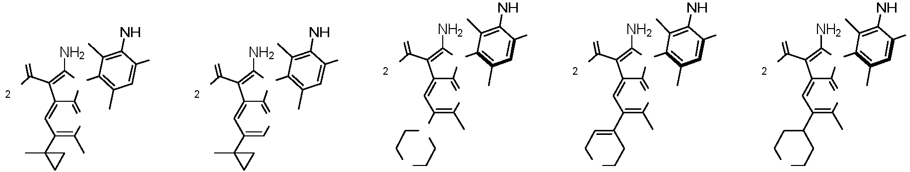

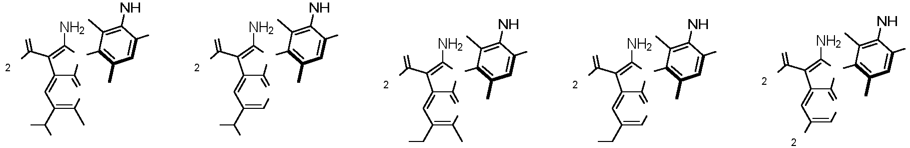

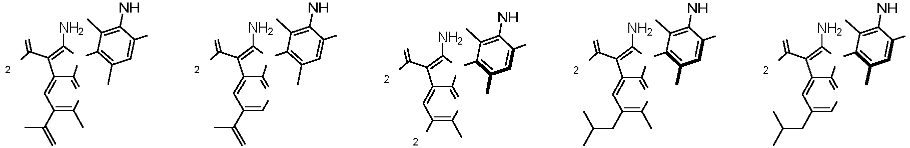

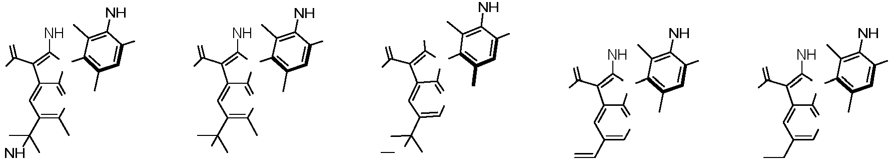

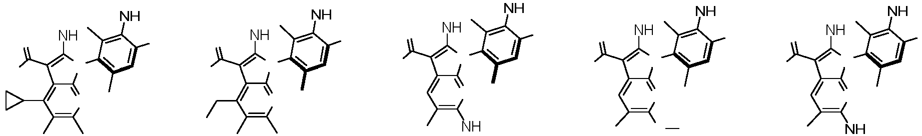

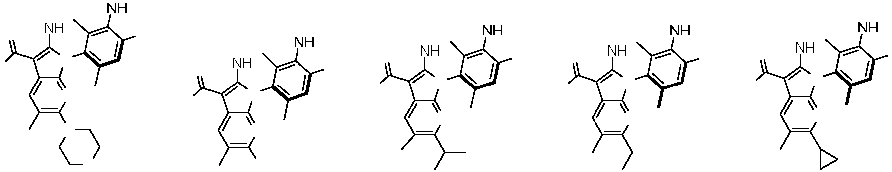

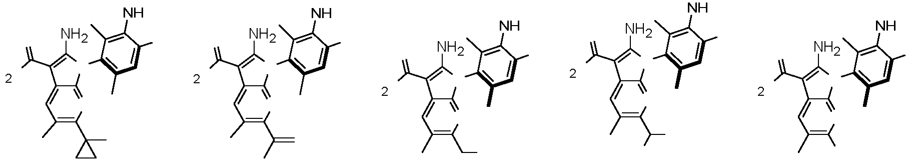

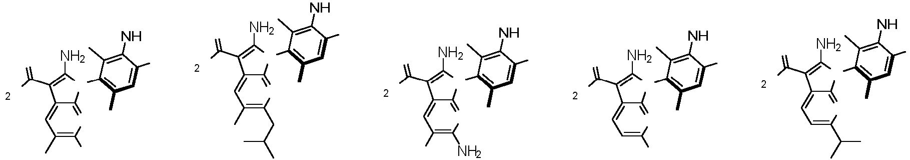

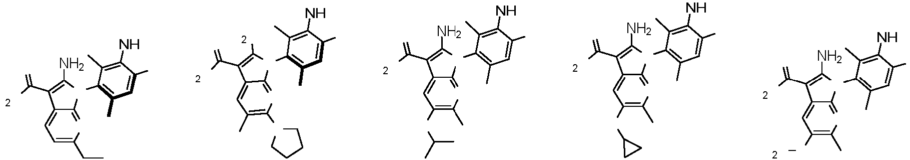

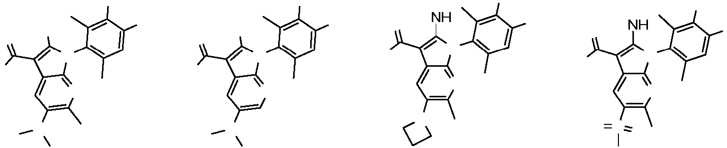

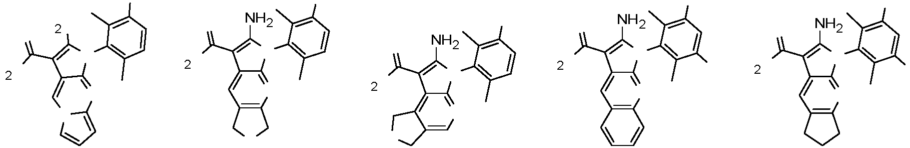

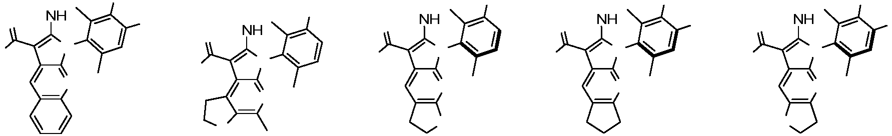

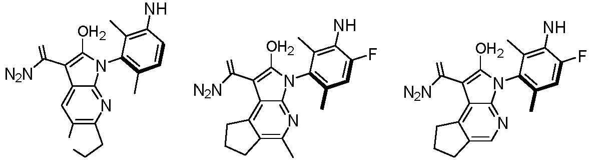

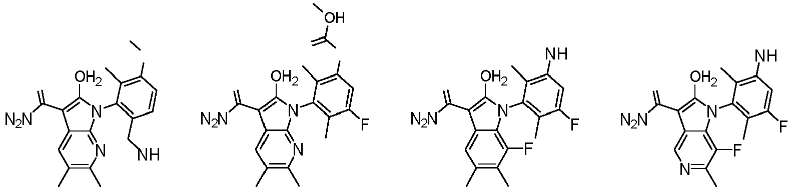

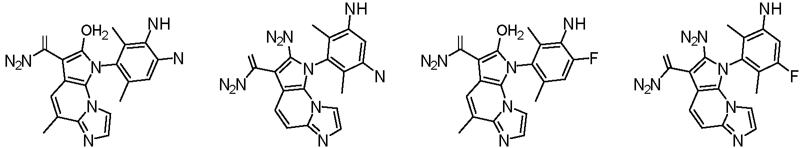

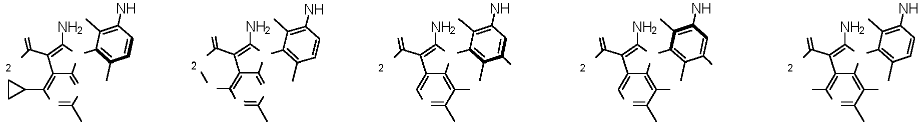

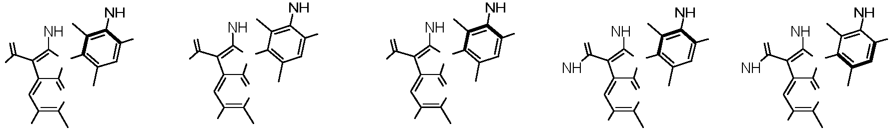

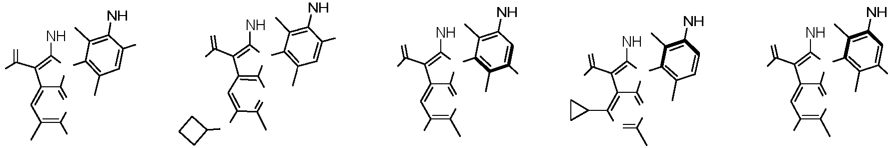

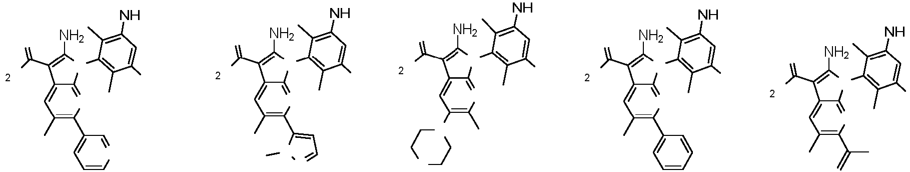

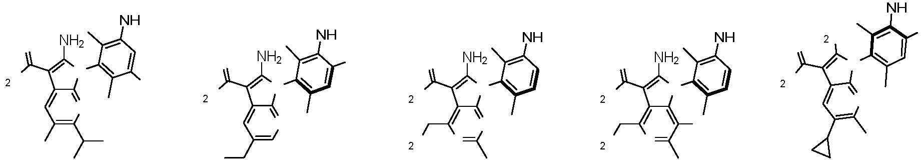

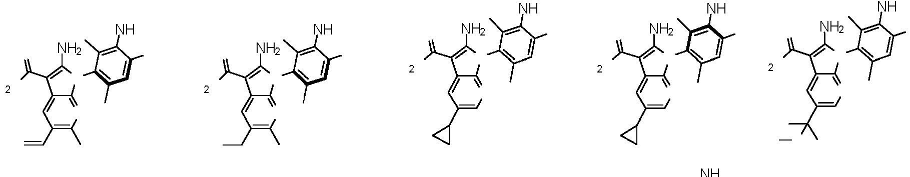

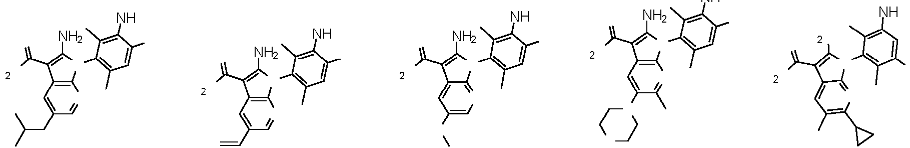

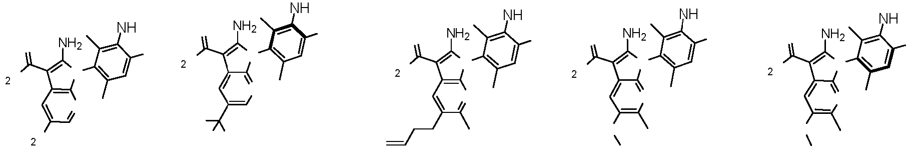

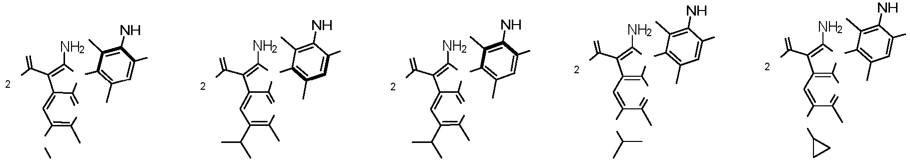

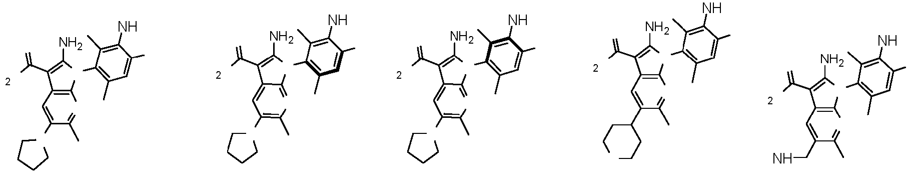

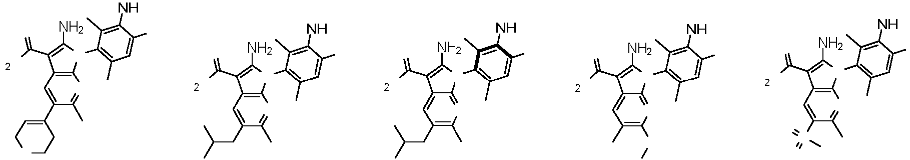

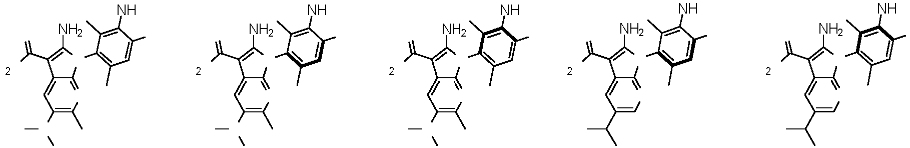

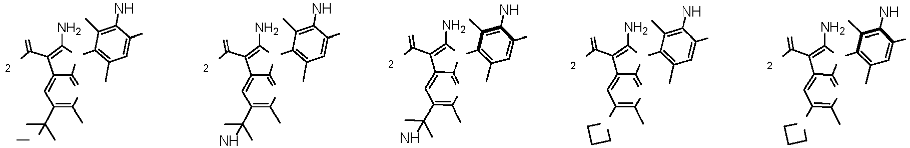

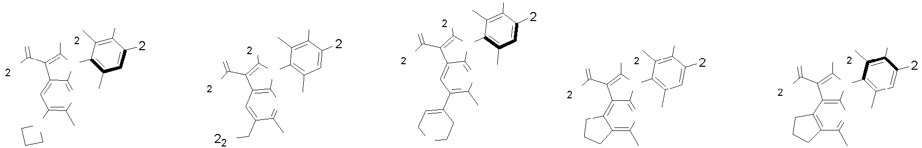

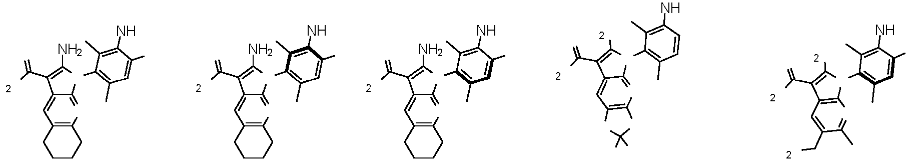

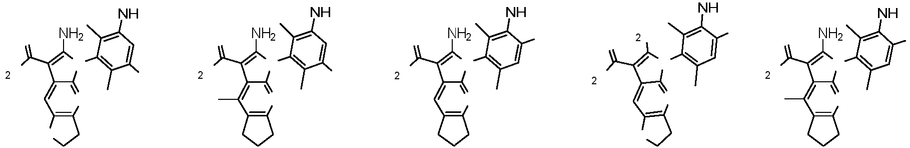

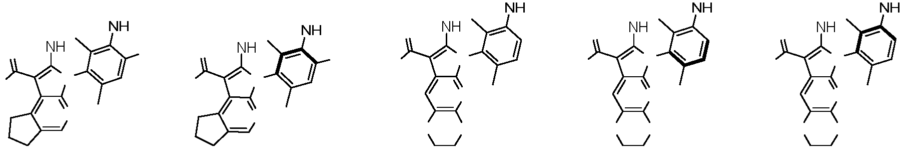

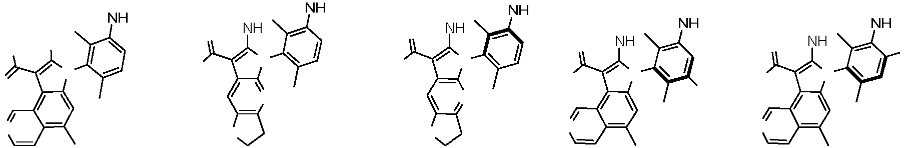

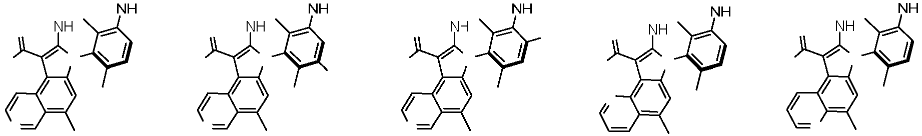

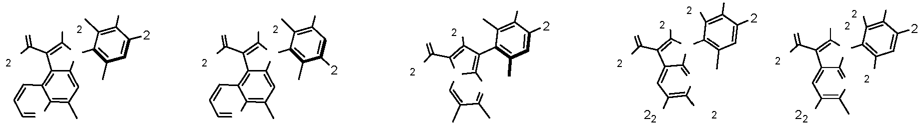

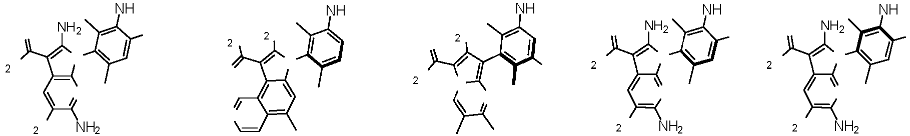

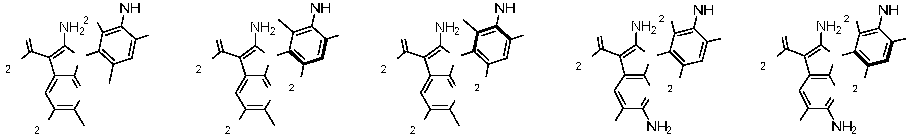

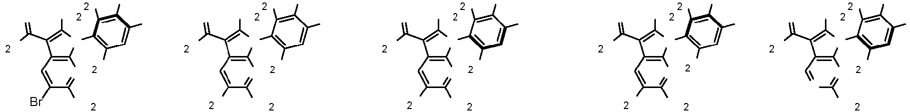

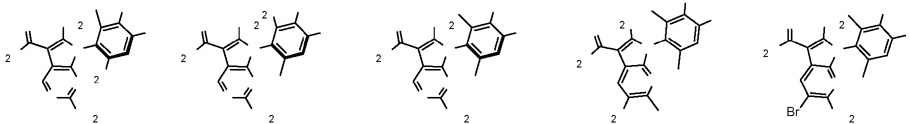

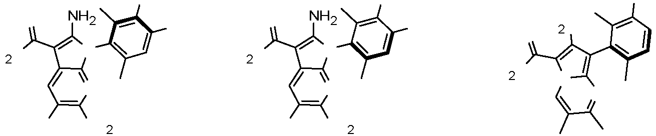

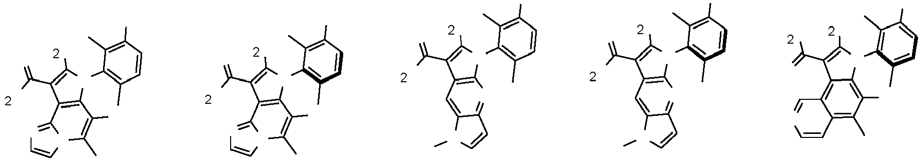

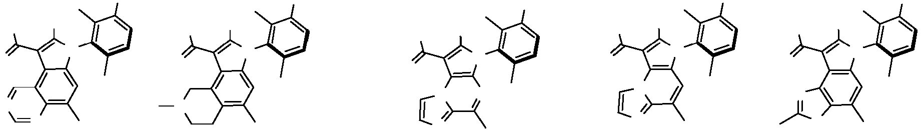

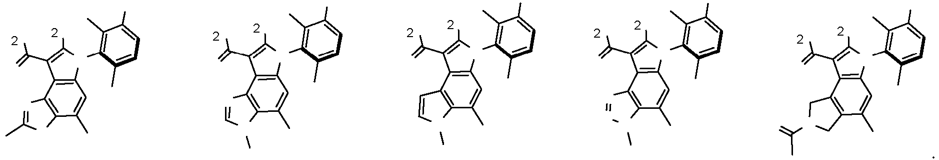

In a second aspect, the present disclosure also provides a pharmaceutical preparation composition, comprising the compound, the pharmaceutically acceptable salt thereof, or the isomer thereof described in any one of the embodiments of the present disclosure, and one or more pharmaceutically acceptable excipients. The pharmaceutical preparation composition can be in any pharmaceutically acceptable dosage form.

According to the present disclosure, a pharmaceutically acceptable excipient is a substance that is non-toxic, compatible with the active ingredient, and biologically suitable for organisms in other aspects. The selection of a specific excipient will depend on the mode of administration used to treat a specific patient or the type and state of the disease. Examples of the pharmaceutically acceptable excipients include, but are not limited to, solvents, diluents, dispersants, suspending agents, surfactants, isotonic agents, thickeners, emulsifiers, binders, lubricants, stabilizers, hydrating agents, emulsion accelerators, buffers, absorbents, colorants, ion exchangers, release agents, coating agents, flavoring agents, antioxidants, and the like conventional in the pharmaceutical art. If necessary, flavorings, preservatives, and sweeteners and the like may also be added to the pharmaceutical preparation composition.

In a third aspect, the present disclosure also provides a medicament comprising the compound, the pharmaceutically acceptable salt thereof, or the isomer thereof described in any one of the embodiments of the present disclosure, for use in the prevention and/or treatment of a tumor disease mediated by PKMYT1.

In another embodiment, the tumor disease mediated by PKMYT1 is a tumor disease caused by CCNE1 overexpression and/or FBXW7 inactivating mutation.

On the other hand, the present disclosure also provides a medicament comprising the compound, the pharmaceutically acceptable salt thereof, or the isomer thereof described in any one of the embodiments of the present disclosure, for use in the prevention and/or treatment of a tumor disease caused by CCNE1 overexpression and/or FBXW7 inactivating mutation.

In another embodiment, the tumor disease is selected from one or more of ovarian cancer, breast cancer, gastric cancer, esophageal cancer, lung cancer, endometrial cancer, and colorectal cancer.

In a fourth aspect, the present disclosure also provides a method for treating a tumor disease mediated by PKMYT1, comprising administering a therapeutically effective amount of the compound, the pharmaceutically acceptable salt thereof, or the isomer thereof described in any one of the embodiments of the present disclosure to a subject.

In another embodiment, the tumor disease mediated by PKMYT1 is a tumor disease caused by CCNE1 overexpression and/or FBXW7 inactivation mutation.

In another embodiment, the tumor disease is selected from one or more of ovarian cancer, breast cancer, gastric cancer, esophageal cancer, lung cancer, endometrial cancer, and colorectal cancer.

In another aspect, the present disclosure also provides a method for treating a tumor disease caused by CCNE1 overexpression and/or FBXW7 inactivating mutation, comprising administering a therapeutically effective amount of the compound, the pharmaceutically acceptable salt thereof, or the isomer thereof described in any one of the embodiments of the present disclosure to a subject.

### Technical effect

The compounds of the present disclosure have good PKMYT1 inhibitory activity, good tumor inhibitory activity *in vitro* and *in vivo,* and pharmacokinetic properties; the compounds of the present disclosure also have good physicochemical properties, such as good solubility, which is more conducive to drug absorption and preparation development.

### Description and definition

In the present disclosure, unless otherwise specified, the scientific and technical terms used herein have the meanings commonly understood by those skilled in the art. However, for a better understanding of the present disclosure, definitions of some terms are provided below. When the definitions of terms provided in the present disclosure are inconsistent with the meanings commonly understood by those skilled in the art, the definitions and explanations of the terms provided in the present disclosure shall prevail.

The "diseases mediated by PKMYT1" described in the present disclosure refer to diseases related to the PKMYT1 target, which may be diseases caused by abnormal expression due to mutation or deletion of PKMYT1 itself, or diseases caused by abnormal expression of PKMYT1 due to abnormalities of other related genes/targets (such as genes/targets that have a synthetic lethal relationship with PKMYT1).

The "CCNE1 overexpression" described in the present disclosure means that the expression level of CCNE1 is higher than that of normal cells. Compared with normal cells, CCNE1-overexpressing cells show higher CCNE1 activity. For example, in normal diploid cells, which exhibit 2 copies, and in CCNE1-overexpressing cells, which exhibit at least 3 copies. CCNE1 overexpression can be measured by identifying the expression level of the gene product in the cell (e.g., mRNA transcript counts of CCNE1 or CCNE1 protein levels).

The "FBXW7 inactivating mutation" described in the present disclosure includes various types of mutations that inactivate FBXW7 gene expression, including but not limited to base insertion, deletion, mutation, substitution, chemical modification, *etc.*

The term "pharmaceutically acceptable" refers to those compounds, materials, compositions and/or dosage forms, which are suitable for use in contact with human and animal tissues within the scope of reasonable medical judgment, without excessive toxicity, irritation, allergic reaction or other problems or complications, and is commensurate with a reasonable benefit/risk ratio.

The "pharmaceutically acceptable salts" described in the present disclosure refer to salts formed between acidic functional groups (*e.g.,* -COOH, -OH, -SO₃H) present in the compound and suitable inorganic or organic cations (bases), including salts formed with alkali metals or alkaline earth metals, ammonium salts, and salts formed with nitrogen-containing organic bases; and salts formed between basic functional groups (*e.g.,* -NH₂) present in the compound and suitable inorganic or organic anions (acids), including salts formed with inorganic acids or organic acids (e.g., carboxylic acids).

The terms "therapeutically effective amount" and "effective amount" refer to an amount sufficient to produce a beneficial or desired effect when the compound of the present disclosure is administered to a subject and is within the dosage range that the subject can tolerate; the effect may be preventing the occurrence of tumors, and/or inhibiting tumor growth, and/or limiting tumor spread, and/or reducing tumor volume, and/or improving clinical symptoms or indicators related to cancer. It will be recognized, however, that the total daily usage of the compounds described in the present disclosure will be decided by the attending physician within the scope of sound medical judgment.

The "isomers" described in the present disclosure include geometric isomers and stereoisomers, such as atropisomers, *cis-trans* isomers, enantiomers, diastereomers, tautomers, and racemic mixtures and other mixtures thereof, all of which are within the scope of the present disclosure. The term "enantiomer" refers to stereoisomers that are mirror images of each other. The term "tautomer" refers to a type of functional group isomer that has different hydrogen attachment points due to one or more double bond shifts, for example, a ketone and its enol form are keto-enol tautomers. The term "diastereomer" refers to stereoisomers in which the molecules have two or more chiral centers and the relationship between the molecules is not mirror images. The term *"cis-trans* isomer" refers to a different spatial configuration in which a double bond or a single bond of a ring carbon atom in a molecule cannot rotate freely. The term "atropisomer" refers to stereoisomers that can be separated because the rotation of the single bond is blocked or very slow.

Stereoisomers of the compounds of the present disclosure may be prepared by chiral synthesis or chiral reagents or other conventional techniques. For example, an enantiomer of a certain compound of the present disclosure may be prepared by an asymmetric catalysis technique or a chiral auxiliary derivatization technique. Alternatively, compounds with a single stereo configuration may be obtained from a mixture by a chiral resolution technique. Alternatively, it can be prepared directly from chiral starting materials. Separation of optically pure compounds in the present disclosure is usually accomplished by preparative chromatography, and a chiral chromatographic column is used to achieve the purpose of separating chiral compounds.

The compounds of the present disclosure may exist in atropisomers, and such structures can be regarded as extensions of chiral centers. Looking along the C1-C1' axis, the groups on both sides of the carbon center C1 closer to the observer are ranked first and second in priority; the groups on both sides of C1' at the other end are ranked third and fourth, and then ranked according to the priority of the groups, with clockwise being R configuration and counterclockwise being S configuration. It should be noted that the results are the same no matter which end of the C-C' axis is viewed. In the present disclosure, when the compound structure adopts a solid bond 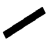 , it means that the compound structure is located above the plane where most atoms in the compound molecule are located, such as 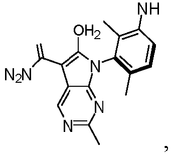 taking the bi-fused ring as the plane, the structure represented by the solid bonds in the benzene ring structure is located above the plane.

It is known to those skilled in the art that when a cyclic compound has a coplanar delocalized system and the number of π electrons is 4n + 2, the ring is aromatic. The aromatic structure in the compound can be expressed by using dotted lines to represent electron delocalization or by alternating single and double bonds. For example, the structure of a benzene ring can be drawn as

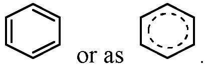

The "optionally substituted" described in the present disclosure refers to two situations in which one or more hydrogen atoms of the substituted group can be "substituted" or "unsubstituted" by one or more substituents.

"PMB" refers to*p*-methoxybenzyl.

When a bond truncated by 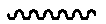 appears in the substituent structure, it means that the bond is a connecting bond of the substituent, for example, 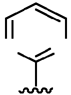 means that the pyrimidine ring is connected to a given group or a given structural formula through a C atom. The dash "-" in the substituent structure indicates the connection point for the substituent, for example, -SCH₃ is connected to a given group or a given structural formula through a sulfur atom.

When the bond of a substituent may be cross-connected to two atoms on a ring, the substituent may be bonded to any atom on the ring. For example, the structure moiety 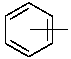 indicates that the substituent R may be substituted at any position on a benzene ring.

When a substituent is listed without indicating the atom via which the substituent is connected to a given group or to a given structural formula, then the substituent may be connected via any bondable atom thereof.

In the present disclosure, when R₃ and R₆ defined in the structural moiety 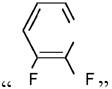 together with the carbon atom to which they are attached form the following cyclic structure: 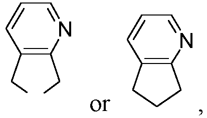 at this time, the newly formed ring 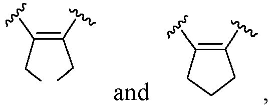 due to the presence of a shared double bond, are defined as "heterocycloalkenyl" and "cycloalkenyl", respectively. In the present disclosure, the "R² and R⁸ together with the atom to which they are attached form a C₅₋₆ cycloalkenyl group", "R³ and R⁶ together with the atom to which they are attached form a phenyl, 5- to 6-membered heterocycloalkenyl, C₅₋₆ cycloalkenyl group", "R⁴ and R⁶ together with the atom to which they are attached form a phenyl, 5- to 6-membered heterocycloalkenyl, C₅₋₆ cycloalkenyl group" shall be construed in accordance with this definition.

The "alkyl" described in the present disclosure refers to a group derived from a branched or straight-chain saturated aliphatic alkane with a specified number of carbon atoms by removing a hydrogen atom. For example, "C₁₋₁₀ alkyl" refers to C₁, C₂, C₃, C₄, C₅, C₆, C₇, C₈, C₉, and C₁₀ alkyl, including "C₁₋₆ alkyl", "C₁₋₄ alkyl", and "C₁₋₃ alkyl"; specific examples include but are not limited to: methyl, ethyl, n-propyl, isopropyl, sec-butyl, 2-methylbutyl, 1,1-dimethylbutyl, *etc.*

The "haloalkyl" described in the present disclosure refers to a group obtained by substituting one or more hydrogen atoms in an alkyl group by halogen, such as "fluoromethyl" including monofluoromethyl, difluoromethyl, and trifluoromethyl; preferably, the "haloalkyl" described in the present disclosure is "halo-C₁₋₆ alkyl" or "halo-C₁₋₄ alkyl". Alkyl is as defined above.

The "hydroxyalkyl" described in the present disclosure refers to a group obtained by substituting one or more hydrogen atoms in an alkyl group by hydroxyl. The "hydroxyalkyl" described in the present disclosure includes "hydroxy-C₁₋₆ alkyl" and "hydroxy-C₁₋₄ alkyl". Specific examples include but are not limited to -CH₂OH, -CH₂CH₂OH, -CH(OH)CH₃, - CH₂CH₂CH₂OH, 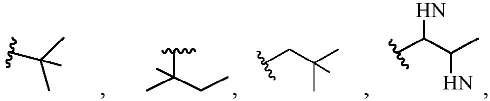 *etc.*

The "alkoxy" described in the present disclosure refers to an alkyl group as defined herein connected to other groups through an oxygen atom, *i.e.,* "alkyl-O-". Including "C₁₋₆ alkoxy" (structure is C₁₋₆ alkyl-O-) and "C₁₋₄ alkoxy", specific examples include but are not limited to methoxy, ethoxy, propoxy, 1-methylethoxy, butoxy, 2-methylbutoxy, 3-methylbutoxy, 1,1-dimethylpropoxy, 1,2-dimethylpropoxy, *etc.;* preferably, the "alkoxy" described in the present disclosure is C₁₋₄ alkoxy, more preferably C₁₋₃ alkoxy.

The "haloalkoxy" described in the present disclosure refers to a group obtained by substituting one or more hydrogen atoms in an alkoxy group with halogen. Preferably, the "haloalkoxy" described in the present disclosure is "halo-C₁₋₆ alkoxy" or "halo-C₁₋₄ alkoxy". Specific examples such as "fluoromethoxy" described in the present disclosure include monofluoromethoxy, difluoromethoxy, and trifluoromethoxy; and also include but are not limited to: -OCH₂CF₃, -OCHFCH₃, *etc.* Alkoxy is as defined above.

The "alkenyl" described in the present disclosure refers to a group derived from a straight-chain or branched alkene (containing at least one double bond) by removing a hydrogen atom, including "C₂₋₆ alkenyl", "C₂₋₅ alkenyl", "C₂₋₄ alkenyl", and "C₂₋₃ alkenyl", and specific examples include but are not limited to: -CH=CH₂, -CH=CHCH₃, -C(CH₂)= CH₂, -CH=CHCH₂CH₃, -CH₂CH=CHCH₃, *etc.*

The "alkynyl" described in the present disclosure refers to a group derived from a straight-chain or branched alkyne (containing at least one triple bond) by removing a hydrogen atom, including "C₂₋₅ alkynyl", "C₂₋₄ alkynyl", and "C₂₋₃ alkynyl", and specific examples include but are not limited to: -C=CH, -C=CHCH₃, -CH=CHCH₂-, -CH=C-C=C-, *etc.*

The "alkylene" described in the present disclosure refers to a group derived from a branched or straight-chain saturated aliphatic alkane by removing two hydrogen atoms, and the removed hydrogen atoms may be derived from the same carbon atom or different carbon atoms; the "alkylene" described in the present disclosure is preferably a "straight-chain alkylene"; the "alkylene" includes "C₁₋₆ alkylene", "C₁₋₄ alkylene", and "C₁₋₂ alkylene"; specific examples include but are not limited to -CH₂-, -CH₂CH₂-, -CH₂CH₂CH₂-, -CH(CH₂)CH₂-, - CH₂CH₂CH₂CH₂-, -CH(CH₂)CH₂CH₂-, -CH(CH₂CH₂)CH₂-, -C(CH₂)(CH₂)CH₂-, - CH₂CH₂CH₂CH₂CH₂-, *etc.*

The "alkenylene" described in the present disclosure refers to a group derived from a straight-chain or branched alkene (containing at least one double bond) by removing two hydrogen atoms, and the removed hydrogen atoms may be derived from the same carbon atom or different carbon atoms. The "alkenylene" includes "C₂₋₆ alkenylene" and "C₂₋₄ alkenylene". Specific examples include but are not limited to -CH=CH-, -CH=CHCH₂-, -CH=C(CH₃)CH₂-, -CH=CHCH₂CH=CH-, -CH₂CH=CHCH₂-, and -CH₂CH=CHCH₂CH₂-.

The "alkynylene" described in the present disclosure refers to a group derived from a straight-chain or branched alkyne (containing at least one triple bond) by removing two hydrogen atoms, and the removed hydrogen atoms may be derived from the same carbon atom or different carbon atoms. The "alkynylene" includes "C₂₋₆ alkynylene" and "C₂₋₄ alkynylene". Specific examples include but are not limited to -C≡C-, -C≡C-CH₂-, -C≡C-CH₂CH₂CH₂-, -CH₂-C≡C-CH₂CH₂CH₂-, -C≡C-CH₂C≡C-, and -C≡C-CH₂CH=CH-.

The "cycloalkyl" described in the present disclosure refers to a saturated cyclic group derived from a monocyclic cycloalkane by removing a hydrogen atom. In the cycloalkyl, except for the carbon atoms bonded to a given group or a given structural formula, other ring carbon atoms can be further oxidized, that is, to form C(O). The cycloalkyl includes "3- to 8-membered cycloalkyl", "3- to 6-membered cycloalkyl", "3- to 5-membered cycloalkyl", and "4- to 6-membered cycloalkyl". Specific examples include, but are not limited to: cyclopropyl, cyclobutyl, cyclopentyl, cyclohexyl, cycloheptyl, and cyclooctyl.

The "cycloalkenyl" described in the present disclosure refers to a "cycloalkyl" group in which one or more ring-forming bonds are double bonds and the cycloalkenyl is not aromatic. In the cycloalkenyl, except for the carbon atoms bonded to a given group or a given structural formula, other ring carbon atoms can be further oxidized, that is, to form C(O). The cycloalkenyl includes "3- to 8-membered cycloalkenyl", "3- to 6-membered cycloalkenyl", "3-to 5-membered cycloalkenyl", and "5- to 6-membered cycloalkenyl". Specific examples include, but are not limited to

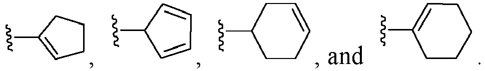

The "heterocyclyl" described in the present disclosure refers to a saturated cyclic group obtained by replacing one or more ring carbon atoms in a cycloalkyl group by a heteroatom. The heteroatom is generally selected from N, O, and S; the carbon atoms or heteroatoms in the heterocyclyl can be further oxidized, *i.e.,* forming C(O), N(O), SO, SO₂; preferably, the heteroatom is independently selected from 1-3 N and/or O. The heterocyclyl includes "3- to 8-membered heterocyclyl", "3- to 6-membered heterocyclyl", "3- to 5-membered heterocyclyl", "4- to 6-membered heterocyclyl", and "5- to 6-membered heterocyclyl". Specific examples include but are not limited to azetidinyl, pyrrolidinyl, tetrahydrofuranyl, piperidinyl, piperazinyl, tetrahydropyranyl, morpholinyl, *etc.*

The "heterocycloalkenyl" described in the present disclosure means that one or more ring-forming bonds in the "heterocyclyl" are double bonds and the heterocycle is not aromatic. Preferably, the heteroatom is independently selected from 1-3 N and/or O. The heterocyclyl includes "3- to 8-membered heterocycloalkenyl", "3- to 6-membered heterocycloalkenyl", "3-to 5-membered heterocycloalkenyl", and "5- to 6-membered heterocycloalkenyl". Specific examples include but are not limited to: 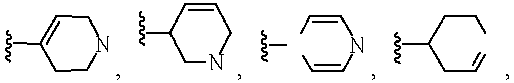 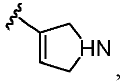 *etc.*

The "aryl" described in the present disclosure refers to a monocyclic or polycyclic group consisting of cyclic carbon atoms with aromatic properties, specific examples include but are not limited to: phenyl and naphthyl.

The "heteroaryl" described in the present disclosure refers to a monocyclic group with aromatic properties in which at least one ring atom is a heteroatom, and the heteroatom is generally selected from N, O, and S; the carbon atoms or heteroatoms in the heterocyclyl may be further oxidized, and the oxidization generally conforms to the rules and conditions for valence bond formation, that is, forming C(O), N(O), SO, or SO₂; preferably, the heteroatoms are independently selected from 1-3 N and/or O. The heteroaryl includes "5- to 6-membered heteroaryl"; specific examples include but are not limited to pyrrolyl, furanyl, thienyl, pyrazolyl, imidazolyl, pyrazinyl, pyridazinyl, triazinyl, oxazolyl, isoxazolyl, thiazolyl, isothiazolyl, pyridinyl, and pyrimidinyl.

The combination of substituents and/or variables described in the present disclosure should be allowed only when these combinations produce stable compounds or usable synthetic intermediates. Those skilled in the art can exclude situations that are obviously beyond the conventional knowledge in the field and unreasonable situations from the present disclosure. A stable compound or stable structure is a compound that is stable enough to undergo chemical reactions, be isolated in a useful purity, and be formulated into an effective therapeutic drug.

### DETAILED DESCRIPTION OF THE PREFERRED EMBODIMENT

In the examples of the present disclosure, the nomenclature of the title compound is converted from the compound structure by means of ChemDraw. If there is any inconsistency between the compound name and the compound structure, it may be determined by synthesizing relevant information and reaction routes; if it cannot be confirmed by other methods, the given structural formula of the compound shall prevail.

The preparation methods of some compounds in the present disclosure refer to the preparation methods of the aforementioned similar compounds. Those skilled in the art should know that when using or referring to the preparation methods cited therein, the feed ratio of reactants, reaction solvent, reaction temperature, *etc.* may be appropriately adjusted according to the different reactants.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

### 1. Summary of experimental instruments:

The structure of the compound of the present disclosure is determined by nuclear magnetic resonance (NMR) and/or liquid chromatography-mass spectrometry (LC-MS). NMR chemical shift (δ) is given in units of parts per million (ppm). NMR is determined using a Varian 400M or Bruker Ascend 400 NMR instrument with deuterated dimethyl sulfoxide (DMSO-*d*₆), deuterated methanol (CD₃OD), deuterated chloroform (CDCl₃), or heavy water (D₂O) as a solvent and tetramethylsilane (TMS) as an internal standard.

The starting materials in the examples of the present disclosure are known and commercially available, or can be synthesized by using or following methods known in the art.

### 2. Synthesis examples

The purification methods described in the examples of the present disclosure include but are not limited to methods known in the art, such as silica gel chromatography, preparative HPLC chromatography, *etc.*

The compounds of the present disclosure are mainly synthesized using the following schemes:

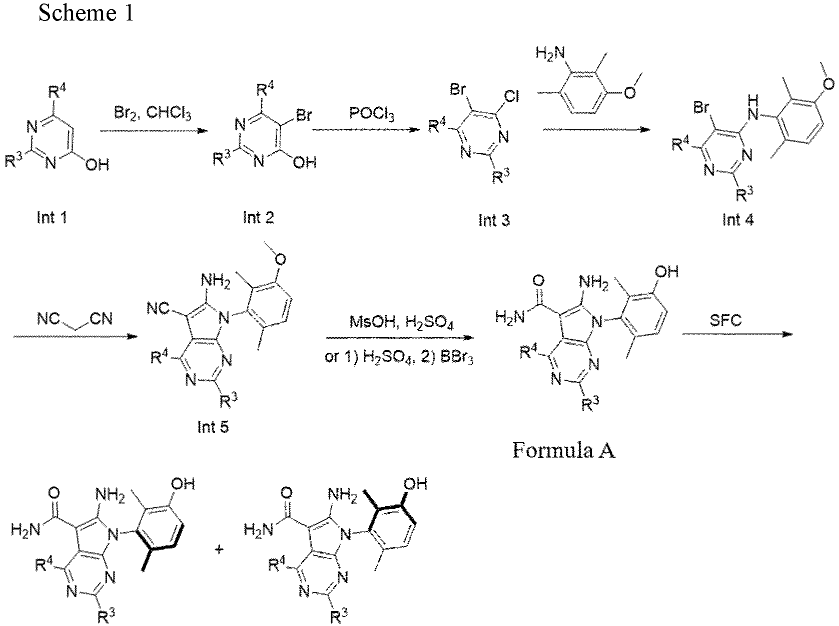

The compound represented by formula A can be prepared from intermediate 1 by the following steps: intermediate 1 is brominated to prepare intermediate 2, intermediate 2 is hydroxychlorinated to obtain intermediate 3, and then subjected to a substitution reaction with 3-methoxy-2,6-dimethylaniline in a base and an organic solvent or a coupling reaction catalyzed by palladium/chiral phosphine ligands to prepare intermediate 4. Intermediate 5 is prepared by cyclization-coupling of intermediate 4 with malononitrile. Intermediate 5 is prepared into a compound represented by formula A by adding an acid in an organic solvent, and the compound represented by formula A is subjected to a chiral preparation method to obtain two chiral isomers.

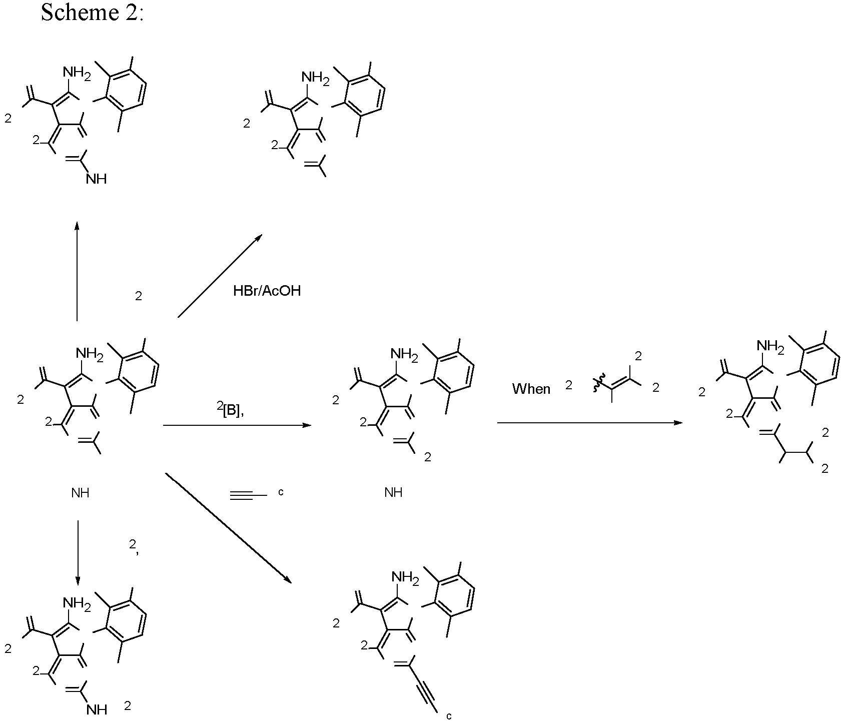

As shown in Scheme 2, products 7 and 12 can be prepared from intermediate 6 as the starting reactant: intermediate 6 is subjected to a cyano substitution or bromination reaction to prepare products 7 and 8, and intermediate 6 is subjected to a coupling reaction with an organic boron reagent to prepare intermediate 9; when R³ is an alkene substituent, intermediate 9 is subjected to a reduction reaction to prepare product 10. Intermediate 6 is subjected to a coupling reaction with an alkyne reagent to prepare product 11, and intermediate 6 is subjected to a substitution reaction with an amine compound to prepare product 12.

R^{m}, R^{P}, and R^{q} are independently H, C₁₋₄ alkyl, C₃₋₆ cycloalkyl, or R^{m} and R^{q} together with the group to which they are attached form a C₃₋₆ cycloalkenyl or 3- to 6-membered heterocycloalkenyl group.

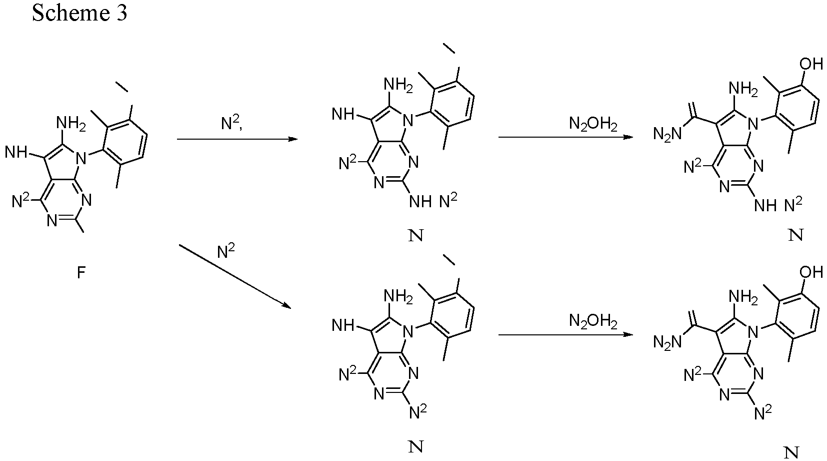

As shown in Scheme 3, 12-15 can be prepared from intermediate 9: intermediate 9 is subjected to a substitution reaction with an amine to prepare intermediate 13, and intermediate 13 is acid-hydrolyzed as described in Scheme 1 above to prepare product 12. Intermediate 9 is subjected to a coupling reaction with an organic boron reagent to prepare intermediate 14, and intermediate 14 is acid-hydrolyzed to prepare product 15.

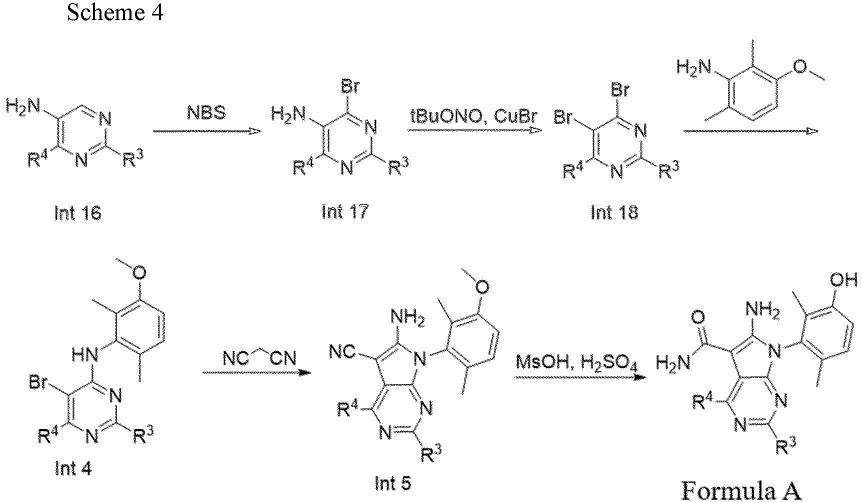

As shown in Scheme 4, the compound represented by formula A can be prepared from the compound represented by intermediate 16 by the following steps: intermediate 16 is subjected to a bromination reaction to obtain intermediate 17, intermediate 17 is subjected to a Sandermeyer bromination reaction of the amino group to prepare intermediate 18, and intermediate 18 is subjected to aromatic amination, malononitrile coupling cyclization, and acid hydrolysis as described in Scheme 1 above to obtain the compound represented by formula A.

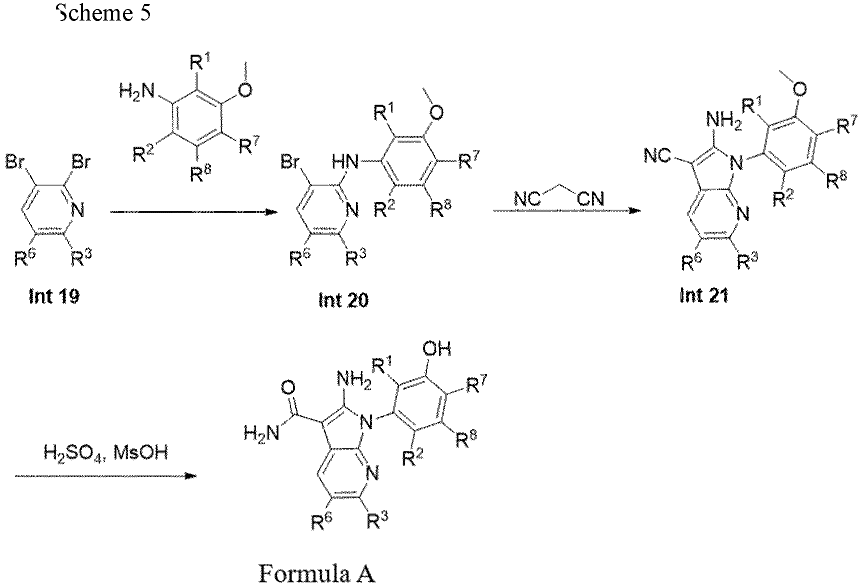

As shown in Scheme 5, the compound represented by formula A can be prepared from the intermediate 19 by aromatic amination, malononitrile coupling cyclization, and acid hydrolysis as described in Scheme 1 above.

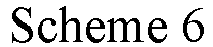

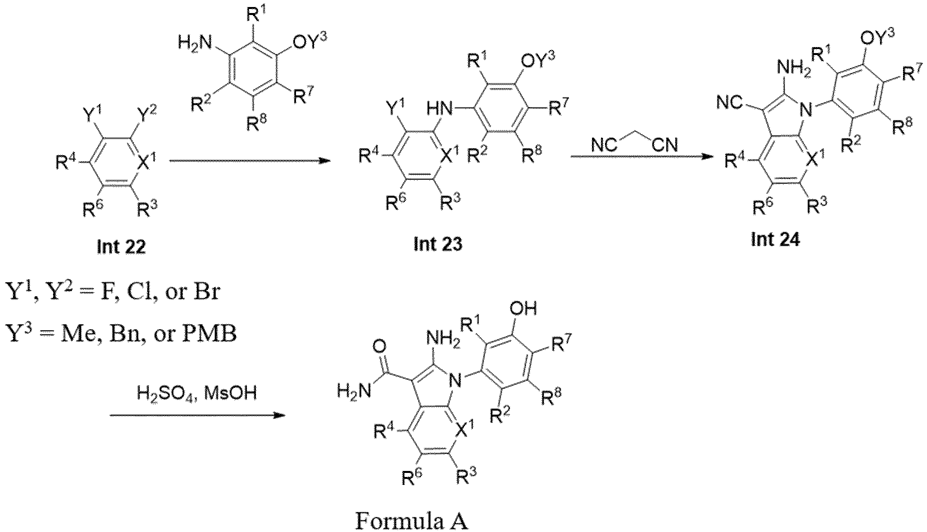

As shown in Scheme 5, the compound represented by formula A can be prepared from the intermediate 22 by aromatic amination, malononitrile coupling cyclization, and acid hydrolysis as described in Scheme 1 above.

The products and racemic intermediate compounds in the above Schemes 2-5 can be prepared by the chiral preparation method shown in Scheme 1 to obtain two chiral isomers respectively.

Specific compound synthesis examples:

### Example 1

### 6-Amino-7-(3-hydroxy-2,6-dimethylphenyl)-2-methyl-7H-pyrrolo[2,3-d]pyrimidine-5-carboxamide

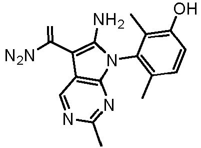

In a single-necked flask, 5-bromo-4-chloro-2-methylpyrimidine (9 g, 43.48 mmol), 3-methoxy-2,6-dimethylaniline (7.88 g, 52.19 mmol), and 2,6-dimethylpyridine (8.61 g, 86.96 mmol) were dissolved in N-methylpyrrolidone (180 mL) and stirred at 130°C for 40 hours. After the complete of the reaction was monitored by TLC, saturated ammonium chloride aqueous solution (300 mL) and ethyl acetate (200 mL) were added to the reaction mixture for extraction. After phase separation, the ethyl acetate phase was collected, and the aqueous phase was extracted again with ethyl acetate (100 mL). The organic phases were combined, washed with saturated ammonium chloride (100 mL × 2), water (100 mL), and saturated brine (100 mL) in sequence, dried over anhydrous sodium sulfate, and concentrated under reduced pressure to remove the organic solvent to obtain a crude product. The crude product was purified to obtain 5-bromo-N-(3-methoxy-2,6-dimethylphenyl)-2-methylpyrimidin-4-amine (2.3 g). MS (ESI) m/z: 323.7 [M+H⁺].

Under nitrogen atmosphere, malononitrile (2.34 g, 35.50 mmol) and sodium *tert-*butoxide (3.40 g, 35.50 mmol) were dissolved in tetrahydrofuran (44 mL) and reacted at room temperature for 30 minutes. 5-Bromo-N-(3-methoxy-2,6-dimethylphenyl)-2-methylpyrimidin-4-amine (2.3 g, 7.10 mmol) and 1,1'-bis(diphenylphosphino)ferrocene-palladium(II)dichloride dichloromethane complex (520 mg, 0.71 mmol) were added thereto, then the system was replaced with nitrogen again, and the reaction was carried out at 100°C for 3 hours. After TLC monitoring showed that the raw material disappeared, water (60 mL) and ethyl acetate (40 mL) were added and stirred for phase separation. The organic phase was washed with saturated brine (40 mL), dried over anhydrous sodium sulfate, and concentrated under reduced pressure to obtain a crude product. The crude product was purified to obtain 6-amino-7-(3-methoxy-2,6-dimethylphenyl)-2-methyl-7H-pyrrolo[2,3-d]pyrimidine-5-carbonitrile (1.2 g). MS (ESI) m/z: 308.0 [M+H⁺].

6-Amino-7-(3-methoxy-2,6-dimethylphenyl)-2-methyl-7H-pyrrolo[2,3-d]pyrimidine-5-carbonitrile (1.2 g, 3.91 mmol) was dissolved in sulfuric acid/water (13/1, 6 mL), and methanesulfonic acid (14 g, 144.63 mmol) was added and stirred at room temperature for 2 hours. DL-Methionine (2.33 g, 15.64 mmol) was then added to the reaction mixture and stirred at 40°C overnight. After TLC monitoring showed that the raw material disappeared, the reaction was stopped. The pH was adjusted to about 7 by dropwise adding sodium hydroxide/dipotassium hydrogen phosphate aqueous solution to the reaction mixture, and the mixture was filtered to obtain a crude product, which was purified to obtain 6-amino-7-(3-hydroxy-2,6-dimethylphenyl)-2-methyl-7H-pyrrolo[2,3-d]pyrimidine-5-carboxamide (690 mg). MS (ESI) m/z: 311.8 [M+H⁺]. ¹H NMR (400 MHz, DMSO-*d*₆) δ 9.62 (s, 1H), 8.88 (s, 1H), 7.11-7.03 (m, 3H), 6.94 (d, *J =* 8.3 Hz, 1H), 6.85 (s, 2H), 2.44 (s, 3H), 1.75 (s, 3H), 1.67 (s, 3H). C₁₆H₁₇N₅O₂.

### Example 2

### S-6-Amino-7-(3-hydroxy-2,6-dimethylphenyl)-2-methyl-7H-pyrrolo[2,3-d]pyrimidine-5-carboxamide

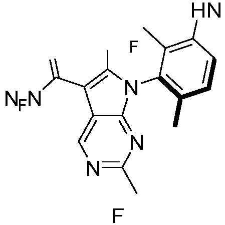

Compound 2 was prepared by chiral HPLC from 100 mg of the compound of Example 1 (29.9 mg, yield of 30%). [α]²⁰_{D} = +55.54° (c = 0.088, MeOH), >99% ee.

Chiral HPLC analysis conditions are: Chiral column: Daicel OZ-3 4.6 * 100 mm 3 µm, temperature: 40°C, mobile phase: CO₂/IPA [1% NH₃ (7M in MeOH)], flow rate: 3.0 mL/min, back pressure: 2000 psi, detection wavelength: 280 nm, cycle time: 3 min, RT = 1.117 min.

SFC separation conditions are: equipment: SFC-150mgm (waters), chiral column: Daicel OZ (25 * 250 mm, 10 µm), temperature: 30°C, mobile phase: CO₂/IPA [0.5% NH₃ (7M in MeOH)] = 65/35, flow rate: 100 mL/min, back pressure: 100 bar, detection wavelength: 214 nm, cycle time: 5.36 min, sample solution: 100 mg dissolved in 45 mL MeOH, injection volume: 2.1 mL.

MS (ESI) m/z: 312.2 [M+H⁺]. ¹H NMR (400 MHz, DMSO) δ 9.58 (s, 1H), 8.88 (s, 1H), 7.08 (d, *J =* 8.3 Hz, 1H), 7.04 (s, 2H), 6.93 (d, *J =* 8.3 Hz, 1H), 6.83 (s, 2H), 2.44 (s, 3H), 1.75 (s, 3H), 1.67 (s, 3H).

### Example 3

### R-6-Amino-7-(3-hydroxy-2,6-dimethylphenyl)-2-methyl-7H-pyrrolo[2,3-d]pyrimidine-5-carboxamide

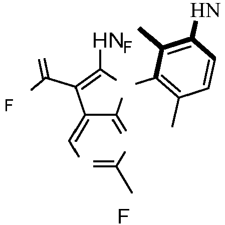

Compound 3 was prepared by chiral HPLC from 100 mg of the compound of Example 1 (32.1 mg, yield of 32%). [α]²⁰_{D} = -1.99° (c = 0.100, MeOH), 98% ee.

Chiral HPLC analysis conditions are: Chiral column: Daicel OZ-3 4.6 * 100 mm 3 µm, temperature: 40°C, mobile phase: CO₂/IPA [1% NH₃ (7M in MeOH)], flow rate: 3.0 mL/min, back pressure: 2000 psi, detection wavelength: 280 nm, cycle time: 3 min, RT = 1.511 min.

SFC separation conditions are: equipment: SFC-150mgm (waters), chiral column: Daicel OZ (25 * 250 mm, 10 µm), temperature: 30°C, mobile phase: CO₂/IPA [0.5% NH₃ (7M in MeOH)] = 65/35, flow rate: 100 mL/min, back pressure: 100 bar, detection wavelength: 214 nm, cycle time: 5.36 min, sample solution: 100 mg dissolved in 45 mL MeOH, injection volume: 2.1 mL.

MS (ESI) m/z: 312.2 [M+H⁺]. ¹H NMR (400 MHz, DMSO) δ 9.58 (s, 1H), 8.88 (s, 1H), 7.08 (d, *J =* 8.3 Hz, 1H), 7.04 (s, 2H), 6.93 (d, *J =* 8.3 Hz, 1H), 6.83 (s, 2H), 2.44 (s, 3H), 1.75 (s, 3H), 1.67 (s, 3H).

### Example 4

### 6-Amino-7-(3-hydroxy-2,6-dimethylphenyl)-2-(trifluoromethyl)-7H-pyrrolo[2,3-d]pyrimidine-5-carboxamide

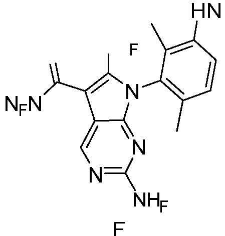

2-(Trifluoromethyl)pyrimidin-5-amine (9.5 g, 58.3 mmol) was dissolved in acetonitrile (100 mL), then N-bromosuccinimide (12.5 g, 70 mmol) was added, and the reaction was carried out at room temperature overnight. After TLC monitoring showed that the raw material disappeared, the reaction was stopped, and water (200 mL) and ethyl acetate (200 mL) were added to extract the reaction. The organic phase was washed with saturated brine (200 mL), dried over anhydrous sodium sulfate, and concentrated under reduced pressure to remove the organic solvent to obtain a crude product. The crude product was purified to obtain 4-bromo-2-(trifluoromethyl)pyrimidin-5-amine (9.2 g). ¹H NMR (400 MHz, CDCl₃) δ 8.19 (s, 1H), 4.56 (br, 2H). C₅H₃BrF₃N₃.

4-Bromo-2-(trifluoromethyl)pyrimidin-5-amine (4 g, 16.6 mmol) was dissolved in acetonitrile (40 mL), and *tert*-butyl nitrite (2.56 g, 25 mmol) was added in an ice-water bath, and then cuprous bromide (2.83 g, 19.92 mmol) was added and stirred at 60°C for 2 hours. After TLC monitoring showed that the raw material disappeared, water (100 mL) and ethyl acetate (100 mL) were added to extract the reaction system, and the organic phase was collected. The organic phase was then washed with saturated brine (100 mL), dried over anhydrous sodium sulfate, and concentrated under reduced pressure to obtain a crude product, which was purified to obtain 4,5-dibromo-2-(trifluoromethyl)pyrimidine (700 mg). ¹H NMR (400 MHz, CDCl₃) δ 8.91 (s, 1H). C₅HBr₂F₃N₃.

4,5-Dibromo-2-(trifluoromethyl)pyrimidine (700 mg, 2.31 mmol), 3-methoxy-2,6-dimethylaniline (384 mg, 2.54 mmol), and 2.6-dimethylpyridine (400 mg, 3.74 mmol) were dissolved in *N,N*-dimethylpyrrolidone (20 mL), and the mixture was stirred at 95°C overnight. After TLC monitoring showed that the raw material disappeared, water (30 mL) and ethyl acetate (30 mL) were added and stirred for phase separation. After phase separation, the organic phase was washed with saturated brine (20 mL), dried over anhydrous sodium sulfate, and concentrated under reduced pressure to obtain a crude product. The crude product was purified to obtain 5-bromo-N-(3-methoxy-2,6-dimethylphenyl)-2-(trifluoromethyl)pyrimidin-4-amine (650 mg). MS (ESI) m/z: 378.2 [M+H⁺].

Malononitrile (585 mg, 8.6 mmol) was dissolved in ethylene glycol dimethyl ether (40 mL), and then sodium tert-butoxide (845 mg, 8.6 mmol) was added to the reaction system, and the reaction system was replaced with nitrogen. After stirring at room temperature for 30 minutes, 5-bromo-*N*-(3-methoxy-2,6-dimethylphenyl)-2-(trifluoromethyl)pyrimidin-4-amine (650 mg, 1.72 mmol) and 1,1'-bis(diphenylphosphino)ferrocene-palladium(II)dichloride dichloromethane complex (130 mg, 0.16 mmol) were added to the reaction system and reacted at 120°C for 4 hours. After TLC monitoring showed that the raw material disappeared, water (20 mL) and ethyl acetate (40 mL) were added and stirred for phase separation. The organic phase was collected and washed with saturated brine (10 mL), dried over anhydrous sodium sulfate, and concentrated under reduced pressure to obtain a crude product. The crude product was purified to obtain 6-amino-7-(3-methoxy-2,6-dimethylphenyl)-2-(trifluoromethyl)-7H-pyrrolo[2,3-d]pyrimidine-5-carbonitrile (300 mg). MS (ESI) m/z: 361.9 [M+H⁺].

6-Amino-2-chloro-7-(3-methoxy-2,6-dimethylphenyl)-7H-pyrrolo[2,3-d]pyrimidine-5-carbonitrile (50 mg, 0.14 mmol) was dissolved in sulfuric acid/water (0.1 mL, sulfuric acid/water = 13/1), and then methanesulfonic acid (500 mg, 5.14 mmol) was added to the reaction system and stirred at room temperature for 2 hours. DL-Methionine (83 mg, 0.55 mmol) was added to the reaction mixture and stirred at 40°C overnight. After TLC monitoring showed that the raw material disappeared, sodium hydroxide/dipotassium hydrogen phosphate aqueous solution (20 mL) was added dropwise to the reaction system to adjust the pH to about 7. Dichloromethane and methanol were added for phase separation, and the organic phase was collected and washed with saturated brine (10 mL), dried over anhydrous sodium sulfate, and concentrated under reduced pressure to obtain a crude product. The crude product was purified to obtain 6-amino-7-(3-hydroxy-2,6-dimethylphenyl)-2-(trifluoromethyl)-7H-pyrrolo[2,3-d]pyrimidine-5-carboxamide (10.43 mg). MS (ESI) m/z: 365.5 [M+H⁺]. ¹H NMR (400 MHz, DMSO-*d*₆) δ 9.71 (s, 1H), 9.13 (s, 1H), 7.51 (s, 2H), 7.16-7.09 (m, 3H), 6.98 (d, *J =* 8.3 Hz, 1H), 1.75 (s, 3H), 1.67 (s, 3H). C₁₆H₁₄F₃N₅O₂.

### Example 5

### 6-Amino-2-chloro-7-(3-hydroxy-2,6-dimethylphenyl)-7H-pyrrolo[2,3-d]pyrimidine-5-carboxamide

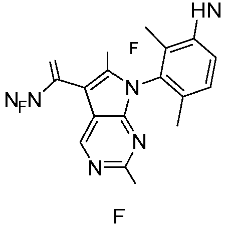

5-Bromo-2,4-dichloropyrimidine (2 g, 8.78 mmol) was dissolved in NMP (20 mL), then 3-methoxy-2,6-dimethylaniline (1.5 g, 9.65 mmol) and 2,6-dimethylpyridine (1.5 g, 14.22 mmol) were added, and the reaction mixture was stirred overnight at 95°C. After TLC monitoring showed that the raw material disappeared, the reaction was stopped. Then water (50 mL) and ethyl acetate (100 mL) were added to the cooled reaction mixture, stirred and extracted, and the organic phase was collected after phase separation. The organic phase was washed with saturated brine (50 mL), dried over anhydrous sodium sulfate, and concentrated under reduced pressure to remove the solvent to obtain a crude product. The crude product was purified to obtain 5-bromo-2-chloro-N-(3-methoxy-2,6-dimethylphenyl)pyrimidin-4-amine (1.8 g). MS (ESI) m/z: 342.0 [M+H⁺].

Malononitrile (288.6 mg, 4.37 mmol) was dissolved in ethylene glycol dimethyl ether (7.8 mL), and then sodium tert-butoxide (420.3 mg, 4.37 mmol) was added to the reaction system. After nitrogen replacement, the mixture was stirred at room temperature for 30 minutes. 5-Bromo-2-chloro-N-(3-methoxy-2,6-dimethylphenyl)pyrimidin-4-amine (300 mg, 0.87 mmol) and 1,1'-bis(diphenylphosphino)ferrocene-palladium(II)dichloride dichloromethane complex (55 mg, 0.067 mmol) were added to the reaction system under nitrogen atmosphere, and the reaction mixture was stirred at 120°C for 4 hours. After TLC monitoring showed that the raw material disappeared, water (20 mL) and ethyl acetate (20 mL) were added and stirred for phase separation. The organic phase was collected and washed with saturated brine (10 mL), dried over anhydrous sodium sulfate, and concentrated under reduced pressure to obtain a crude product. The crude product was purified to obtain 6-amino-2-chloro-7-(3-methoxy-2,6-dimethylphenyl)-7H-pyrrolo[2,3-d]pyrimidine-5-carbonitrile (120 mg). MS (ESI) m/z: 328.44 [M+H⁺].

6-Amino-2-chloro-7-(3-methoxy-2,6-dimethylphenyl)-7H-pyrrolo[2,3-d]pyrimidine-5-carbonitrile (120 mg, 0.37 mmol) was dissolved in sulfuric acid/water (13/1, 0.3 mL), and methanesulfonic acid (1.3 g, 13.6 mmol) was added and stirred at room temperature for 2 hours. DL-Methionine (219 mg, 1.47 mmol) was then added to the reaction mixture, and the reaction mixture was stirred at 40°C overnight. After TLC monitoring showed that the raw material disappeared, sodium hydroxide/dipotassium hydrogen phosphate aqueous solution was added to the system to adjust the pH to about 7, and solids were precipitated, and then the mixture was filtered to obtain a crude product. The crude product was purified to obtain 6-amino-2-chloro-7-(3-hydroxy-2,6-dimethylphenyl)-7H-pyrrolo[2,3-d]pyrimidine-5-carboxamide (50 mg). MS (ESI) m/z: 331.3 [M+H⁺]. ¹H NMR (400 MHz, DMSO-*d*₆) δ 9.69 (s, 1H), 8.89 (s, 1H), 7.33 (s, 2H), 7.15 - 6.85 (m, 4H), 1.76 (s, 3H), 1.68 (s, 3H). C₁₅H₁₄ClN₅O₂.

### Example 6

### 6-Amino-7-(3-hydroxy-2,6-dimethylphenyl)-2-vinyl-7H-pyrrolo[2,3-d]pyrimidine-5-carboxamide

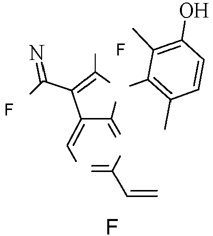

Step A: 6-Amino-2-chloro-7-(3-hydroxy-2,6-dimethylphenyl)-7H-pyrrolo[2,3-d]pyrimidine-5-carboxamide (200 mg, 0.6 mmol), 4,4,5,5-tetramethyl-2-vinyl-1,3,2-dioxaborolane (279.2 mg, 1.8 mmol), potassium carbonate (250.2 mg, 1.8 mmol), and tetrakis(triphenylphosphine)palladium (69.8 mg, 0.06 mmol) were dissolved in *N,N-*dimethylformamide/water (5/1, 7.2 mL), reacted at 100°C under nitrogen atmosphere overnight. After LCMS monitoring showed that the raw material disappeared, the reaction mixture was cooled to room temperature. Water (20 mL) and ethyl acetate (20 mL) were used for extraction post-treatment. The collected organic phase was washed with saturated brine (10 mL), dried over anhydrous sodium sulfate, and concentrated under reduced pressure to obtain a crude product. The crude product was purified to obtain 6-amino-7-(3-hydroxy-2,6-dimethylphenyl)-2-vinyl-7H-pyrrolo[2,3-d]pyrimidine-5-carboxamide (83.84 mg). MS (ESI) m/z: 324.5 [M+H⁺]. ¹H NMR (400 MHz, DMSO-*d*₆) δ 9.62 (s, 1H), 8.97 (s, 1H), 7.19 (s, 2H), 7.09 (d, *J* = 8.3 Hz, 1H), 6.98 - 6.90 (m, 3H), 6.63 (dd, *J =* 17.2, 10.6 Hz, 1H), 6.18 (dd, *J =* 17.2, 2.3 Hz, 1H), 5.40 (dd, J= 10.5, 2.2 Hz, 1H), 1.77 (s, 3H), 1.68 (s, 3H). C₁₇H₁₇N₅O₂.

### Example 7

### 6-Amino-2-bromo-7-(3-hydroxy-2,6-dimethylphenyl)-7H-pyrrolo[2,3-d]pyrimidine-5-carboxamide

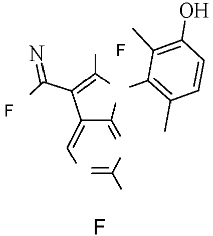

6-Amino-2-chloro-7-(3-hydroxy-2,6-dimethylphenyl)-7H-pyrrolo[2,3-d]pyrimidine-5-carboxamide (100 mg, 0.3 mmol) was dissolved in 33% hydrobromic acid/acetic acid solution (1 mL), heated to 80°C, and reacted overnight. After LCMS monitoring showed that the raw material disappeared, the reaction mixture was cooled to room temperature. Saturated sodium bicarbonate aqueous solution (20 mL) and ethyl acetate (20 mL) were added and stirred, and the phases were separated. The collected organic phase was washed with saturated brine (10 mL), dried over anhydrous sodium sulfate, and concentrated under reduced pressure to obtain a crude product. The crude product was purified to obtain 6-amino-2-bromo-7-(3-hydroxy-2,6-dimethylphenyl)-7H-pyrrolo[2,3-d]pyrimidine-5-carboxamide (45.58 mg). MS (ESI) m/z: 376.0 [M+H⁺]. ¹H NMR (400 MHz, DMSO-*d*₆) δ 9.70 (s, 1H), 8.85 (s, 1H), 7.33 (s, 2H), 7.11 (d, *J* = 8.3 Hz, 1H), 7.02- 6.93 (m, 3H), 1.76 (s, 3H), 1.68 (s, 3H). C₁₅H₁₄BrN₅O₂.

### Example 8

### 6-Amino-2-ethyl-7-(3-hydroxy-2,6-dimethylphenyl)-7H-pyrrolo[2,3-d]pyrimidine-5-carboxamide

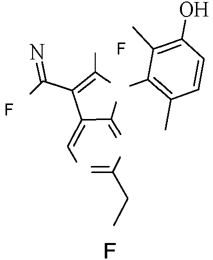

6-Amino-7-(3-hydroxy-2,6-dimethylphenyl)-2-vinyl-7H-pyrrolo[2,3-d]pyrimidine-5-carboxamide (80 mg, 0.25 mmol) was dissolved in methanol (10 mL), then 10% palladium on carbon (100 mg) was added and reacted under hydrogen atmosphere at normal pressure for 3 hours. After LCMS monitoring showed that the raw material disappeared, the mixture was filtered and the filtrate was concentrated to obtain a crude product. The crude product was purified to obtain 6-amino-2-ethyl-7-(3-hydroxy-2,6-dimethylphenyl)-7H-pyrrolo[2,3-d]pyrimidine-5-carboxamide (17.13 mg). MS (ESI) m/z: 326.4 [M+H⁺]. ¹H NMR (400 MHz, DMSO-*d*₆) δ 9.61 (s, 1H), 8.90 (s, 1H), 7.12 - 7.03 (m, 3H), 6.93 (d, *J =* 8.2 Hz, 1H), 6.86 (s, 2H), 2.69 (q, *J =* 7.3 Hz, 2H), 1.76 (s, 3H), 1.67 (s, 3H), 1.15 (t, *J =* 7.6 Hz, 3H). C₁₇H₁₉N₅O₂.

### Example 9

### 6-Amino-2-(cyclohex-1-en-1-yl)-7-(3-hydroxy-2,6-dimethylphenyl)-7H-pyrrolo[2,3-d]pyrimidine-5-carboxamide

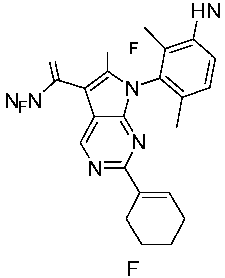

Same as Example 6, 6-amino-2-chloro-7-(3-hydroxy-2,6-dimethylphenyl)-7H-pyrrolo[2,3-d]pyrimidine-5-carboxamide (100 mg, 0.30 mmol) and 2-(cyclohex-1-en-1-yl)-4,4,5,5-tetramethyl-1,3,2-dioxaborolane (115 mg, 0.91 mmol) were reacted to obtain 6-amino-2-(cyclohex-1-en-1-yl)-7-(3-hydroxy-2,6-dimethylphenyl)-7H-pyrrolo[2,3-d]pyrimidine-5-carboxamide (19.8 mg). MS (ESI) m/z: 377.5 [M+H⁺]. ¹H NMR (400 MHz, DMSO-*d*₆) δ 9.59 (s, 1H), 8.92 (s, 1H), 7.16 - 7.05 (m, 3H), 6.93 (m, 1H), 6.87 (m, 2H) 6.82 - 6.75 (m, 1H), 2.39 (m, 2H), 2.18 - 2.05 (m, 2H), 1.77 (s, 3H), 1.69 (s, 3H), 1.66 - 1.51 (m, 4H). C₂₁H₂₃N₅O₂.

### Example 10

### 6-Amino-2-cyclohexyl-7-(3-hydroxy-2,6-dimethylphenyl)-7H-pyrrolo[2,3-d]pyrimidine-5-carboxamide

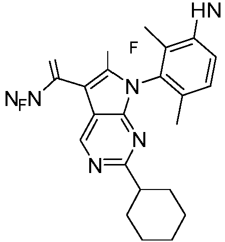

6-Amino-2-(cyclohex-1-en-1-yl)-7-(3-hydroxy-2,6-dimethylphenyl)-7H-pyrrolo[2,3-d]pyrimidine-5-carboxamide (15 mg, 0.04 mmol) was dissolved in methanol (2 mL), then 10% Pd/C (3 mg) was added, and the reaction was carried out under hydrogen atmosphere at normal pressure and room temperature for 1.5 hours. After LCMS monitoring showed that the raw material disappeared, the reaction mixture was filtered to collect the filtrate, and the filtrate was concentrated to obtain 6-amino-2-cyclohexyl-7-(3-hydroxy-2,6-dimethylphenyl)-7H-pyrrolo[2,3-d]pyrimidine-5-carboxamide (13.89 mg). MS (ESI) m/z: 380.3 [M+H⁺]. ¹H NMR (400 MHz, DMSO-*d*₆) δ 9.73 (s, 1H), 8.89 (s, 1H), 7.12 - 7.01 (m, 3H), 6.95 (d, *J= 8.3* Hz, 1H), 6.84 (s, 2H), 2.59 (tt, *J=* 11.6, 3.5 Hz, 1H), 1.85 - 1.57 (m, 10H), 1.47 (qt, *J =* 12.5, 3.0 Hz, 2H), 1.34 - 1.12 (m, 4H). C₂₁H₂₅N₅O₂.

### Example 11

### 6-Amino-7-(3-hydroxy-2,6-dimethylphenyl)-2-phenyl-7H-pyrrolo[2,3-d]pyrimidine-5-carboxamide

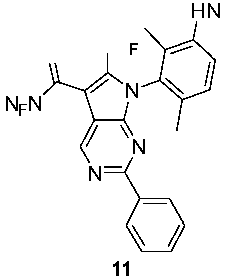

6-Amino-2-chloro-7-(3-methoxy-2,6-dimethylphenyl)-7H-pyrrolo[2,3-d]pyrimidine-5-carbonitrile (50 mg, 0.15 mmol), phenylboronic acid (37.3 mg, 0.31 mmol), potassium carbonate (63.3 mg, 0.46 mmol), and [1,1'-bis(diphenylphosphino)ferrocene]dichloropalladium(II) (7.8 mg, 0.012 mmol) were dissolved in dioxane/water (5/1, 3 mL) and reacted at 120°C for 4.5 hours under nitrogen atmosphere. LCMS monitoring showed that the raw material was completely reacted, and then the reaction mixture was cooled to room temperature. Water (20 mL) and ethyl acetate (20 mL) were added to the reaction system, and the mixture was stirred and extracted. The organic phase was collected and washed with saturated brine (10 mL), dried over anhydrous sodium sulfate, and concentrated under reduced pressure to obtain a crude product. The crude product was purified to obtain 6-amino-7-(3-methoxy-2,6-dimethylphenyl)-2-phenyl-7H-pyrrolo[2,3-d]pyrimidine-5-carbonitrile (50 mg). MS (ESI) m/z: 370.3 [M+H⁺].

6-Amino-7-(3-methoxy-2,6-dimethylphenyl)-2-phenyl-7H-pyrrolo[2,3-d]pyrimidine-5-carbonitrile (50 mg, 0.14 mmol) was dissolved in sulfuric acid/water (13/1, 0.5 mL), and then methanesulfonic acid (483.2 mg, 5.03 mmol) was added to the reaction system and stirred at room temperature for 2 hours. Then, DL-methionine (81.1 mg, 0.54 mmol) was added to the reaction mixture, and the reaction mixture was stirred at 40°C overnight. After TLC monitoring showed that the raw material was completely reacted, sodium hydroxide/dipotassium hydrogen phosphate aqueous solution was added to the reaction system to adjust the pH to about 7, and the mixture was filtered to obtain a crude product. The crude product was purified to obtain 6-amino-7-(3-hydroxy-2,6-dimethylphenyl)-2-phenyl-7H-pyrrolo[2,3-d]pyrimidine-5-carboxamide (5.82 mg). MS (ESI) m/z: 373.3 [M+H⁺]. ¹H NMR (400 MHz, DMSO-*d*₆) δ 9.63 (s, 1H), 9.09 (s, 1H), 8.17 - 8.10 (m, 2H), 7.45 - 7.32 (m, 3H), 7.23 (s, 2H), 7.13 (d, *J =* 8.3 Hz, 1H), 7.01 - 6.93 (m, 3H), 1.82 (s, 3H), 1.73 (s, 3H). C₂₁H₁₉N₅O₂.

### Example 12

### 6-Amino-2-(cyclopropylethynyl)-7-(3-hydroxy-2,6-dimethylphenyl)-7H-pyrrolo[2,3-d]pyrimidine-5-carboxamide

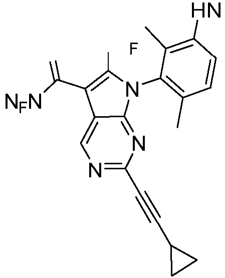

6-Amino-2-chloro-7-(3-hydroxy-2,6-dimethylphenyl)-7H-pyrrolo[2,3-d]pyrimidine-5-carboxamide (100 mg, 0.30 mmol), cyclopropylacetylene (100 mg, 1.52 mmol), bis(acetonitrile)dichloropalladium(II) (10 mg, 0.04 mmol), cesium carbonate (165 mg, 0.51 mmol), and 2-dicyclohexylphosphino-2',6'-dimethoxybiphenyl (28 mg, 0.07 mmol) were dissolved in acetonitrile (4 mL) and stirred at 90°C under nitrogen atmosphere overnight. After TLC monitoring showed that the raw material was completely reacted, the reaction system was cooled to room temperature. Then, water (20 mL) and ethyl acetate (20 mL) were added to the reaction system, and the mixture was stirred and extracted. The organic phase was collected and washed with saturated brine (10 mL), dried over anhydrous sodium sulfate, and concentrated under reduced pressure to obtain a crude product. The crude product was purified to obtain 6-amino-2-(cyclopropylethynyl)-7-(3-hydroxy-2,6-dimethylphenyl)-7*H-*pyrrolo[2,3-d]pyrimidine-5-carboxamide (10.4 mg).

MS (ESI) m/z: 362.3 [M+H⁺]. ¹HNMR (400 MHz, DMSO-*d*₆) δ 9.66 (s, 1H), 8.91 (s, 1H), 7.29 (s, 2H), 7.10 (d, *J=* 8.3 Hz, 1H), 6.99-6.92 (m, 3H), 1.74 (s, 3H), 1.65 (s, 3H), 1.50 (tt, J= 8.2, 5.0 Hz, 1H), 0.90-0.81 (m, 2H), 0.74 (dt, J= 4.9, 3.1 Hz, 2H). C₂₀H₁₉N₅O₂.

### Example 13

### 6-Amino-7-(3-hydroxy-2,6-dimethylphenyl)-2-(piperidin-1-yl)-7H-pyrrolo[2,3-d]pyrimidine-5-carboxamide

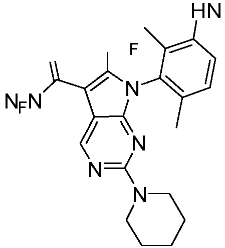

6-Amino-2-chloro-7-(3-methoxy-2,6-dimethylphenyl)-7H-pyrrolo[2,3-d]pyrimidine-5-carbonitrile (100 mg, 0.31 mmol) was dissolved in n-butanol (2 mL), and then piperidine (1 mL) and *N,N-*diisopropylethylamine (118.6 mg, 0.93 mmol) were added to the reaction system. The reaction mixture was stirred at 150°C for 8 hours. TLC monitoring showed that the raw material was completely reacted, and then the reaction mixture was cooled to room temperature. Water (20 mL) and ethyl acetate (20 mL) were added to the reaction system for extraction. After stirring and phase separation, the organic phase was collected and washed with saturated brine (10 mL), dried over anhydrous sodium sulfate, and concentrated under reduced pressure to obtain a crude product. The crude product was purified to obtain 6-amino-7-(3-methoxy-2,6-dimethylphenyl)-2-(piperidin-1-yl)-7H-pyrrolo[2,3-d]pyrimidine-5-carbonitrile (90 mg). MS (ESI) m/z: 377.4 [M+H⁺].

6-Amino-7-(3-methoxy-2,6-dimethylphenyl)-2-(piperidin-1-yl)-7H-pyrrolo[2,3-d]pyrimidine-5-carbonitrile (70 mg, 0.19 mmol) was dissolved in sulfuric acid/water (13/1, 0.6 mL), and methanesulfonic acid (662 mg, 6.9 mmol) was added to the reaction system. After the reaction mixture was stirred at room temperature for 2 hours, DL-methionine (111.1 mg, 0.74 mmol) was added to the reaction mixture and stirred at 40°C overnight. LCMS monitoring showed that the raw material was completely reacted. Sodium hydroxide/dipotassium hydrogen phosphate aqueous solution was added to the reaction system to adjust the pH of the system to about 7. The crude product was purified to obtain 6-amino-7-(3-hydroxy-2,6-dimethylphenyl)-2-(piperidin-1-yl)-7*H*-pyrrolo[2,3-*d*]pyrimidine-5-carboxamide (18.9 mg). MS (ESI) m/z: 380.2 [M+H⁺]. ¹H NMR (400 MHz, DMSO-*d*₆) δ 9.53 (s, 1H), 8.60 (s, 1H), 7.06 (d, *J =* 8.2 Hz, 1H), 6.90 (d, *J =* 8.2 Hz, 1H), 6.73 (s, 2H), 6.66 (s, 2H), 3.49 (t, *J* = 5.4 Hz, 4H), 1.80 (s, 3H), 1.71 (s, 3H), 1.52 (m, 2H), 1.42 (m, 4H). C₂₀H₂₄N₆O₂.

### Example 14

### 6-Amino-2-cyano-7-(3-hydroxy-2,6-dimethylphenyl)-7H-pyrrolo[2,3-d]pyrimidine-5-carboxamide

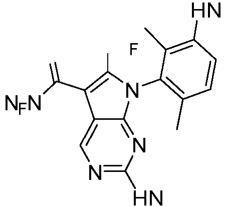

6-Amino-2-chloro-7-(3-hydroxy-2,6-dimethylphenyl)-7H-pyrrolo[2,3-d]pyrimidine-5-carboxamide (90 mg, 0.27 mmol), zinc cyanide (25 mg, 0.21 mmol), 1,1'-bis(diphenylphosphino)ferrocene (12 mg, 0.02 mmol), zinc powder (6 mg, 0.09 mmol), and tris(dibenzylideneacetone)dipalladium (6 mg, 0.006 mmol) were dissolved in dimethylacetamide (3.5 mL) and stirred at 150°C under nitrogen atmosphere for 3 hours. TLC monitoring showed that the raw material was completely reacted, and then the reaction mixture was cooled to room temperature. Water (20 mL) and ethyl acetate (20 mL) were added to the reaction system for extraction. After stirring and phase separation, the organic phase was collected and washed with saturated brine (10 mL), dried over anhydrous sodium sulfate, and concentrated under reduced pressure to obtain a crude product. The crude product was purified to obtain 6-amino-2-cyano-7-(3-hydroxy-2,6-dimethylphenyl)-7H-pyrrolo[2,3-d]pyrimidine-5-carboxamide (25.55 mg). MS (ESI) m/z: 322.4 [M+H⁺]. ¹H NMR (400 MHz, DMSO-*d*₆) δ 9.72 (s, 1H), 9.10 (s, 1H), 7.69 (d, *J =* 6.0 Hz, 2H), 7.17 (s, 2H), 7.12 (d, *J* = 8 Hz, 1H), 7.03-6.95 (d, J= 8 Hz, 1H), 1.75 (s, 3H), 1.67 (s, 3H). C₁₆H₁₄N₆O₂.

### Example 15

### 6-Amino-7-(3-hydroxy-2,6-dimethylphenyl)-2-((4-methoxybenzyl)amino)-7H-pyrrolo[2,3-d]pyrimidine-5-carboxamide

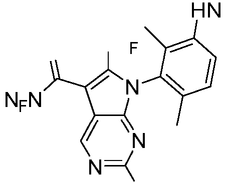

6-Amino-2-chloro-7-(3-hydroxy-2,6-dimethylphenyl)-7H-pyrrolo[2,3-d]pyrimidine-5-carboxamide (100 mg, 0.3 mmol) was dissolved in n-butanol (2 mL), and then 4-methoxybenzylamine (1 mL) and N,N-diisopropylethylamine (117.1 mg, 0.91 mmol) were added to the reaction mixture and stirred at 150°C for 8 hours. TLC monitoring showed that the raw material was completely reacted, and then the reaction mixture was cooled to room temperature. Water (20 mL) and ethyl acetate (20 mL) were added for extraction. The organic phase was collected and washed with saturated brine (10 mL), dried over anhydrous sodium sulfate, and concentrated under reduced pressure to obtain a crude product. The crude product was purified to obtain 6-amino-7-(3-hydroxy-2,6-dimethylphenyl)-2-((4-methoxybenzyl)amino)-7H-pyrrolo[2,3-d]pyrimidine-5-carboxamide (64.44 mg). MS (ESI) m/z: 432.19 [M+H⁺]. ¹H NMR (400 MHz, DMSO-*d*₆) δ 9.54 (s, 1H), 8.51 (s, 1H), 7.21 - 7.02 (m, 4H), 6.96 - 6.83 (m, 1H), 6.80 - 6.70 (m, 2H), 6.69 - 6.58 (m, 4H), 4.22 *(d, J=* 5.8 Hz, 2H), 3.71 (s, 3H), 1.78 (s, 3H), 1.69 (s, 3H). C₂₃H₂₄N₆O₃.

### Example 16

### 6-Amino-2-(3,6-dihydro-2H-pyran-4-yl)-7-(3-hydroxy-2,6-dimethylphenyl)-7H-pyrrolo[2,3-d]pyrimidine-5-carboxamide

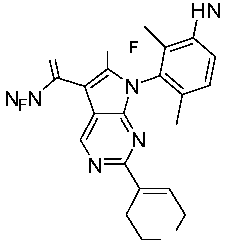

Same as Example 6, 6-amino-2-chloro-7-(3-hydroxy-2,6-dimethylphenyl)-7H-pyrrolo[2,3-d]pyrimidine-5-carboxamide (120 mg, 0.36 mmol) and 2-(3,6-dihydro-2H-pyran-4-yl)-4,4,5,5-tetramethyl-1,3,2-dioxaborolane (240 mg, 1.14 mmol) were reacted to obtain 6-amino-2-(3,6-dihydro-2H-pyran-4-yl)-7-(3-hydroxy-2,6-dimethylphenyl)-7H-pyrrolo[2,3-d]pyrimidine-5-carboxamide (25.21 mg). MS (ESI) m/z: 379.2 [M+H⁺]. ¹H NMR (400 MHz, DMSO-*d*₆) δ 9.59 (s, 1H), 8.96 (s, 1H), 7.16 (d, *J=* 9.4 Hz, 2H), 7.09 (d, *J =* 8.3 Hz, 1H), 6.97 -6.88 (m, 3H), 6.78 - 6.71 (m, 1H), 4.20 (q, *J =* 2.8 Hz, 2H), 3.74 (t, *J =* 5.4 Hz, 2H), 1.78 (s, 3H), 1.69 (s, 3H). C₂₀H₂₁N₅O₃.

### Example 17

### 6-Amino-2-(diethylamino)-7-(3-hydroxy-2,6-dimethylphenyl)-7H-pyrrolo[2,3-d]pyrimidine-5-carboxamide

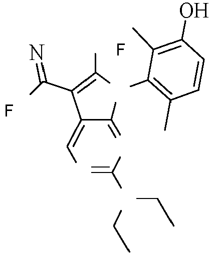

Same as Example 15, 6-amino-2-chloro-7-(3-hydroxy-2,6-dimethylphenyl)-7H-pyrrolo[2,3-d]pyrimidine-5-carboxamide (100 mg, 0.3 mmol) and diethylamine (1 mL) were reacted to obtain 6-amino-2-(diethylamino)-7-(3-hydroxy-2,6-dimethylphenyl)-7H-pyrrolo[2,3-d]pyrimidine-5-carboxamide (9.29 mg). MS (ESI) m/z: 369.0 [M+H⁺]. ¹H NMR (400 MHz, DMSO-*d*₆) δ 9.50 (s, 1H), 8.58 (s, 1H), 7.05 (d, *J* = 8.3 Hz, 1H), 6.89 (d, *J=* 8.2 Hz, 1H), 6.68 (s, 2H), 6.62 (s, 2H), 3.45 -3.35 (m, 4H), 1.80 (s, 3H), 1.72 (s, 3H), 0.99 (t, *J* = 6.9 Hz, 6H). C₁₉H₂₄N₆O₂.

### Example 18

### 6-Amino-7-(3-hydroxy-2,6-dimethylphenyl)-2-(tetrahydro-2H-pyran-4-yl)-7H-pyrrolo[2,3-d]pyrimidine-5-carboxamide

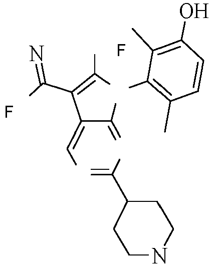

6-Amino-2-(3,6-dihydro-2H-pyran-4-yl)-7-(3-hydroxy-2,6-dimethylphenyl)-7H-pyrrolo[2,3-d]pyrimidine-5-carboxamide (19 mg, 0.05 mmol) was dissolved in methanol (2 mL), then 10% Pd/C (10 mg) was added, and the reaction was carried out under hydrogen atmosphere at normal pressure and room temperature for 1.5 hours. After LCMS monitoring showed that the raw material disappeared, the reaction mixture was filtered to collect the filtrate, and the filtrate was concentrated to obtain 6-amino-7-(3-hydroxy-2,6-dimethylphenyl)-2-(tetrahydro-2H-pyran-4-yl)-7H-pyrrolo[2,3-d]pyrimidine-5-carboxamide (7.03 mg). MS (ESI) m/z: 382.3 [M+H⁺]. ¹H NMR (400 MHz, DMSO-*d*₆) δ 9.80 (s, 1H), 8.98 (s, 1H), 7.20 - 7.10 (m, 3H), 7.11 (d, *J* = 8.4 Hz, 1H), 6.92 (s, 2H), 3.92 (dt, *J* = 11.1, 3.3 Hz, 2H), 2.93 (ddd, J= 15.4, 9.0, 7.0 Hz, 1H), 1.84 - 1.69 (m, 10H). C₂₀H₂₃N₅O₃.

### Example 19

### 6-Amino-7-(3-hydroxy-2,6-dimethylphenyl)-2,4-dimethyl-7H-pyrrolo[2,3-d]pyrimidine-5-carboxamide

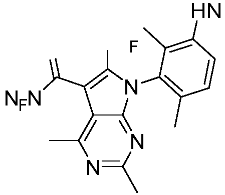

2,6-Dimethylpyrimidin-4-ol (2.0 g, 16.11 mmol) was dissolved in chloroform (20 mL), and liquid bromine (3.2 mL, 64.5 mmol) was added dropwise to the reaction mixture under an ice bath. After the dropwise addition was completed, the reaction system was heated to 80°C and stirred overnight. After TLC monitoring showed that the raw material disappeared, the solvent was removed under reduced pressure, ethyl acetate (50 mL) was added thereto, and the mixture was concentrated under reduced pressure again, and the process was repeated for three times. The crude product was adjusted to pH = 7-8 with saturated NaHCO₃. Ethyl acetate (80 mL × 3), dichloromethane/isopropanol system (7/3, 50 mL × 3) were used to extract, and the organic phases were combined, washed with saturated brine (50 mL × 3), dried over anhydrous sodium sulfate, and concentrated under reduced pressure to obtain crude 5-bromo-2,6-dimethylpyrimidin-4-ol (1.316 g). MS (ESI) m/z: 205.1 [M+H⁺]. ¹H NMR (400 MHz, DMSO) δ 12.76 (s, 1H), 2.33 (s, 3H), 2.24 (s, 3H).

In a single-necked flask, a solution of 5-bromo-2,6-dimethylpyrimidin-4-ol (1.316 g, 6.48 mmol) in phosphorus oxychloride (20 mL) was added, and the reaction system was stirred at 110°C overnight. After TLC monitoring showed that the raw material disappeared, the solvent was removed under reduced pressure. Ethyl acetate (50 mL) was then added to dissolve, and the pH was adjusted to 8-9 with saturated NaHCO₃ under an ice bath. The mixture was extracted with ethyl acetate (80 mL × 3), and the organic phases were separated and combined, washed with saturated brine (50 mL × 3), dried over anhydrous sodium sulfate, and concentrated under reduced pressure to obtain a crude product, which was purified to obtain 5-bromo-4-chloro-2,6-dimethylpyrimidine (1.096 g). MS (ESI) m/z: 223.0 [M+H⁺]. ¹H NMR (400 MHz, DMSO) δ 2.60 (s, 3H), 2.54 (s, 3H).

In a 20 mL sealed tube, a solution of 5-bromo-4-chloro-2,6-dimethylpyrimidine (1.096 g, 4.9 mmol) in N-methylpyrrolidone (7 mL) was added, and 3-methoxy-2,6-dimethylaniline (0.9 g, 5.9 mmol) and 2,6-dimethylpyridine (1.06 g, 9.9 mmol) were slowly added thereto under nitrogen atmosphere, and the reaction was carried out under microwave radiation at 130°C for 54 hours. LCMS detected the formation of products. The reaction mixture was extracted with ethyl acetate (50 mL × 3), and then the organic phases were combined, washed with saturated brine (20 mL × 3), dried over anhydrous sodium sulfate, and concentrated under reduced pressure to obtain a crude product. The crude product was purified to obtain 5-bromo-N-(3-methoxy-2,6-dimethylphenyl)-2,6-dimethylpyrimidin-4-amine (883 mg). MS (ESI) m/z: 336.2 [M+H⁺].

Sodium tert-butoxide (71 mg, 0.744 mmol), ethylene glycol dimethyl ether (1 mL), and malononitrile (49 mg, 0.744 mmol) were added to a 10 mL sealed tube under nitrogen flow. The container was sealed, and the reaction mixture was stirred at room temperature for 30 minutes. Then, under nitrogen flow, 5-bromo-N-(3-methoxy-2,6-dimethylphenyl)-2,6-dimethylpyrimidin-4-amine (50 mg, 0.148 mmol) and PdCl₂(dppf)₂ (10 mg, 0.015 mmol) were added to the sealed tube. The reaction tube was sealed and heated to 100°C in an oil bath and stirred overnight. TLC detection showed that new spots were generated, and the reaction mixture was poured into ice water. The reaction mixture was extracted with ethyl acetate (10 mL × 3). The organic phases were combined, then washed with saturated brine (5 mL × 3), dried over anhydrous sodium sulfate, and concentrated under reduced pressure to obtain a crude product. The crude product was purified to obtain 6-amino-7-(3-methoxy-2,6-dimethylphenyl)-2,4-dimethyl-7H-pyrrolo[2,3-d]pyrimidine-5-carbonitrile (30 mg). MS (ESI) m/z: 322.2 [M+H⁺].

6-Amino-7-(3-methoxy-2,6-dimethylphenyl)-2,4-dimethyl-7H-pyrrolo[2,3-d]pyrimidine-5-carbonitrile (30 mg, 0.09 mmol) was dissolved in concentrated sulfuric acid (1 mL) at room temperature. The reaction mixture was stirred at room temperature for 5 hours. After LCMS monitoring showed that the raw material disappeared, crushed ice was added to the reaction mixture to quench, and then the pH value of the reaction mixture was adjusted to 9 with concentrated ammonia water. The mixture was extracted with ethyl acetate (5 mL × 3). The organic phases were combined, then washed with saturated brine (5 mL), dried over anhydrous sodium sulfate, and concentrated under reduced pressure to obtain a crude product. The crude product was purified to obtain 6-amino-7-(3-methoxy-2,6-dimethylphenyl)-2,4-dimethyl-7H-pyrrolo[2,3-d]pyrimidine-5-carboxamide (10 mg). MS (ESI) m/z: 340.4 [M+H⁺].

In a 50 mL three-necked flask, 6-amino-7-(3-methoxy-2,6-dimethylphenyl)-2,4-dimethyl-7H-pyrrolo[2,3-d]pyrimidine-5-carboxamide (10 mg, 0.029 mmol) and anhydrous dichloromethane (0.5 mL) were added. A solution of BBr₃ in dichloromethane (1 mol of dichloromethane solution, 1 mL) was added dropwise to the reaction system under an ice bath, and the reaction mixture was stirred at room temperature for 1 hour. After TLC monitoring showed that the raw material disappeared, the reaction mixture was quenched with ice water, and the pH was adjusted to 9 with concentrated ammonia water, and then the mixture was concentrated under reduced pressure to obtain a crude product. The crude product was purified to obtain 6-amino-7-(3-hydroxy-2,6-dimethylphenyl)-2,4-dimethyl-7H-pyrrolo[2,3-d]pyrimidine-5-carboxamide (4 mg). MS (ESI) m/z: 326.2 [M+H⁺]. ¹H NMR (400 MHz, DMSO) δ 9.55 (s, 1H), 7.06 (d, *J =* 8.3 Hz, 1H), 6.92 (d, *J =* 8.3 Hz, 1H), 6.85 (s, 2H), 6.47 (s, 2H), 2.70 (s, 3H), 2.38 (s, 3H), 1.75 (s, 3H), 1.66 (s, 3H).

### Example 20

### 6-Amino-7-(3-hydroxy-2,6-dimethylphenyl)-2,4-dimethyl-7H-pyrrolo[2,3-d]pyrimidine-5-carboxamide

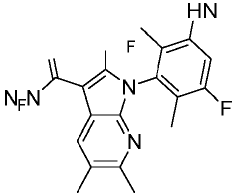

3-Fluoro-5-methoxyaniline (8.0 g, 56.68 mmol) was dissolved in *N,N-*dimethylformamide (160 mL), and N-chlorosuccinimide (7.57 g, 56.68 mmol) was added to the reaction mixture at room temperature. The reaction system was stirred at room temperature overnight. After TLC monitoring showed that the raw material disappeared, the reaction mixture was poured into 600 mL of water. The mixture was extracted with ethyl acetate (300 mL × 3). The organic phase was washed with saturated brine (100 mL × 3), dried over anhydrous sodium sulfate, filtered, and concentrated under reduced pressure. The resulting mixture was purified by silica gel column chromatography to obtain 4-chloro-3-fluoro-5-methoxyaniline (6.025 g, yield of 60.74%). ¹H NMR (400 MHz, DMSO) δ 6.19 - 6.13 (m, 1H), 6.09 (dd, J = 11.6, 2.3 Hz, 1H), 5.58 (s, 2H), 3.76 (s, 3H).

In a single-necked flask, a solution of 4-chloro-3-fluoro-5-methoxyaniline (6.0 g, 34.3 mmol) in acetonitrile (170 mL) was added, and N-bromosuccinimide (24.4 g, 137.1 mmol) was added to the reaction mixture at room temperature, and then the reaction system was stirred at room temperature overnight. After TLC monitoring showed that the raw material disappeared, the solvent was removed under reduced pressure. The resulting mixture was purified by silica gel column chromatography to obtain 2,6-dibromo-4-chloro-3-fluoro-5-methoxyaniline (9.783 g, yield of 85.56%). ¹H NMR (400 MHz, DMSO) δ 5.94 (s, 2H), 3.32 (s, 3H).

2,6-Dibromo-4-chloro-3-fluoro-5-methoxyaniline (4.5 g, 13.5 mmol) was dissolved in 1,4-dioxane/water (60 mL/6 mL), then potassium carbonate (6.53 g, 47.24 mmol), methylboric acid (4.04 g, 67.5 mmol), and PdCl₂(dppf) (493 mg, 0.67 mmol) were slowly added under nitrogen atmosphere, and the reaction system was stirred at 100°C overnight. After TLC monitoring showed that the raw material disappeared, the solvent was removed under reduced pressure and 50 mL of pure water was added to the system. The mixture was extracted with ethyl acetate (150 mL × 3). The organic phases were combined, washed with saturated brine (50 mL × 3), dried over anhydrous sodium sulfate, filtered, and concentrated under reduced pressure. The resulting mixture was purified by silica gel column chromatography to obtain 4-chloro-3-fluoro-5-methoxy-2,6-dimethylaniline (1.919 g, yellow liquid, yield of 69.80%).

In a single-necked flask, a solution of 4-chloro-3-fluoro-5-methoxy-2,6-dimethylaniline (2.0 g, 9.82 mmol) in methanol (80 mL) was added, and ammonium formate (12.4 g, 196.43 mmol) and Pd/C (2.0 g, 18.8 mmol) were slowly added under nitrogen atmosphere, and the reaction system was stirred at 65°C overnight. After TLC monitoring showed that the raw material disappeared, the reaction mixture was filtered and concentrated under reduced pressure. The resulting residue was purified by silica gel column chromatography to obtain 3-fluoro-5-methoxy-2,6-dimethylaniline (1.417 g, yield of 85.38%). ¹H NMR (400 MHz, DMSO) δ 6.07 (d, J = 12.1 Hz, 1H), 4.84 (s, 2H), 3.67 (s, 3H), 1.95-1.82 (m, 6H). MS (ESI) M/Z: 170.0 [M+H⁺].

At room temperature, 3-fluoro-5-methoxy-2,6-dimethylaniline (707 mg, 4.18 mmol) was dissolved in ethylene glycol dimethyl ether (25 mL), and 2,3-dibromo-5,6-dimethylpyridine (1.0 g, 3.8 mmol), cesium carbonate (3.095 g, 9.5 mmol), Pd₂dba₃ (348 mg, 0.38 mmol), and XantPhos (440 mg, 0.76 mmol) were added at room temperature. The reaction system was stirred at 100°C overnight. After LCMS monitoring showed that the raw material disappeared, pure water was added to the reaction mixture to quench, and the mixture was extracted with ethyl acetate (150 mL × 3). The organic phases were combined, then washed with saturated brine (50 mL × 3), dried over anhydrous sodium sulfate, filtered, and finally concentrated under reduced pressure. The resulting residue was purified by silica gel plate chromatography to obtain 3-bromo-N-(3-fluoro-5-methoxy-2,6-dimethylphenyl)-5,6-dimethylpyridin-2-amine (1.166 g, yield of 91.9%). ¹H NMR (400 MHz, DMSO) δ 7.60 (s, 1H), 7.41 (s, 1H), 6.77 (d, J = 11.8 Hz, 1H), 3.78 (s, 3H), 2.07 (s, 3H), 2.05 (s, 3H), 1.92 (d, J = 2.0 Hz, 3H), 1.90 (s, 3H). MS (ESI) M/Z: 355.0 [M+H⁺].

In a 20 mL sealed tube, sodium tert-butoxide (1.578 g, 16.42 mmol) was added, and ethylene glycol dimethyl ether (3 mL) was added under nitrogen flow, and then malononitrile (1.084 g, 16.42 mmol) was added dropwise at room temperature. After the dropwise addition was completed, the container was sealed, and the reaction mixture was stirred at room temperature for 30 minutes. Under nitrogen flow, 3-bromo-N-(3-fluoro-5-methoxy-2,6-dimethylphenyl)-5,6-dimethylpyridin-2-amine (1.16 g, 3.28 mmol) and PdCl₂(dppf) (240 mg) were added to the sealed tube. The reaction tube was sealed, heated to 100°C in an oil bath, and stirred overnight. TLC detection showed that new spots were generated, and the reaction mixture was poured into pure water. The reaction mixture was extracted with ethyl acetate (150 mL × 3). The organic phases were combined, washed with saturated brine (50 mL × 3), dried over anhydrous sodium sulfate, filtered, and concentrated under reduced pressure. The resulting mixture was purified by silica gel column chromatography to obtain 2-amino-1-(3-fluoro-5-methoxy-2,6-dimethylphenyl)-5,6-dimethyl-1H-pyrrolo[2,3-b]pyridine-3-carbonitrile (950 mg, yield of 85.6%). ¹H NMR (400 MHz, DMSO) δ 7.40 (s, 1H), 7.10 (d, J = 11.7 Hz, 1H), 6.92 (s, 2H), 3.86 (s, 3H), 2.25 (s, 3H), 2.25 (s, 3H), 1.69 (d, J = 1.7 Hz, 3H), 1.64 (s, 3H). MS (ESI) M/Z: 339.1 [M+H⁺].

At 0°C, 2-amino-1-(3-fluoro-5-methoxy-2,6-dimethylphenyl)-5,6-dimethyl-1H-pyrrolo[2,3-b]pyridine-3-carbonitrile (950 mg, 2.8 mmol) was dissolved in concentrated sulfuric acid (8 mL). The reaction mixture was stirred at room temperature for 3 hours. After LCMS monitoring showed that the raw material disappeared, crushed ice was added to the reaction mixture to quench, and the pH value was adjusted to 7-8 with saturated sodium bicarbonate aqueous solution. The mixture was extracted with ethyl acetate (100 mL × 3). The organic phases were combined, first washed with saturated brine (50 mL × 3), dried over anhydrous sodium sulfate, filtered, and finally concentrated under reduced pressure. The resulting residue was purified by silica gel chromatography to obtain 2-amino-1-(3-fluoro-5-methoxy-2,6-dimethylphenyl)-5,6-dimethyl-1H-pyrrolo[2,3-b]pyridine-3-carboxamide (732 mg, yield of 73.65%). ¹H NMR (400 MHz, DMSO) δ 7.85 (s, 1H), 7.09 (d, J = 11.8 Hz, 1H), 6.87 (s, 2H), 6.67 (s, 2H), 3.86 (s, 3H), 2.26 (s, 3H), 2.24 (s, 3H), 1.69 (d, J = 1.5 Hz, 3H), 1.65 (s, 3H). MS (ESI) M/Z: 357.2 [M+H]⁺.

In a 50 mL three-necked flask, 2-amino-1-(3-fluoro-5-methoxy-2,6-dimethylphenyl)-5,6-dimethyl-1H-pyrrolo[2,3-b]pyridine-3-carboxamide (732 mg, 2.05 mmol) and anhydrous dichloromethane (20 mL) were added. A solution of BBr₃ in dichloromethane (2N, 10.3 mL, 20.5 mmol) was added dropwise in an ice bath, and the reaction mixture was stirred at room temperature for 2 hours. TLC monitoring showed that the starting material disappeared. The reaction mixture was quenched with methanol, adjusted to pH = 8 with concentrated ammonia water, and concentrated under reduced pressure to obtain a crude product. The crude product was purified by preparative high-performance liquid chromatography. The final product 2-amino-1-(3-fluoro-5-hydroxy-2,6-dimethylphenyl)-5,6-dimethyl-1H-pyrrolo[2,3-b]pyridine-3-carboxamide (4 mg, yield of 71.3%) was obtained. ¹H NMR (400 MHz, DMSO) δ 9.95 (s, 1H), 7.84 (s, 1H), 6.85 (s, 2H), 6.79 (d, J = 11.1 Hz, 1H), 6.66 (s, 2H), 2.26 (s, 3H), 2.25 (s, 3H), 1.65 (d, J *=* 1.6 Hz, 3H), 1.62 (s, 3H). MS (ESI) M/Z: 343.3 [M+H]⁺.

### Example 21

### S-6-Amino-7-(3-hydroxy-2,6-dimethylphenyl)-2,4-dimethyl-7H-pyrrolo[2,3-d]pyrimidine-5-carboxamide

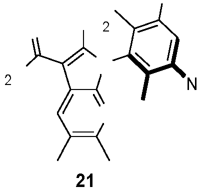

Compound 21 was prepared by chiral HPLC from 100 mg of the compound of Example 20. SFC separation method: instrument: SFC-150mgm (waters), chiral column: Daicel-OZ (25 * 250 mm, 10 µm), temperature: 30°C, mobile phase: CO₂/IPA [0.5% NH₃ (7N in MeOH)] = 60/40, flow rate: 100 mL/min, back pressure: 100 bar, detection wavelength: 214 nm, cycle time: 8.68 min, RT = 1.643 min. [α]²⁰_{D} = +66.47° (c = 0.10065, MeOH), 100% ee. ¹H NMR (400 MHz, DMSO) δ 9.97 (s, 1H), 7.84 (s, 1H), 6.84 (s, 2H), 6.79 (d, J = 11.1 Hz, 1H), 6.66 (s, 2H), 2.26 (s, 3H), 2.25 (s, 3H), 1.65 (d, J = 1.6 Hz, 3H), 1.62 (s, 3H). MS (ESI) M/Z: 343.3 [M+H]⁺.

### Example 22

### R-6-Amino-7-(3-hydroxy-2,6-dimethylphenyl)-2,4-dimethyl-7H-pyrrolo[2,3-d]pyrimidine-5-carboxamide

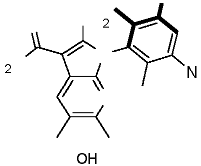

Compound 22 was prepared by chiral HPLC from 100 mg of the compound of Example 20. The SFC method was the same as that of Example 21, RT = 2.472 min. [α]²⁰_{D} = -6.42° (c = 0.10275, MeOH), 96% ee. ¹H NMR (400 MHz, DMSO) δ 9.95 (s, 1H), 7.84 (s, 1H), 6.85 (s, 2H), 6.79 (d, J = 11.1 Hz, 1H), 6.66 (s, 2H), 2.26 (s, 3H), 2.25 (s, 3H), 1.65 (d, J = 1.6 Hz, 3H), 1.62 (s, 3H). MS (ESI) M/Z: 343.3 [M+H]⁺.

### Example 23

### 2-Amino-1-(4-fluoro-3-hydroxy-2,6-dimethylphenyl)-5,6-dimethyl-1H-pyrrolo[2,3-b]pyridine-3-carboxamide

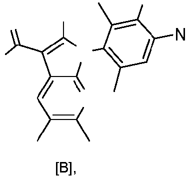

4-Fluoro-3-methoxyaniline (1 g, 7.09 mmol) was dissolved in dichloromethane (5 mL) and methanol (5 mL), and the reaction mixture was added dropwise to a mixture of liquid bromine (0.96 mL), dichloromethane (5 mL), and methanol (5 mL), and stirred at room temperature for 4 hours. After LCMS monitoring showed that the raw material disappeared, sodium thiosulfate aqueous solution (20 mL) and sodium carbonate aqueous solution (20 mL) were added and stirred for 10 minutes, and then the mixture was extracted with ethyl acetate (40 mL × 2). The organic phases were combined, washed with saturated brine (50 mL), and dried over anhydrous sodium sulfate. Finally, the resulting residue obtained by concentrating under reduced pressure was purified by silica gel column chromatography to obtain 2,6-dibromo-4-fluoro-3-methoxyaniline (1.9 g). ¹H NMR (400 MHz, CDCl₃) δ 7.24 (d, *J =* 10.4 Hz, 1H), 4.31 (br, 2H), 3.94 (d, J = 1.6 Hz, 3H). C₇H₆Br₂FNO. MS (ESI) M/Z: 299.9 [M+H⁺].

2,6-Dibromo-4-fluoro-3-methoxyaniline (500 mg, 1.67 mmol) was dissolved in dioxane/water (10/1, 5.5 mL), then methylboric acid (301 mg, 5.02 mmol), potassium carbonate (692.3 mg, 5.02 mmol), and [1,1'-bis(diphenylphosphino)ferrocene]dichloropalladium(II) (61.3 mg, 0.08 mmol) were added, and the reaction system was replaced with nitrogen for three times. The reaction mixture was stirred at 100°C overnight. After LCMS monitoring showed that the raw material disappeared, the reaction mixture was added with water (30 mL) and extracted with ethyl acetate (20 mL × 2). The organic phases were combined and washed with saturated brine (20 mL), and then dried over anhydrous sodium sulfate. Finally, the resulting residue obtained by concentrating under reduced pressure was purified by silica gel column chromatography to obtain 4-fluoro-3-methoxy-2,6-dimethylaniline (600 mg). ¹H NMR (400 MHz, CDCl₃) δ 6.70 (d, *J* = 12 Hz, 1H), 3.83 (s, 3H), 3.47 (br, 2H), 2.12 (d, *J* = 4.4 Hz, 6H). C₉H₁₂FNO. MS (ESI) M/Z: 169.7 [M+H⁺].

2,3-Dibromo-5,6-dimethylpyridine (200 mg, 0.76 mmol), 4-fluoro-3-methoxy-2,6-dimethylaniline (135.5 mg, 0.8 mmol), 4,5-bis(diphenylphosphino)-9,9-dimethylxanthene (39.7 mg, 0.069 mmol), cesium carbonate (622.1 mg, 1.91 mmol), and tris(dibenzylideneacetone)dipalladium (35 mg, 0.038 mmol) were added to ethylene glycol dimethyl ether (5 mL), and the reaction system was replaced with nitrogen for three times, and reacted at 90°C overnight. After LCMS monitoring showed that the raw material disappeared, the reaction mixture was added with water (30 mL) and extracted with ethyl acetate (20 mL × 2). The organic phases were combined, washed with saturated brine (10 mL), and dried over anhydrous sodium sulfate. Finally, the resulting residue obtained by concentrating under reduced pressure was purified by silica gel column chromatography to obtain 3-bromo-N-(4-fluoro-3-methoxy-2,6-dimethylphenyl)-5,6-dimethylpyridin-2-amine (165 mg). MS (ESI) m/z: 353.2 [M+H⁺].

Malononitrile (92.8 mg, 1.41 mmol) was dissolved in dioxane (2 mL), then sodium tert-butoxide (225.2 mg, 2.34 mmol) was added, and the reaction system was replaced with nitrogen, and the reaction was carried out at room temperature for 30 minutes. 3-Bromo-N-(4-fluoro-3-methoxy-2,6-dimethylphenyl)-5,6-dimethylpyridin-2-amine (165 mg, 0.47 mmol) and [1,1'-bis(diphenylphosphino)ferrocene]dichloropalladium(II) (34 mg, 0.047 mmol) were added, and the reaction system was replaced with nitrogen, and the reaction was carried out at 120°C for 2.5 hours. After LCMS monitoring showed that the raw material disappeared, the reaction mixture was added with water (10 mL) and extracted with ethyl acetate (20 mL × 2). The organic phases were combined, washed with saturated brine (10 mL), and dried over anhydrous sodium sulfate. Finally, the resulting residue obtained by concentrating under reduced pressure was purified by silica gel column chromatography to obtain 2-amino-1-(4-fluoro-3-methoxy-2,6-dimethylphenyl)-5,6-dimethyl-1H-pyrrolo[2,3-b]pyridine-3-carbonitrile (100 mg). MS (ESI) m/z: 339.2 [M+H⁺].

2-Amino-1-(4-fluoro-3-methoxy-2,6-dimethylphenyl)-5,6-dimethyl-1H-pyrrolo[2,3-b]pyridine-3-carbonitrile (100 mg, 0.3 mmol) was dissolved in sulfuric acid/water (0.5 mL), and methanesulfonic acid (1.05 g, 10.95 mmol) was added. The reaction was carried out at room temperature for 1.5 hours, and methionine (176.6 mg, 1.18 mmol) was added. The reaction was carried out at 40°C overnight. After LCMS monitoring showed that the raw material disappeared, the reaction mixture was cooled, and the pH value was adjusted to neutral with sodium hydroxide and dipotassium hydrogen phosphate aqueous solution, and the mixture was extracted with ethyl acetate (20 mL × 2). The organic phase was dried and concentrated to dryness to obtain a crude product. The crude product was purified to obtain 2-amino-1-(4-fluoro-3-hydroxy-2,6-dimethylphenyl)-5,6-dimethyl-1H-pyrrolo[2,3-b]pyridine-3-carboxamide (5.02 mg). MS (ESI) m/z: 343.5 [M+H⁺]. ¹H NMR (400 MHz, DMSO-*d*₆) δ 9.56 (s, 1H), 7.83 (s, 1H), 7.10 (d, *J =* 11.5 Hz, 1H), 6.83 (s, 2H), 6.66 (s, 2H), 2.25 (d, J= 7.7 Hz, 6H), 1.76 (s, 3H), 1.70 (s, 3H). C₁₈H₁₉FN₄O₂.

### Example 24

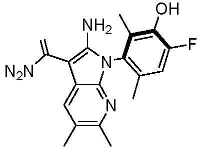

Compound 24 was prepared by chiral HPLC from the compound of Example 23. SFC separation method: instrument: SFC-150mgm (waters), chiral column: YMC Cellulose-SC (20 * 250 mm, 5 µm), temperature: 30°C, mobile phase: CO₂/MeOH [0.2% NH₃ (7M in MeOH)] = 65/35, flow rate: 50 mL/min, back pressure: 100 bar, detection wavelength: 214 nm, cycle time: 8.68 min. [α]²⁰_{D} = -35.95° (c = 0.10015, MeOH); >99% ee. MS (ESI) m/z: 343.3 [M+H⁺]. ¹H NMR (400 MHz, DMSO) δ 9.53 (s, 1H), 7.82 (s, 1H), 7.09 (d, *J =* 11.5 Hz, 1H), 6.81 (s, 2H), 6.64 (s, 2H), 2.26 (s, 3H), 2.24 (s, 3H), 1.75 (s, 3H), 1.70 (s, 3H).

### Example 25

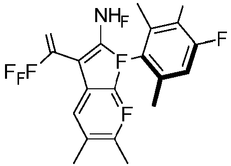

Compound 25 was prepared by chiral HPLC from the compound of Example 23. SFC separation method: instrument: SFC-150mgm (waters), chiral column: YMC Cellulose-SC (20 * 250 mm, 5 µm), temperature: 30°C, mobile phase: CO₂/MeOH [0.2% NH₃ (7M in MeOH)] = 65/35, flow rate: 50 mL/min, back pressure: 100 bar, detection wavelength: 214 nm, cycle time: 8.68 min. [α]²⁰_{D} = +46.32° (c = 0.10125, MeOH ); >99% ee. MS (ESI) m/z: 343.3 [M+H⁺]. ¹H NMR (400 MHz, DMSO) δ 9.54 (s, 1H), 7.82 (s, 1H), 7.09 (d, *J =* 11.5 Hz, 1H), 6.81 (s, 2H), 6.64 (s, 2H), 2.26 (s, 3H), 2.24 (s, 3H), 1.75 (s, 3H), 1.70 (s, 3H).

### Example 26

### 6-Amino-7-(3-hydroxy-2,6-dimethylphenyl)-2-(3-hydroxy-3-methylbut-1-yn-1-yl)-7H-pyrrolo[2,3-d]pyrimidine-5-carboxamide

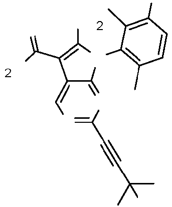

The synthesis method was the same as that of Example 12, using 6-amino-2-bromo-7-(3-hydroxy-2,6-dimethylphenyl)-7*H*-pyrrolo[2,3-*d*]pyrimidine-5-carboxamide and 2-methylbutyn-3-2-ol as raw materials to prepare 6-amino-7-(3-hydroxy-2,6-dimethylphenyl)-2-(3-hydroxy-3-methylbut-1-yn-1-yl)-7*H*-pyrrolo[2,3-*d*]pyrimidine-5-carboxamide (5.01 mg). MS (ESI) m/z: 380.1 [M+H⁺]. ¹H NMR (400 MHz, DMSO-*d*₆) δ 9.67 (s, 1H), 8.95 (s, 1H), 7.32 (s, 2H), 7.10 (d, *J* = 8.3 Hz, 1H), 7.01 - 6.92 (m, 3H), 5.54 (s, 1H), 1.74 (s, 3H), 1.66 (s, 3H), 1.42 (s, 6H). C₂₀H₂₁N₅O₃.

### Example 27

### 6-Amino-2-(3-fluoro-3-methylprop-1-yn-1-yl)-7-(3-hydroxy-2,6-dimethylphenyl)-7H-pyrrolo[2,3-d]pyrimidine-5-carboxamide

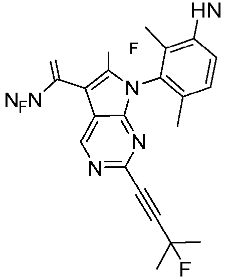

MS (ESI) m/z: 382.37 [M+H⁺].

### Example 28

### 6-Amino-4-chloro-7-(3-hydroxy-2,6-dimethylphenyl)-2-methyl-7H-pyrrolo[2,3-d]pyrimidine-5-carboxamide

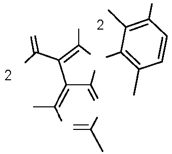

The synthesis route of Scheme 1 was adopted. In a single-necked flask, a solution of 2-methylpyrimidine-4,6-diol (5 g, 39.65 mmol) in chloroform (50 mL) was added. The reaction system was replaced with nitrogen and then cooled to 0°C. After bromine (4.1 mL, 79.3 mmol) was slowly added to the reaction system, the reaction system was stirred at 80°C overnight. After TLC monitoring showed that the raw material disappeared, the reaction mixture was concentrated under reduced pressure. The reaction mixture was washed with ethyl acetate (100 mL × 3) and filtered to obtain a light yellow solid. The resulting solid was adjusted to pH = 7-8 with saturated sodium bicarbonate, and the crude product 5-bromo-2-methylpyrimidine-4,6-diol (11 g) was obtained after rotary evaporation to dryness. The crude product was then added to phosphorus oxychloride (15 mL) and stirred at 110°C overnight under nitrogen atmosphere. After the reaction was completed, the mixture was diluted with ethyl acetate (100 mL), adjusted to pH = 8 with saturated sodium bicarbonate, and then extracted with ethyl acetate (100 mL × 3), dried over anhydrous sodium sulfate, filtered, and concentrated under reduced pressure. The resulting mixture was purified by silica gel column chromatography to obtain 5-bromo-4,6-dichloro-2-methylpyrimidine (1.2 g). Using 5-bromo-4,6-dichloro-2-methylpyrimidine as a raw material, referring to the synthetic route of Example 1, 6-amino-4-chloro-7-(3-hydroxy-2,6-dimethylphenyl)-2-methyl-7*H*-pyrrolo[2,3-*d*]pyrimidine-5-carboxamide (1.47 mg) was prepared. MS (ESI) m/z: 346.1 [M+H]⁺. ¹H NMR (400 MHz, MeOD) δ 6.85 (d, J = 8.3 Hz, 1H), 6.66 (d, J = 8.2 Hz, 1H), 2.22 (s, 3H), 2.00 (s, 3H), 1.96 (s, 3H).

### Example 29

### 2-Amino-7-fluoro-1-(3-hydroxy-2,6-dimethylphenyl)-6-methyl-1H-pyrrolo[3,2-c]nicotinamide

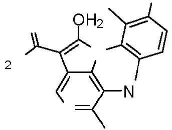

Diisopropylamine (319 mg, 3.16 mmol) was dissolved in tetrahydrofuran (10 mL), cooled to -78°C, then 1.8 mol/L *n*-butyllithium (1.75 mL) was slowly added, and reacted at - 78°C for 15 minutes, followed by the addition of a solution of 5-bromo-3-fluoro-2-methylpyridine (500 mg, 2.63 mmol) in tetrahydrofuran (20 mL). The reaction mixture was stirred at this temperature for another 30 minutes, and then a solution of iodine (1 g, 3.95 mmol) in tetrahydrofuran (5 mL) was slowly added and stirred at room temperature for 2 hours. After LCMS monitoring showed that the raw material disappeared, the reaction mixture was added to ammonium chloride aqueous solution (80 mL), and extracted with ethyl acetate (40 mL × 2). The organic phases were combined, first washed with sodium thiosulfate (50 mL × 2), then washed with saturated brine (40 mL), and then dried over anhydrous sodium sulfate. Finally, the resulting residue obtained by concentrating under reduced pressure was purified by silica gel column chromatography to obtain 5-bromo-3-fluoro-4-iodo-2-methylpyridine (500 mg).

5-Bromo-3-fluoro-4-iodo-2-methylpyridine (470 mg, 1.49 mmol) was dissolved in ethylene glycol dimethyl ether (10 mL), and 3-methoxy-2,6-dimethylaniline (247 mg, 1.64 mmol), cesium carbonate (1.21 g, 3.72 mmol), 4,5-bis(diphenylphosphino)-9,9-dimethylxanthene (77 mg, 0.13 mmol), and tris(dibenzylideneacetone)dipalladium (68 mg, 0.07 mmol) were added. The reaction system was replaced with nitrogen for three times, and the reaction mixture was stirred at 90°C overnight. After LCMS monitoring showed that the raw material disappeared, the reaction mixture was added with water (30 mL) and extracted with ethyl acetate (20 mL). The organic phases were combined, washed with saturated brine (20 mL), dried over anhydrous sodium sulfate, concentrated under reduced pressure, and purified by silica gel column chromatography to obtain 5-bromo-3-fluoro-*N*-(3-methoxy-2,6-dimethylphenyl)-2-methylpyridin-4-amine (400 mg).

2-Amino-7-fluoro-1-(3-hydroxy-2,6-dimethylphenyl)-6-methyl-1*H*-pyrrolo[3,2-*c*]nicotinamide (15.18 mg) was prepared according to the scheme of Example 1 using 5-bromo-3-fluoro-*N*-(3-methoxy-2,6-dimethylphenyl)-2-methylpyridin-4-amine as a raw material. MS (ESI) m/z: 328.5 [M+H⁺]. ¹H NMR (400 MHz, DMSO-*d*₆) δ 9.61 (s, 1H), 8.64 (d, *J =* 1.6 Hz, 1H), 7.06 (d, *J =* 8.3 Hz, 1H), 6.89 (dd, *J =* 22.5, 6.9 Hz, 5H), 2.33 (d, *J =* 3.2 Hz, 3H), 1.80 (s, 3H), 1.72 (s, 3H). C₁₇H₁₇FN₄O₂.

### Example 30

### 2-Amino-1-(3-hydroxy-2,6-dimethylphenyl)-1H-pyrrolo[3,2-c]pyridine-3-carboxamide

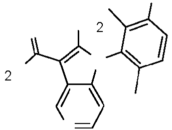

2-Amino-1-(3-hydroxy-2,6-dimethylphenyl)-1*H*-pyrrolo[3,2-*c*]pyridine-3-carboxamide (5.6 mg) was prepared by referring to the route of Scheme 5 using 3,4-dibromopyridine as a raw material. MS (ESI) M/Z:297.4 [M+H⁺]. ¹H NMR (400 MHz, DMSO) δ 9.64 (s, 1H), 8.92 (s, 1H), 8.01 (d, *J =* 5.3 Hz, 1H), 7.12 (d, *J =* 8.3 Hz, 1H), 6.96 (d, *J* = 8.3 Hz, 1H), 6.90 (s, 2H), 6.80 (s, 2H), 6.47 (d, *J =* 5.3 Hz, 1H), 1.75 (s, 3H), 1.66 (s, 3H).

### Example 31

### 2-Amino-1-(3-hydroxy-2,6-dimethylphenyl)-6-methyl-1H-pyrrolo[3,2-c]pyridine-3-carboxamide

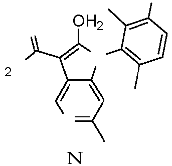

2-Amino-1-(3-hydroxy-2,6-dimethylphenyl)-6-methyl-1*H*-pyrrolo[3,2-*c*]pyridine-3-carboxamide (3.22 mg) was prepared according to the route of Scheme 5 using 5-bromo-4-chloro-2-methylpyridine as a raw material. MS (ESI) m/z: 311.5 [M+H⁺]. ¹H NMR (400 MHz, DMSO-*d₆*) δ 9.65 (s, 1H), 8.77 (s, 1H), 7.11 (d, *J* = 8.3 Hz, 1H), 6.95 (d, *J* = 8.3 Hz, 1H), 6.83 (s, 2H), 6.75 (s, 2H), 6.31 (s, 1H), 2.37 (s, 3H), 1.75 (s, 3H), 1.66 (s, 3H). C₁₇H₁₈N₄O₂.

### Example 32

### 6-Amino-7-(2,6-diethyl-3-hydroxyphenyl)-2-methyl-7H-pyrrolo[2,3-d]pyrimidine-5-carboxamide

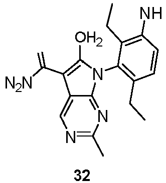

5-Bromo-4-chloro-2-methylpyrimidine (1 g, 4.83 mmol) and 2,6-diethyl-3-methoxyaniline (690 mg, 3.86 mmol) were dissolved in tetrahydrofuran (30 mL), and lithium bis(trimethylsilyl)amide (1M, 14.5 mL, 14.5 mmol) was added in an ice-water bath, and the reaction was carried out at 50°C overnight. After TLC monitoring showed that the raw material disappeared, the reaction mixture was quenched with ammonium chloride aqueous solution (20 mL), extracted with ethyl acetate (40 mL), and the organic phase was washed with saturated brine (30 mL), dried over anhydrous sodium sulfate, and concentrated to dryness under reduced pressure. The resulting residue was subjected to column chromatography to obtain 5-bromo-amino-(2,6-diethyl-3-methoxyphenyl)-2-methylpyrimidin-4-amine (150 mg). According to the method of Example 1, 5-bromo-4-chloro-2-methylpyrimidine and 2,6-diethyl-3-methoxyaniline were used as raw materials to prepare 6-amino-7-(2,6-diethyl-3-hydroxyphenyl)-2-methyl-7H-pyrrolo[2,3-d]pyrimidine-5-carboxamide (15.87 mg). MS (ESI) m/z: 339.1 [M+H⁺]. ¹H NMR (400 MHz, DMSO-*d*₆) δ 9.60 (s, 1H), 8.87 (s, 1H), 7.12 (d, *J* = 8.4 Hz, 1H), 7.06 - 6.96 (m, 3H), 6.86 (s, 2H), 2.43 (s, 3H), 2.21 - 1.89 (m, 4H), 0.86 (dt, *J* = 27.7, 7.5 Hz, 6H). C₁₈H₂₁N₅O₂.

### Example 33

### 6-Amino-7-(3-hydroxy-2,6-dimethylphenyl)-2-(phenylamino)-7H-pyrrolo[2,3-d]pyrimidine-5-carboxamide

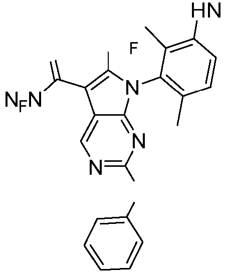

¹H NMR (400 MHz, DMSO-*d*₆) δ 9.57 (s, 1H), 9.15 (s, 1H), 8.73 (s, 1H), 7.71 (d, *J =* 8.1 Hz, 2H), 7.18 - 7.05 (m, 3H), 6.96 - 6.88 (m, 3H), 6.82 - 6.69 (m, 3H), 1.83 (s, 3H), 1.74 (s, 3H). C₂₁H₂₀N₆O₂.

MS (ESI) m/z: 388.3 [M+H⁺].

### Example 34

### 6-Amino-7-(3-hydroxy-2,6-dimethylphenyl)-2-morpholino-7H-pyrrolo[2,3-d]pyrimidine-5-carboxamide

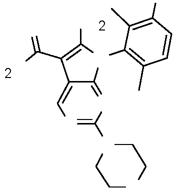

The preparation method was the same as that of Example 13. 6-Amino-7-(3-hydroxy-2,6-dimethylphenyl)-2-morpholino-7*H*-pyrrolo[2,3-*d*]pyrimidine-5-carboxamide (23.97 mg) was prepared by reacting 6-amino-2-chloro-7-(3-hydroxy-2,6-dimethylphenyl)-7*H*-pyrrolo[2,3-*d*]pyrimidine-5-carboxamide with morpholine. MS (ESI) m/z: 383.1 [M+H⁺]. ¹H NMR (400 MHz, DMSO-*d₆*) δ 9.54 (s, 1H), 8.66 (s, 1H), 7.06 (d, *J =* 8.3 Hz, 1H), 6.90 (d, *J =* 8.2 Hz, 1H), 6.79 (s, 2H), 6.70 (s, 2H), 3.59 (t, *J =* 4.8 Hz, 4H), 3.42 (t, *J =* 4.8 Hz, 4H), 1.79 (s, 3H), 1.70 (s, 3H). C₁₉H₂₂N₆O₃.

### Example 35

### 6-Amino-7-(3-hydroxy-2,6-dimethylphenyl)-2-(pyrrolidin-1-yl)-7H-pyrrolo[2,3-d]pyrimidine-5-carboxamide

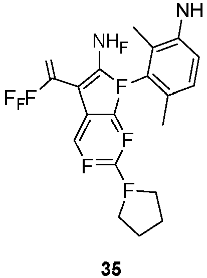

The preparation method was the same as that of Example 13. 6-Amino-7-(3-hydroxy-2,6-dimethylphenyl)-2-(pyrrolidin-1-yl)-7*H*-pyrrolo[2,3-*d*]pyrimidine-5-carboxamide (6.97 mg) was prepared by reacting 6-amino-2-chloro-7-(3-hydroxy-2,6-dimethylphenyl)-7*H*-pyrrolo[2,3-d]pyrimidine-5-carboxamide with pyrrolidine. MS (ESI) m/z: 367.2 [M+H⁺]. ¹H NMR (400 MHz, DMSO-*d*₆) δ 9.52 (s, 1H), 8.59 (s, 1H), 7.05 (d, *J* = 8.3 Hz, 1H), 6.89 (d, *J* = 8.2 Hz, 1H), 6.71 - 6.61 (m, 4H), 3.34 - 3.23 (m, 4H), 1.91 - 1.75 (m, 7H), 1.72 (s, 3H). C₁₉H₂₂N₆O₂.

### Example 36

### 6-Amino-2-(dimethylamino)-7-(3-hydroxy-2,6-dimethylphenyl)-7H-pyrrolo[2,3-d]pyrimidine-5-carboxamide

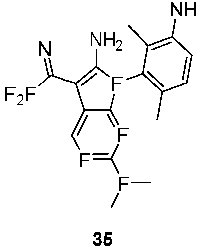

The preparation method was the same as that of Example 13. 6-Amino-2-(dimethylamino)-7-(3-hydroxy-2,6-dimethylphenyl)-7*H*-pyrrolo[2,3-*d*]pyrimidine-5-carboxamide (10.82 mg) was prepared by reacting 6-amino-2-chloro-7-(3-hydroxy-2,6-dimethylphenyl)-7*H*-pyrrolo[2,3-*d*]pyrimidine-5-carboxamide with dimethylamine hydrochloride. MS (ESI) m/z: 341.3 [M+H⁺]. ¹H NMR (400 MHz, DMSO-*d₆*) δ 9.53 (s, 1H), 8.60 (s, 1H), 7.06 (d, *J =* 8.2 Hz, 1H), 6.89 (d, *J =* 8.2 Hz, 1H), 6.71 (s, 2H), 6.65 (s, 2H), 2.93 (s, 6H), 1.80 (s, 3H), 1.71 (s, 3H). C₁₇H₂₀N₆O₂.

### Example 37

### 6-Amino-2-(4,4-difluoropiperidin-1-yl)-7-(3-hydroxy-2,6-dimethylphenyl)-7H-pyrrolo[2,3-d]pyrimidine-5-carboxamide

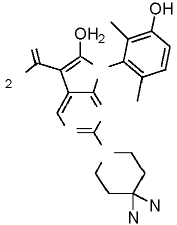

The preparation method was the same as that of Example 13. 6-Amino-2-(4,4-difluoropiperidin-1-yl)-7-(3-hydroxy-2,6-dimethylphenyl)-7*H*-pyrrolo[2,3-*d*]pyrimidine-5-carboxamide (21.39 mg) was prepared by reacting 6-amino-2-chloro-7-(3-hydroxy-2,6-dimethylphenyl)-7*H*-pyrrolo[2,3-*d*]pyrimidine-5-carboxamide with 4,4-difluoropyridine. MS (ESI) m/z: 417.3 [M+H⁺]. ¹H NMR (400 MHz, DMSO-*d₆*) δ 9.55 (s, 1H), 8.67 (s, 1H), 7.07 (d, *J =* 8.3 Hz, 1H), 6.90 (d, *J =* 8.2 Hz, 1H), 6.81 (s, 2H), 6.71 (s, 2H), 3.66 (t, *J* = 5.7 Hz, 4H), 1.97 - 1.82 (m, 4H), 1.80 (s, 3H), 1.71 (s, 3H). C₂₀H₂₂F₂N₆O₂.

### Example 40

### 2,6-Diamino-7-(3-hydroxy-2,6-dimethylphenyl)-7H-pyrrolo[2,3-d]pyrimidine-5-carboxamide

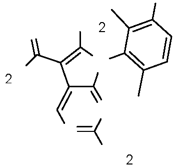

6-Amino-2-chloro-7-(3-methoxy-2,6-dimethylphenyl)-7*H*-pyrrolo[2,3-*d*]pyrimidine-5-carbonitrile (150 mg, 0.46 mmol) was dissolved in n-butanol (3 mL), then *N,N-*diisopropylethylamine (177.9 mg, 1.38 mmol) and *p*-methoxybenzylamine (1.5 mL) were added, and the reaction mixture was stirred at 150°C for 12 hours. Water (30 mL) was added, and the mixture was extracted with ethyl acetate (20 mL × 2). The organic phases were combined, washed with saturated brine (50 mL), dried over anhydrous sodium sulfate, concentrated under reduced pressure, and then purified by silica gel column chromatography (eluent: petroleum ether/ethyl acetate = 1/1) to obtain 6-amino-7-(3-methoxy-2,6-dimethylphenyl)-2-((4-methoxybenzyl)amino)-7*H*-pyrrolo[2,3-*d*]pyrimidine-5-carbonitrile (168 mg). Then the compound was dissolved in sulfuric acid/water (13/1, 1 mL), and methanesulfonic acid (1.4 g, 14.52 mmol) was added. The reaction was carried out at room temperature for 1.5 hours, and methionine (234.3 mg, 1.57 mmol) was added. The reaction was carried out at 40°C overnight. After LCMS monitoring showed that the raw material disappeared, sodium hydroxide and dipotassium hydrogen phosphate aqueous solution was added to adjust the pH to neutral, and the mixture was directly concentrated to dryness to obtain a crude product to prepare 2,6-diamino-7-(3-hydroxy-2,6-dimethylphenyl)-7*H*-pyrrolo[2,3-*d*]pyrimidine-5-carboxamide (2.01 mg). MS (ESI) m/z: 313.3 [M+H⁺]. ¹H NMR (400 MHz, DMSO-*d₆*) δ 9.55 (s, 1H), 8.52 (s, 1H), 7.08 - 7.00 (m, 1H), 6.93 - 6.85 (m, 1H), 6.77 - 6.60 (m, 4H), 5.99 (s, 2H), 1.80 (s, 3H), 1.71 (s, 3H). C₁₅H₁₆N₆O₂.

### Example 41

### 6-Amino-2-cyclopropyl-7-(3-hydroxy-2,6-dimethylphenyl)-7H-pyrrolo[2,3-d]pyrimidine-5-carboxamide

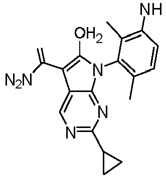

5-Bromo-4-chloro-2-(methylthio)pyrimidine (4 g, 16.81 mmol) was dissolved in tetrahydrofuran (30 mL), then potassium peroxymonosulfate complex salt (15.5 g, 50.42 mmol) was dissolved in water (20 mL) and added dropwise to the reaction mixture, and stirred overnight at room temperature. After the reaction was completed, the reaction mixture was extracted with ethyl acetate (60 mL × 2). The organic phases were combined, first washed with saturated brine (50 mL), then dried over anhydrous sodium sulfate, and finally concentrated under reduced pressure to obtain 5-bromo-4-chloro-2-(methylsulfonyl)pyrimidine (4.1 g).

5-Bromo-4-chloro-2-(methylsulfonyl)pyrimidine (2 g, 7.33 mmol) was dissolved in tetrahydrofuran (60 mL), then cyclopropylmagnesium bromide (17.6 mL, 8.8 mmol) was added, and the reaction mixture was stirred overnight at room temperature. After the reaction was completed, the reaction mixture was added with water (30 mL) and extracted with ethyl acetate (20 mL × 2). The organic phases were combined, washed with saturated brine (20 mL), and dried over anhydrous sodium sulfate. Finally, the resulting residue obtained by concentrating under reduced pressure was purified by silica gel column chromatography to obtain 5-bromo-4-chloro-2-cyclopropylpyrimidine (510 mg). 6-Amino-2-cyclopropyl-7-(3-hydroxy-2,6-dimethylphenyl)-7*H*-pyrrolo[2,3-*d*]pyrimidine-5-carboxamide (5.34 mg) was prepared according to the method of Example 1 using 5-bromo-4-chloro-2-cyclopropylpyrimidine as a raw material. MS (ESI) m/z: 338.2 [M+H⁺]. ¹H NMR (400 MHz, DMSO-*d*₆) δ 9.61 (s, 1H), 8.82 (s, 1H), 7.09 (d, *J =* 8.3 Hz, 1H), 7.02 (s, 2H), 6.94 (d, *J* = 8.3 Hz, 1H), 6.83 (s, 2H), 1.97 (tt, *J =* 7.8, 5.0 Hz, 1H), 1.76 (s, 3H), 1.68 (s, 3H), 0.84 (tt, *J* = 7.7, 2.8 Hz, 4H). C₁₈H₁₉N₅O₂.

### Example 42

### 6-Amino-7-(3-hydroxy-2,6-dimethylphenyl)-2-(2-methylprop-1-en-1-yl)-7H-pyrrolo[2,3-d]pyrimidine-5-carboxamide

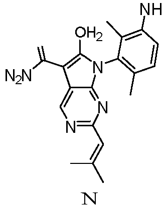

The preparation method was the same as that of Example 6. 6-Amino-7-(3-hydroxy-2,6-dimethylphenyl)-2-(2-methylprop-1-en-1-yl)-7*H*-pyrrolo[2,3-*d*]pyrimidine-5-carboxamide (70 mg) was prepared by reacting 6-amino-2-chloro-7-(3-hydroxy-2,6-dimethylphenyl)-7*H*-pyrrolo[2,3-*d*]pyrimidine-5-carboxamide with (2-methylprop-1-en-1-yl)boric acid. MS (ESI) m/z: 352.2 [M+H⁺]. ¹HNMR (400 MHz, DMSO-*d₆*) δ 9.58 (s, 1H), 8.95 (s, 1H), 7.15 (s, 2H), 7.08 (d, *J =* 8.3 Hz, 1H), 6.96 - 6.85 (m, 3H), 6.16 (s, 1H), 2.07 (s, 3H), 1.84 (s, 3H), 1.77 (s, 3H), 1.68 (s, 3H). C₁₉H₂₁N₅O₂.

### Example 43

### 2-Amino-1-(4-chloro-3-hydroxy-2,6-dimethylphenyl)-5,6-dimethyl-1H-pyrrolo[2,3-b]pyridine-3-carboxamide

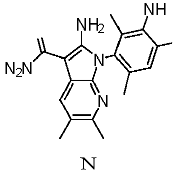

2-Amino-1-(3-hydroxy-2,6-dimethylphenyl)-5,6-dimethyl-1*H*-pyrrolo[2,3-*b*]pyridine-3-carboxamide (200 mg, 0.62 mmol) was dissolved in trifluoroacetic acid (6 mL), then *N*-chlorosuccinimide (197 mg, 1.47 mmol) was added, and the reaction mixture was stirred at room temperature. After the reaction was completed, sodium bicarbonate (30 mL) and dichloromethane (30 mL) were added, and the reaction mixture was stirred, and the phases were separated. The organic phase was washed with saturated brine, dried over sodium sulfate, filtered, and the filtrate was concentrated. The resulting crude product was used to prepare 2-amino-1-(4-chloro-3-hydroxy-2,6-dimethylphenyl)-5,6-dimethyl-1*H*-pyrrolo[2,3-b]pyridine-3-carboxamide (2.44 mg). MS (ESI) m/z: 359.2 [M+H⁺]. ¹H NMR (400 MHz, DMSO-*d₆*) δ 7.81 (s, 1H), 7.15 (s, 1H), 6.74 (s, 2H), 6.63 (s, 2H), 2.25 (d, *J =* 6.6 Hz, 6H), 1.69 (d, *J* = 10.9 Hz, 6H). C₁₈H₁₉ClN₄O₂.

### Example 44

### 2-Amino-1-(4-chloro-3-fluoro-5-hydroxy-2,6-dimethylphenyl)-5,6-dimethyl-1H-pyrrolo[2,3-b]pyridine-3-carboxamide

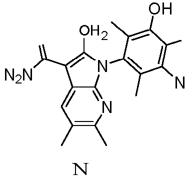

Using the route of Scheme 5, the preparation method was the same as that of Example 20, and 2-amino-1-(4-chloro-3-fluoro-5-hydroxy-2,6-dimethylphenyl)-5,6-dimethyl-1*H-*pyrrolo[2,3-*b*]pyridine-3-carboxamide (4 mg) was prepared by using 2,3-dibromo-5,6-dimethylpyridine and 3-fluoro-4-chloro-5-methoxy-2,6-dimethylaniline as raw materials. MS (ESI) m/z: 377.1, 379.1 [M+H]⁺. ¹H NMR (400 MHz, DMSO) δ 9.82 (s, 1H), 7.85 (s, 1H), 6.95 (s, 2H), 6.67 (s, 2H), 2.26 (d, *J =* 7.0 Hz, 6H), 1.74 - 1.66 (m, 6H).

### Example 45

### 6-Amino-7-(3-hydroxy-2,6-dimethylphenyl)-7H-pyrrolo[2,3-d]pyrimidine-5-carboxamide

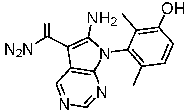

The preparation method was the same as that of Example 1, and 6-amino-7-(3-hydroxy-2,6-dimethylphenyl)-7*H*-pyrrolo[2,3-*d*]pyrimidine-5-carboxamide (59.74 mg) was prepared by using 5-bromo-4-chloropyrimidine as a raw material. MS (ESI) m/z: 298.0 [M+H⁺]. ¹H NMR (400 MHz, DMSO-*d*₆) δ 9.62 (s, 1H), 9.00 (s, 1H), 8.41 (s, 1H), 7.21 (s, 2H), 7.09 (d, *J =* 8.3 Hz, 1H), 6.98 - 6.90 (m, 3H), 1.75 (s, 3H), 1.67 (s, 3H). C₁₅H₁₅N₅O₂.

### Example 46

### 6-Amino-7-(3-hydroxy-2,6-dimethylphenyl)-2-methoxy-7H-pyrrolo[2,3-d]pyrimidine-5-carboxamide

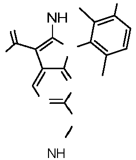

1-Methoxy-2,4-dimethyl-3-nitrobenzene (3.0 g, 17.95 mmol) was dissolved in dichloromethane (30 mL), and 1M dichloromethane solution of boron tribromide (25 mL) was added dropwise at -40°C. After the addition was completed, the reaction mixture was slowly warmed to room temperature and stirred overnight. After the reaction was completed, water (150 mL) and potassium dihydrogen phosphate (11.2 g) were added to the reaction mixture, and then extracted with dichloromethane (100 mL). The organic phase was washed with saturated brine (50 mL), dried over anhydrous sodium sulfate, and concentrated under reduced pressure. The resulting residue was purified by silica gel column chromatography to obtain 2,4-dimethyl-3-nitrophenol (1.9 g). 2,4-Dimethyl-3-nitrophenol (200.0 mg, 1.3 mmol) and 4-methoxybenzyl alcohol (270.8 mg, 1.9 mmol) were dissolved in tetrahydrofuran, then triphenylphosphine (514.1 mg, 1.9 mmol) was added, and diisopropyl azodicarboxylate was added dropwise at 0°C, and stirred at room temperature overnight. After the reaction was completed, water (20 mL) and ethyl acetate (20 mL) were added to the reaction mixture for extraction, and the organic phase was washed with saturated brine (10 mL), dried over anhydrous sodium sulfate, and concentrated under reduced pressure. The resulting residue was purified by silica gel column chromatography to obtain 1-((4-methoxybenzyl)oxy)-2,4-dimethyl-3-nitrobenzene (110 mg). The reaction was then scaled up to prepare 1-((4-methoxybenzyl)oxy)-2,4-dimethyl-3-nitrobenzene (1.06 g).

Reduced iron powder (1.34 g, 24.0 mmol), silica (2.7 g, 45.6 mmol), and ammonium chloride (790 mg, 14.7 mmol) were added to ethanol (20 mL) and water (20 mL), and the reaction system was heated to 60°C. 1-((4-Methoxybenzyl)oxy)-2,4-dimethyl-3-nitrobenzene (1.06 g, 3.7 mmol) was dissolved in tetrahydrofuran (20 mL) and added to the reaction mixture. After stirring at 70°C for 3 hours, saturated brine (30 mL) and ethyl acetate (50 mL) were added to the reaction mixture for phase separation. The organic phase was washed with saturated brine (30 mL), dried over anhydrous sodium sulfate, and concentrated under reduced pressure. The resulting residue was purified by silica gel column chromatography to obtain 3-((4-methoxybenzyl)oxy)-2,6-dimethyl-aniline (830 mg). Referring to Example 1, 5-bromo-2,4-dichloropyrimidine (800 mg, 3.5 mmol) was dissolved in N-methylpyrrolidone (10 mL), then 2,6-dimethylpyridine (608.5 mg, 5.7 mmol) and 3-((4-methoxybenzyl)oxy)-2,6-dimethyl-aniline (992.5 mg, 3.9 mmol) were added, and the reaction was carried out to prepare 5-bromo-2-chloro-*N*-(3-((4-methoxybenzyl)oxy)-2,6-dimethylphenyl)pyrimidin-4-amine (1.0 g).

5-Bromo-2-chloro-*N*-(3-((4-methoxybenzyl)oxy)-2,6-dimethylphenyl)pyrimidin-4-amine (400 mg, 0.9 mmol) was dissolved in methanol (8 mL), then sodium methoxide (241.6 mg, 4.5 mmol) was added, and the reaction system was heated to 80°C, and stirred overnight. After LCMS monitoring showed that the raw material disappeared, water (30 mL) and ethyl acetate (30 mL) were added to the reaction mixture for extraction, and the organic phase was washed with saturated brine, dried over anhydrous sodium sulfate, and concentrated under reduced pressure to obtain 5-bromo-2-methoxy-*N*-(3-((4-methoxybenzyl)oxy)-2,6-dimethylphenyl)pyrimidin-4-amine (311.1 mg). MS (ESI) m/z: 446.2 [M+H⁺].

Referring to Example 1, 6-amino-2-methoxy-7-(3-((4-methoxybenzyl)oxy)-2,6-dimethylphenyl)-7*H*-pyrrolo[2,3-*d*]pyrimidine-5-carbonitrile (60 mg) was prepared by reacting 5-bromo-2-methoxy-*N*-(3-((4-methoxybenzyl)oxy)-2,6-dimethylphenyl)pyrimidin-4-amine with malononitrile.

6-Amino-2-methoxy-7-(3-((4-methoxybenzyl)oxy)-2,6-dimethylphenyl)-7*H-*pyrrolo[2,3-*d*]pyrimidine-5-carbonitrile (60 mg, 0.14 mmol) was dissolved in concentrated sulfuric acid (1 mL). After stirring at room temperature for 1 hour, saturated sodium bicarbonate aqueous solution (20 mL) and ethyl acetate (20 mL) were added to the reaction mixture for extraction. The organic phase was washed with saturated brine (10 mL), dried over anhydrous sodium sulfate, and concentrated under reduced pressure. 6-Amino-7-(3-hydroxy-2,6-dimethylphenyl)-2-methoxy-7H-pyrrolo[2,3-d]pyrimidine-5-carboxamide (4.11 mg) was prepared. MS (ESI) m/z: 328.3 [M+H⁺]. ¹H NMR (400 MHz, DMSO-*d*₆) δ 9.61 (s, 1H), 8.73 (s, 1H), 7.08 (d, *J =* 8.3 Hz, 1H), 6.96 (dd, *J =* 26.8, 9.2 Hz, 3H), 6.82 (s, 2H), 3.77 (s, 3H), 1.77 (s, 3H), 1.68 (s, 3H). C₁₆H₁₇N₅O₃.

### Example 47

### 6-Amino-2-ethynyl-7-(3-hydroxy-2,6-dimethylphenyl)-7H-pyrrolo[2,3-d]pyrimidine-5-carboxamide

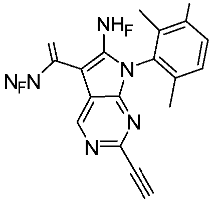

6-Amino-2-bromo-7-(3-hydroxy-2,6-dimethylphenyl)-7*H*-pyrrolo[2,3-*d*]pyrimidine-5-carboxamide (200 mg, 0.535 mmol), (triisopropylsilyl)acetylene (481 mg, 2.67 mmol), tetrakis(triphenylphosphine)palladium (124 mg, 0.11 mmol), and cuprous iodide (6 mg, 0.005 mmol) were dissolved in *N*,*N*-dimethylformamide (6 mL) and triethylamine (6 mL), and the reaction system was replaced with nitrogen, and reacted at 90°C. After the reaction was completed, the reaction mixture was extracted with water (20 mL) and ethyl acetate (30 mL), washed with saturated brine (20 mL), dried over anhydrous sodium sulfate, and concentrated under reduced pressure. The resulting mixture obtained by concentrating under reduced pressure was subjected to column chromatography to obtain 6-amino-7-(3-hydroxy-2,6-dimethylphenyl)-2-((triisopropylsilyl)ethynyl)-7H-pyrrolo[2,3-d]pyrimidine-5-carboxamide (70 mg).

6-Amino-7-(3-hydroxy-2,6-dimethylphenyl)-2-((triisopropylmethylsilyl)ethynyl)-7H-pyrrolo[2,3-*d*]pyrimidine-5-carboxamide (70 mg, 0.146 mmol) was dissolved in tetrahydrofuran (6 mL), then tetrabutylammonium fluoride (230 mg, 0.732 mmol) was added in an ice-water bath. The reaction mixture was stirred in an ice-water bath for 40 minutes, extracted with water (20 mL) and ethyl acetate (30 mL), washed with saturated brine (20 mL), and dried over anhydrous sodium sulfate. The resulting mixture obtained by concentrating under reduced pressure was purified to obtain 6-amino-2-ethynyl-7-(3-hydroxy-2,6-dimethylphenyl)-7*H*-pyrrolo[2,3-*d*]pyrimidine-5-carboxamide (3.46 mg). MS (ESI) m/z: 321.2 [M+H⁺]. ¹H NMR (400 MHz, DMSO-*d₆*) δ 9.67 (s, 1H), 8.96 (s, 1H), 7.37 (s, 2H), 7.10 (d, *J* = 8.3 Hz, 1H), 7.04 - 6.93 (m, 3H), 4.03 (s, 1H), 1.75 (s, 3H), 1.66 (s, 3H). C₁₇H₁₅N₅O₂.

### Example 48

### 2-Amino-1-(3-chloro-5-hydroxy-2,6-dimethylphenyl)-5,6-dimethyl-1H-pyrrolo[2,3-b]nicotinamide

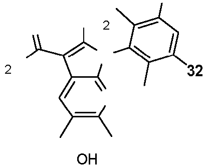

Using the route of Scheme 5, the preparation method was the same as that of Example 20, and 2-amino-1-(3-chloro-5-hydroxy-2,6-dimethylphenyl)-5,6-dimethyl-1*H*-pyrrolo[2,3-*b*]nicotinamide (24.61 mg) was prepared by using 2,3-dibromo-5,6-dimethylpyridine and 3-chloro-5-methoxy-2,6-dimethylaniline as raw materials. MS (ESI) m/z: 359.1 [M+H⁺]. ¹H NMR (400 MHz, DMSO-*d*₆) δ 10.33 (s, 1H), 7.86 (s, 1H), 7.21 (s, 1H), 6.88 (s, 2H), 6.70 (s, 2H), 2.25 (d, *J* = 7.0 Hz, 6H), 1.75 (s, 3H), 1.62 (s, 3H). C₁₈H₁₉ClN₄O₂.

### Example 50

### 2-Amino-1-(2-hydroxy-3,5-dimethylpyridin-4-yl)-5,6-dimethyl-1H-pyrrolo[2,3-b]pyridine-3-carboxamide

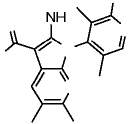

Using the route of Scheme 5, the preparation method was the same as that of Example 20, and 2-amino-1-(2-hydroxy-3,5-dimethylpyridin-4-yl)-5,6-dimethyl-1*H*-pyrrolo[2,3-*b]pyridine-3-carboxamide (3.17 mg) was prepared by using 2,3-dibromo-5,6-dimethylpyridine* and 2-methoxy-3,5-dimethylpyridin-4-amine as raw materials. MS (ESI) m/z: 326.4 [M+H⁺]. ¹H NMR (400 MHz, DMSO-*d*₆) δ 7.84 (s, 1H), 7.27 (s, 1H), 7.06 (s, 2H), 6.69 (s, 2H), 2.27 (d, *J=* 5.1 Hz, 6H), 1.61 (s, 3H), 1.55 (s, 3H). C₁₇H₁₉N₅O₂.

### Example 51

### 2-Amino-1-(5-hydroxy-2,4-dimethylpyrimidin-3-yl)-5,6-dimethyl-1H-pyrrolo[2,3-b]pyrimidine-3-carboxamide

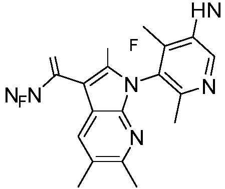

¹H NMR (400 MHz, DMSO-*d*₆) δ 9.95 (s, 1H), 8.15 (s, 1H), 7.85 (s, 1H), 6.97 (s, 2H), 6.69 (s, 2H), 2.25 (d, *J =* 8.5 Hz, 6H), 1.94 (s, 3H), 1.72 (s, 3H). C₁₇H₁₉N₅O₂.

MS (ESI) m/z: 326.2 [M+H⁺].

### Example 53

### 2-Amino-1-(3-hydroxy-2,5,6-trimethylphenyl)-5,6-dimethyl-1H-pyrrolo[2,3-b]pyridine-3-carboxamide

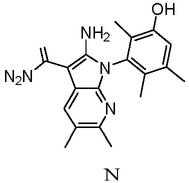

Using the route of Scheme 5, the preparation method was the same as that of Example 20. 2-Amino-1-(3-hydroxy-2,5,6-trimethylphenyl)-5,6-dimethyl-1*H*-pyrrolo[2,3-b]pyridine-3-carboxamide (5.13 mg) was prepared by using 2,3-dibromo-5,6-dimethylpyridine and 3-methoxy-2,5,6-trimethylaniline as raw materials. MS (ESI) m/z: 339.4 [M+H⁺]. ¹H NMR (400 MHz, DMSO-*d*₆) δ 9.40 (s, 1H), 7.83 (d, *J* = 4.7 Hz, 1H), 6.82 (d, *J* = 5.5 Hz, 1H), 6.75 - 6.56 (m, 4H), 2.30 - 2.18 (m, 9H), 1.66 - 1.57 (m, 6H). C₁₉H₂₂N₄O₂.

### Example 54

### 2-Amino-1-(3,4-difluoro-5-hydroxy-2,6-dimethylphenyl)-5,6-dimethyl-1H-pyrrolo[2,3-b]pyrimidine-3-carboxamide

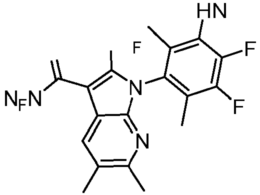

¹H NMR (400 MHz, DMSO-*d*₆) δ 10.16 (s, 1H), 7.85 (s, 1H), 6.93 (s, 2H), 6.69 (s, 2H), 2.26 (d, *J =* 5.7 Hz, 6H), 1.71 (s, 3H), 1.67 (s, 3H). C₁₈H₁₈F₂N₄O₂.

MS (ESI) m/z: 359.9 [M+H⁺].

### Example 57

### 6-Amino-7-(3-hydroxy-2,6-dimethylphenyl)-2-isobutyl-7H-pyrrolo[2,3-d]pyrimidine-5-carboxamide

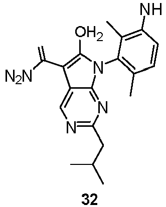

6-Amino-7-(3-hydroxy-2,6-dimethylphenyl)-2-(2-methylprop-1-en-1-yl)-7*H-*pyrrolo[2,3-*d*]pyrimidine-5-carboxamide (60 mg, 0.17 mmol) was dissolved in methanol, then 10% Pd/C (3 mg) was added and reacted under hydrogen atmosphere at normal pressure and room temperature for 2 hours. After LCMS monitoring showed that the raw material disappeared, the reaction mixture was filtered, and the filtrate was concentrated to obtain 6-amino-7-(3-hydroxy-2,6-dimethylphenyl)-2-isobutyl-7*H*-pyrrolo[2,3-*d*]pyrimidine-5-carboxamide (37.03 mg). MS (ESI) m/z: 354.0 [M+H⁺]. ¹H NMR (400 MHz, DMSO-*d₆*) δ 9.66 (s, 1H), 8.90 (s, 1H), 7.12 - 7.03 (m, 3H), 6.94 (d, *J =* 8.2 Hz, 1H), 6.85 (s, 2H), 2.55 (d, *J =* 7.2 Hz, 2H), 2.02 (dt, *J =* 13.5, 6.8 Hz, 1H), 1.75 (s, 3H), 1.66 (s, 3H), 0.80 (d, *J =* 6.4 Hz, 6H). C₁₉H₂₃N₅O₂.

### Example 58

### 2-Amino-5-bromo-1-(4-fluoro-3-hydroxy-2,6-dimethylphenyl)-6-methyl-1H-pyrrolo[2,3-b]pyridine-3-carboxamide

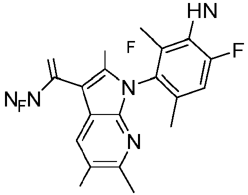

3,5-Dibromo-2-fluoro-6-methylpyridine (1 g, 3.72 mmol) was dissolved in tetrahydrofuran (10 mL), then 4-fluoro-3-methoxy-2,6-dimethylaniline (628 mg, 3.72 mmol) was added, and lithium bis(trimethylsilyl)amide (7.4 mL, 7.44 mmol) was added dropwise, and the reaction mixture was stirred at room temperature for 0.5 hours. After LCMS monitoring showed that the raw material disappeared, the reaction mixture was added with ammonium chloride solution (20 mL), stirred for 10 minutes, and extracted with ethyl acetate (40 mL × 2). The organic phases were combined, first washed with saturated brine (50 mL), and then dried over anhydrous sodium sulfate. Finally, the resulting residue obtained by concentrating under reduced pressure was purified by silica gel column chromatography to obtain 3,5-dibromo-*N-*(4-fluoro-3-methoxy-2,6-dimethylphenyl)-6-methylpyridin-2-amine (1.2 g). MS (ESI) m/z: 418.6 [M+H⁺].

Malononitrile (189.5 mg, 2.89 mmol) was dissolved in dioxane (10 mL), then sodium tert-butoxide (331 mg, 3.44 mmol) was added, and the reaction system was replaced with nitrogen, and the reaction was carried out at room temperature for 30 minutes. 3,5-Dibromo-N-(4-fluoro-3-methoxy-2,6-dimethylphenyl)-6-methylpyridin-2-amine (1.2 g, 2.87 mmol) and [1,1'-bis(diphenylphosphino)ferrocene]dichloropalladium(II) (234 mg, 0.29 mmol) were added, and the reaction system was replaced with nitrogen, and the reaction was carried out at 120°C for 3 hours. After LCMS monitoring showed that the raw material disappeared, the reaction mixture was added with water (10 mL) and extracted with ethyl acetate (20 mL × 2). The organic phases were combined, washed with saturated brine (10 mL), and then dried over anhydrous sodium sulfate. Finally, the resulting residue obtained by concentrating under reduced pressure was purified by silica gel column chromatography to obtain 2-amino-5-bromo-1-(4-fluoro-3-methoxy-2,6-dimethylphenyl)-6-methyl-1*H*-pyrrolo[2,3-*b*]pyridine-3-carbonitrile (499 mg). MS (ESI) m/z: 402.85 [M+H⁺].

2-Amino-5-bromo-1-(4-fluoro-3-methoxy-2,6-dimethylphenyl)-6-methyl-1*H-*pyrrolo[2,3-*b*]pyridine-3-carbonitrile (400 mg, 1 mmol) was dissolved in sulfuric acid/water (3 mL), and methanesulfonic acid (3.5 g, 36.8 mmol) was added. The reaction was carried out at room temperature for 1.5 hours, and methionine (594 mg, 3.98 mmol) was added. The reaction was carried out at 40°C overnight. After LCMS monitoring showed that the raw material disappeared, the reaction mixture was cooled, and the pH value was adjusted to neutral with sodium hydroxide and dipotassium hydrogen phosphate aqueous solution, and the mixture was extracted with ethyl acetate (20 mL × 2). The organic phase was dried, and concentrated to dryness to obtain a crude product. The crude product was purified to obtain 2-amino-5-bromo-1-(4-fluoro-3-hydroxy-2,6-dimethylphenyl)-6-methyl-1*H*-pyrrolo[2,3-*b*]pyridine-3-carboxamide (98.91 mg). ¹H NMR (400 MHz, DMSO-*d*₆) δ 9.63 (s, 1H), 8.25 (s, 1H), 7.16 - 7.06 (m, 3H), 6.82 (s, 2H), 2.39 (s, 3H), 1.76 (s, 3H), 1.71 (s, 3H). C₁₇H₁₆BrFN₄O₂. MS (ESI) M/Z: 407.9 [M+H⁺].

### Examples 58.1 and 58.2

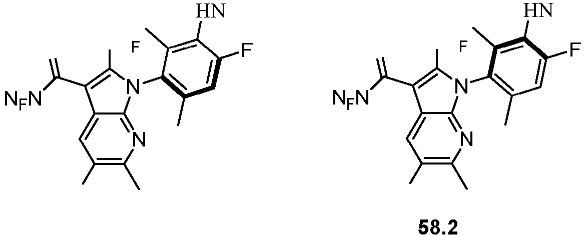

SFC method: instrument: SFC-150mgm (waters), chiral column: Daicel OZ-3 (25 * 250 mm,10 µm), temperature: 30°C, mobile phase: CO₂/MeOH [0.2% NH₃ (7M MeOH)] = 45/55, flow rate: 100 mL/min, back pressure: 100 bar, detection wavelength: 214 nm, cycle time: 4.84 min.

Example 58.1: 40.4 mg. SFC Rt = 1.448 min. [α]_{D}²⁰ = +35.7 ° (c = 0.10135, MeOH). ¹H NMR (400 MHz, DMSO) δ 9.59 (s, 1H), 8.25 (s, 1H), 7.11 (d, *J* = 11.5 Hz, 1H), 7.06 (s, 2H), 6.79 (s, 2H), 2.40 (s, 3H), 1.76 (s, 3H), 1.71 (s, 3H). MS (ESI) M/Z: 407.8 [M+H⁺].

Example 58.2: 39.8 mg. SFC Rt = 2.031 min. [α]_{D}²⁰ = -16.5° (c = 0.1033 g, MeOH). ¹H NMR (400 MHz, DMSO) δ 9.60 (s, 1H), 8.25 (s, 1H), 7.11 (d, *J* = 11.6 Hz, 1H), 7.06 (s, 2H), 6.79 (s, 2H), 2.39 (s, 3H), 1.76 (s, 3H), 1.71 (s, 3H). MS (ESI) M/Z: 407.8 [M+H⁺].

### Example 59

### 2-Amino-1-(4-fluoro-3-hydroxy-2,6-dimethylphenyl)-6-methyl-5-(trifluoromethyl)-1H-pyrrolo[2,3-b]pyridine-3-carboxamide

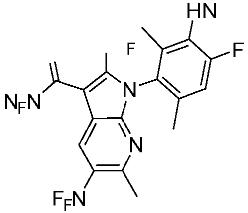

In a single-necked flask, 6-chloro-5-(trifluoromethyl)pyridin-2-amine (39 g, 0.19 mol) was dissolved in dioxane/water (10: 1), and then methylboric acid (23.4 g, 0.39 mol), potassium carbonate (82 g, 0.59 mol), and PdCl₂(dppf) (8 g, 0.011 mol) were added, and the reaction system was replaced with a nitrogen system, and stirred at 110°C for 16 hours. LCMS detected that the raw material disappeared and the product was generated. Pure water was added to the reaction mixture to quench. The mixture was extracted with ethyl acetate (700 mL × 3). The organic phases were combined, washed with saturated brine (100 mL × 3), dried over anhydrous sodium sulfate, filtered, and concentrated under reduced pressure. The resulting mixture was purified by silica gel column chromatography to obtain 6-methyl-5-(trifluoromethyl)pyridin-2-amine (24 g).

In a single-necked flask, 6-methyl-5-(trifluoromethyl)pyridin-2-amine (24 g, 0.14 mol) was dissolved in acetonitrile (300 mL), and *N*-bromosuccinimide (29.13 g, 0.164 mol) was added in batches under an ice bath. The reaction system was stirred at room temperature for 16 hours. LCMS detected that the raw material disappeared and the product was generated, and the reaction mixture was poured into 500 mL of water. The reaction mixture was extracted with ethyl acetate (400 mL × 3). The organic phases were combined, washed with saturated brine (100 mL × 3), dried over anhydrous sodium sulfate, filtered, concentrated under reduced pressure, and the resulting mixture was purified by silica gel column chromatography to obtain 3-bromo-6-methyl-5-(trifluoromethyl)pyridin-2-amine (26.3 g). MS (ESI) m/z: 257.0 [M+H⁺]. ¹H NMR (400 MHz, CDCl₃) δ 8.04 (s, 1H), 5.30 (s, 1H,-NH₂), 2.73 - 2.59 (m, 3H).

In a three-necked flask, 3-bromo-6-methyl-5-(trifluoromethyl)pyridin-2-amine (26.3 g, 0.103 mol) was dissolved in bromoform, and bromine (19.9 g, 0.124 mol) was slowly added dropwise at 0°C. Then, *tert*-butyl nitrite (32.61 g, 0.31 mol) was slowly added dropwise to the reaction mixture at 0°C. The mixture was reacted at room temperature for 3 hours. LCMS detected that the raw material disappeared and the product was generated. The reaction mixture was quenched by adding ice water, and the organic phase was washed three times with saturated sodium bicarbonate solution. The resulting organic phase was concentrated under reduced pressure using an oil pump to remove bromoform. The resulting mixture was purified by silica gel column chromatography to obtain 2,3-dibromo-6-methyl-5-(trifluoromethyl)pyridine (26.5 g). MS (ESI) m/z: 320.0 [M+H⁺].

In a single-necked flask, 2,3-dibromo-6-methyl-5-(trifluoromethyl)pyridine (15.1 g, 0.047 mol) was dissolved in ethylene glycol dimethyl ether (150 mL), and then 4-fluoro-3-methoxy-2,6-dimethylaniline (8.8 g, 0.053 mol), cesium carbonate (38.75 g, 0.12 mol), Pd₂dba₃ (4.34 g, 4.74 mmol), and XantPhos (5.48 g, 9.48 mmol) were added at room temperature. The reaction system was replaced with a nitrogen system and reacted at 110°C for 12 hours. LCMS detected that the raw material disappeared and the product was generated. Pure water was added to the reaction mixture to quench. The mixture was extracted with ethyl acetate (400 mL × 3). The organic phases were combined, washed with saturated brine (100 mL × 3), dried over anhydrous sodium sulfate, filtered, and concentrated under reduced pressure. The resulting mixture was purified by silica gel column chromatography to obtain 3-bromo-*N-*(4-fluoro-3-methoxy-2,6-dimethylphenyl)-6-methyl-5-(trifluoromethyl)pyridin-2-amine (10.5 g). MS (ESI) m/z: 407.1 [M+H]⁺.

In a 250 mL three-necked flask, sodium *tert*-butoxide (12.42 g, 129.3 mmol) was added, ethylene glycol dimethyl ether (30 mL) was added under nitrogen atmosphere, and then a solution of malononitrile (8.53 g, 129.3 mmol) in ethylene glycol dimethyl ether (5 mL) was added dropwise at room temperature. After the dropwise addition was completed, the reaction mixture was stirred at room temperature for 30 minutes. Then, under nitrogen atmosphere, a solution of 3-bromo-*N*-(4-fluoro-3-methoxy-2,6-dimethylphenyl)-6-methyl-5-(trifluoromethyl)pyridin-2-amine (10.5 g, 25.86 mmol) in ethylene glycol dimethyl ether (30 mL) and PdCl₂(dppf) (1.89 g, 2.586 mmol) were added. The reaction system was reacted at 110°C for 16 hours. LCMS detected the formation of products, and the reaction mixture was poured into ice water. The reaction mixture was extracted with ethyl acetate (300 mL × 3), and the organic phases were combined, washed with saturated brine (50 mL × 3), dried over anhydrous sodium sulfate, filtered, and concentrated under reduced pressure. The resulting mixture was purified by silica gel column chromatography to obtain 2-amino-1-(4-fluoro-3-methoxy-2,6-dimethylphenyl)-6-methyl-5-(trifluoromethyl)-1*H*-pyrrolo[2,3-*b*]pyridine-3-carbonitrile (5.7 g, yield of 56.3%). MS (ESI) m/z: 393.2 [M+H]⁺.

2-Amino-1-(4-fluoro-3-methoxy-2,6-dimethylphenyl)-6-methyl-5-(trifluoromethyl)-1H-pyrrolo[2,3-*b*]pyridine-3-carbonitrile (5.7 g, 14.55 mmol) was dissolved in concentrated sulfuric acid (50 mL) under an ice bath. The reaction mixture was stirred at room temperature for 2 hours. After LCMS monitoring showed that the raw material disappeared, the reaction mixture was poured into ice water, and then the pH value was adjusted to 8 with saturated sodium bicarbonate solution. The mixture was extracted with ethyl acetate (30 mL × 3). The organic phases were combined, first washed with saturated brine (50 mL × 3), then dried over anhydrous sodium sulfate, filtered, and finally concentrated under reduced pressure. The resulting mixture was purified by silica gel column chromatography to obtain 2-amino-1-(4-fluoro-3-methoxy-2,6-dimethylphenyl)-6-methyl-5-(trifluoromethyl)-1H-pyrrolo[2,3-*b*]pyridine-3-carboxamide (4.4 g). ¹H NMR (400 MHz, DMSO) δ 8.28 (s, 1H), 7.26 (d, *J =* 12.2 Hz, 1H), 7.19 (s, 2H), 6.94 (s, 2H), 3.87 (d, *J =* 1.0 Hz, 3H), 2.45 (d, *J =* 1.6 Hz, 3H), 1.82 (s, 3H), 1.78 (s, 3H). MS (ESI) M/Z: 411.3 [M+H]⁺.

In a 250 mL three-necked flask, 2-amino-1-(4-fluoro-3-methoxy-2,6-dimethylphenyl)-6-methyl-5-(trifluoromethyl)-1*H*-pyrrolo[2,3-*b*]pyridine-3-carboxamide (4.4 g, 10.7 mmol) and anhydrous dichloromethane (40 mL) were added. A solution of BBr₃ (1N, 107 mL) in dichloromethane was added dropwise under an ice bath, and the reaction mixture was stirred at room temperature for 2 hours. LCMS detected that the raw material disappeared and the product was generated. The reaction mixture was quenched with ice water, and the pH was adjusted to 9 with saturated sodium bicarbonate solution. The mixture was extracted with ethyl acetate (20 mL × 3), and the organic phases were combined. The organic phases were first washed with saturated brine (50 mL × 3), then dried over anhydrous sodium sulfate, filtered, and finally concentrated under reduced pressure. The resulting residue was purified by slurrying with acetonitrile to obtain 2-amino-1-(4-fluoro-3-hydroxy-2,6-dimethylphenyl)-6-methyl-5-(trifluoromethyl)-1*H*-pyrrolo[2,3-*b*]pyridine-3-carboxamide (3.1 g). ¹H NMR (400 MHz, DMSO) δ 9.63 (s, 1H), 8.27 (s, 1H), 7.19-7.07 (m, 3H), 6.93 (s, 2H), 2.45 (d, *J=* 1.5Hz, 3H), 1.77 (s, 3H), 1.72 (s, 3H). MS (ESI) M/Z: 397.1 [M+H⁺].

### Examples 59.1 and 59.2

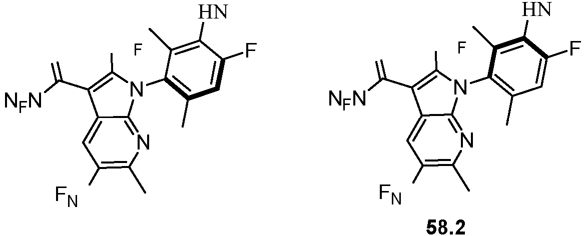

SFC method: instrument: SFC-150mgm (waters), chiral column: Daicel OZ (25 * 250 mm, 10 µm), temperature: 30°C, mobile phase: CO₂/MeOH [0.2% NH₃ (7M MeOH)] = 65/35, flow rate: 100 mL/min, back pressure: 100 bar, detection wavelength: 214 nm, cycle time: 10 min.

Example 59.1: 2.13 mg. SFC Rt = 1.770 min. [α]_{D}²⁰ = +42.4° (0.10018, MeOH). ¹H NMR (400 MHz, DMSO) δ 9.63 (s, 1H), 8.26 (s, 1H), 7.19 - 7.05 (m, 3H), 6.92 (s, 2H), 2.45 (d, *J* = 1.6 Hz, 3H), 1.77 (s, 3H), 1.72 (s, 3H). MS (ESI) M/Z: 397.1 [M+H⁺].

Example 59.2: 2.36 mg. SFC Rt = 2.480 min. [α]_{D}²⁰ = -26.0° (0.10100, MeOH). ¹H NMR (400 MHz, DMSO) δ 9.63 (s, 1H), 8.26 (s, 1H), 7.13 (t, *J =* 5.6 Hz, 3H), 6.92 (s, 2H), 2.45 (d, *J =* 1.6 Hz, 3H), 1.76 (s, 3H), 1.72 (s, 3H). MS (ESI) M/Z: 397.1 [M+H⁺].

### Example 120

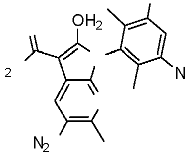

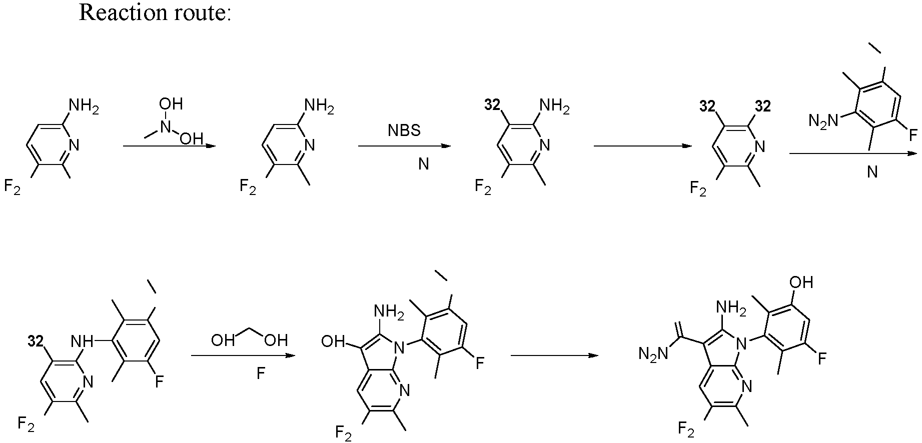

6-Chloro-5-(trifluoromethyl)pyridin-2-amine (1 g, 5.01 mmol) was dissolved in dioxane/water (10/1, 11 mL), then methylboric acid (910.6 mg, 15.2 mmol), potassium carbonate (2.1 g, 15.2 mmol), and [1,1'-bis(diphenylphosphino)ferrocene]dichloropalladium(II) (206.6 mg, 0.25 mmol) were added, and the reaction system was replaced with nitrogen for three times. The reaction mixture was stirred at 100°C overnight. After LCMS monitoring showed that the raw material disappeared, the reaction mixture was added with water (30 mL) and extracted with ethyl acetate (30 mL × 2). The organic phases were combined, first washed with saturated brine (30 mL), and then dried over anhydrous sodium sulfate. Finally, the resulting residue obtained by concentrating under reduced pressure was purified by silica gel column chromatography to obtain 6-methyl-5-(trifluoromethyl)pyridin-2-amine (710 mg). MS (ESI) m/z: 177.0 [M+H⁺].

6-Methyl-5-(trifluoromethyl)pyridin-2-amine (710 mg, 4.03 mmol) was dissolved in acetonitrile (8 mL), then *N*-bromosuccinimide (861.7 mg, 4.84 mmol) was added, and the reaction mixture was stirred at room temperature overnight. After LCMS monitoring showed that the raw material disappeared, sodium thiosulfate aqueous solution (20 mL) was added, and ethyl acetate (40 mL × 2) was added for extraction. The organic phases were combined, first washed with saturated brine (50 mL), and then dried over anhydrous sodium sulfate. Finally, the resulting residue obtained by concentrating under reduced pressure was purified by silica gel column chromatography to obtain 3-bromo-6-methyl-5-(trifluoromethyl)pyridin-2-amine (880 mg). MS (ESI) m/z: 254.7 [M+H⁺].

3-Bromo-6-methyl-5-(trifluoromethyl)pyridin-2-amine (580 mg, 2.28 mmol) was dissolved in bromoform (4 mL), and liquid bromine (301 mg, 1.9 mmol) was added. *tert-*Butyl nitrite (500 mg, 4.74 mmol) was added dropwise, and the reaction mixture was stirred at room temperature for 2 hours. After LCMS monitoring showed that the raw material disappeared, the reaction mixture was added with water (30 mL) and extracted with ethyl acetate (20 mL × 2). The organic phases were combined, first washed with saturated brine (20 mL), and then dried over anhydrous sodium sulfate. Finally, the resulting residue obtained by concentrating under reduced pressure was purified by silica gel column chromatography to obtain 2,3-dibromo-6-methyl-5-(trifluoromethyl)pyridine (280 mg). ¹H NMR (400 MHz, CDCl₃) δ 8.04 (s, 1H), 2.66 (s, 3H). C₇H₅BrF₃NO.

2,3-Dibromo-6-methyl-5-(trifluoromethyl)pyridine (280 mg, 0.92 mmol), 3-fluoro-5-methoxy-2,6-dimethylaniline (155 mg, 0.92 mmol), 4,5-bis(diphenylphosphino)-9,9-dimethylxanthene (48 mg, 0.083 mmol), cesium carbonate (748 mg, 2.3 mmol), and tris(dibenzylideneacetone)dipalladium (42 mg, 0.046 mmol) were added to ethylene glycol dimethyl ether (15 mL), and the reaction system was replaced with nitrogen for three times, and reacted at 90°C overnight. After LCMS monitoring showed that the raw material disappeared, the reaction mixture was added with water (30 mL) and extracted with ethyl acetate (20 mL × 2). The organic phases were combined, first washed with saturated brine (10 mL), and then dried over anhydrous sodium sulfate. Finally, the resulting residue obtained by concentrating under reduced pressure was purified by silica gel column chromatography to obtain 3-bromo-N-(3-fluoro-5-methoxy-2,6-dimethylphenyl)-6-methyl-5-(trifluoromethyl)pyridin-2-amine (40 mg). MS (ESI) m/z: 407.0 [M+H⁺].

Malononitrile (20 mg, 0.3 mmol) was dissolved in dioxane (5 mL), then sodium *tert-*butoxide (50 mg, 0.5 mmol) was added, and the reaction system was replaced with nitrogen, and the reaction was carried out at room temperature for 30 minutes. 3-Bromo-N-(3-fluoro-5-methoxy-2,6-dimethylphenyl)-6-methyl-5-(trifluoromethyl)pyridin-2-amine (40 mg, 0.1 mmol) and [1,1'-bis(diphenylphosphino)ferrocene]dichloropalladium(II) (8.15 mg, 0.01 mmol) were added, and the reaction system was replaced with nitrogen, and the reaction was carried out at 120°C for 3 hours. After LCMS monitoring showed that the raw material disappeared, the reaction mixture was added with water (10 mL) and extracted with ethyl acetate (20 mL × 2). The organic phases were combined, washed with saturated brine (10 mL), and then dried over anhydrous sodium sulfate. Finally, the resulting residue obtained by concentrating under reduced pressure was purified by silica gel column chromatography to obtain 2-amino-1-(3-fluoro-5-methoxy-2,6-dimethylphenyl)-6-methyl-5-(trifluoromethyl)-1*H*-pyrrolo[2,3-*b*]pyridine-3-carbonitrile (40 mg). MS (ESI) m/z: 393.2 [M+H⁺].

2-Amino-1-(3-fluoro-5-methoxy-2,6-dimethylphenyl)-6-methyl-5-(trifluoromethyl)-1*H*-pyrrolo[2,3-*b*]pyridine-3-carbonitrile (40 mg, 0.102 mmol) was dissolved in sulfuric acid/water (0.15 mL), and methanesulfonic acid (400 mg, 3.78 mmol) was added. The reaction was carried out at room temperature for 1.5 hours, and then methionine (61 mg, 0.41 mmol) was added. The reaction was carried out at 40°C overnight. After LCMS monitoring showed that the raw material disappeared, the reaction mixture was cooled, and the pH value was adjusted to neutral with sodium hydroxide and dipotassium hydrogen phosphate aqueous solution, and the mixture was extracted with ethyl acetate (20 mL × 2). The organic phase was dried, and concentrated to dryness to obtain a crude product. The crude product was purified to obtain 2-amino-1-(3-fluoro-5-hydroxy-2,6-dimethylphenyl)-6-methyl-5-(trifluoromethyl)-1*H*-pyrrolo[2,3-*b*]pyridine-3-carboxamide (2.27 mg). MS (ESI) m/z: 397.0 [M+H⁺]. ¹H NMR (400 MHz, DMSO-*d*₆) δ 10.09 (s, 1H), 8.28 (s, 1H), 7.18 (s, 2H), 6.96 (s, 2H), 6.83 (d, *J =* 11.1 Hz, 1H), 2.46 (s, 3H), 1.65 (d, *J =* 16.0 Hz, 6H).

### Example 189

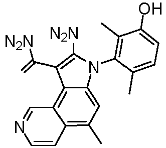

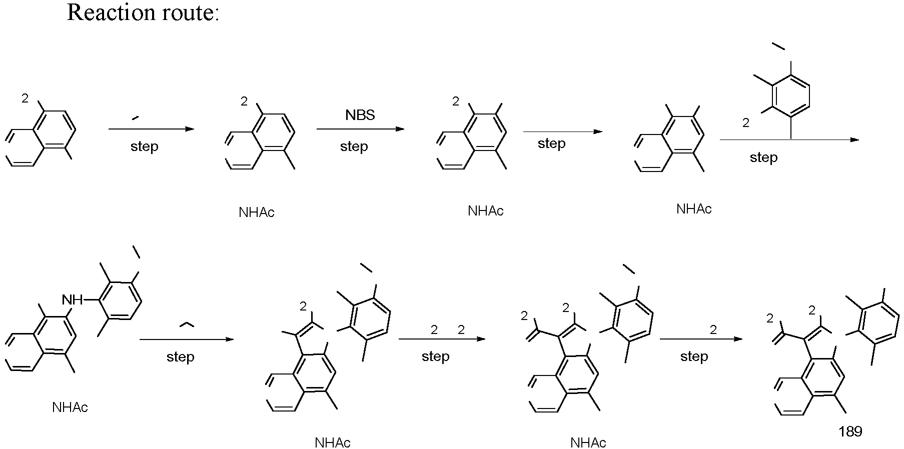

5-Bromoisoquinolin-8-amine (5.0 g, 22.4 mmol), methylboronic acid (4.0 g, 67.2 mmol), and potassium carbonate (9.27 g, 67.2 mmol) were dissolved in dioxane (100 mL) and water (10 mL), and [1,1'-bis(diphenylphosphino)ferrocene]dichloropalladium(II) (900 mg, 1.1 mmol) was added. The reaction system was replaced with nitrogen for three times, and the reaction mixture was stirred at 100°C overnight. After TLC monitoring showed that the raw material disappeared, the reaction mixture was added with water (100 mL) and extracted with ethyl acetate (100 mL × 2). The organic phases were combined, first washed with saturated brine (80 mL), and then dried over anhydrous sodium sulfate. Finally, the resulting residue obtained by concentrating under reduced pressure was purified by silica gel column chromatography to obtain 5-methylisoquinolin-8-amine (3.5 g). MS (ESI) m/z: 158.7 [M+H⁺].

5-Methylisoquinolin-8-amine (3.5 g, 27.2 mmol) was dissolved in acetonitrile (80 mL), and N-bromosuccinimide (4.85 g, 27.2 mmol) was added. The reaction mixture was stirred at room temperature overnight. After TLC monitoring showed that the raw material disappeared, the reaction mixture was added with water (100 mL) and extracted with ethyl acetate (100 mL × 2). The organic phases were combined, first washed with saturated brine (50 mL), and then dried over anhydrous sodium sulfate. Finally, the resulting residue obtained by concentrating under reduced pressure was purified by silica gel column chromatography to obtain 7-bromo-5-methylisoquinolin-8-amine (3.2 g). MS (ESI) m/z: 237.2 [M+H⁺]. ¹H NMR (400 MHz, CDCl₃) δ 9.33 (s, 1H), 8.56 (d, *J =* 5.9 Hz, 1H), 7.67 (d, *J=* 5.9 Hz, 1H), 7.54 (s, 1H), 4.80 (s, 2H), 2.53 (s, 3H). C₁₀H₉BrN₂.

7-Bromo-5-methylisoquinolin-8-amine (1.5 g, 6.3 mmol) was dissolved in hydrochloric acid (37%, 7 mL) and water (1.8 mL), cooled to -15°C, and a solution of sodium nitrite (480 mg, 6.9 mmol) in water (8.4 mL) was added dropwise. After the addition was completed, the reaction mixture was stirred at 0°C for 30 minutes. The reaction mixture was slowly added dropwise to a solution of potassium iodide (9.4 g, 56.7 mmol) in water (84 mL). After the addition was completed, the mixture was stirred at room temperature overnight. After TLC monitoring showed that the raw material disappeared, the mixture was cooled, quenched by adding saturated sodium sulfite solution (30 mL) and saturated sodium bicarbonate solution (30 mL), and extracted with dichloromethane (50 mL × 2). The organic phase was washed with brine, dried over sodium sulfate, and concentrated to dryness to obtain a crude product. Finally, the resulting residue obtained by concentrating under reduced pressure was purified by silica gel column chromatography to obtain 7-bromo-8-chloro-5-methylisoquinoline (1.1 g). ¹H NMR (400 MHz, CDCl₃) δ 9.55 (s, 1H), 8.65 (d, *J* = 5.8 Hz, 1H), 7.68 (d, *J* = 5.9 Hz, 1H), 7.62 (s, 1H), 2.65 (s, 3H).

7-Bromo-8-chloro-5-methylisoquinoline (1.1 g, 4.26 mmol), 3-methoxy-2,6-dimethylaniline (600 mg, 4.26 mmol), 4,5-bis(diphenylphosphino)-9,9-dimethylxanthene (240 mg, 0.43 mmol), and cesium carbonate (3.4 g, 10.6 mmol) were dissolved in toluene (22 mL), and tris(dibenzylideneacetone)dipalladium (180 mg, 0.2 mmol) was added. The reaction mixture was replaced with nitrogen for three times, and the reaction was carried out at 100°C overnight. After TLC monitoring showed that the raw material disappeared, the reaction mixture was cooled, added with water (50 mL), and extracted with ethyl acetate (20 mL × 2). The organic phase was washed with brine, dried over sodium sulfate, and concentrated to dryness to obtain a crude product. Finally, the resulting residue obtained by concentrating under reduced pressure was purified by silica gel column chromatography to obtain 8-chloro-*N*-(3-methoxy-2,6-dimethylphenyl)-5-methylisoquinolin-7-amine (1.3 g). MS (ESI) m/z: 327.39 [M+H⁺].

Malononitrile (728.83 mg, 11.04 mmol) was dissolved in *N,N*-dimethylformamide (15 mL), then sodium *tert*-butoxide (1.59 g, 16.56 mmol) was added, and the reaction system was replaced with nitrogen, and the reaction was carried out at room temperature for 30 minutes. 8-Chloro-*N*-(3-methoxy-2,6-dimethylphenyl)-5-methylisoquinolin-7-amine (900 mg, 2.76 mmol) and [1,1'-bis(diphenylphosphino)ferrocene]dichloropalladium(II) (225 mg, 0.28 mmol) were added, and the reaction system was replaced with nitrogen, and the reaction was carried out at 130°C for 3 hours. After LCMS monitoring showed that the raw material disappeared, the reaction mixture was added with water (30 mL) and extracted with ethyl acetate (20 mL × 2). The organic phases were combined, washed with saturated brine (30 mL), and then dried over anhydrous sodium sulfate. Finally, the resulting residue obtained by concentrating under reduced pressure was purified by silica gel column chromatography to obtain 8-amino-7-(3-methoxy-2,6-dimethylphenyl)-5-methyl-7*H*-pyrrolo[2,3-*h*]isoquinoline-9-carbonitrile (420 mg). MS (ESI) m/z: 357.39 [M+H⁺].

8-Amino-7-(3-methoxy-2,6-dimethylphenyl)-5-methyl-7*H*-pyrrolo[2,3-*h*]isoquinoline-9-carbonitrile (420 mg, 1.18 mmol) was dissolved in sulfuric acid (5 mL) and reacted at room temperature for 1 hour. After TLC monitoring showed that the raw material disappeared, the reaction mixture was cooled, and the pH value was adjusted to neutral with sodium hydroxide aqueous solution. The mixture was extracted with ethyl acetate (30 mL × 2), and the organic phase was dried, concentrated to dryness to obtain a crude product, and finally concentrated under reduced pressure to obtain 8-amino-7-(3-methoxy-2,6-dimethylphenyl)-5-methyl-7*H*-pyrrolo[2,3-*h*]isoquinoline-9-carboxamide (500 mg).

8-Amino-7-(3-methoxy-2,6-dimethylphenyl)-S-methyl-7*H*-pyrrolo[2,3-h]isoquinoline-9-carboxamide (500 mg, 1.37 mmol) was dissolved in dichloromethane (20 mL), and a 2M dichloromethane solution of boron tribromide (4 mL, 8.0 mmol) was added, and the reaction mixture was stirred at room temperature overnight. After LCMS monitoring showed that the raw material disappeared, sodium bicarbonate aqueous solution (50 mL) was slowly added. The mixture was extracted with ethyl acetate, washed with saturated brine, dried over sodium sulfate, and filtered. Finally, the resulting residue obtained by concentrating under reduced pressure was purified to obtain 8-amino-7-(3-hydroxy-2,6-dimethylphenyl)-S-methyl-7*H*-pyrrolo[2,3-*h*]isoquinoline-9-carboxamide (3.36 + 52.84 mg). MS (ESI) m/z: 361.44 [M+H⁺]. ¹H NMR (400 MHz, DMSO-*d*₆) δ 9.79 (s, 1H), 8.26 - 8.16 (m, 2H), 8.15 (s, 1H), 7.76 (d, *J =* 5.9 Hz, 1H), 7.23 (d, *J =* 8.3 Hz, 1H), 7.07 (d, *J =* 8.2 Hz, 1H), 6.93 (s, 2H), 6.53 (s, 2H), 2.67 (s, 3H), 1.77 (s, 3H), 1.68 (s, 3H).

The preparation of the following compounds refers to the preparation method of the above examples.

| Example number | Structure | Characterization data |
|---|---|---|
| 60 | 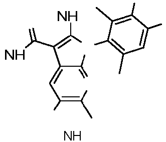 | 20.3 mg |
| | | ¹H NMR (400 MHz, DMSO-*d₆*) δ 9.60 (s, 1H), 8.11 (s, 1H), 7.14 - 7.02 (m, 3H), 6.79 (s, 2H), 2.36 (s, 3H), 1.76 (s, 3H), 1.71 (s, 3H). |
| | | MS (ESI) m/z: 363.1 [M+H⁺] |
| 60.1 | 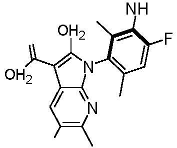 | 54.38 mg |
| | | SFC Rt = 0.866 min |
| | | (Rt represents retention time) |
| | | Optical rotation: -48.4° (c = 0.10600, MeOH) |
| | | ¹H NMR (400 MHz, DMSO-*d₆*) δ 9.61 (d, *J* = 1.3 Hz, 1H), 8.11 (s, 1H), 7.12 (d, *J =* 11.5 Hz, 1H), 7.05 (d, *J =* 9.6 Hz, 2H), 6.79 (s, 2H), 2.36 (s, 3H), 1.76 (s, 3H), 1.71 (s, 3H). |
| | | MS (ESI) m/z: 363.1 [M+H⁺] |
| 60.2 | 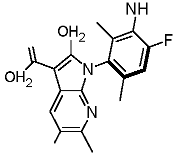 | 51.43 mg |
| | | SFC Rt = 1.008 min |
| | | [a]_{D}²⁰ = +37.3° (c=0.10250, MeOH) |
| | | ¹H NMR (400 MHz, DMSO-*d₆*) δ 9.61 (s, 1H), 8.11 (s, 1H), 7.12 (d, *J =* 11.4 Hz, 1H), 7.05 (d, *J =* 9.3 Hz, 2H), 6.79 (s, 2H), 2.36 (s, 3H), 1.76 (s, 3H), 1.71 (s, 3H). |
| | | MS (ESI) m/z: 363.1 [M+H⁺] |
| The chiral separation method of Example 60.1 and Example 60.2 is as follows: SFC method: instrument: SFC-150mgm (waters), chiral column: Daicel OZ-3 (25 * 250 mm, 10 µm), temperature: 30°C, mobile phase: CO₂/MeOH [0.2% NH₃ (7M MeOH)] = 45/55, flow rate: 100 mL/min, back pressure: 100 bar, detection wavelength: 214 nm, cycle time: 4.84 min. | | |
| 42.1 | 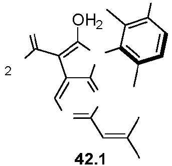 | 59.12 mg |
| | | SFC Rt = 1.292 min |
| | | [a]_{D}²⁰ = +22.87° (c=0.10015, MeOH) |
| | | ¹H NMR (400 MHz, DMSO-*d₆*) δ 9.55 (s, 1H), 8.95 (s, 1H), 7.12 (s, 2H), 7.08 (d, J = 8.3 Hz, 1H), 6.93 (d, J = 8.3 Hz, 1H), 6.85 (s, 2H), 6.18 - 6.14 (m, 1H), 2.07 (d, J = 0.9 Hz, 3H), 1.84 (s, 3H), 1.77 (s, 3H), 1.68 (s, 3H). |
| | | MS (ESI) m/z: 352.2 [M+H⁺] |
| 42.2 | 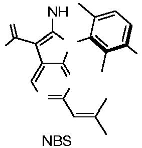 | 51.62 mg |
| | | SFC Rt = 1.854 min |
| | | [a]_{D}²⁰ =+0.59° (c=0.10095, MeOH) |
| | | ¹H NMR (400 MHz, DMSO-*d₆*) δ 9.55 (s, 1H), 8.95 (s, 1H), 7.14 (s, 2H), 7.08 (d, J = 8.2 Hz, 1H), 6.93 (d, J = 8.3 Hz, 1H), 6.86 (s, 2H), 6.17 (s, 1H), 2.06 (s, 3H), 1.84 (s, 3H), 1.77 (s, 3H), 1.68 (s, 3H) |
| | | MS (ESI) m/z: 352.2 [M+H⁺] |
| The chiral separation method of Example 42.1 and Example 42.2 is as follows: SFC method-instrument: SFC-150mgm (waters), chiral column: Daicel OZ (25 * 250 mm,10 µm), temperature: 30°C, mobile phase: CO₂/IPA [0.5% NH₃ (7M MeOH)] = 60/40, flow rate: 100 mL/min, back pressure: 100 bar, detection wavelength: 214 nm, cycle time: 13 min. | | |
| 19.1 | 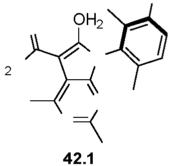 | 25.09 mg |
| | | SFC Rt = 3.641 min |
| | | [a]_{D}²⁰= -14.16° (c= 0.09180, MeOH) |
| | | ¹H NMR (400 MHz, DMSO-*d₆*) δ 9.55 (s, 1H), 7.06 (d, J = 8.3 Hz, 1H), 6.92 (d, J = 8.3 Hz, 1H), 6.85 (s, 2H), 6.46 (d, J = 9.8 Hz, 2H), 2.70 (s, 3H), 2.38 (s, 3H), 1.75 (s, 3H), 1.66 (s, 3H). |
| | | MS (ESI) m/z: 326.2 [M+H⁺] |
| 19.2 | 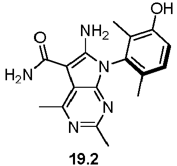 | 23.22 mg |
| | | SFC Rt = 4.096 |
| | | [a]_{D}²⁰= +24.5° (c= 0.09180, MeOH) |
| | | 1H NMR (400 MHz, DMSO-*d₆*) δ 9.58 (s, 1H), 7.06 (d, J = 8.3 Hz, 1H), 6.92 (d, J = 8.3 Hz, 1H), 6.85 (s, 2H), 6.47 (s, 2H), 2.70 (s, 3H), 2.38 (s, 3H), 1.75 (s, 3H), 1.66 (s, 3H). |
| | | MS(ESI) M/Z: 326.2 [M+H⁺] |
| The chiral separation method of Example 19.1 and Example 19.2 is as follows: | | |
| SFC method-instrument: SFC-150mgm (waters), chiral column: YMC Cellulose-SC (20 * 250 mm, 5 µm), temperature: 30°C, mobile phase: CO₂/MeOH [0.2% NH₃ (7M MeOH)] = 75/25, flow rate: 50 mL/min, back pressure: 100 bar, detection wavelength: 214 nm, cycle time: 16 min. | | |
| 61 | 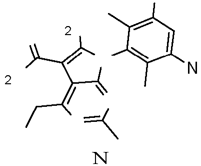 | 6.44 mg |
| | | ¹H NMR (400 MHz, DMSO-*d₆*) δ 10.05 (s, 1H), 6.89 (s, 2H), 6.81 (d, *J =* 11.1 Hz, 1H), 6.46 (s, 2H), 3.15 - 3.08 (m, 2H), 2.41 (s, 3H), 1.68 - 1.60 (m, 6H), 1.21 (t, *J* = 7.5 Hz, 3H). |
| | | MS (ESI) m/z: 358.1 [M+H⁺] |
| 62 | 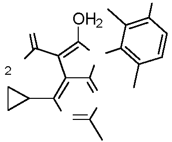 | 18.95 mg |
| | | ¹H NMR (400 MHz, DMSO-*d₆*) δ 9.54 (s, 1H), 7.06 (d, J = 8.3 Hz, 1H), 6.92 (d, J = 8.3 Hz, 1H), 6.85 (s, 2H), 6.46 (s, 2H), 2.76 (s, 1H), 2.33 (s, 3H), 1.76 (s, 3H), 1.66 (s, 3H), 1.19 - 1.15 (m, 2H), 1.02 - 0.97 (m, 2H). |
| | | MS (ESI) m/z: 352.2 [M+H⁺] |
| 62.1 | 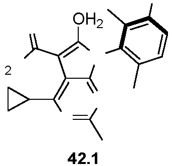 | 2.2 mg |
| | | SFC Rt = 0.910 min |
| | | ¹H NMR (400 MHz, DMSO-*d₆*) δ 9.54 (s, 1H), 7.06 (d, J = 8.3 Hz, 1H), 6.92 (d, J = 8.3 Hz, 1H), 6.85 (s, 2H), 6.46 (s, 2H), 2.82 - 2.68 (m, 1H), 2.33 (s, 3H), 1.76 (s, 3H), 1.66 (s, 3H), 1.20 - 1.15 (m, 2H), 1.01-0.94 (m, 2H). |
| | | MS (ESI) m/z: 352.2 [M+H⁺] |
| 62.2 | 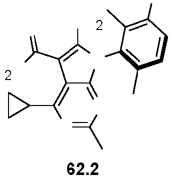 | 2.02 mg |
| | | SFC Rt = 1.029 min |
| | | ¹H NMR (400 MHz, DMSO-*d₆*) δ 9.54 (s, 1H), 7.06 (d, J = 8.3 Hz, 1H), 6.92 (d, J = 8.3 Hz, 1H), 6.85 (s, 2H), 6.46 (s, 2H), 2.82 - 2.68 (m, 1H), 2.33 (s, 3H), 1.76 (s, 3H), 1.66 (s, 3H), 1.20 - 1.15 (m, 2H), 1.01-0.94 (m, 2H). |
| | | MS (ESI) m/z: 352.2 [M+H⁺] |
| The chiral separation method of Example 62.1 and Example 62.2 is as follows: | | |
| SFC method-instrument: SFC-150mgm (waters), chiral column: Daicel IC-3 (25 * 250 mm, 10 µm), temperature: 30°C, mobile phase: CO₂/EtOH [0.5% NH₃ (7M MeOH)] = 60/40, flow rate: 100 mL/min, back pressure: 100 bar, detection wavelength: 214 nm, cycle time: 7 min. | | |
| 63 | 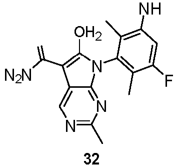 | 38.4 mg |
| | | ¹H NMR (400 MHz, DMSO-*d₆*) δ 10.08 (s, 1H), 8.89 (s, 1H), 7.16 (s, 2H), 6.92 - 6.77 (m, 3H), 2.44 (s, 3H), 1.66 (d, J = 1.6 Hz, 3H), 1.62 (s, 3H). |
| | | MS (ESI) m/z: 330.4 [M+H⁺] |
| 63.1 | 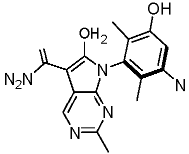 | 22.88 mg |
| | | SFC Rt = 0.821 min |
| | | [a]_{D}²⁰= +25.74° (c= 0.10064, MeOH) |
| | | ¹H NMR (400 MHz, Methanol-*d₄*) δ 8.81 (s, 1H), 6.80 (d, J = 10.8 Hz, 1H), 2.58 (s, 3H), 1.86 - 1.71 (m, 6H). |
| | | MS (ESI) m/z: 330.4 [M+H⁺] |
| 63.2 | 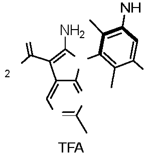 | 25.19 mg |
| | | SFC Rt = 0.936 min |
| | | [a]_{D}²⁰ = -12.65° (c= 0.0972, MeOH) |
| | | ¹H NMR (400 MHz, Methanol-*d₄*) δ 8.78 (s, 1H), 6.77 (d, J = 10.8 Hz, 1H), 2.56 (s, 3H), 1.78 - 1.74 (m, 6H). |
| | | MS (ESI) m/z: 330.4 [M+H⁺] |
| The chiral separation method of Example 63.1 and Example 63.2 is as follows: | | |
| SFC method-instrument: SFC-150mgm (waters), chiral column: Daicel OJ (25 * 250 mm, 10 µm), temperature: 30°C, mobile phase: CO₂/MeOH [0.2% NH₃ (7M MeOH)] = 80/20, flow rate: 100 mL/min, back pressure: 100 bar, detection wavelength: 214 nm, cycle time: 3.5 min. | | |
| 64 | 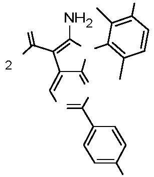 | 2.24 mg |
| | | H¹NMR: (400 MHz, DMSO-*d₆*) δ 9.64 (s, 1H), 9.08 (s, 1H), 8.20 - 8.12 (m, 2H), 7.28 - 7.18 (m, 4H), 7.13 (d, *J* = 8.3 Hz, 1H), 6.97 (d, *J* = 7.0 Hz, 3H), 1.81 (s, 3H), 1.72 (s, 3H). |
| | | MS (ESI) m/z: 392.2 [M+H⁺]. |
| 65 | 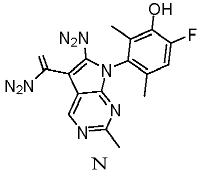 | 49.39 mg |
| | | ¹H NMR (400 MHz, DMSO-*d*₆) δ 9.60 (s, 1H), 8.88 (s, 1H), 7.21 - 7.03 (m, 3H), 6.84 (s, 2H), 2.44 (s, 3H), 1.77 (s, 3H), 1.72 (s, 3H). |
| | | MS (ESI) m/z: 330.0 [M+H⁺] |
| 66 | 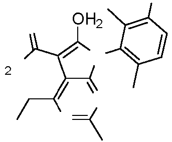 | 26.97 mg |
| | | ¹H NMR (400 MHz, Methanol-*d₄*) δ 7.11 (d, J = 8.3 Hz, 1H), 6.94 (d, J = 8.3 Hz, 1H), 3.25 (q, J = 7.6 Hz, 2H), 2.55 (s, 3H), 1.85 (s, 3H), 1.80 (s, 3H), 1.30 (t, J = 7.6 Hz, 3H). |
| | | MS (ESI) m/z: 340.1 [M+H⁺] |
| 66.1 | 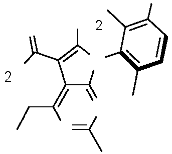 | 8.68 mg |
| | | SFC Rt = 1.199 min |
| | | ¹H NMR (400 MHz, Methanol-*d₄*) δ 7.13 (d, J = 8.3 Hz, 1H), 6.95 (d, J = 8.3 Hz, 1H), 3.24 (q, J = 7.6 Hz, 2H), 2.54 (s, 3H), 1.87 (s, 3H), 1.82 (s, 3H), 1.30 (t, J = 7.6 Hz, 3H). |
| | | MS (ESI) m/z: 340.1 [M+H⁺] |
| 66.2 | 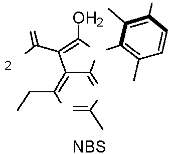 | 7.9 mg |
| | | SFC Rt = 1.064 min |
| | | ¹H NMR (400 MHz, Methanol-*d₄*) δ 7.13 (d, J = 8.3 Hz, 1H), 6.95 (d, J = 8.3 Hz, 1H), 3.24 (q, J = 7.6 Hz, 2H), 2.54 (s, 3H), 1.87 (s, 3H), 1.82 (s, 3H), 1.30 (t, J = 7.6 Hz, 3H). |
| | | MS (ESI) m/z: 340.1 [M+H⁺] |
| The chiral separation method of Example 66.1 and Example 66.2 is as follows: | | |
| SFC method-instrument: SFC-150mgm (waters), chiral column: Daicel IC (25 * 250 mm, 10 µm), temperature: 30°C, mobile phase: CO₂/MeOH [0.2% NH₃ (7M MeOH)] = 65/35, flow rate: 100 mL/min, back pressure: 100 bar, detection wavelength: 214 nm, cycle time: 5 min. | | |
| 67 | 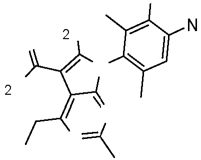 | 80.64 mg |
| | | ¹H NMR (400 MHz, DMSO-*d*₆) δ 9.50 (s, 1H), 7.11 (d, J = 11.4 Hz, 1H), 6.86 (s, 2H), 6.43 (s, 2H), 3.11 (q, J = 7.5 Hz, 2H), 2.40 (s, 3H), 1.76 (s, 3H), 1.71 (s, 3H), 1.21 (t, J = 7.5 Hz, 3H). |
| | | MS (ESI) m/z: 358.1 [M+H⁺] |
| 68 | 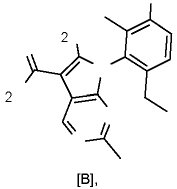 | 14.06 mg |
| | | ¹H NMR (400 MHz, DMSO-*d*₆) δ 9.59 (s, 1H), 8.87 (s, 1H), 7.11 (d, J = 8.4 Hz, 1H), 7.03 (s, 2H), 6.99 (d, J = 8.4 Hz, 1H), 6.83 (s, 2H), 2.43 (s, 3H), 2.11-1.99 (m, 2H), 1.64 (s, 3H), 0.90 (t, J = 7.6 Hz, 3H). |
| | | MS (ESI) m/z: 326.2 [M+H⁺] |
| 69 | 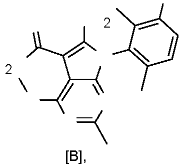 | 16.34 mg |
| | | ¹H NMR: (400 MHz, DMSO-*d*₆) δ 9.47 (s, 1H), 7.79 (s, 1H), 7.12 (d, *J* = 3.4 Hz, 1H), 7.02 (d, *J* = 8.3 Hz, 1H), 6.88 (d, *J* = 8.3 Hz, 1H), 6.78 (s, 2H), 3.66 (s, 3H), 2.50 (s, 3H), 1.78 (s, 3H), 1.70 (s, 3H). |
| | | MS(ESI)M/Z: 342.3 [M+H⁺] |
| 70 | 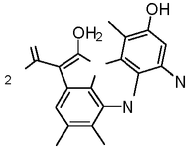 | 1.02 mg |
| | | ¹H NMR: (400 MHz, DMSO-*d*₆) δ 10.00 (s, 1H), 7.36 (s, 1H), 6.77 (d, *J =* 11.1 Hz, 1H), 6.68 (s, 4H), 2.27 (s, 3H), 2.04 (d, *J =* 2.1 Hz, 3H), 1.70 (s, 3H), 1.66 (s, 3H). |
| | | MS (ESI) m/z: 359.8 [M+H⁺]. |
| 71 | 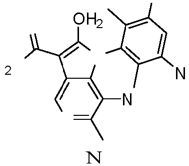 | 111.85 mg |
| | | ¹H NMR (400 MHz, DMSO-*d*₆) δ 8.64 (d, *J* = 1.6 Hz, 1H), 6.95 (s, 2H), 6.88 (s, 2H), 6.76 (d, *J* = 11.4 Hz, 1H), 2.34 (d, *J =* 3.2 Hz, 3H), 1.70 (s, 3H), 1.66 (s, 3H). |
| | | MS (ESI) m/z: 347.2 [M+H⁺] |
| 71.1 | 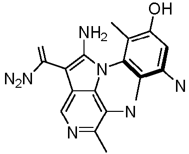 | 33.22 mg |
| | | SFC Rt = 1.206 min |
| | | [a]_{D}²⁰ = +31.4° (c=0.09306, MeOH) |
| | | ¹H NMR (400 MHz, DMSO-*d*₆) δ 10.09 (s, 1H), 8.64 (d, *J =* 1.4 Hz, 1H), 6.96 (s, 2H), 6.87 (s, 2H), 6.81 (d, *J =* 11.2 Hz, 1H), 2.34 (d, *J =* 3.2 Hz, 3H), 1.72 (d, *J =* 1.2 Hz, 3H), 1.67 (s, 3H). |
| | | MS (ESI) m/z: 347.2 [M+H⁺] |
| 71.2 | 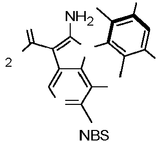 | 33 mg |
| | | SFC Rt = 1.595 min |
| | | [a]_{D}²⁰ = -25.5° (c=0.09517, MeOH) |
| | | ¹H NMR (400 MHz, DMSO-*d*₆) δ 10.10 (s, 1H), 8.64 (d, *J =* 1.4 Hz, 1H), 6.96 (s, 2H), 6.87 (s, 2H), 6.81 (d, *J =* 11.2 Hz, 1H), 2.34 (d, *J* = 3.2 Hz, 3H), 1.72 (d, *J =* 1.4 Hz, 3H), 1.67 (s, 3H). |
| | | MS (ESI) m/z: 347.2 [M+H⁺] |
| The chiral separation method of Example 71.1 and Example 71.2 is as follows: | | |
| SFC method-instrument: SFC-150mgm (waters), chiral column: Daicel OZ-3 (25 * 250 mm, 10 µm), temperature: 30°C, mobile phase: CO₂/MeOH [0.2% NH₃ (7M MeOH)] = 60/40, flow rate: 100 mL/min, back pressure: 100 bar, detection wavelength: 214 nm, cycle time: 6.5 min. | | |
| 72 | 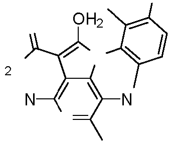 | 3.49 mg |
| | | ¹H NMR (400 MHz, DMSO-*d*₆) δ 9.63 (s, 1H), 7.09 - 7.01 (m, 3H), 6.93 (d, *J* = 8.4 Hz, 1H), 2.26 (d, *J* = 3.2 Hz, 3H), 1.81 (s, 3H), 1.73 (s, 3H). |
| | | MS (ESI) m/z: 347.0 [M+H⁺] |
| 73 | 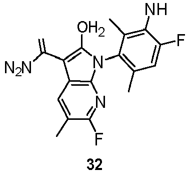 | 178.72 mg |
| | | ¹H NMR (400 MHz, Methanol-*d*₄) δ 7.94 (dd, *J* = 9.0, 0.8 Hz, 1H), 7.01 (d, *J* = 11.3 Hz, 1H), 2.33 (s, 3H), 1.85 (s, 3H), 1.83 (s, 3H). |
| | | MS (ESI) M/Z:347.4 [M+H⁺] |
| 74 | 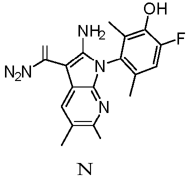 | 53.27 mg |
| | | ¹H NMR (400 MHz, DMSO-*d*₆) δ 9.63 (s, 1H), 8.08 (s, 1H), 7.13 (d, J *=* 11.5 Hz, 1H), 7.06 (s, 2H), 6.80 (s, 2H), 2.33 (s, 3H), 1.77 (s, 3H), 1.72 (s, 3H). |
| | | MS (ESI) m/z: 363.0 [M+H⁺] |
| 75 | 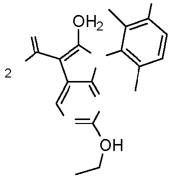 | 5.07 mg |
| | | ¹H NMR: (400 MHz, DMSO-*d₆*) δ 9.51 (s, 1H), 8.53 (s, 1H), 7.04 (d, *J* = 8.3 Hz, 1H), 6.88 (d, *J* = 8.2 Hz, 1H), 6.69 (s, 2H), 6.62 (s, 2H), 6.49 (t, *J =* 5.5 Hz, 1H), 3.22 - 3.10 (m, 2H), 1.80 (s, 3H), 1.71 (s, 3H), 1.03 (t, *J* = 7.1 Hz, 3H). |
| | | MS (ESI) m/z: 341.0 [M+H⁺]. |
| 76 | 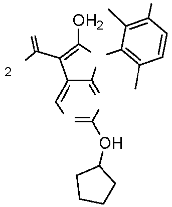 | 15.39 mg |
| | | ¹H NMR: (400 MHz, DMSO-*d*₆) δ 9.51 (s, 1H), 8.53 (s, 1H), 7.03 (d, *J* = 8.2 Hz, 1H), 6.88 (d, *J* = 8.2 Hz, 1H), 6.68 (s, 2H), 6.61 (s, 2H), 6.48 (d, *J* = 7.0 Hz, 1H), 4.03 (q, *J* = 6.8 Hz, 1H), 1.87 - 1.75 (m, 5H), 1.71 (s, 3H), 1.66 - 1.52 (m, 2H), 1.49 - 1.33 (m, 4H). |
| | | MS (ESI) m/z: 381.2 [M+H⁺]. |
| 77 | 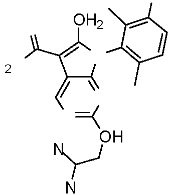 | 2.16 mg |
| | | ¹H NMR: (400 MHz, DMSO-*d*₆) δ 9.53 (s, 1H), 8.58 (s, 1H), 7.05 (d, *J* = 8.3 Hz, 1H), 6.97 (t, *J* = 6.0 Hz, 1H), 6.89 (d, *J* = 8.3 Hz, 1H), 6.79 (s, 2H), 6.68 (s, 2H), 6.19 - 5.84 (m, 1H), 3.53 (t, *J =* 15.2 Hz, 2H), 1.79 (s, 3H), 1.70 (s, 3H). |
| | | MS (ESI) m/z: 376.76 [M+H⁺]. |
| 78 | 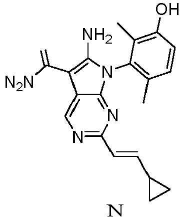 | 4.15 mg |
| | | ¹H NMR: (400 MHz, DMSO-*d*₆) δ 9.62 (s, 1H), 8.89 (s, 1H), 7.13 - 7.05 (m, 3H), 6.98 - 6.87 (m, 3H), 6.38 (d, *J=* 15.4 Hz, 1H), 6.29 (dd, *J=* 15.4, 9.2 Hz, 1H), 1.76 (s, 3H), 1.67 (s, 3H), 1.58 (tt, *J=* 8.3, 3.9 Hz, 1H), 0.85 - 0.75 (m, 2H), 0.57 - 0.48 (m, 2H). |
| | | MS (ESI) m/z: 364.2 [M+H⁺] |
| 79 | 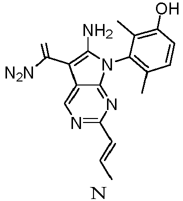 | 4.82 mg |
| | | ¹H NMR: (400 MHz, DMSO-*d*₆) δ 9.61 (s, 1H), 8.92 (s, 1H), 7.16 - 7.05 (m, 3H), 6.98 - 6.87 (m, 3H), 6.77 - 6.63 (m, 1H), 6.34 (dq, *J* = 15.4, 1.5 Hz, 1H), 1.85 - 1.79 (m, 3H), 1.76 (s, 3H), 1.68 (s, 3H). |
| | | MS (ESI) m/z: 338.2 [M+H⁺]. |
| 80 | 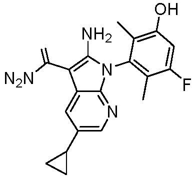 | 10 mg |
| | | ¹H NMR: (400 MHz, DMSO-*d*₆) δ 10.00 (s, 1H), 7.64 (d, *J* = 1.8 Hz, 1H), 7.56 (d, *J* = 2.0 Hz, 1H), 7.04 (s, 2H), 6.83 - 6.75 (m, 3H), 1.92 (ddd, *J* = 13.6, 8.6, 5.2 Hz, 1H), 1.64 (s, 3H), 1.61 (s, 3H), 0.96 - 0.82 (m, 2H), 0.85 - 0.76 (m, 2H). |
| | | MS (ESI) m/z: 355.0 [M+H⁺] |
| 81 | 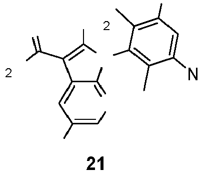 | 60 mg |
| | | ¹H NMR: (400 MHz, DMSO-*d*₆) δ 8.16 (d, *J* = 2.2 Hz, 1H), 7.76 (d, *J =* 2.1 Hz, 1H), 7.25 (s, 2H), 6.89 (s, 2H), 6.79 (d, *J* = 11.3 Hz, 1H), 1.65 (s, 3H), 1.61 (s, 3H). |
| | | MS (ESI) m/z: 349.4 [M+H⁺]. |
| 82 | 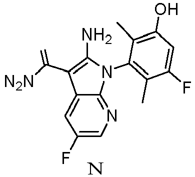 | 19.4 mg |
| | | ¹H NMR: (400 MHz, DMSO-*d*₆) δ 10.04 (s, 1H), 7.99 (dd, *J* = 10.5, 2.6 Hz, 1H), 7.71 (dd, *J* = 2.5, 1.4 Hz, 1H), 7.23 (s, 2H), 6.88 - 6.76 (m, 3H), 1.65 (s, 3H), 1.61 (s, 3H). |
| | | MS (ESI) m/z: 333.1 [M+H⁺]. |
| 83 | 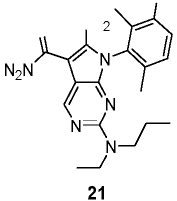 | 10.58 mg |
| | | ¹H NMR: (400 MHz, DMSO-*d*₆) δ 9.48 (s, 1H), 8.57 (s, 1H), 7.05 (d, *J* = 8.2 Hz, 1H), 6.89 (d, *J* = 8.2 Hz, 1H), 6.70 (s, 2H), 6.61 (s, 2H), 3.49 - 3.23 (m, 4H), 1.80 (s, 3H), 1.71 (s, 3H), 1.45 (h, *J* = 7.2 Hz, 2H), 0.98 (t, *J* = 6.9 Hz, 3H), 0.73 (t, *J =* 7.3 Hz, 3H). |
| | | MS (ESI) m/z: 383.25 [M+H⁺]. |
| 84 | 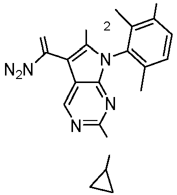 | 3.05 mg |
| | | ¹H NMR: (400 MHz, DMSO-*d*₆) δ 9.52 (s, 1H), 8.57 (s, 1H), 7.03 (d, *J* = 8.2 Hz, 1H), 6.88 (d, *J* = 8.3 Hz, 1H), 6.79 (d, *J* = 3.5 Hz, 1H), 6.72 (s, 2H), 6.65 (s, 2H), 2.61 (tq, *J =* 7.2, 3.7 Hz, 1H), 1.79 (s, 3H), 1.71 (s, 3H), 0.53 (dt, *J =* 6.6, 3.2 Hz, 2H), 0.39 - 0.30 (m, 2H). |
| | | MS (ESI) m/z: 353.3 [M+H⁺]. |
| 85 | 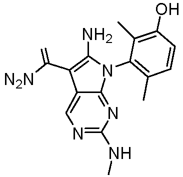 | 9.54 mg |
| | | ¹H NMR: (400 MHz, DMSO-*d*₆) δ 9.51 (s, 1H), 8.55 (s, 1H), 7.04 (d, *J* = 8.3 Hz, 1H), 6.88 (d, *J* = 8.2 Hz, 1H), 6.70 (s, 2H), 6.63 (s, 2H), 6.48 (q, *J =* 4.7 Hz, 1H), 2.68 (d, *J =* 4.7 Hz, 3H), 1.79 (s, 3H), 1.71 (s, 3H). |
| | | MS (ESI) m/z: 327.3 [M+H⁺]. |
| 86 | 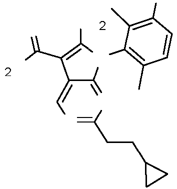 | 4.18 mg |
| | | ¹H NMR: (400 MHz, DMSO-*d*₆) δ 9.61 (s, 1H), 8.89 (s, 1H), 7.12 - 7.02 (m, 3H), 6.94 (d, *J =* 8.3 Hz, 1H), 6.85 (s, 2H), 2.76 (t, *J* = 7.6 Hz, 2H), 1.75 (s, 3H), 1.66 (s, 3H), 1.53 (q, *J* = 7.2 Hz, 2H), 0.69 - 0.58 (m, 1H), 0.33 - 0.24 (m, 2H), -0.03 - -0.12 (m, 2H). |
| | | MS (ESI) m/z: 366.3 [M+H⁺]. |
| 87 | 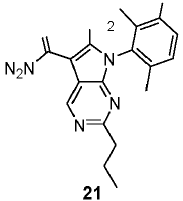 | 12.04 mg |
| | | ¹H NMR: (400 MHz, DMSO-*d*₆) δ 9.67 (s, 1H), 8.90 (s, 1H), 7.11 - 7.02 (m, 3H), 6.94 (d, *J* = 8.3 Hz, 1H), 6.85 (s, 2H), 2.65 (dd, *J =* 8.3, 6.7 Hz, 2H), 1.75 (s, 3H), 1.67 (s, 3H), 1.65 - 1.60 (m, 2H), 0.83 (t, *J =* 7.4 Hz, 3H). |
| | | MS (ESI) m/z: 340.2 [M+H⁺]. |
| 57.1 | 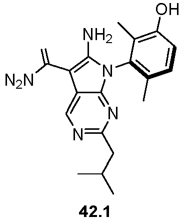 | 42.14 mg |
| | | SFC Rt = 0.934 min |
| | | [a]_{D}²⁰ = +41.32° (c=0.09970, MeOH) |
| | | ¹H NMR (400 MHz, DMSO-*d*₆) δ 9.58 (s, 1H), 8.89 (s, 1H), 7.08 (d, *J =* 8.4 Hz, 1H), 7.03 (s, 2H), 6.93 (d, *J =* 8.2 Hz, 1H), 6.83 (s, 2H), 2.55 (d, *J =* 7.4 Hz, 2H), 2.05-1.98 (m, 1H), 1.75 (s, 3H), 1.67 (s, 3H), 0.81 (s, 3H), 0.79 (s, 3H). MS (ESI) m/z: 354.2 [M+H⁺] |
| 57.2 | 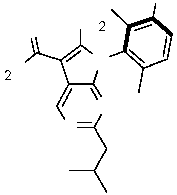 | 44.02 mg |
| | | SFC Rt =1.353 min |
| | | [a]_{D}²⁰ = -3.81° (c=0.09705, MeOH) |
| | | ¹H NMR (400 MHz, DMSO-*d*₆) δ 9.58 (s, 1H), 8.89 (s, 1H), 7.08 (d, *J =* 8.4 Hz, 1H), 7.04 (s, 2H), 6.93 (d, *J =* 8.4 Hz, 1H), 6.83 (s, 2H), 2.55 (d, *J =* 7.2 Hz, 2H), 2.05-1.98 (m, 1H), 1.75 (s, 3H), 1.67 (s, 3H), 0.81 (s, 3H), 0.79 (s, 3H). |
| | | MS (ESI) m/z: 354.2 [M+H⁺] |
| The chiral separation method of Example 57.1 and Example 57.2 is as follows: | | |
| SFC method-instrument: SFC-150mgm (waters), chiral column: Daicel OZ (25 * 250 mm, 10 µm), temperature: 30°C, mobile phase: CO₂/IPA [0.5% NH₃ (7M MeOH)] = 65/35, flow rate: 100 mL/min, back pressure: 100 bar, detection wavelength: 214 nm, cycle time: 5.71 min. | | |
| 88 | 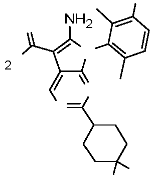 | 15.5 mg |
| | | ¹H NMR: (400 MHz, DMSO-*d*₆) δ 9.60 (s, 1H), 8.92 (s, 1H), 7.16 - 7.05 (m, 3H), 6.93 (d, *J* = 8.2 Hz, 1H), 6.87 (s, 2H), 2.87 - 2.75 (m, 1H), 2.07 - 1.78 (m, 8H), 1.75 (s, 3H), 1.67 (s, 3H). |
| | | MS (ESI) m/z: 416.14 [M+H⁺]. |
| 89 | 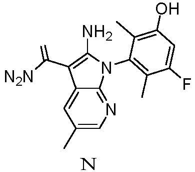 | 5 mg |
| | | ¹H NMR: (400 MHz, DMSO-*d*₆) δ 9.99 (s, 1H), 7.89 (d, *J* = 1.9 Hz, 1H), 7.60 (d, *J* = 1.8 Hz, 1H), 7.02 (s, 2H), 6.83 - 6.70 (m, 3H), 2.31 (s, 3H), 1.65 (s, 3H), 1.61 (s, 3H). |
| | | MS (ESI) m/z: 328.9 [M+H⁺]. |
| 90 | 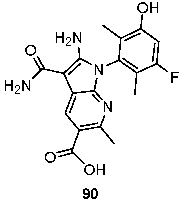 | 7.15 mg |
| | | ¹H NMR (400 MHz, Methanol-*d₄*) δ 8.42 (s, 1H), 6.76 (d, J = 10.9 Hz, 1H), 2.67 (s, 3H), 1.76 (d, J = 2.1 Hz, 6H). |
| | | MS (ESI) m/z: 373.0 [M+H⁺] |
| 91 | 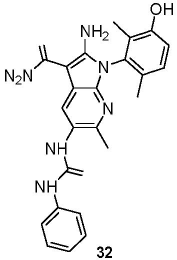 | 2.7 mg |
| | | ¹H NMR: (400 MHz, DMSO-*d*₆) δ 9.54 (s, 1H), 8.93 (s, 1H), 8.05 (s, 1H), 7.92 (s, 1H), 7.52 - 7.44 (m, 2H), 7.31 - 7.22 (m, 2H), 7.07 (d, *J* = 8.3 Hz, 1H), 6.99 - 6.88 (m, 2H), 6.79 (s, 2H), 6.62 (s, 2H), 2.26 (s, 3H), 1.78 (s, 3H), 1.69 (s, 3H). |
| | | MS (ESI)M/Z: 445.2 [M+H⁺] |
| 92 | 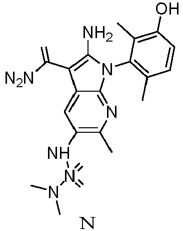 | 5.67 mg |
| | | ¹H NMR: (400 MHz, DMSO-*d*₆) δ 9.52 (s, 1H), 8.91 (s, 1H), 7.80 (s, 1H), 7.06 (d, *J =* 8.2 Hz, 1H), 6.91 (d, *J =* 8.3 Hz, 1H), 6.83 (s, 2H), 6.66 (s, 2H), 2.76 (s, 6H), 2.35 (s, 3H), 1.75 (s, 3H), 1.67 (s, 3H). |
| | | MS (ESI) m/z: 432.3 [M+H⁺] |
| 93 | 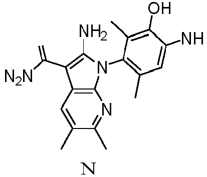 | 5.57 mg |
| | | ¹H NMR: (400 MHz, DMSO-*d*₆) δ 7.85 (s, 1H), 7.53 (s, 1H), 6.92 (s, 2H), 6.69 (s, 2H), 2.25 (d, *J* = 9.0 Hz, 6H), 1.76 (s, 3H), 1.73 (s, 3H). |
| | | MS (ESI) m/z: 350.1 [M+H⁺]. |
| 94 | 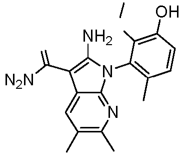 | 0.62 mg |
| | | ¹H NMR: (400 MHz, DMSO-*d*₆) δ 9.48 (s, 1H), 7.81 (s, 1H), 6.97 (d, *J* = 2.8 Hz, 2H), 6.81 (s, 2H), 6.64 (s, 2H), 3.48 (s, 3H), 2.26 (d, *J* = 7.2, 6H), 1.78 (s, 3H). |
| | | MS (ESI) m/z: 341.1 [M+H⁺]. |
| 95 | 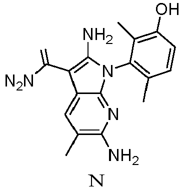 | 2.07 mg |
| | | ¹H NMR: (400 MHz, DMSO-*d*₆) δ 9.42 (s, 1H), 7.67 (s, 1H), 7.01 (d, *J* = 8.2 Hz, 1H), 6.86 (d, *J* = 8.2 Hz, 1H), 6.48 (s, 2H), 6.32 (s, 2H), 5.13 (s, 2H), 2.07 (s, 3H), 1.77 (s, 3H), 1.68 (s, 3H). |
| | | MS (ESI) m/z: 325.8 [M+H⁺] |
| 96 | 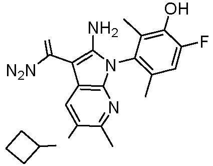 | 53.21 mg |
| | | ¹H NMR (400 MHz, DMSO-*d*₆) δ 9.52 (s, 1H), 7.43 (s, 1H), 7.09 (d, *J* = 11.4 Hz, 1H), 6.82 (s, 2H), 6.70 (s, 2H), 4.90 - 4.81 (m, 1H), 2.50 - 2.41 (m, 2H), 2.19 (s, 3H), 2.07 - 1.98 (m, 2H), 1.85 - 1.53 (m, 8H). |
| | | MS (ESI) M/Z: 398.8 [M+H⁺] |
| 97 | 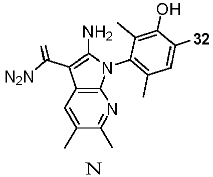 | 2.74 mg |
| | | ¹HNMR: (400 MHz, DMSO-*d*₆) δ 9.00 (s, 1H), 7.84 (s, 1H), 7.47 (s, 1H), 6.88 (s, 2H), 6.67 (s, 2H), 2.26 (s, 3H), 2.24 (s, 3H), 1.76 (s, 3H), 1.75 (s, 3H). |
| | | MS(ESI) M/Z: 402.5 [M+H⁺]. |
| 98 | 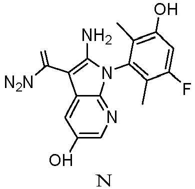 | 8.78 mg |
| | | ¹H NMR: (400 MHz, DMSO-*d*₆) δ 10.14 (s, 1H), 8.49 (d, *J* = 1.9 Hz, 1H), 8.22 (d, *J* = 1.8 Hz, 1H), 7.42 (s, 2H), 6.99 (s, 2H), 6.83 (d, *J* = 11.1 Hz, 1H), 1.65 (s, 3H), 1.61 (s, 3H). |
| | | MS (ESI) m/z: 340.1 [M+H⁺]. |
| 99 | 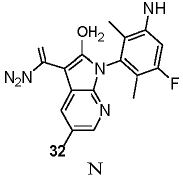 | 49.77 mg |
| | | ¹H NMR: (400 MHz, DMSO-*d*₆) δ 10.06 (s, 1H), 8.26 (d, *J* = 2.1 Hz, 1H), 7.83 (d, *J* = 2.0 Hz, 1H), 7.26 (s, 2H), 6.89 (s, 2H), 6.81 (d, *J* = 11.1 Hz, 1H), 1.65 (s, 3H), 1.61 (s, 3H). |
| | | MS (ESI) m/z: 393.14 [M+H⁺]. |
| 100 | 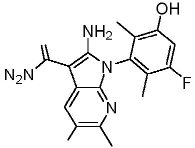 | 2.12 mg |
| | | ¹H NMR (400 MHz, DMSO-*d*₆) δ 10.00 (s, 1H), 8.13 (s, 1H), 7.10 (s, 2H), 6.88 - 6.72 (m, 3H), 2.37 (s, 3H), 1.66 (d, *J =* 1.4 Hz, 3H), 1.62 (s, 3H). |
| | | MS (ESI) m/z: 363.1 [M+H⁺] |
| 100.1 | 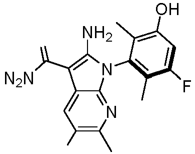 | 18.39 mg |
| | | SFC Rt = 1.153 min |
| | | [a]_{D}²⁰ = +29.25° (c= 0.0988, MeOH) |
| | | ¹H NMR (400 MHz, DMSO-*d*₆) δ 10.02 (s, 1H), 8.13 (s, 1H), 7.10 (s, 2H), 6.87 - 6.72 (m, 3H), 2.37 (s, 3H), 1.66 (d, *J =* 1.6 Hz, 3H), 1.62 (s, 3H). |
| | | MS (ESI) m/z: 363.6 [M+H⁺] |
| 100.2 | 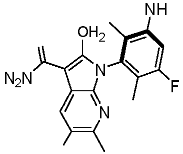 | 26.63 mg |
| | | SFC Rt = 1.633 min |
| | | [a]_{D}²⁰ = -32.26° (c= 0.09608, MeOH) |
| | | ¹H NMR (400 MHz, DMSO-*d*₆) δ 10.04 (s, 1H), 8.13 (s, 1H), 7.10 (s, 2H), 6.89 - 6.70 (m, 3H), 2.37 (s, 3H), 1.66 (d, *J =* 1.6 Hz, 3H), 1.62 (s, 3H). |
| | | MS (ESI) m/z: 363.6 [M+H⁺] |
| The chiral separation method of Example 100.1 and Example 100.2 is as follows: | | |
| SFC method-instrument: SFC-150mgm (waters), chiral column: Daicel OZ (25 * 250 mm, 10 µm), temperature: 30°C, mobile phase: CO₂/IPA [0.5% NH₃ (7M MeOH)] = 55/45, flow rate: 100 mL/min, back pressure: 100 bar, detection wavelength: 214 nm, cycle time: 7.5 min. | | |
| 101 | 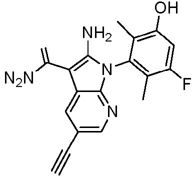 | 5.03 mg |
| | | ¹H NMR: (400 MHz, DMSO-*d*₆) δ 10.00 (s, 1H), 8.18 (d, *J* = 1.8 Hz, 1H), 7.90 (d, *J* = 1.7 Hz, 1H), 7.22 (s, 2H), 6.89 - 6.77 (m, 3H), 4.17 (s, 1H), 1.65 (s, 3H), 1.61 (s, 3H). |
| | | MS (ESI) m/z: 339.2 [M+H⁺]. |
| 102 | 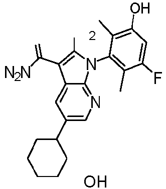 | 24.94 mg |
| | | ¹H NMR: (400 MHz, DMSO-*d*₆) δ 10.47 (s, 1H), 7.90 (d, *J* = 1.9 Hz, 1H), 7.63 (d, *J* = 1.8 Hz, 1H), 7.02 (s, 2H), 6.85 - 6.76 (m, 3H), 1.80 (d, *J =* 11.6 Hz, 4H), 1.76 - 1.50 (m, 10H), 1.40 (d, *J =* 12.4 Hz, 1H), 1.24-1.40 (m, *J =* 28.9, 13.9 Hz, 2H). |
| | | MS (ESI) m/z: 397.0 [M+H⁺]. |
| 103 | 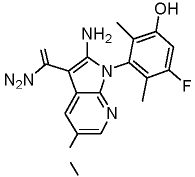 | 9.82 mg |
| | | ¹H NMR: (400 MHz, DMSO-*d*₆) δ 10.00 (s, 1H), 7.67 (d, *J* = 2.6 Hz, 1H), 7.48 (d, *J* = 2.5 Hz, 1H), 7.06 (s, 2H), 6.83 - 6.75 (m, 3H), 3.83 (s, 3H), 1.68 - 1.59 (m, 6H). |
| | | MS (ESI) m/z: 343.8 [M+H⁺] |
| 104 | 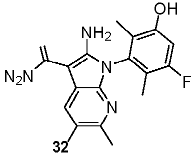 | 111.19 mg |
| | | ¹H NMR: (400 MHz, DMSO-*d*₆) δ 10.07 (s, 1H), 8.27 (s, 1H), 7.12 (s, 2H), 6.86 - 6.77 (m, 3H), 2.41 (s, 3H), 1.66 (s, 3H), 1.62 (s, 3H). |
| | | MS (ESI) m/z: 407.0 [M+H⁺] |
| 104.1 | 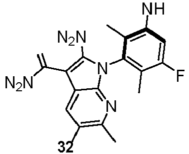 | 23.44 mg |
| | | SFC Rt = 1.456 min |
| | | Optical rotation: -48.6° (c=0.07475, MeOH) |
| | | ¹H NMR (400 MHz, DMSO-*d*₆) δ 10.08 (s, 1H), 8.26 (s, 1H), 7.10 (s, 2H), 6.85 - 6.71 (m, 3H), 2.40 (s, 3H), 1.66 (d, J = 1.6 Hz, 3H), 1.62 (s, 3H). |
| | | MS (ESI) m/z: 406.9 [M+H⁺] |
| 104.2 | 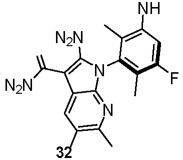 | 16.72 mg |
| | | SFC Rt = 1.634 min |
| | | [a]_{D}²⁰ = +32.6° (c=0.07735, MeOH) |
| | | ¹H NMR(400 MHz, DMSO-*d*₆) δ 10.04 (s, 1H), 8.26 (s, 1H), 7.10 (s, 2H), 6.87 - 6.70 (m, 3H), 2.40 (s, 3H), 1.66 (s, 3H), 1.62 (s, 3H). |
| | | MS (ESI) m/z: 406.9 [M+H⁺] |
| The chiral separation method of Example 104.1 and Example 104.2 is as follows: | | |
| SFC method-instrument: SFC-150mgm (waters), chiral column: Daicel AD (25 * 250 mm, 10 µm), temperature: 30°C, mobile phase: CO₂/EtOH [0.5% NH₃ (7M MeOH)] = 85/15, flow rate: 100 mL/min, back pressure: 100 bar, detection wavelength: 214 nm, cycle time: 12 min. | | |
| 105 | 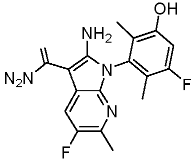 | 18.48 mg |
| | | ¹H NMR: (400 MHz, DMSO-*d*₆) δ 7.95 (d, *J* = 11.2 Hz, 1H), 7.07 (s, 2H), 6.84 - 6.76 (m, 3H), 2.27 (d, *J* = 3.1 Hz, 3H), 1.66 (s, 3H), 1.62 (s, 3H). |
| | | MS (ESI) m/z: 347.1 [M+H⁺]. |
| 106 | 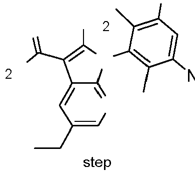 | 17.25 mg |
| | | ¹H NMR: (400 MHz, DMSO-*d*₆) δ 9.98 (s, 1H), 7.90 (d, *J* = 1.9 Hz, 1H), 7.62 (d, *J* = 1.8 Hz, 1H), 7.01 (s, 2H), 6.83 - 6.72 (m, 3H), 2.62 (q, *J* = 7.6 Hz, 2H), 1.65 (s, 3H), 1.61 (s, 3H), 1.25 (t, *J =* 7.6 Hz, 3H). |
| | | MS (ESI) m/z: 342.5 [M+H⁺]. |
| 107 | 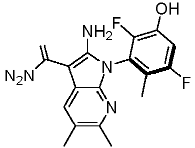 | 4.01 mg |
| | | ¹H NMR (400 MHz, DMSO-*d*₆) δ 10.56 (s, 1H), 7.84 (s, 1H), 7.06 (s, 2H), 6.95 (dd, J = 10.9, 7.3 Hz, 1H), 6.69 (s, 2H), 2.26 (s, 6H), 1.73 (d, J = 1.6 Hz, 3H). |
| | | MS (ESI) m/z: 347.0 [M+H⁺] |
| 108 | 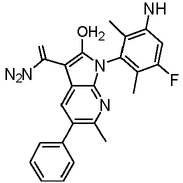 | 25.61 mg |
| | | ¹H NMR: (400 MHz, DMSO-*d*₆) δ 7.89 (s, 1H), 7.49 - 7.30 (m, 5H), 6.99 (s, 2H), 6.82 (d, *J* = 11.0 Hz, 1H), 6.75 (s, 2H), 2.26 (s, 3H), 1.71 (s, 3H), 1.67 (s, 3H). |
| | | MS (ESI) m/z: 403.8 [M+H⁺]. |
| 109 | 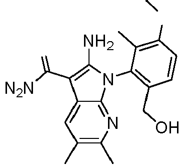 | 2.1 mg |
| | | ¹H NMR (400 MHz, Methanol-*d₄*) δ 7.81 - 7.77 (m, 1H), 7.52 (d, *J* = 8.6 Hz, 1H), 7.20 (d, *J* = 8.6 Hz, 1H), 4.20 (d, *J =* 1.8 Hz, 2H), 3.92 (s, 3H), 2.35 (d, *J* = 7.0 Hz, 6H), 1.77 (s, 3H). |
| | | MS (ESI) m/z: 355.0 [M+H⁺] |
| 110 | 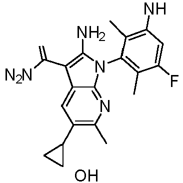 | 14.71 mg |
| | | ¹H NMR: (400 MHz, DMSO-*d*₆) δ 10.00 (s, 1H), 7.55 (s, 1H), 6.87 (s, 2H), 6.83 - 6.71 (m, 3H), 2.40 (s, 3H), 1.89 (ddd, *J =* 13.7, 8.5, 5.4 Hz, 1H), 1.65 (s, 3H), 1.61 (s, 3H), 0.93 - 0.74 (m, 4H). |
| | | MS (ESI) m/z: 369.0 [M+H⁺]. |
| 111 | 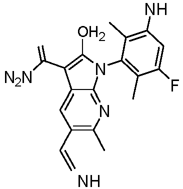 | 33.8 mg |
| | | ¹H NMR (400 MHz, DMSO-*d*₆) δ 10.00 (s, 1H), 8.11 (s, 1H), 6.98 (d, *J =* 6.2 Hz, 2H), 6.95 - 6.86 (m, 1H), 6.80 (d, *J* = 10.8 Hz, 3H), 5.94 (dd, *J* = 17.6, 1.6 Hz, 1H), 5.27 (dd, *J* = 11.2, 1.4 Hz, 1H), 2.34 (s, 3H), 1.67 (d, *J* = 1.6 Hz, 3H), 1.63 (s, 3H). |
| | | MS (ESI) m/z: 355.6 [M+H⁺] |
| 111.1 | 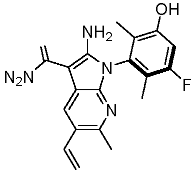 | 13.86 mg |
| | | SFC Rt = 1.138 min |
| | | [a]_{D}²⁰ = +34.8° (c = 0.09165, MeOH) |
| | | ¹H NMR (400 MHz, DMSO-*d*₆) δ 10.01 (s, 1H), 8.11 (s, 1H), 6.99 (s, 2H), 6.96 - 6.88 (m, 1H), 6.81 (s, 2H), 6.78 (s, 1H), 5.94 (d, J = 17.4 Hz, 1H), 5.27 (d, J = 12.3 Hz, 1H), 2.35 (s, 3H), 1.67 (s, 3H), 1.63 (s, 3H). |
| | | MS (ESI) m/z: 355.1 [M+H⁺] |
| 111.2 | 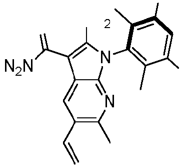 | 33.8 mg |
| | | SFC Rt = 1.359 min |
| | | [a]_{D}²⁰ = -30.5° (c = 0.0614, MeOH) |
| | | ¹H NMR (400 MHz, DMSO-*d*₆) δ 10.00 (s, 1H), 8.11 (s, 1H), 6.98 (d, J = 6.0 Hz, 2H), 6.95 - 6.86 (m, 1H), 6.81 (s, 2H), 6.79 (s, 1H), 5.94 (d, J = 17.5 Hz, 1H), 5.27 (d, J = 11.2 Hz, 1H), 2.35 (s, 3H), 1.67 (s, 3H), 1.63 (s, 3H). |
| | | MS (ESI) m/z: 355.1 [M+H⁺] |
| The chiral separation method of Example 111.1 and Example 111.2 is as follows: | | |
| SFC method-instrument: SFC-150mgm (waters), chiral column: Daicel OZ-3 (25 * 250 mm, 10 µm), temperature: 30°C, mobile phase: CO₂/MeOH [0.2% NH₃ (7M MeOH)] = 60/40, flow rate: 100 mL/min, back pressure: 100 bar, detection wavelength: 214 nm, cycle time: 6.5 min. | | |
| 112 | 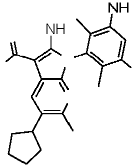 | 32.52 mg |
| | | ¹H NMR: (400 MHz, DMSO-*d*₆) δ 10.05 (s, 1H), 7.83 (s, 1H), 6.87 (s, 2H), 6.83 - 6.71 (m, 3H), 3.09 (td, J = 9.8, 9.3, 6.5 Hz, 1H), 2.33 (s, 3H), 1.96 (d, J = 11.2 Hz, 2H), 1.83 (d, J = 8.7 Hz, 2H), 1.71 - 1.59 (m, 10H). |
| | | MS(ESI)M/Z: 397.05 [M+H⁺]. |
| 113 | 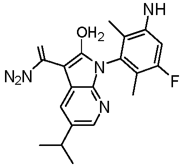 | 7.72 mg |
| | | ¹H NMR: (400 MHz, DMSO-*d*₆) δ 10.74 (s, 1H), 7.92 (d, *J* = 1.9 Hz, 1H), 7.65 (d, *J* = 1.9 Hz, 1H), 7.01 (s, 2H), 6.85 - 6.73 (m, 3H), 2.93 (p, *J* = 6.9 Hz, 1H), 1.66 - 1.58 (m, 6H), 1.28 (d, *J* = 6.9 Hz, 6H). |
| | | MS (ESI) m/z: 356.9 [M+H⁺] |
| 113.1 | 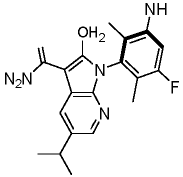 | 24.47 mg |
| | | SFC Rt = 1.102 min |
| | | [a]_{D}²⁰ = -23.4° (c = 0.10145, MeOH) |
| | | ¹H NMR (400 MHz, DMSO-*d*₆) δ 9.98 (s, 1H), 7.91 (d, *J =* 1.8 Hz, 1H), 7.65 (d, *J =* 1.8 Hz, 1H), 7.01 (s, 2H), 6.79 (d, *J* = 11.2 Hz, 3H), 2.93 (dt, *J* = 13.8, 6.8 Hz, 1H), 1.65 (d, *J =* 1.6 Hz, 3H), 1.62 (s, 3H), 1.29 (s, 3H), 1.27 (s, 3H). |
| | | MS (ESI) m/z: 357.2 [M+H⁺] |
| 113.2 | 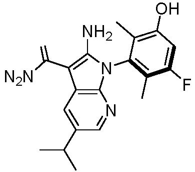 | 21.63 mg |
| | | SFC Rt = 1.322 min |
| | | [a]_{D}²⁰ = +23.6° (c = 0.10145, MeOH) |
| | | ¹H NMR (400 MHz, DMSO-*d*₆) δ 9.97 (s, 1H), 7.91 (d, *J =* 1.8 Hz, 1H), 7.65 (d, *J =* 1.8 Hz, 1H), 7.02 (s, 2H), 6.79 (d, *J* = 11.8 Hz, 3H), 2.93 (dt, *J* = 13.8, 6.8 Hz, 1H), 1.65 (d, *J =* 1.6 Hz, 3H), 1.62 (s, 3H), 1.29 (s, 3H), 1.27 (s, 3H). |
| | | MS (ESI) m/z: 357.2 [M+H⁺] |
| The chiral separation method of Example 113.1 and Example 113.2 is as follows: | | |
| SFC method-instrument: SFC-150mgm (waters), chiral column: Daicel IC-3 (25 * 250 mm, 10 µm), temperature: 30°C, mobile phase: CO₂/EtOH [0.5% NH₃ (7M MeOH)] = 70/30, flow rate: 100 mL/min, back pressure: 100 bar, detection wavelength: 214 nm, cycle time: 8.5 min. | | |
| 114 | 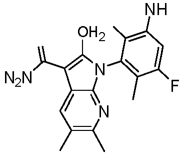 | 23.79 mg |
| | | ¹H NMR (400 MHz, MeOD) δ 7.75 (s, 1H), 6.90 (d, J = 10.8 Hz, 1H), 2.36 (s, 6H), 1.84 (d, J = 1.9 Hz, 3H). |
| | | MS (ESI) m/z: 362.9 [M+H⁺] |
| 114.1 | 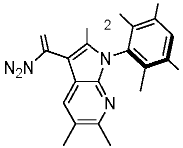 | 83.85 mg |
| | | SFC Rt = 1.116 min |
| | | [a]_{D}²⁰= +44.5° (c = 0.10255, MeOH) |
| | | ¹H NMR (400 MHz, DMSO-*d*₆) δ 10.84 (s, 1H), 7.83 (s, 1H), 7.04 - 6.92 (m, 3H), 6.66 (s, 2H), 2.25 (d, J = 5.8 Hz, 6H), 1.73 (d, J = 1.6 Hz, 3H). |
| | | MS (ESI) m/z: 363.8 [M+H⁺] |
| The chiral separation method of Example 114.1 is as follows: | | |
| SFC method-instrument: SFC-150mgm (waters), chiral column: Daicel IC-3 (25 * 250 mm, 10 µm), temperature: 30°C, mobile phase: CO₂/MeOH [0.2% NH₃ (7M MeOH)] = 60/40, flow rate: 100 mL/min, back pressure: 100 bar, detection wavelength: 214 nm, cycle time: 3.6 min. | | |
| 115 | 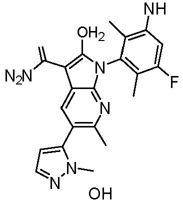 | 1.76 mg |
| | | ¹H NMR: (400 MHz, DMSO-*d*₆) δ 10.06 (s, 1H), 7.97 (s, 1H), 7.50 (d, *J =* 1.8 Hz, 1H), 7.08 (s, 2H), 6.86 - 6.74 (m, 3H), 6.32 (d, *J* = 1.9 Hz, 1H), 3.63 (s, 3H), 2.14 (s, 3H), 1.70 (s, 3H), 1.67 (s, 3H). |
| | | MS (ESI) m/z: 408.4 [M+H⁺]. |
| 116 | 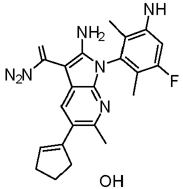 | 26.47 mg |
| | | ¹H NMR: (400 MHz, DMSO-*d*₆) δ 10.00 (s, 1H), 7.80 (s, 1H), 6.94 (s, 2H), 6.80 (d, *J =* 11.1 Hz, 1H), 6.72 (s, 2H), 5.78 (p, *J =* 2.2 Hz, 1H), 2.72 (ddt, *J =* 7.8, 5.7, 2.3 Hz, 2H), 2.50 (m, 2H), 2.33 (s, 3H), 1.96 (p, *J =* 7.5 Hz, 2H), 1.67 (s, 3H), 1.63 (s, 3H). |
| | | MS (ESI) m/z: 395.03 [M+H⁺]. |
| 117 | 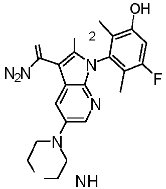 | 0.86 mg |
| | | ¹H NMR: (400 MHz, DMSO-*d*₆) δ 7.63 (s, 1H), 7.48 (s, 1H), 7.03 (s, 2H), 6.78 (s, 3H), 3.76 (s, 4H), 3.13 (s, 4H), 1.65 (s, 3H), 1.61 (s, 3H). |
| | | MS(ESI)M/Z: 400.07 [M+H⁺] |
| 118 | 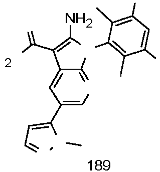 | 13.55 mg |
| | | ¹H NMR: (400 MHz, DMSO-*d*₆) δ 10.05 (s, 1H), 8.16 (d, *J* = 1.9 Hz, 1H), 7.90 (d, *J* = 1.9 Hz, 1H), 7.48 (d, *J* = 1.9 Hz, 1H), 7.23 (s, 2H), 6.91 - 6.78 (m, 3H), 6.45 (d, *J* = 1.9 Hz, 1H), 3.87 (s, 3H), 1.69 (s, 3H), 1.66 (s, 3H). |
| | | MS (ESI) m/z: 395.0 [M+H⁺]. |
| 118.1 | 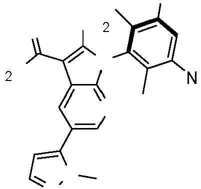 | 48.48 mg |
| | | SFC Rt = 1.430 min |
| | | [a]_{D}²⁰ = -16.5° (c = 0.10125, MeOH) |
| | | ¹H NMR (400 MHz, DMSO-*d*₆) δ 10.03 (s, 1H), 8.15 (d, *J =* 1.8 Hz, 1H), 7.89 (d, *J =* 1.8 Hz, 1H), 7.48 (d, *J* = 1.8 Hz, 1H), 7.21 (s, 2H), 6.86 (s, 2H), 6.82 (d, *J* = 11.1 Hz, 1H), 6.45 (d, *J =* 1.8 Hz, 1H), 3.87 (s, 3H), 1.69 (d, *J =* 1.5 Hz, 3H), 1.66 (s, 3H). |
| | | MS (ESI) m/z: 395.8 [M+H⁺] |
| 118.2 | 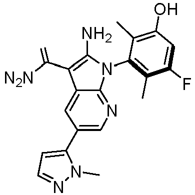 | 48.44 mg |
| | | SFC Rt = 1.602 min |
| | | [a]_{D}²⁰ = +37.8° (c = 0.10225, MeOH) |
| | | ¹H NMR (400 MHz, DMSO-*d*₆) δ 10.03 (s, 1H), 8.15 (d, *J =* 1.9 Hz, 1H), 7.89 (d, *J =* 1.8 Hz, 1H), 7.48 (d, *J* = 1.8 Hz, 1H), 7.21 (s, 2H), 6.86 (s, 2H), 6.82 (d, *J* = 11.1 Hz, 1H), 6.45 (d, *J =* 1.8 Hz, 1H), 3.87 (s, 3H), 1.69 (d, *J =* 1.5 Hz, 3H), 1.66 (s, 3H). |
| | | MS (ESI) m/z: 395.8 [M+H⁺] |
| The chiral separation method of Example 118.1 and Example 118.2 is as follows: | | |
| SFC method-instrument: SFC-150mgm (waters), chiral column: Daicel IC-3 (25 * 250 mm, 10 µm), temperature: 30°C, mobile phase: CO₂/MeOH [0.2% NH₃ (7M MeOH)] = 55/45, flow rate: 100 mL/min, back pressure: 100 bar, detection wavelength: 214 nm, cycle time: 9.5 min. | | |
| 119 | 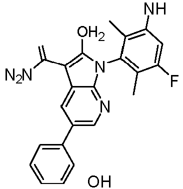 | 4.81 mg |
| | | ¹H NMR: (400 MHz, DMSO-*d*₆) δ 10.04 (s, 1H), 8.25 (d, *J =* 2.0 Hz, 1H), 8.06 *(d, J =* 2.0 Hz, 1H), 7.81 - 7.74 (m, 2H), 7.47 (t, *J* = 7.6 Hz, 2H), 7.40 - 7.31 (m, 1H), 7.15 (s, 2H), 6.93 (s, 2H), 6.82 (d, *J =* 11.1 Hz, 1H), 1.70 (s, 3H), 1.66 (s, 3H). |
| | | MS (ESI) m/z: 391.1 [M+H⁺]. |
| 119.1 | 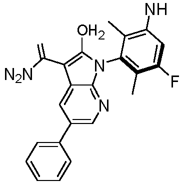 | 43.43 mg |
| | | SFC Rt = 1.215 min |
| | | [a]_{D}²⁰ = +44.7° (c = 0.1055, MeOH) |
| | | ¹H NMR (400 MHz, DMSO-*d*₆) δ 10.02 (s, 1H), 8.25 (d, *J =* 2.0 Hz, 1H), 8.06 *(d, J =* 2.0 Hz, 1H), 7.82 - 7.72 (m, 2H), 7.47 (t, *J* = 7.8 Hz, 2H), 7.35 (t, *J =* 7.4 Hz, 1H), 7.14 (s, 2H), 6.91 (s, 2H), 6.82 (d, *J =* 11.2 Hz, 1H), 1.70 (d, *J =* 1.5 Hz, 3H), 1.66 (s, 3H). |
| | | MS (ESI) m/z: 391.3 [M+H⁺] |
| 119.2 | 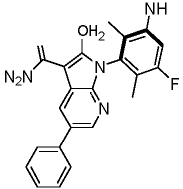 | 43.92 mg |
| | | SFC Rt = 2.146 min |
| | | [a]_{D}²⁰ = -30.8° (c = 0.10285, MeOH) |
| | | ¹H NMR (400 MHz, DMSO-*d*₆) δ 9.97 (s, 1H), 8.25 (d, *J =* 1.8 Hz, 1H), 8.06 (d, *J* = 1.8 Hz, 1H), 7.77 (d, *J* = 7.8 Hz, 2H), 7.47 (t, *J =* 7.6 Hz, 2H), 7.35 (t, *J =* 7.3 Hz, 1H), 7.14 (s, 2H), 6.91 (s, 2H), 6.82 (d, *J =* 11.2 Hz, 1H), 1.69 (s, 3H), 1.66 (s, 3H). |
| | | MS (ESI) m/z: 391.3 [M+H⁺] |
| The chiral separation method of Example 119.1 and Example 119.2 is as follows: | | |
| SFC method-instrument: SFC-150 mgm (waters), chiral column: Daicel OJ-3 (25 * 250 mm, 10 µm), temperature: 30°C, mobile phase: CO₂/MeOH [0.2% NH₃ (7M MeOH)] = 65/35, flow rate: 100 mL/min, back pressure: 100 bar, detection wavelength: 214 nm, cycle time: 4.48 min. | | |
| 120 | 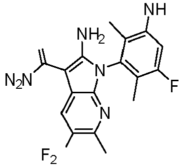 | 2.27 mg |
| | | ¹H NMR: (400 MHz, DMSO-*d*₆) δ 10.09 (s, 1H), 8.28 (s, 1H), 7.18 (s, 2H), 6.96 (s, 2H), 6.83 (d, *J* = 11.1 Hz, 1H), 2.46 (s, 3H), 1.65 (d, *J =* 16.0 Hz, 6H). |
| | | MS (ESI) m/z: 397.0 [M+H⁺]. |
| 120.1 | 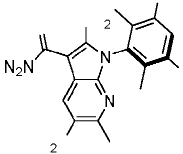 | 82.38 mg |
| | | SFC Rt = 0.862 min |
| | | [a]_{D}²⁰ = +34.5° (c = 0.09853, MeOH) |
| | | ¹H NMR: (400 MHz, DMSO-*d*₆) δ 10.12 (s, 1H), 8.27 (s, 1H), 7.13 (s, 2H), 6.92 (s, 2H), 6.83 (d, *J =* 11.2 Hz, 1H), 2.46 (d, *J* = 1.6 Hz, 3H), 1.67 (d, *J* = 1.6 Hz, 3H), 1.63 (s, 3H). |
| | | MS (ESI) m/z: 396.8 [M+H⁺] |
| 120.2 | 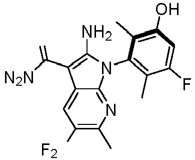 | 56.42 mg |
| | | SFC Rt = 0.961 min |
| | | [a]_{D}²⁰ = -22.2° (c = 0.09202, MeOH) |
| | | ¹H NMR: (400 MHz, DMSO-*d*₆) δ 10.16 (s, 1H), 8.27 (s, 1H), 7.13 (s, 2H), 6.92 (s, 2H), 6.83 (d, *J =* 11.2 Hz, 1H), 2.46 (d, *J* = 1.6 Hz, 3H), 1.67 (d, *J* = 1.6 Hz, 3H), 1.63 (s, 3H). |
| | | MS (ESI) m/z: 396.8 [M+H⁺] |
| The chiral separation method of Example 120.1 and Example 120.2 is as follows: | | |
| SFC method: instrument: SFC-150mgm (waters), chiral column: Daicel OJ (25 * 250 mm, 10 µm), temperature: 30°C, mobile phase: CO₂/MeOH [0.2% NH₃ (7M MeOH)] = 80/20, flow rate: 100 mL/min, back pressure: 100 bar, detection wavelength: 214 nm, cycle time: 1.61 min. | | |
| 121 | 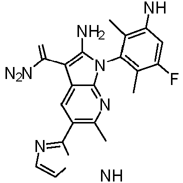 | 12.7 mg |
| | | ¹H NMR: (400 MHz, DMSO-*d*₆) δ 10.06 (s, 1H), 8.43 (s, 1H), 8.25 (s, 1H), 7.42 (s, 1H), 7.08 (s, 2H), 6.88 - 6.79 (m, 3H), 2.62 (s, 3H), 1.69 (s, 3H), 1.65 (s, 3H). |
| | | MS (ESI) m/z: 396.1 [M+H⁺]. |
| 122 | 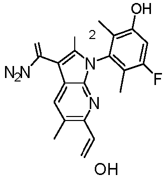 | 5 mg |
| | | ¹H NMR: (400 MHz, DMSO-*d*₆) δ 9.97 (s, 1H), 7.84 (s, 1H), 7.09 (s, 2H), 6.91 (dd, J = 16.8, 10.5 Hz, 1H), 6.79 (d, J = 11.1 Hz, 1H), 6.73 (s, 2H), 5.74 (dd, J = 16.8, 2.8 Hz, 1H), 5.12 (dd, J = 10.6, 2.8 Hz, 1H), 2.37 (s, 3H), 1.69 (d, J = 1.6 Hz, 3H), 1.65 (s, 3H). |
| | | MS (ESI) m/z: 355.2 [M+H⁺] |
| 123 | 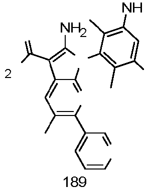 | 10.31 mg |
| | | ¹H NMR: (400 MHz, DMSO-*d*₆) δ 9.96 (s, 1H), 8.59 (d, *J* = 1.6 Hz, 1H), 8.50 (dd, J = 4.8, 1.6 Hz, 1H), 8.03 (s, 1H), 7.84 - 7.78 (m, 1H), 7.46 - 7.33 (m, 1H), 7.12 (s, 2H), 6.88 - 6.68 (m, 3H), 2.38 (s, 3H), 1.71 (d, J = 1.5 Hz, 3H), 1.67 (s, 3H). |
| | | MS (ESI) m/z: 406.0 [M+H⁺] |
| 124 | 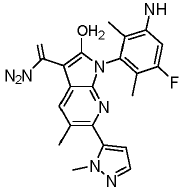 | 11.93 mg |
| | | ¹H NMR: (400 MHz, DMSO-*d*₆) δ 9.99 (s, 1H), 8.04 (s, 1H), 7.40 (d, *J=* 1.9 Hz, 1H), 7.17 (s, 2H), 6.89 - 6.80 (m, 2H), 6.77 (s, 1H), 6.38 (d, *J* = 1.9 Hz, 1H), 3.57 (s, 3H), 2.32 (s, 3H), 1.71 - 1.62 (m, 6H). |
| | | MS (ESI) m/z: 409.1 [M+H⁺] |
| 125 | 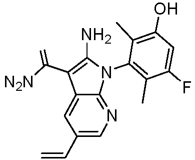 | 2 mg |
| | | ¹H NMR: (400 MHz, DMSO-*d*₆) δ 8.20 (d, *J* = 1.9 Hz, 1H), 7.81 (d, *J=* 1.8 Hz, 1H), 7.15 (s, 2H), 6.87 (s, 2H), 6.84 - 6.68 (m, 2H), 5.99 (dd, *J* = 17.7, 1.3 Hz, 1H), 5.23 (dd, *J=* 10.9, 1.3 Hz, 1H), 1.69 - 1.60 (m, 6H). |
| | | MS (ESI) m/z: 340.2 [M+H⁺]. |
| 126 | 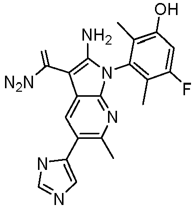 | 3.65 mg |
| | | ¹H NMR: (400 MHz, DMSO-*d*₆) δ 10.04 (s, 1H), 9.16 (d, *J* = 0.8 Hz, 1H), 8.04 (d, *J* = 7.2 Hz, 2H), 7.08 (s, 2H), 6.87 - 6.76 (m, 3H), 2.37 (s, 3H), 1.72 - 1.62 (m, 6H). |
| | | MS (ESI) m/z: 411.9 [M+H⁺]. |
| 127 | 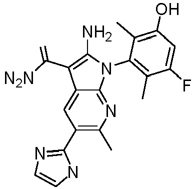 | 3.24 mg |
| | | ¹H NMR (400 MHz, DMSO-*d*₆) δ 10.04 (s, 1H), 8.22 (s, 1H), 7.96 (d, J = 3.3 Hz, 1H), 7.85 (d, J = 3.3 Hz, 1H), 7.07 (s, 2H), 6.84 (s, 2H), 6.81 (s, 1H), 3.32 (s, 3H), 1.70 (d, J = 1.4 Hz, 3H), 1.66 (s, 3H). |
| | | MS (ESI) m/z: 412.3 [M+H⁺] |
| 128 | 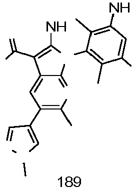 | 28.95 mg |
| | | ¹H NMR (400 MHz, DMSO-*d*₆) δ 9.99 (s, 1H), 7.94 (d, J = 4.6 Hz, 2H), 7.75 (s, 1H), 6.95 (s, 2H), 6.80 (d, J = 11.1 Hz, 1H), 6.76 (s, 2H), 3.89 (s, 3H), 2.39 (s, 3H), 1.69 (d, J = 1.5 Hz, 3H), 1.65 (s, 3H). |
| | | MS (ESI) m/z: 409.1 [M+H⁺] |
| 129 | 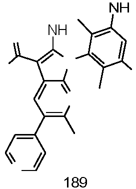 | 16.99 mg |
| | | ¹H NMR: (400 MHz, DMSO-*d*₆) δ 8.66 - 8.60 (m, 2H), 7.95 (s, 1H), 7.56 - 7.49 (m, 2H), 7.05 (s, 2H), 6.88 - 6.75 (m, 3H), 2.31 (s, 3H), 1.70 (s, 3H), 1.67 (s, 3H). |
| | | MS (ESI) m/z: 406.1 [M+H⁺]. |
| 130 | 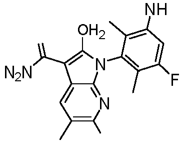 | 2.13 mg |
| | | 1H NMR (400 MHz, DMSO) δ 10.07 (s, 1H), 8.09 (s, 1H), 7.10 (s, 2H), 6.82 (d, J = 11.0 Hz, 3H), 2.34 (s, 3H), 1.66 (d, J = 1.5 Hz, 3H), 1.62 (s, 3H). |
| | | MS (ESI) m/z: 363.2 [M+H]⁺ |
| 131 | 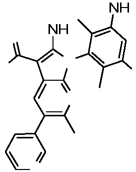 | 36.2 mg |
| | | ¹H NMR: (400 MHz, DMSO-*d*₆) δ 10.04 (s, 1H), 8.68 (d, *J* = 2.3 Hz, 1H), 8.57 (dd, *J* = 4.9, 1.6 Hz, 1H), 7.99 - 7.87 (m, 2H), 7.48 (dd, *J* = 7.8, 4.8 Hz, 1H), 7.04 (s, 2H), 6.86 - 6.77 (m, 3H), 2.28 (s, 3H), 1.74 - 1.65 (m, 6H). |
| | | MS (ESI) m/z: 406.2 [M+H⁺]. |
| 132 | 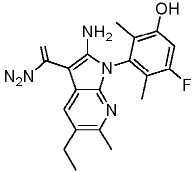 | 3.35 mg |
| | | ¹H NMR: (400 MHz, DMSO-*d*₆) δ 9.93 (s, 1H), 7.81 (s, 1H), 6.85 (s, 2H), 6.79 (d, *J =* 11.2 Hz, 1H), 6.68 (s, 2H), 2.62 (q, *J =* 7.6 Hz, 2H), 2.29 (s, 3H), 1.65 (d, *J* = 1.6 Hz, 3H), 1.62 (s, 3H), 1.21 (t, *J =* 7.5 Hz, 3H). |
| | | MS (ESI) m/z: 357.2 [M+H⁺] |
| 132.1 | 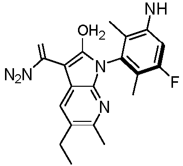 | 7.06 mg |
| | | SFC Rt = 1.091 min |
| | | [a]_{D}²⁰ = +16.7° (c = 0.067015, MeOH) |
| | | ¹H NMR: (400 MHz, Methanol-*d₄*) δ 7.75 (s, 1H), 6.74 (d, *J =* 11.0 Hz, 1H), 2.74 (q, *J =* 7.6 Hz, 2H), 2.40 (s, 3H), 1.75 (s, 6H), 1.26 (t, *J =* 7.6 Hz, 3H). |
| | | MS (ESI) m/z: 357.0 [M+H⁺] |
| The chiral separation method of Example 132.1 is as follows: | | |
| SFC method-instrument: SFC-150mgm (waters), chiral column: Daicel AD-3 (25 * 250 mm, 10 µm), temperature: 30°C, mobile phase: CO₂/EtOH [0.5% NH₃ (7M MeOH)] = 80/20, flow rate: 100 mL/min, back pressure: 100 bar, detection wavelength: 214 nm, cycle time: 1.41 min. | | |
| 133 | 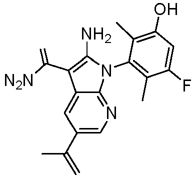 | 2.32 mg |
| | | ¹H NMR: (400 MHz, DMSO-*d*₆) δ 10.00 (s, 1H), 8.08 (d, *J* = 2.0 Hz, 1H), 7.91 (d, *J* = 1.9 Hz, 1H), 7.12 (s, 2H), 6.87 (s, 2H), 6.80 (d, *J =* 11.1 Hz, 1H), 5.49 (s, 1H), 5.06 (s, 1H), 2.18 (s, 3H), 1.66 (s, 3H), 1.62 (s, 3H). |
| | | MS (ESI) m/z: 354.9 [M+H⁺]. |
| 133.1 | 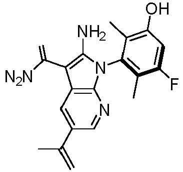 | 34.85 mg |
| | | SFC Rt =1.035 min |
| | | [a]_{D}²⁰ = +23.8° (c = 0.1048, MeOH) |
| | | ¹H NMR: (400 MHz, DMSO-*d*₆) δ 9.99 (s, 1H), 8.08 (d, *J* = 1.8 Hz, 1H), 7.91 (d, *J =* 1.8 Hz, 1H), 7.11 (s, 2H), 6.85 (s, 2H), 6.80 (d, *J =* 11.2 Hz, 1H), 5.48 (s, 1H), 5.06 (s, 1H), 2.18 (s, 3H), 1.66 (d, *J =* 1.4 Hz, 3H), 1.63 (s, 3H). |
| | | MS (ESI) m/z: 355.2 [M+H⁺] |
| The chiral separation method of Example 133.1 is as follows: | | |
| SFC method-instrument: SFC-150mgm (waters), chiral column: Daicel OJ-3 (25 * 250 mm, 10 µm), temperature: 30°C, mobile phase: CO₂/MeOH [0.2% NH₃ (7M MeOH)] = 80/20, flow rate: 100 mL/min, back pressure: 100 bar, detection wavelength: 214 nm, cycle time: 3.58 min. | | |
| 134 | 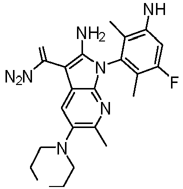 | 3.11 mg |
| | | ¹H NMR: (400 MHz, DMSO-*d*₆) δ 9.99 (s, 1H), 7.77 (s, 1H), 6.89 (s, 2H), 6.83 - 6.72 (m, 3H), 3.77 - 3.70 (m, 4H), 2.90 (t, *J* = 4.5 Hz, 4H), 2.30 (s, 3H), 1.65 (s, 3H), 1.61 (s, 3H). |
| | | MS (ESI) m/z: 414.0 [M+H⁺] |
| 135 | 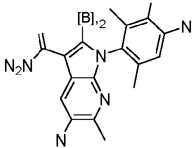 | 13.35 mg |
| | | ¹H NMR: (400 MHz, DMSO-*d*₆) δ 9.56 (s, 1H), 7.92 (d, J = 11.2 Hz, 1H), 7.11 (d, J = 11.5 Hz, 1H), 7.02 (s, 2H), 6.75 (s, 2H), 2.26 (d, J = 3.1 Hz, 3H), 1.76 (s, 3H), 1.71 (s, 3H). |
| | | MS (ESI) m/z: 347.0 [M+H⁺] |
| 136 | 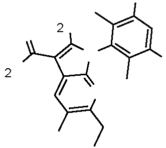 | 14.31 mg |
| | | ¹H NMR (400 MHz, DMSO) δ 9.95 (s, 1H), 7.82 (s, 1H), 6.86 (s, 2H), 6.79 (d, J = 11.1 Hz, 1H), 6.66 (s, 2H), 2.57 (q, J = 7.5 Hz, 2H), 2.31 (s, 3H), 1.69 - 1.59 (m, 6H), 1.02 (t, J = 7.5 Hz, 3H). |
| | | MS (ESI) m/z: 357.0 [M+H]⁺ |
| 137 | 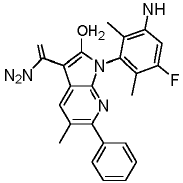 | 6.11 mg |
| | | ¹H NMR: (400 MHz, Methanol-*d₄*) δ 7.89 (s, 1H), 7.35 (dd, J = 10.4, 2.8 Hz, 4H), 7.33 - 7.26 (m, 1H), 6.69 (d, J = 10.9 Hz, 1H), 2.38 (s, 3H), 1.79 (d, J = 3.1 Hz, 6H). |
| | | MS (ESI) m/z: 405.0 [M+H⁺] |
| 138 | 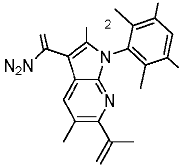 | 48.99 mg |
| | | ¹H NMR: (400 MHz, DMSO-*d*₆) δ 9.97 (s, 1H), 7.90 (s, 1H), 7.00 (s, 2H), 6.79 (d, J = 11.1 Hz, 1H), 6.71 (s, 2H), 5.24 (s, 1H), 4.90 (s, 1H), 2.36 (s, 3H), 1.89 (s, 3H), 1.67 (d, J = 1.2 Hz, 3H), 1.64 (s, 3H). |
| | | MS (ESI) m/z: 369.2 [M+H⁺] |
| 139 | 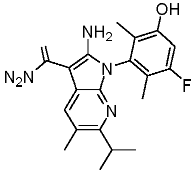 | 1.83 mg |
| | | ¹H NMR: (400 MHz, Methanol-*d₄*) δ 7.65 (s, 1H), 6.72 (d, J = 10.8 Hz, 1H), 3.20 (dt, J = 13.6, 6.8 Hz, 1H), 2.40 (s, 3H), 1.76 (s, 3H) x2, 1.10 (d, J = 6.7 Hz, 6H). |
| | | MS (ESI) m/z: 371.0 [M+H⁺] |
| 140 | 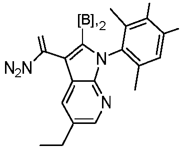 | 28.92 mg |
| | | ¹H NMR: (400 MHz, DMSO-*d*₆) δ 9.52 (s, 1H), 7.89 (d, *J =* 1.9 Hz, 1H), 7.61 (d, *J* = 1.8 Hz, 1H), 7.10 (d, *J =* 11.4 Hz, 1H), 6.98 (s, 2H), 6.73 (s, 2H), 2.62 (q, *J =* 7.5 Hz, 2H), 1.75 (s, 3H), 1.70 (s, 3H), 1.24 (t, *J =* 7.6 Hz, 3H) |
| | | MS(ESI)M/Z: 342.6 [M+H⁺] |
| 141 | 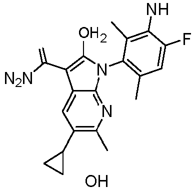 | 2.02 mg |
| | | ¹H NMR: (400 MHz, DMSO-*d*₆) δ 9.55 (s, 1H), 7.54 (s, 1H), 7.10 (d, *J =* 11.4 Hz, 1H), 6.83 (s, 2H), 6.70 (s, 2H), 2.40 (s, 3H), 1.91 1.85 (m, 1H), 1.75 (s, 3H), 1.70 (s, 3H), 0.91 - 0.75 (m, 4H). |
| | | MS (ESI) m/z: 369.2 [M+H⁺] |
| 141.1 | 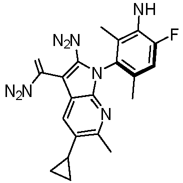 | 22.23 mg, chiral isomer 1 |
| | | SFC Rt = 2.106 min |
| | | ¹H NMR: (400 MHz, DMSO-*d*₆) δ 9.53 (s, 1H), 7.53 (s, 1H), 7.09 (d, J = 11.5 Hz, 1H), 6.82 (s, 2H), 6.69 (s, 2H), 2.40 (s, 3H), 1.93 - 1.83 (m, 1H), 1.75 (s, 3H), 1.70 (s, 3H), 0.92 - 0.84 (m, 2H), 0.78 (dt, J = 9.9, 5.1 Hz, 2H). |
| | | MS (ESI) m/z: 369.3 [M+H⁺] |
| 141.2 | 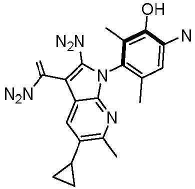 | 22.71 mg, chiral isomer 2 |
| | | SFC Rt = 3.156 min |
| | | ¹H NMR: (400 MHz, DMSO-*d*₆) δ 9.53 (s, 1H), 7.53 (s, 1H), 7.09 (d, J = 11.5 Hz, 1H), 6.82 (s, 2H), 6.69 (s, 2H), 2.40 (s, 3H), 1.89 (dq, J = 8.4, 5.5 Hz, 1H), 1.75 (s, 3H), 1.70 (s, 3H), 0.92 - 0.83 (m, 2H), 0.78 (dt, J = 10.0, 5.1 Hz, 2H). |
| | | MS (ESI) m/z: 369.3 [M+H⁺] |
| The chiral separation method of Example 141.1 and Example 141.2 is as follows: | | |
| SFC method-instrument: SFC-150mgm (waters), chiral column: Daicel OZ-3 (25 * 250 mm, 10 µm), temperature: 30°C, mobile phase: CO₂/MeOH [0.2% NH₃ (7M MeOH)] = 50/50, flow rate: 100 mL/min, back pressure: 100 bar, detection wavelength: 214 nm, cycle time: 14.5 min. | | |
| 142 | 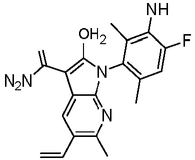 | 2.37 mg |
| | | ¹H NMR: (400 MHz, DMSO-*d*₆) δ 9.58 (s, 1H), 8.10 (s, 1H), 7.11 (d, *J* = 11.5 Hz, 1H), 6.97 (s, 2H), 6.97 - 6.87 (m, 1H), 6.81 (s, 2H), 5.94 (dd, *J =* 17.3, 1.6 Hz, 1H), 5.27 (dd, *J* = 11.0, 1.6 Hz, 1H), 2.34 (s, 3H), 1.77 (s, 3H), 1.72 (s, 3H). |
| | | MS (ESI) m/z: 355.2 [M+H⁺]. |
| 142.1 | 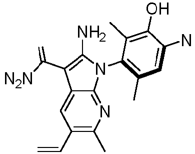 | 34.1 mg |
| | | SFC Rt = 2.136 min |
| | | ¹H NMR (400 MHz, DMSO-*d*₆) δ 9.56 (s, 1H), 8.09 (s, 1H), 7.11 (d, *J =* 11.6 Hz, 1H), 6.94 (dt, *J =* 17.6, 8.8 Hz, 3H), 6.79 (s, 2H), 5.94 (dd, *J =* 17.4, 1.4 Hz, 1H), 5.31 - 5.22 (m, 1H), 2.34 (s, 3H), 1.77 (s, 3H), 1.72 (s, 3H). |
| | | MS (ESI) m/z: 355.3 [M+H⁺] |
| The chiral separation method of Example 142.1 is as follows: | | |
| SFC method-instrument: SFC-150mgm (waters), chiral column: Daicel OZ-3 (25 * 250 mm, 10 µm), temperature: 30°C, mobile phase: CO₂/MeOH [0.2% NH₃ (7M MeOH)] = 55/45, flow rate: 100 mL/min, back pressure: 100 bar, detection wavelength: 214 nm, cycle time: 9.8 min. | | |
| 143 | 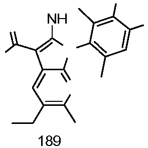 | 3.42 mg |
| | | ¹H NMR: (400 MHz, DMSO-*d*₆) δ 7.80 (s, 1H), 7.10 (d, *J =* 11.5 Hz, 1H), 6.83 (s, 2H), 6.67 (s, 2H), 2.61 (q, *J =* 7.4 Hz, 2H), 2.28 (s, 3H), 1.76 (s, 3H), 1.71 (s, 3H), 1.20 (t, *J =* 7.5 Hz, 3H). |
| | | MS (ESI) m/z: 357.3 [M+H⁺]. |
| 143.1 | 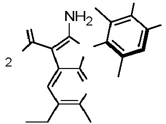 | 50.56 mg |
| | | SFC Rt = 1.772 min |
| | | ¹H NMR: (400 MHz, DMSO-*d*₆) δ 9.57 (s, 1H), 7.79 (s, 1H), 7.08 (d, J = 11.5 Hz, 1H), 6.81 (s, 2H), 6.66 (s, 2H), 2.62 (q, J = 7.5 Hz, 2H), 2.28 (s, 3H), 1.76 (s, 3H), 1.71 (s, 3H), 1.20 (t, J = 7.5 Hz, 3H). |
| | | MS (ESI) m/z: 357.3 [M+H⁺] |
| The chiral separation method of Example 143.1 is as follows: | | |
| SFC method-instrument: SFC-150mgm (waters), chiral column Daicel OZ-3 (25 * 250 mm, 10 µm), | | |
| temperature: 30°C, mobile phase: CO₂/EtOH [0.5%NH₃ (7M MeOH)] = 55/45, flow rate: 100 mL/min, back pressure: 100 bar, detection wavelength: 214 nm, cycle time: 13 min. | | |
| 144 | 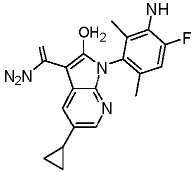 | 98.45 mg |
| | | ¹H NMR: (400 MHz, DMSO-*d*₆) δ 9.56 (s, 1H), 7.63 (d, *J =* 1.8 Hz, 1H), 7.55 (d, *J =* 1.9 Hz, 1H), 7.10 (d, *J =* 11.5 Hz, 1H), 6.99 (s, 2H), 6.76 (s, 2H), 1.91 (td, *J =* 8.4, 4.2 Hz, 1H), 1.75 (s, 3H), 1.70 (s, 3H), 0.95 - 0.76 (m, 4H). |
| | | MS (ESI) m/z: 355.1 [M+H⁺] |
| 144.1 | 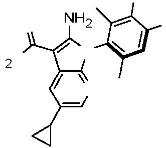 | 38.44 mg, chiral isomer 1 |
| | | SFC Rt = 2.796 min |
| | | ¹H NMR: (400 MHz, DMSO-*d*₆) δ 9.54 (s, 1H), 7.62 (d, J = 1.8 Hz, 1H), 7.55 (d, J = 1.8 Hz, 1H), 7.09 (d, J = 11.5 Hz, 1H), 6.98 (s, 2H), 6.74 (s, 2H), 1.96 - 1.87 (m, 1H), 1.75 (s, 3H), 1.70 (s, 3H), 0.95 - 0.87 (m, 2H), 0.82 - 0.75 (m, 2H). |
| | | MS (ESI) m/z: 355.2 [M+H⁺] |
| 144.2 | 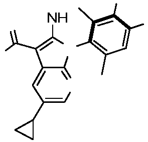 | 34.25 mg, chiral isomer 2 |
| | | SFC Rt = 4.089 min |
| | | ¹H NMR: (400 MHz, DMSO-*d*₆) δ 9.53 (s, 1H), 7.62 (d, J = 1.8 Hz, 1H), 7.55 (d, J = 1.8 Hz, 1H), 7.09 (d, J = 11.5 Hz, 1H), 6.98 (s, 2H), 6.74 (s, 2H), 1.97 - 1.85 (m, 1H), 1.75 (s, 3H), 1.70 (s, 3H), 0.93 - 0.85 (m, 2H), 0.85 - 0.75 (m, 2H). |
| | | MS (ESI) m/z: 355.2 [M+H⁺] |
| The chiral separation method of Example 144.1 and Example 144.2 is as follows: | | |
| SFC method-instrument: SFC-150mgm (waters), chiral column: Daicel OZ-3 (25 * 250 mm, 10 µm), temperature: 30°C, mobile phase: CO₂/MeOH [0.2% NH₃ (7M MeOH)] = 50/50, flow rate: 100 mL/min, back pressure: 100 bar, detection wavelength: 214 nm, cycle time: 11 min. | | |
| 145 | 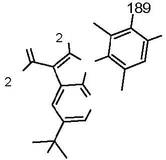 | 6.25 mg |
| | | ¹H NMR: (400 MHz, DMSO-*d*₆) δ 9.52 (s, 1H), 7.95 (dd, *J =* 14.1, 2.0 Hz, 2H), 7.10 (d, *J =* 11.4 Hz, 1H), 6.97 (s, 2H), 6.74 (s, 2H), 4.95 (s, 1H), 1.75 (s, 3H), 1.70 (s, 3H), 1.51 (s, 6H). |
| | | MS(ESI) m/z: 373.1 [M+H⁺] |
| 146 | 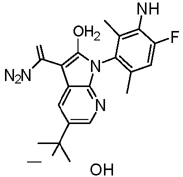 | 2.37 mg |
| | | ¹H NMR: (400 MHz, DMSO-*d*₆) δ 9.56 (s, 1H), 7.91 (d, *J =* 2.0 Hz, 1H), 7.81 (d, *J* = 2.0 Hz, 1H), 7.11 (d, *J =* 12 Hz, 1H), 7.01(s, 2H), 6.78 (s, 2H), 2.98 (s, 3H), 1.76 (s, 3H), 1.71 (s, 3H), 1.53 (s, 6H). |
| | | MS(ESI) m/z: 387.3 [M+H⁺] |
| 147 | 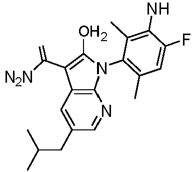 | 70 mg |
| | | ¹H NMR: (400 MHz, DMSO-*d*₆) δ 7.86 (d, *J =* 1.8 Hz, 1H), 7.55 (d, *J* = 1.8 Hz, 1H), 7.06 (d, *J =* 11.5 Hz, 1H), 6.96 (s, 2H), 6.72 (s, 2H), 2.45 (d, *J =* 7.2 Hz, 2H), 1.91 (dq, *J* = 13.3, 6.7 Hz, 1H), 1.74 (s, 3H), 1.69 (s, 3H), 0.88(s, 3H), 0.87 (s, 3H). |
| | | MS(ESI) m/z: 371.3 [M+H⁺] |
| 148 | 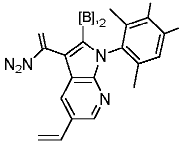 | 2.47 mg |
| | | ¹H NMR: (400 MHz, DMSO-*d*₆) δ 9.58 (s, 1H), 8.18 (d, *J* = 1.9 Hz, 1H), 7.80 (d, *J =* 1.8 Hz, 1H), 7.11 (t, *J =* 5.8 Hz, 3H), 6.85 (s, 2H), 6.74 (dd, *J =* 17.7, 11.1 Hz, 1H), 5.99 (dd, *J =* 17.7, 1.3 Hz, 1H), 5.22 (dd, *J =* 11.0, 1.3 Hz, 1H), 1.76 (s, 3H) 1.71 (s, 3H) |
| | | MS(ESI) m/z: 341.3 [M+H⁺] |
| 149 | 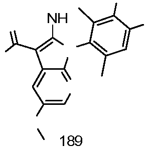 | 119.2 mg |
| | | ¹H NMR: (400 MHz, DMSO-*d*₆) δ 9.57 (s, 1H), 7.66 (d, *J =* 2.5 Hz, 1H), 7.47 (d, *J =* 2.4 Hz, 1H), 7.10 (d, *J =* 11.5 Hz, 1H), 7.03 (s, 2H), 6.79 (s, 2H), 3.82 (s, 3H), 2.08 (s, 3H), 1.76 (s, 3H) 1.71 (s, 3H). |
| | | MS(ESI) m/z: 345.19 [M+H⁺] |
| 150 | 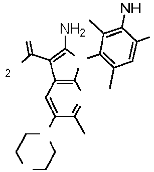 | 0.87 mg |
| | | ¹H NMR: (400 MHz, DMSO-*d*₆) δ 9.55 (s, 1H), 7.76 (s, 1H), 7.10 (d, *J =* 11.5 Hz, 1H), 6.86 (s, 2H), 6.75 (s, 2H), 3.73 (s, 4H), 2.93 - 2.86 (s, 4H), 2.30 (s, 3H), 1.76 (s, 3H), 1.71 (s, 3H). |
| | | MS (ESI) m/z: 413.5 [M+H⁺] |
| 151 | 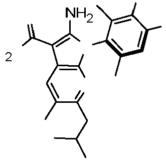 | 160.67 mg |
| | | ¹H NMR (400 MHz, Methanol-*d₄*) δ 7.73 (s, 1H), 7.00 (d, *J =* 11.3 Hz, 1H), 2.58 (d, *J =* 7.3 Hz, 2H), 2.39 (s, 3H), 1.98 - 1.88 (m, 1H), 1.88 - 1.81 (m, 6H), 0.90 - 0.80 (m, 6H). |
| | | MS (ESI) m/z: 385.4 [M+H⁺] |
| 152 | 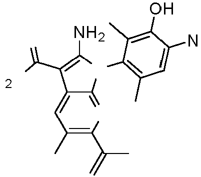 | 22.04 mg |
| | | ¹H NMR (400 MHz, DMSO) δ 9.54 (s, 1H), 7.88 (s, 1H), 7.10 (d, J = 11.5 Hz, 1H), 6.96 (s, 2H), 6.69 (s, 2H), 5.24 (s, 1H), 4.90 (s, 1H), 2.36 (s, 3H), 1.89 (s, 3H), 1.78 (s, 3H), 1.73 (s, 3H). |
| | | MS (ESI) m/z: 369.3 [M+H]⁺ |
| 153 | 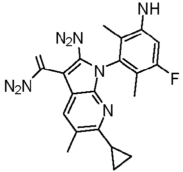 | 15.7 mg |
| | | ¹H NMR: (400 MHz, DMSO-*d*₆) δ 9.92 (s, 1H), 7.81 (s, 1H), 6.84 (s, 2H), 6.77 (d, *J =* 11.2 Hz, 1H), 6.64 (s, 2H), 2.41 (s, 3H), 2.02 (d, *J* = 4.6 Hz, 1H), 1.63 (s, 3H), 1.60 (s, 3H), 0.73 (dd, *J* = 8.0, 2.6 Hz, 2H), 0.57 (s, 2H). |
| | | MS (ESI) m/z: 368.41 [M+H⁺] |
| 154 | 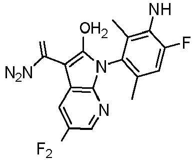 | 177.36 mg |
| | | ¹H NMR (400 MHz, Methanol-*d₄*) δ 8.27 - 8.25 (m, 1H), 8.12 - 8.08 (m, 1H), 7.05 (d, *J =* 11.3 Hz, 1H), 1.86 (s, 3H), 1.83 (s, 3H). |
| | | MS (ESI) m/z: 383.2 [M+H⁺] |
| 154.1 | 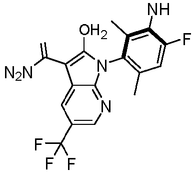 | 59.59 mg |
| | | SFC Rt = 0.821 min |
| | | [a]_{D}²⁰ = -27.5° (c = 0.10763, MeOH) |
| | | ¹H NMR; (400 MHz, DMSO-*d*₆) δ 9.63 (s, 1H), 8.37 (s, 1H), 8.12 (s, 1H), 7.31 (s, 2H), 7.14 (d, *J* = 11.2 Hz, 1H), 6.98 (s, 2H), 1.76 (s, 3H), 1.71 (s, 3H). |
| | | MS (ESI) m/z: 383.2 [M+H⁺] |
| 154.2 | 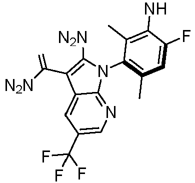 | 51.43 mg |
| | | SFC Rt = 0.928 min |
| | | [a]_{D}²⁰ = +16.7° (c = 0.10219, MeOH) |
| | | ¹H NMR: (400 MHz, DMSO-*d*₆) δ 9.63 (s, 1H), 8.37 (s, 1H), 8.12 (s, 1H), 7.31 (s, 2H), 7.14 (d, *J =* 11.4 Hz, 1H), 6.98 (s, 2H), 1.76 (s, 3H), 1.71 (s, 3H). |
| | | MS (ESI) m/z: 383.2 [M+H⁺] |
| The chiral separation method of Example 154.1 and Example 154.2 is as follows: | | |
| SFC method-instrument: SFC-150mgm (waters), chiral column: Daicel IC (25 * 250 mm, 10 µm), temperature: 30°C, mobile phase: CO₂/MeOH [0.2% NH₃ (7M MeOH)] = 65/35, flow rate: 100 mL/min, back pressure: 100 bar, detection wavelength: 214 nm, cycle time: 2.13 min. | | |
| 155 | 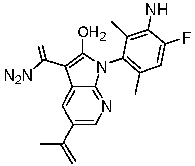 | 2.29 mg |
| | | ¹H NMR: (400 MHz, DMSO-*d*₆) δ 8.07 (s, 1H), 7.90 (s, 1H), 7.17 - 7.02 (m, 3H), 6.84 (s, 2H), 5.48 (s, 1H), 5.06 (s, 1H), 2.17 (s, 3H), 1.76 (s, 3H), 1.72 (s, 3H). |
| | | MS (ESI) m/z: 355.1 [M+H⁺] |
| 156 | 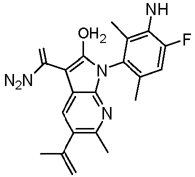 | 75.4 mg |
| | | ¹H NMR: (400 MHz, DMSO-*d*₆) δ 9.57 (d, *J =* 1.4 Hz, 1H), 7.76 (s, 1H), 7.11 (d, *J* = 11.5 Hz, 1H), 6.90 (s, 2H), 6.69 (s, 2H), 5.24 (s, 1H), 4.88 (s, 1H), 2.29 (s, 3H), 2.08 (s, 3H), 1.77 (s, 3H), 1.72 (s, 3H). MS (ESI) m/z: 369.3 [M+H⁺] |
| 157 | 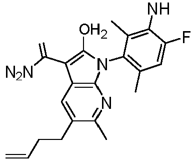 | 31.5 mg |
| | | ¹H NMR: (400 MHz, DMSO-*d*₆) δ 9.54 (s, 1H), 7.83 (s, 1H), 7.10 (d, *J =* 11.4 Hz, 1H), 6.84 (s, 2H), 6.68 (s, 2H), 5.93 (ddt, *J =* 16.8, 10.2, 6.5 Hz, 1H), 5.10 (dq, *J* = 17.2, 1.7 Hz, 1H), 5.00 (ddd, *J* = 10.2, 2.2, 1.2 Hz, 1H), 2.72 - 2.63 (m, 2H), 2.40 - 2.27 (m, 5H), 1.76 (s, 3H), 1.71 (s, 3H). |
| | | MS(ESI) m/z: 383.2 [M+H⁺] |
| 158 | 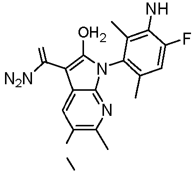 | 64.29 mg |
| | | ¹H NMR: (400 MHz, DMSO-*d*₆) δ 9.54 (s, 1H), 7.62 (s, 1H), 7.09 (d, *J* = 11.5 Hz, 1H), 6.83 (s, 2H), 6.77 (s, 2H), 3.86 (s, 3H), 2.19 (s, 3H), 1.76 (s, 3H), 1.71 (s, 3H). |
| | | MS(ESI) m/z: 359.2 [M+H⁺] |
| 158.1 | 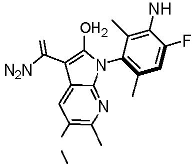 | 11.5 mg |
| | | SFC Rt = 1.467 min |
| | | [a]_{D}²⁰ = +41.3° (c = 0.05200, MeOH) |
| | | ¹H NMR: (400 MHz, DMSO-*d*₆) δ 9.53 (d, *J* = 1.4 Hz, 1H), 7.62 (s, 1H), 7.09 (d, *J* = 11.5 Hz, 1H), 6.82 (s, 2H), 6.76 (s, 2H), 3.86 (s, 3H), 2.19 (s, 3H), 1.76 (s, 3H), 1.71 (s, 3H). |
| | | MS (ESI) m/z: 359.0 [M+H⁺] |
| 158.2 | 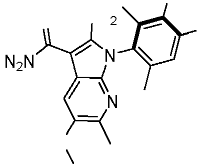 | 11.12 mg |
| | | SFC Rt = 2.260 min |
| | | [a]_{D}²⁰ = -35.5° (c = 0.05074, MeOH) |
| | | ¹H NMR (400 MHz, DMSO-*d*₆) δ 9.53 (s, 1H), 7.62 (s, 1H), 7.09 (d, *J* = 11.4 Hz, 1H), 6.80 (d, *J* = 11.2 Hz, 2H), 6.76 (s, 2H), 3.86 (s, 3H), 2.19 (s, 3H), 1.76 (s, 3H), 1.71 (s, 3H). |
| | | MS (ESI) m/z: 358.9 [M+H⁺] |
| The chiral separation method of Example 158.1 and Example 158.2 is as follows: | | |
| SFC method: instrument: SFC-150mgm (waters), chiral column: Daicel OZ (25 * 250 mm, 10 µm), temperature: 30°C, mobile phase: CO₂/MeOH [0.2% NH₃ (7M MeOH)] = 50/50, flow rate: 100 mL/min, back pressure: 100 bar, detection wavelength: 214 nm, cycle time: 6.03 min. | | |
| 159 | 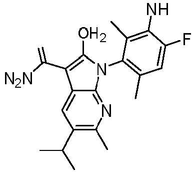 | 169.45 mg |
| | | ¹H NMR: (400 MHz, DMSO-*d*₆) δ 9.56 (d, *J =* 1.4 Hz, 1H), 7.82 (s, 1H), 7.10 (d, *J* = 11.4 Hz, 1H), 6.85 (s, 2H), 6.73 (s, 2H), 3.08 (p, *J =* 6.8 Hz, 1H), 2.32 (s, 3H), 1.76 (s, 3H), 1.71 (s, 3H), 1.26 (d, *J =* 6.8 Hz, 6H). |
| | | MS (ESI) m/z: 371.2 [M+H⁺] |
| 159.1 | 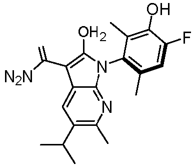 | 48.68 mg |
| | | SFC Rt = 1.700 min |
| | | [a]_{D}²⁰ = +30.2° (c = 0.10480, MeOH) |
| | | ¹H NMR: (400 MHz, DMSO-*d*₆) δ 9.53 (s, 1H), 7.82 (s, 1H), 7.09 (d, *J* = 11.6 Hz, 1H), 6.83 (s, 2H), 6.71 (s, 2H), 3.09 (dt, *J =* 13.8, 6.8 Hz, 1H), 2.32 (s, 3H), 1.76 (s, 3H), 1.71 (s, 3H), 1.26 (d, *J =* 6.8 Hz, 6H). |
| | | MS (ESI) m/z: 371.3 [M+H⁺] |
| 159.2 | 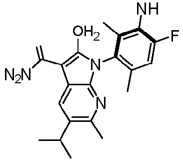 | 37.78 mg |
| | | SFC Rt = 2.452 min |
| | | [a]_{D}²⁰ = -28.9° (c = 0.10344, MeOH) |
| | | ¹H NMR: (400 MHz, DMSO-*d*₆) δ 9.53 (s, 1H), 7.82 (s, 1H), 7.09 (d, *J* = 11.6 Hz, 1H), 6.83 (s, 2H), 6.71 (s, 2H), 3.09 (dt, *J =* 13.8, 7.0 Hz, 1H), 2.32 (s, 3H), 1.76 (d, *J* = 2.8 Hz, 3H), 1.71 (s, 3H), 1.26 (d, *J* = 6.8 Hz, 6H). |
| | | MS (ESI) m/z: 371.3 [M+H⁺] |
| The chiral separation method of Example 159.1 and Example 159.2 is as follows: | | |
| SFC method-instrument: SFC-150mgm (waters), chiral column: Daicel OZ (25 * 250 mm, 10 µm), temperature: 30°C, mobile phase: CO₂/MeOH [0.2% NH₃ (7M MeOH)] = 50/50, flow rate: 100 mL/min, back pressure: 100 bar, detection wavelength: 214 nm, cycle time: 7.78 min. | | |
| 160 | 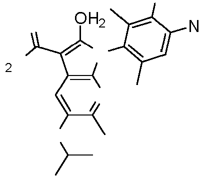 | 6.11 mg |
| | | ¹H NMR: (400 MHz, DMSO-*d*₆) δ 9.54 (s, 1H), 7.65 (s, 1H), 7.09 (d, *J* = 11.5 Hz, 1H), 6.84 (s, 2H), 6.72 (s, 2H), 4.67 (p, *J =* 6.0 Hz, 1H), 2.18 (s, 3H), 1.76 (s, 3H), 1.71 (s, 3H), 1.27 (d, *J =* 6.0 Hz, 6H) |
| | | MS(ESI) m/z: 387.4 [M+H⁺] |
| 161 | 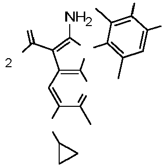 | 2.12 mg |
| | | ¹H NMR: (400 MHz, DMSO-*d*₆) δ 9.56 (s, 1H), 9.07 (s, 1H), 7.37 (s, 1H), 7.13 - 7.01 (m, 2H), 6.97 (s, 2H), 4.65 (d, *J* = 5.6 Hz, 1H), 2.18 (s, 3H), 1.82 - 1.68 (m, 8H), 0.86 (td, *J* = 7.4, 3.5 Hz, 2H). |
| | | MS(ESI) m/z: 385.1 [M+H⁺] |
| 162 | 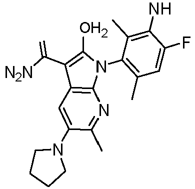 | 165.37mg |
| | | ¹H NMR (400 MHz, DMSO-*d*₆) δ 9.55 (s, 1H), 7.63 (s, 1H), 7.09 (d, *J =* 11.5 Hz, 1H), 6.81 (s, 2H), 6.69 (s, 2H), 3.11 - 3.02 (m, 4H), 2.27 (s, 3H), 1.92 - 1.83 (m, 4H), 1.76 (s, 3H), 1.71 (s, 3H). |
| | | MS (ESI) m/z: 397.8 [M+H⁺] |
| 162.1 | 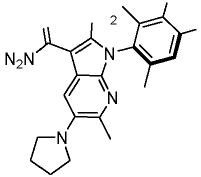 | 41.63 mg |
| | | SFC Rt = 1.996 min |
| | | [a]_{D}²⁰ = +42.4° (c = 0.05141, MeOH) |
| | | ¹H NMR: (400 MHz, DMSO-*d*₆) δ7.63 (s, 1H), 7.09 (d, J = 11.5 Hz, 1H), 6.79 (s, 2H), 6.67 (s, 2H), 3.07 (t, J = 6.0 Hz, 4H), 2.27 (s, 3H), 1.91 - 1.85 (m, 4H), 1.76 (s, 3H), 1.72 (s, 3H). |
| | | MS (ESI) m/z: 398.4 [M+H⁺] |
| 162.2 | 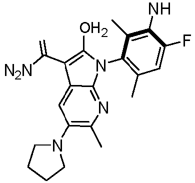 | 36.39 mg |
| | | SFC Rt = 3.158 min |
| | | [a]_{D}²⁰ = -31.0° (c = 0.05097, MeOH) |
| | | ¹H NMR: (400 MHz, DMSO-*d*₆) δ7.63 (s, 1H), 7.09 (d, J = 11.5 Hz, 1H), 6.79 (s, 2H), 6.67 (s, 2H), 3.07 (t, J = 6.0 Hz, 4H), 2.27 (s, 3H), 1.91 - 1.85 (m, 4H), 1.76 (s, 3H), 1.72 (s, 3H). |
| | | MS (ESI) m/z: 398.4 [M+H⁺] |
| The chiral separation method of Example 162.1 and Example 162.2 is as follows: | | |
| SFC method: instrument: SFC-150mgm (waters), chiral column: Daicel OZ (25 * 250 mm, 10 µm), temperature: 30°C, mobile phase: CO₂/MeOH [0.2% NH₃ (7M MeOH)] = 50/50, flow rate: 100 mL/min, back pressure: 100 bar, detection wavelength: 214 nm, cycle time: 9.12 min. | | |
| 163 | 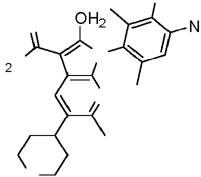 | 13.72 mg |
| | | ¹H NMR: (400 MHz, DMSO-*d*₆) δ 9.54 (s, 1H), 7.85 (s, 1H), 7.09 (d, *J* = 11.4 Hz, 1H), 6.85 (s, 2H), 6.74 (s, 2H), 3.98 (dd, *J =* 10.8, 4.0 Hz, 2H), 3.47 (dd, *J =* 12.5, 10.5 Hz, 2H), 2.93 (t, *J* = 12.0 Hz, 1H), 2.34 (s, 3H), 1.96 (qd, *J* = 12.4, 4.4 Hz, 2H), 1.76 (s, 3H), 1.71 (s, 3H), 1.63 - 1.55 (m, 2H). |
| | | MS(ESI) M/Z: 413.24 [M+H⁺] |
| 163.1 | 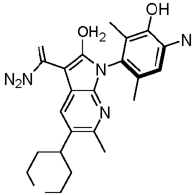 | 26.08 mg |
| | | ¹H NMR: (400 MHz, DMSO-*d*₆) δ 9.53 (s, 1H), 7.85 (s, 1H), 7.09 (d, J = 11.5 Hz, 1H), 6.85 (d, J = 11.2 Hz, 2H), 6.75 (s, 2H), 3.98 (dd, J = 10.8, 3.6 Hz, 2H), 3.47 (t, J = 11.0 Hz, 2H), 2.93 (t, J = 11.9 Hz, 1H), 2.34 (s, 3H), 1.96 (qd, J = 12.3, 3.9 Hz, 2H), 1.76 (s, 3H), 1.71 (s, 3H), 1.59 (d, J = 12.6 Hz, 2H). |
| | | MS (ESI) m/z: 413.2 [M+H⁺] |
| 164 | 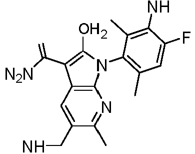 | 2.5 mg |
| | | ¹H NMR: (400 MHz, DMSO-*d*₆) δ 9.54 (s, 1H), 7.94 (s, 1H), 7.11 (d, *J =* 11.5 Hz, 1H), 6.84 (s, 2H), 6.66 (s, 2H), 4.92 (t, *J =* 5.3 Hz, 1H), 4.52 (d, *J =* 5.3 Hz, 2H), 2.32 (s, 3H), 1.76 (s, 3H), 1.71 (s, 3H). |
| | | MS(ESI)M/Z: 359.2 [M+H⁺] |
| 165 | 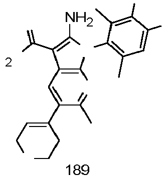 | 2.92 mg |
| | | ¹H NMR: (400 MHz, DMSO-*d*₆) δ 9.49 (s, 1H), 7.77 (s, 1H), 7.10 (d, *J =* 11.4 Hz, 1H), 6.90 (s, 2H), 6.70 (s, 2H), 5.72 - 5.66 (m, 1H), 4.20 (q, *J* = 2.7 Hz, 2H), 3.84 (t, *J =* 5.4 Hz, 2H), 2.37 (s, 2H), 2.28 (s, 3H), 1.77 (s, 3H), 1.72 (s, 3H) |
| | | MS (ESI) m/z: 411.3 [M+H⁺] |
| 166 | 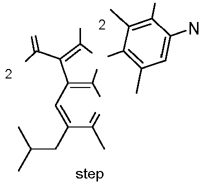 | 22.93 mg |
| | | ¹H NMR: (400 MHz, DMSO-*d*₆) δ 9.52 (s, 1H), 7.77 (s, 1H), 7.09 (d, *J* = 11.5 Hz, 1H), 6.84 (s, 2H), 6.65 (s, 2H), 2.47 (s, 2H), 2.27 (s, 3H), 1.90 (dt, *J =* 13.4, 6.7 Hz, 1H), 1.76 (s, 3H), 1.71 (s, 3H), 0.91 (d, *J =* 6.6 Hz, 6H) |
| | | MS(ESI)M/Z: 385.4 [M+H⁺] |
| 166.1 | 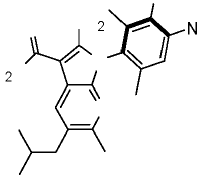 | 49.29 mg |
| | | SFC Rt = 1.335 min |
| | | [a]_{D}²⁰ = -28.2° (c = 0.10241, MeOH) |
| | | ¹H NMR: (400 MHz, DMSO-*d*₆) δ 9.53 (s, 1H), 7.77 (s, 1H), 7.09 (d, *J* = 11.5 Hz, 1H), 6.84 (s, 2H), 6.65 (s, 2H), 2.47 (s, 2H), 2.27 (s, 3H), 1.93-1.87 (m, 1H), 1.76 (s, 3H), 1.71 (s, 3H), 0.91 (d, *J =* 6.6 Hz, 6H). MS (ESI) m/z: 385.0 [M+H⁺] |
| 166.2 | 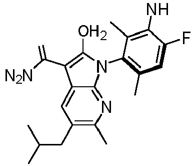 | 51.02 mg |
| | | SFC Rt = 1.657 min |
| | | [a]_{D}²⁰ = +39.7° (c = 0.10193, MeOH) ¹H NMR: (400 MHz, DMSO-*d*₆) δ 9.52 (s, 1H), 7.77 (s, 1H), 7.09 (d, *J* = 11.5 Hz, 1H), 6.84 (s, 2H), 6.65 (s, 2H), 2.47 (s, 2H), 2.27 (s, 3H), 1.93-1.87 (m, 1H), 1.76 (s, 3H), 1.71 (s, 3H), 0.91 (d, *J =* 6.6 Hz, 6H). |
| | | MS (ESI) m/z: 385.2 [M+H⁺] |
| The chiral separation method of Example 166.1 and Example 166.2 is as follows: | | |
| SFC method-instrument: SFC-150mgm (waters), chiral column: Daicel IC (25 * 250 mm, 10 µm), temperature: 30°C, mobile phase: CO₂/MeOH [0.2% NH₃ (7M MeOH)] = 60/40, flow rate: 100 mL/min, back pressure: 100 bar, detection wavelength: 214 nm, cycle time: 3.5 min. | | |
| 167 | 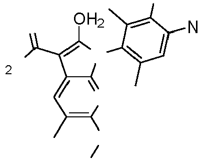 | 1.35 mg |
| | | ¹H NMR: (400 MHz, Methanol-*d₄*) δ 7.73 (s, 1H), 6.98 (d, *J =* 11.3 Hz, 1H), 3.69 (s, 3H), 2.24 (s, 3H), 1.88 (s, 3H), 1.85 (s, 3H). |
| | | MS (ESI) m/z: 359.3 [M+H⁺] |
| 168 | 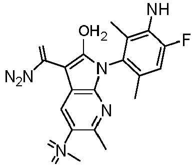 | 6.82 mg |
| | | ¹H NMR: (400 MHz, DMSO-*d*₆) δ 9.65 (s, 1H), 8.36 (s, 1H), 7.18 - 7.08 (m, 3H), 6.91 (s, 2H), 3.26 (s, 3H), 2.64 (s, 3H), 1.77 (s, 3H), 1.72 (s, 3H). |
| | | MS(ESI) m/z: 407.5 [M+H⁺] |
| 169 | 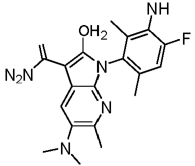 | 4.2 mg |
| | | ¹H NMR: (400 MHz, DMSO-*d*₆) δ 7.74 (s, 1H), 7.09 (d, J = 11.5 Hz, 1H), 6.81(s, 2H), 6.70 (s, 2H), 2.65 (s, 6H), 2.29 (s, 3H), 1.76 (s, 3H),1.71 (s, 3H). |
| | | MS (ESI) m/z: 372.4 [M+H⁺] |
| 169.1 | 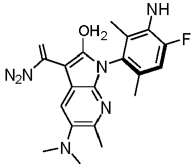 | 10.14 mg |
| | | SFC Rt = 1.582 min |
| | | [a]_{D}²⁰ = +35.9° (c = 0.07996, MeOH) ¹H NMR: (400 MHz, DMSO-*d*₆) δ9.53 (s, 1H), 7.74 (s, 1H), 7.09 (d, J = 11.5 Hz, 1H), 6.79 (s, 1H), 6.70 (s, 1H), 2.65 (s, 6H), 2.29 (s, 3H), 1.76 (s, 3H), 1.71 (s, 3H). |
| | | MS (ESI) m/z: 372.4 [M+H⁺] |
| 169.2 | 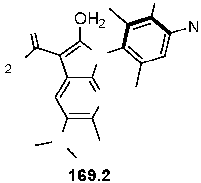 | 10.25 mg |
| | | SFC Rt = 2.351 min |
| | | [a]_{D}²⁰ = -26.5° (c = 0.08071, MeOH) |
| | | ¹H NMR: (400 MHz, DMSO-*d*₆) δ9.53 (s, 1H), 7.74 (s, 1H), 7.09 (d, J = 11.4 Hz, 1H), 6.79 (s, 1H), 6.70 (s, 1H), 2.65 (s, 6H), 2.29 (s, 3H), 1.76 (s, 3H), 1.71 (s, 3H). |
| | | MS (ESI) m/z: 372.4 [M+H⁺] |
| The chiral separation method of Example 169.1 and Example 169.2 is as follows: | | |
| SFC method-instrument: SFC-150mgm (waters), chiral column: Daicel OZ-3 (25 * 250 mm, 10 µm), temperature: 30°C, mobile phase: CO₂/MeOH [0.2% NH₃ (7M MeOH)] = 50/50, flow rate: 100 mL/min, back pressure: 100 bar, detection wavelength: 214 nm, cycle time: 10 min. | | |
| 170 | 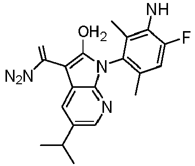 | 130.05 mg |
| | | ¹H NMR: (400 MHz, DMSO-*d*₆) δ 9.53 (s, 1H), 7.90 (d, *J =* 1.6 Hz, 1H), 7.64 (d, *J =* 1.6 Hz, 1H), 7.10 (d, *J =* 11.6 Hz, 1H), 6.98 (s, 2H), 6.75 (s, 2H), 2.98 - 2.86 (m, 1H), 1.75 (s, 3H), 1.71 (s, 3H), 1.28 (d, *J =* 6.8 Hz, 6H). |
| | | MS(ESI) m/z: 357.35 [M+H⁺] |
| 170.1 | 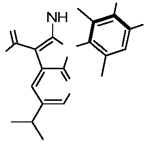 | 42.78 mg |
| | | SFC Rt = 1.248 min |
| | | [a]_{D}²⁰ = -23.3° (c = 0.1046, MeOH) |
| | | ¹H NMR: (400 MHz, DMSO-*d*₆) δ 9.53 (s, 1H), 7.90 (d, *J =* 1.8 Hz, 1H), 7.64 (d, *J =* 1.6 Hz, 1H), 7.09 (d, *J =* 11.4 Hz, 1H), 6.98 (s, 2H), 6.75 (s, 2H), 2.93 (dt, *J =* 13.8, 6.8 Hz, 1H), 1.75 (s, 3H), 1.71 (s, 3H), 1.29 (s, 3H), 1.27 (s, 3H). |
| | | MS (ESI) m/z: [M+H⁺] 357.3 |
| 170.2 | 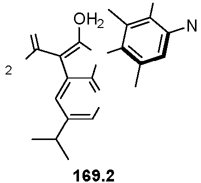 | 39.87 mg |
| | | SFC Rt = 1.504 min |
| | | [a]_{D}²⁰ = +22.9° (c = 0.1046, MeOH) |
| | | ¹H NMR: (400 MHz, DMSO-*d*₆) δ 9.53 (s, 1H), 7.90 (d, *J =* 1.7 Hz, 1H), 7.64 (d, *J =* 1.6 Hz, 1H), 7.09 (d, *J =* 11.6 Hz, 1H), 6.98 (s, 2H), 6.75 (s, 2H), 2.93 (dt, *J =* 13.8, 7.0 Hz, 1H), 1.75 (s, 3H), 1.71 (s, 3H), 1.28 (s, 3H), 1.27 (s, 3H). |
| | | MS (ESI) m/z: 357.3 [M+H⁺] |
| The chiral separation method of Example 170.1 and Example 170.2 is as follows: | | |
| SFC method-instrument: SFC-150mgm (waters), chiral column: Daicel IC (25 * 250 mm, 10 µm), temperature: 30°C, mobile phase: CO₂/MeOH [0.2% NH₃ (7M MeOH)] = 60/40, flow rate: 100 mL/min, back pressure: 100 bar, detection wavelength: 214 nm, cycle time: 4.1 min. | | |
| 171 | 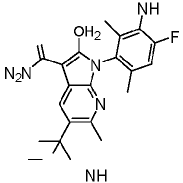 | 2.69 mg |
| | | ¹H NMR: (400 MHz, DMSO-*d*₆) δ 9.54 (s, 1H), 7.71 (s, 1H), 7.10 (d, *J* = 11.5 Hz, 1H), 6.83 (s, 2H), 6.70 (s, 2H), 2.91 (s, 3H), 1.75 (d, *J* = 17.7 Hz, 6H), 1.59 (s, 6H). |
| | | MS(ESI)M/Z: 401.1 [M+H⁺] |
| 172 | 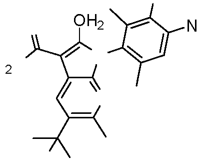 | 72.23 mg |
| | | ¹H NMR: (400 MHz, DMSO-*d*₆) δ 9.56 (s, 1H), 7.84 (s, 1H), 7.10 (d, *J =* 11.4 Hz, 1H), 6.80 (s, 2H), 6.65 (s, 2H), 4.85 (s, 1H), 2.54 (s, 3H), 1.77 (s, 3H), 1.71 (s, 3H), 1.57 (s, 6H). |
| | | MS (ESI) m/z: 387.3 [M+H⁺]. |
| 172.1 | 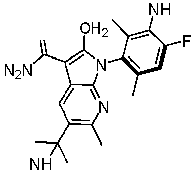 | 88.33 mg |
| | | SFC Rt = 1.540 min |
| | | [a]_{D}²⁰ = +57.3° (c = 0.09460, MeOH) |
| | | ¹H NMR: (400 MHz, DMSO-*d*₆) δ 9.55 (s, 1H), 7.84 (s, 1H), 7.10 (d, J = 11.6 Hz, 1H), 6.79 (s, 2H), 6.64 (s, 2H), 4.84 (s, 1H), 2.54 (s, 3H), 1.77 (s, 3H), 1.71 (s, 3H), 1.57 (s, 6H). |
| | | MS (ESI) m/z: 387.4 [M+H⁺] |
| 172.2 | 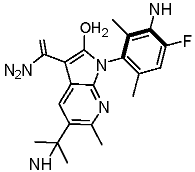 | 87.33 mg |
| | | SFC Rt = 2.137 min |
| | | [a]_{D}²⁰ = -21.9° (c = 0.09451, MeOH) |
| | | ¹H NMR: (400 MHz, DMSO-*d*₆) δ 9.55 (d, J = 1.2 Hz, 1H), 7.84 (s, 1H), 7.10 (d, J = 11.6 Hz, 1H), 6.79 (s, 2H), 6.64 (s, 2H), 4.84 (s, 1H), 2.54 (s, 3H), 1.77 (s, 3H), 1.71 (s, 3H), 1.57 (s, 6H). |
| | | MS (ESI) m/z: 387.4 [M+H⁺] |
| The chiral separation method of Example 172.1 and Example 172.2 is as follows: | | |
| SFC method-instrument: SFC-150mgm (waters), chiral column: Daicel OZ (25 * 250 mm, 10 µm), temperature: 30°C, mobile phase: CO₂/MeOH [0.2% NH₃ (7M MeOH)] = 55/45, flow rate: 100 mL/min, back pressure: 100 bar, detection wavelength: 214 nm, cycle time: 9 min. | | |
| 173 | 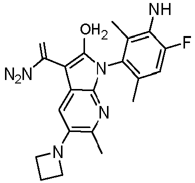 | 378.4 mg |
| | | ¹H NMR: (400 MHz, DMSO-*d*₆) δ 9.52 (s, 1H), 7.14 - 7.04 (m, 2H), 6.76 (s, 2H), 6.66 (s, 2H), 3.82 (t, *J* = 7.1 Hz, 4H), 2.23 - 2.13 (m, 5H), 1.76 (s, 3H), 1.71 (s, 3H). |
| | | MS(ESI) m/z: 384.4 [M+H⁺] |
| 173.1 | 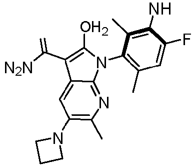 | 156.56 mg |
| | | SFC Rt = 1.702 min |
| | | [a]_{D}²⁰ = -42.4° (c = 0.08885, MeOH) |
| | | ¹H NMR: (400 MHz, DMSO-*d*₆) δ 9.52 (s, 1H), 7.12 (s, 1H), 7.08 (d, J = 11.5 Hz, 1H), 6.75 (s, 2H), 6.65 (s, 2H), 3.82 (t, J = 7.1 Hz, 4H), 2.20 (d, J = 7.2 Hz, 2H), 2.16 (s, 3H), 1.76 (s, 3H), 1.71 (s, 3H). |
| | | MS (ESI) m/z: 384.4 [M+H⁺] |
| 173.2 | 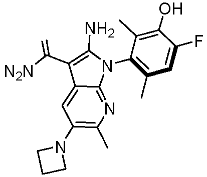 | 99.5 mg |
| | | SFC Rt = 2.062 min |
| | | [a]_{D}²⁰ = +42.5° (c = 0.08517, MeOH) |
| | | ¹H NMR: (400 MHz, DMSO-*d*₆) δ 9.51 (d, J = 1.2 Hz, 1H), 7.12 (s, 1H), 7.08 (d, J = 11.6 Hz, 1H), 6.74 (d, J = 12.0 Hz, 2H), 6.65 (s, 2H), 3.82 (t, J = 7.1 Hz, 4H), 2.19 (d, J = 7.2 Hz, 2H), 2.16 (s, 3H), 1.76 (s, 3H), 1.71 (s, 3H). |
| | | MS (ESI) m/z: 384.4 [M+H⁺] |
| The chiral separation method of Example 173.1 and Example 173.2 is as follows: | | |
| SFC method-instrument: SFC-150mgm (waters), chiral column: Daicel IC (25 * 250 mm, 10 µm), temperature: 30°C, mobile phase: CO₂/MeOH [0.2% NH₃ (7M MeOH)] = 55/45, flow rate: 100 mL/min, back pressure: 100 bar, detection wavelength: 214 nm, cycle time: 6 min. | | |
| 174 | 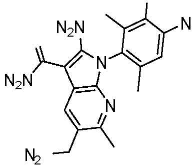 | 7.19 mg |
| | | ¹H NMR: (400 MHz, DMSO-*d*₆) δ 9.57 (s, 1H), 8.00 (s, 1H), 7.11 (d, J = 11.5 Hz, 1H), 6.95 (s, 2H), 6.70 (s, 2H), 3.63 (q, J = 11.3 Hz, 2H), 2.34 (s, 3H), 1.77 (s, 3H), 1.72 (s, 3H). |
| | | MS (ESI) m/z: 411.0 [M+H⁺] |
| 175 | 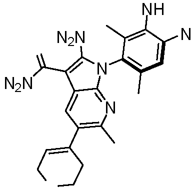 | 20.9 mg |
| | | ¹H NMR: (400 MHz, DMSO-*d*₆) δ 9.55 (s, 1H), 7.77 (s, 1H), 7.10 (d, J = 11.5 Hz, 1H), 6.90 (s, 2H), 6.69 (s, 2H), 5.69 (s, 1H), 4.20 (d, J = 2.5 Hz, 2H), 3.84 (t, J = 5.3 Hz, 2H), 2.37 (s, 2H), 2.28 (s, 3H), 1.77 (s, 3H), 1.72 (s, 3H). |
| | | MS (ESI) m/z: 411.2 [M+H⁺] |
| 176 | 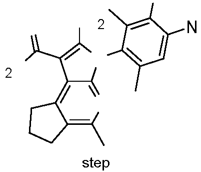 | 191.27 mg |
| | | ¹H NMR: (400 MHz, DMSO-*d*₆) δ 9.54 (s, 1H), 7.08 (d, *J* = 11.5 Hz, 1H), 6.72 (s, 2H), 6.24 (s, 2H), 2.83 (t, *J =* 7.3 Hz, 2H), 2.75 (t, *J =* 7.6 Hz, 2H), 2.22 (s, 3H), 2.10 - 1.86 (m, 2H), 1.75 (s, 3H), 1.70 (s, 3H). |
| | | MS(ESI) m/z: 369.3 [M+H⁺] |
| 176.1 | 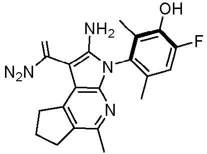 | 8.45 mg |
| | | SFC Rt = 1.954 min |
| | | [a]_{D}²⁰ = -23.1° (c = 0.04686, MeOH) |
| | | ¹H NMR: (400 MHz, DMSO-*d*₆) δ 9.51 (s, 1H), 7.09 (d, *J =* 11.5 Hz, 1H), 6.55 (s, 2H), 6.51 (s, 2H), 3.29 (d, *J =* 7.4 Hz, 2H), 2.8 0 (t, *J =* 7.4 Hz, 2H), 2.22 (s, 3H), 2.10 - 2.02 (m, 2H), 1.76 (s, 3H), 1.70 (s, 3H). |
| | | MS (ESI) m/z: 368.9 [M+H⁺] |
| 176.2 | 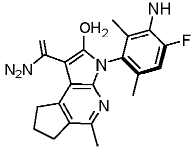 | 8.57 mg |
| | | SFC Rt = 2.444 min |
| | | [a]_{D}²⁰ = +25.9° (c = 0.04949, MeOH) |
| | | ¹H NMR: (400 MHz, DMSO-*d*₆) δ 9.51 (s, 1H), 7.09 (d, *J* = 11.4 Hz, 1H), 6.55 (s, 2H), 6.51 (s, 2H), 3.28 (d, *J =* 7.4 Hz, 2H), 2.80 (t, *J =* 7.6 Hz, 2H), 2.22 (s, 3H), 2.09 - 2.02 (m, 2H), 1.75 (s, 3H), 1.70 (s, 3H). |
| | | MS (ESI) m/z: 369.0 [M+H⁺] |
| The chiral separation method of Example 176.1 and Example 176.2 is as follows: | | |
| SFC method: instrument: SFC-150mgm (waters), chiral column: Daicel IC (25 * 250 mm, 10 µm), temperature: 30°C, mobile phase: CO₂/MeOH [0.2% NH₃ (7M MeOH)] = 55/45, flow rate: 100 mL/min, back pressure: 100 bar, detection wavelength: 214 nm, cycle time: 5.6 min. | | |
| 177 | 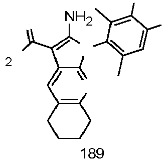 | 222 mg |
| | | ¹H NMR: (400 MHz, DMSO-*d*₆): δ 9.57 (s, 1H), 7.71 (s, 1H), 7.09 (d, 1H), 6.87 (s, 2H), 6.63 (s, 2H), 2.79 (s, 2H), 2.62 (s, 2H), 1.84 - 1.63 (m, 10H). |
| | | MS (ESI) m/z: 369.2 [M+H⁺]. |
| 177.1 | 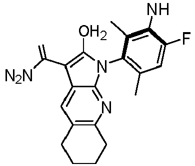 | 90.1 mg |
| | | SFC Rt = 1.435 min |
| | | [a]_{D}²⁰ = -39.3° (0.10271, MeOH) |
| | | ¹H NMR: (400 MHz, DMSO-*d*₆) δ 9.55 (s, 1H), 7.71 (s, 1H), 7.09 (d, *J =* 11.4 Hz, 1H), 6.86 (s, 2H), 6.62 (s, 2H), 2.80 (d, *J =* 5.8 Hz, 2H), 2.62 (t, *J =* 5.6 Hz, 2H), 1.76 (s, 3H), 1.74 (d, *J =* 5.8 Hz, 4H), 1.71 (s, 3H). |
| | | MS (ESI) m/z: 369.2 [M+H⁺] |
| 177.2 | 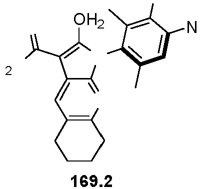 | 88.46 mg |
| | | SFC Rt = 1.845 min |
| | | [a]_{D}²⁰ = +37.0° (0.10593, MeOH) |
| | | ¹H NMR: (400 MHz, DMSO-*d*₆) δ 9.60 (s, 1H), 7.71 (s, 1H), 7.08 (d, *J =* 11.4 Hz, 1H), 6.85 (s, 2H), 6.62 (s, 2H), 2.80 (d, *J =* 5.7 Hz, 2H), 2.62 (t, *J =* 5.6 Hz, 2H), 1.76 (s, 3H), 1.74 (d, *J =* 6.0 Hz, 4H), 1.71 (s, 3H). |
| | | MS (ESI) m/z: 369.2 [M+H⁺] |
| The chiral separation method of Example 177.1 and Example 177.2 is as follows: | | |
| SFC method-instrument: SFC-150mgm (waters), chiral column: Daicel IC (25 * 250 mm, 10 µm), temperature: 30°C, mobile phase: CO₂/MeOH [0.2% NH₃ (7M MeOH)] = 55/45, flow rate: 100 mL/min, back pressure: 100 bar, detection wavelength: 214 nm, cycle time: 4.42 min. | | |
| 178 | 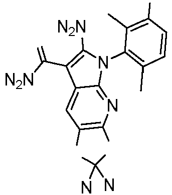 | 8.39 mg |
| | | ¹H NMR: (400 MHz, DMSO-*d*₆) δ 9.57 (s, 1H), 8.22 (s, 1H), 7.10 - 7.06 (m, 1H), 6.95 - 6.90 (m, 3H), 6.89 - 6.85 (m, 2H), 1.75 (s, 3H), 1.67 (s, 3H). |
| | | MS (ESI) m/z: 377.1 [M+H⁺] |
| 179 | 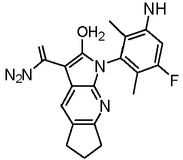 | 21.32 mg |
| | | ¹H NMR: (400 MHz, DMSO-*d*₆) δ 10.01 (s, 1H), 7.90 (s, 1H), 6.88 (s, 2H), 6.79 (d, *J =* 11.1 Hz, 1H), 6.69 (s, 2H), 2.90 (t, *J =* 7.4 Hz, 2H), 2.75 (t, *J =* 7.5 Hz, 2H), 2.10 - 1.97 (m, 2H), 1.65 (s, 3H), 1.62 (s, 3H). |
| | | MS (ESI) m/z: 355.1 [M+H⁺]. |
| 180 | 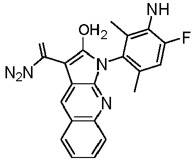 | 27.74 mg |
| | | ¹H NMR (400 MHz, DMSO-*d*₆) δ 9.62 (s, 1H), 8.45 (s, 1H), 7.90 - 7.82 (m, 1H), 7.69 (d, J = 8.0 Hz, 1H), 7.49 (s, 2H), 7.45 - 7.36 (m, 2H), 7.16 (d, J = 11.5 Hz, 1H), 6.87 (s, 2H), 1.81 (s, 3H), 1.76 (s, 3H). |
| | | MS (ESI) m/z: 365.1[M+H⁺] |
| 181 | 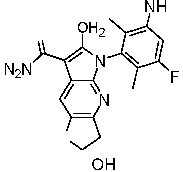 | 13.6 mg |
| | | ¹H NMR: (400 MHz, DMSO-*d*₆) δ 9.98 (s, 1H), 7.52 (s, 1H), 6.88 (s, 2H), 6.79 (d, *J* = 10.8 Hz, 1H), 6.66 (s, 2H), 4.57 - 4.52 (m, 2H), 3.13 - 3.07 (m, 2H), 1.67 - 1.65 (m, 3H), 1.63 (s, 3H). |
| | | MS (ESI) m/z: 357.13 [M+H⁺] |
| 182 | 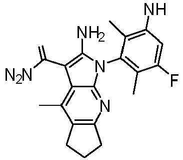 | 2.55 mg |
| | | ¹H NMR: (400 MHz, DMSO-*d*₆) δ 9.97 (s, 1H), 6.81 - 6.72 (m, 3H), 6.27 (s, 2H), 2.83 (t, *J* = 7.4 Hz, 2H), 2.75 (t, *J* = 7.6 Hz, 2H), 2.5 (s, 3H), 2.10 - 1.95 (m, 2H), 1.65 (s, 3H), 1.60 (s, 3H) |
| | | MS (ESI) m/z: 369.3 [M+H⁺] |
| 183 | 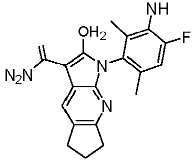 | 159.67 mg |
| | | ¹H NMR: (400 MHz, DMSO-*d*₆) δ 9.57 (d, *J =* 1.3 Hz, 1H), 7.89 (s, 1H), 7.10 (d, *J* = 11.5 Hz, 1H), 6.84 (s, 2H), 6.66 (s, 2H), 2.90 (t, *J =* 7.3 Hz, 2H), 2.74 (t, *J =* 7.5 Hz, 2H), 2.10 - 1.97 (m, 2H), 1.76 (s, 3H), 1.71 (s, 3H) |
| | | MS (ESI) m/z: 354.8 [M+H⁺] |
| 184 | 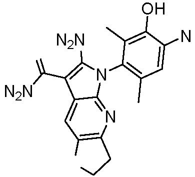 | 60 mg |
| | | ¹H NMR: (400 MHz, DMSO-*d*₆) δ 9.55 (s, 1H), 7.51 (s, 1H), 7.09 (d, J = 11.5 Hz, 1H), 6.84 (s, 2H), 6.65 (s, 2H), 4.54 (t, J = 8.7 Hz, 2H), 3.10 (t, J = 8.7 Hz, 2H), 1.77 (s, 3H), 1.72 (s, 3H). |
| | | MS (ESI) m/z: 357.2 [M+H⁺] |
| 185 | 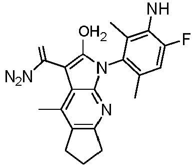 | 51.3 mg |
| | | ¹H NMR: (400 MHz, DMSO-*d*₆) δ 9.54 (s, 1H), 7.09 (d, *J =* 11.5 Hz, 1H), 6.62 - 6.43 (m, 4H), 3.29 (t, *J =* 7.4 Hz, 2H), 2.80 (t, *J =* 7.5 Hz, 2H), 2.22 (s, 3H), 2.12 - 2.00 (m, 2H), 1.75 (s, 3H), 1.70 (s, 3H). |
| | | MS(ESI) m/z: 369.2 [M+H⁺] |
| 186 | 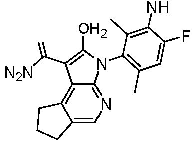 | 74.9 mg |
| | | ¹H NMR: (400 MHz, DMSO-*d*₆) δ 9.51 (s, 1H), 7.68 (s, 1H), 7.09 (d, *J* = 11.5 Hz, 1H), 6.70 (s, 2H), 6.59 (s, 2H), 3.30 (q, *J =* 7.7 Hz, 2H), 2.86 (t, *J =* 7.4 Hz, 2H), 2.12 - 2.00 (m, 2H), 1.75 (s, 3H), 1.70 (s, 3H) |
| | | MS(ESI) m/z: 355.1 [M+H⁺] |
| 186.1 | 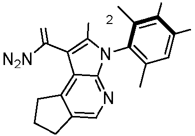 | 17.69 mg |
| | | SFC Rt = 1.750 min |
| | | [a]_{D}²⁰ = -24.4° (c = 0.09304, MeOH) |
| | | ¹H NMR: (400 MHz, DMSO-*d*₆) δ 9.52 (s, 1H), 7.68 (s, 1H), 7.09 (d, *J* = 11.6 Hz, 1H), 6.69 (s, 2H), 6.57 (s, 2H), 3.28 (d, *J* = 7.4 Hz, 2H), 2.86 (t, *J =* 7.4 Hz, 2H), 2.11 - 2.01 (m, 2H), 1.75 (s, 3H), 1.70 (s, 3H). |
| | | MS (ESI) m/z: 355.3 [M+H⁺] |
| 186.2 | 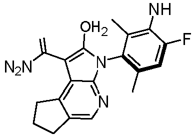 | 19.99 mg |
| | | SFC Rt = 2.224 min |
| | | [a]_{D}²⁰ = +22.3° (c = 0.09151, MeOH) |
| | | ¹H NMR: (400 MHz, DMSO-*d*₆) δ 9.52 (s, 1H), 7.67 (s, 1H), 7.09 (d, *J* = 11.6 Hz, 1H), 6.69 (s, 2H), 6.57 (s, 2H), 3.28 (d, *J =* 7.4 Hz, 2H), 2.86 (t, *J =* 7.4 Hz, 2H), 2.10 - 2.02 (m, 2H), 1.75 (s, 3H), 1.70 (s, 3H). |
| | | MS (ESI) m/z: 355.3 [M+H⁺] |
| The chiral separation method of Example 186.1 and Example 186.2 is as follows: | | |
| SFC method-instrument: SFC-150mgm (waters), chiral column: Daicel IC (25 * 250 mm, 10 µm), temperature: 30°C, mobile phase: CO₂/MeOH [0.2% NH₃ (7M MeOH)] = 55/45, flow rate: 100 mL/min, back pressure: 100 bar, detection wavelength: 214 nm, cycle time: 4.55 min. | | |
| 187 | 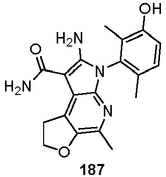 | 1.01 mg |
| | | ¹H NMR: (400 MHz, Methanol-*d₄*) δ 7.08 (d, *J =* 8.3 Hz, 1H), 6.90 (d, *J =* 8.3 Hz, 1H), 4.63 (t, *J =* 8.7 Hz, 2H), 3.66 (t, *J =* 8.6 Hz, 2H), 2.25 (s, 3H), 1.84 (s, 3H), 1.79 (s, 3H). |
| | | MS (ESI) m/z: 353.1 [M+H⁺] |
| 188 | 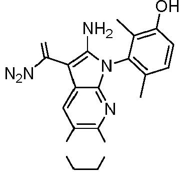 | 242.86 mg |
| | | ¹H NMR: (400 MHz, DMSO-*d*₆) δ 9.48 (s, 1H), 7.67 (s, 1H), 7.06 - 7.02 (m, 1H), 6.91- 6.88 (m,1H), 6.69 (s, 2H), 6.61 (s, 2H), 4.28 - 4.24 (m, 2H), 4.19 - 4.15 (m, 2H), 1.75 (s, 3H), 1.66 (s, 3H). |
| | | MS (ESI) m/z: 355.2 [M+H⁺] |
| 188.1 | 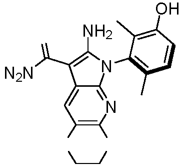 | 91.13 mg |
| | | SFC Rt = 0.931 min |
| | | [a]_{D}²⁰: +53.66° (c = 0.09355, MeOH) |
| | | ¹H NMR: (400 MHz, DMSO-*d*₆) δ 9.48 (s, 1H), 7.67 (s, 1H), 7.04 (d, J = 8.4 Hz, 1H), 6.90 (d, J = 8.2 Hz, 1H), 6.67 (d, J = 12.2 Hz, 2H), 6.61 (s, 2H), 4.27 (dd, J = 5.6, 2.2 Hz, 2H), 4.21 - 4.12 (m, 2H), 1.75 (s, 3H), 1.66 (s, 3H). |
| | | MS (ESI) m/z: 355.3 [M+H⁺] |
| 188.2 | 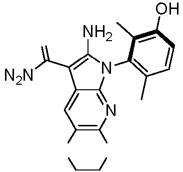 | 85.27 mg |
| | | SFC Rt = 1.106 min |
| | | [a]_{D}²⁰ = -49.60° (c = 0.07904, MeOH) |
| | | ¹H NMR: (400 MHz, DMSO-*d*₆) δ 9.48 (s, 1H), 7.67 (s, 1H), 7.04 (d, J = 8.4 Hz, 1H), 6.90 (d, J = 8.2 Hz, 1H), 6.67 (d, J = 12.2 Hz, 2H), 6.61 (s, 2H), 4.27 (dd, J = 5.6, 2.2 Hz, 2H), 4.21 - 4.12 (m, 2H), 1.75 (s, 3H), 1.66 (s, 3H). |
| | | MS (ESI) m/z: 355.3 [M+H⁺] |
| The chiral separation method of Example 188.1 and Example 188.2 is as follows: | | |
| SFC method-instrument: SFC-150mgm (waters), chiral column: Daicel OJ (25 * 250 mm, 10 µm), temperature: 30°C, mobile phase: CO₂/MeOH [0.2% NH₃ (7M MeOH)] = 80/20, flow rate: 100 mL/min, back pressure: 100 bar, detection wavelength: 214 nm, cycle time: 5 min. | | |
| 189 | 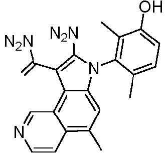 | 3.76 mg |
| | | ¹H NMR: (400 MHz, DMSO-*d*₆) δ 9.78 (s, 1H), 8.26 - 8.16 (m, 2H), 8.15 (s, 1H), 7.76 (d, *J =* 5.9 Hz, 1H), 7.23 (d, *J* = 8.3 Hz, 1H), 7.07 (d, *J* = 8.3 Hz, 1H), 6.93 (s, 2H), 6.53 (s, 2H), 2.67 (s, 3H), 1.77 (s, 3H), 1.68 (s, 3H). |
| | | MS(ESI) m/z: 361.3 [M+H⁺] |
| 189.1 | 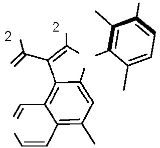 | 13.08 mg |
| | | SFC Rt = 3.06 min |
| | | [a]_{D}²⁰ = -26.17° (0.09401, MeOH) |
| | | ¹H NMR (400 MHz, DMSO) δ 9.79 (s, 1H), 8.23 (d, *J* = 5.8 Hz, 1H), 8.19 (s, 1H), 8.14 (d, *J* =0.8 Hz, 1H), 7.76 (d, *J =* 5.8 Hz, 1H), 7.23 (d, *J =* 8.3 Hz, 1H), 7.07 (d, *J* = 8.3 Hz, 1H), 6.94 (s, 2H), 6.54 (s, 2H), 2.67 (s, 3H), 1.77 (s, 3H), 1.68 (s, 3H). |
| | | MS (ESI) m/z: 361.4[M+H⁺]. |
| 189.2 | 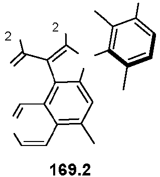 | 14.52 mg |
| | | SFC Rt = 3.34min |
| | | [a]_{D}²⁰ = +39.67° (0.09201, MeOH) |
| | | ¹H NMR (400 MHz, DMSO) δ 9.81 (s, 1H), 8.23 (d, *J* = 5.8 Hz, 1H), 8.17 (d, *J =* 14.9 Hz, 2H), 7.76 (d, *J =* 5.8, 0.8 Hz, 1H), 7.22 (d, *J =* 8.3 Hz, 1H), 7.07 (d, *J =* 8.3 Hz, 1H), 6.93 (s, 2H), 6.54 (s, 2H), 2.67 (s, 3H), 1.76 (s, 3H), 1.68 (s, 3H). |
| | | MS (ESI) m/z: 361.4 [M+H⁺]. |
| The chiral separation method of Example 189.1 and Example 189.2 is as follows: | | |
| SFC method: instrument: SFC-150mgm (waters), chiral column: Daicel IC (25 * 250 mm,10 µm), temperature: 30°C, mobile phase: CO₂/MeOH [0.2% NH₃ (7M MeOH)] = 65/35, flow rate: 100 mL/min, back pressure: 100 bar, detection wavelength: 214 nm, cycle time: 13 min. | | |
| 190 | 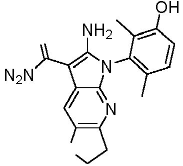 | 7.88 mg |
| | | ¹H NMR: (400 MHz, DMSO-*d*₆) δ 9.48 (s, 1H), 7.51 (s, 1H), 7.05 - 7.02 (m, 1H), 6.91 - 6.88 (m, 1H), 6.75 (s, 2H), 6.64 (s, 2H), 4.54 (t, J = 8.8 Hz, 2H), 3.12 - 3.06 (m,2H), 1.76 (s, 3H), 1.67 (s, 3H). |
| | | MS (ESI) m/z: 339.1 [M+H⁺] |
| 190.1 | 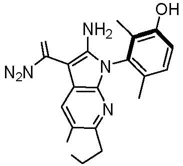 | 33.26 mg |
| | | SFC Rt = 1.224 min |
| | | [a]_{D}²⁰ = -31.1° (c = 0.05270, MeOH) |
| | | ¹H NMR: (400 MHz, DMSO-*d*₆) δ 9.47 (s, 1H), 7.51 (s, 1H), 7.04 (d, *J* = 8.1 Hz, 1H), 6.90 (d, *J =* 8.3 Hz, 1H), 6.75 (s, 2H), 6.64 (s, 2H), 4.54 (t, *J =* 8.8 Hz, 2H), 3.09 (t, *J* = 8.6 Hz, 2H), 1.76 (s, 3H), 1.67 (s, 3H). |
| | | MS (ESI) m/z: 339.2 [M+H⁺] |
| 190.2 | 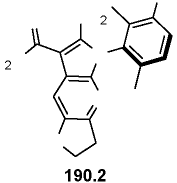 | 36.36 mg |
| | | SFC Rt = 1.426 min |
| | | [a]_{D}²⁰ = +53.1° (c = 0.05081, MeOH) |
| | | ¹H NMR: (400 MHz, DMSO-*d*₆) δ 9.48 (s, 1H), 7.51 (s, 1H), 7.04 (d, *J =* 8.3 Hz, 1H), 6.90 (d, *J =* 8.2 Hz, 1H), 6.75 (s, 2H), 6.64 (s, 2H), 4.54 (t, *J =* 8.6 Hz, 2H), 3.09 (t, *J* = 8.7 Hz, 2H), 1.76 (s, 3H), 1.67 (s, 3H). |
| | | MS (ESI) m/z: 339.2 [M+H⁺] |
| The chiral separation method of Example 190.1 and Example 190.2 is as follows: | | |
| SFC method: instrument: SFC-150mgm (waters), chiral column: Daicel IC (25 * 250 mm, 10 µm), temperature: 30°C, mobile phase: CO₂/MeOH [0.2% NH₃ (7M MeOH)] = 55/45, flow rate: 100 mL/min, back pressure: 100 bar, detection wavelength: 214 nm, cycle time: 3.61 min. | | |
| 191 | 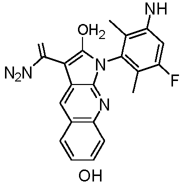 | 18.99 mg |
| | | ¹H NMR: (400 MHz, DMSO-*d*₆) δ: 10.05 (s, 1H), 8.47 (s, 1H), 7.86 (dd, *J =* 8.0, 1.2 Hz, 1H), 7.71 (d, *J =* 8.1 Hz, 1H), 7.53 (br. s, 2H), 7.46 - 7.41 (m, 1H), 7.41 - 7.36 (m, 1H), 6.96 - 6.80 (m, 3H), 1.71 (d, *J* = 1.2 Hz, 3H), 1.67 (s, 3H). |
| | | MS (ESI) m/z: 365.1 [M+H⁺] |
| 192 | 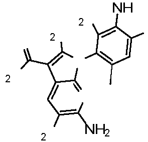 | 101.75 mg |
| | | ¹H NMR: (400 MHz, DMSO-*d*₆) δ 8.26 (s, 1H), 7.14 (d, J = 8.6 Hz, 3H), 6.92 (s, 2H), 1.77 (s, 3H). |
| | | MS (ESI) m/z: 403.4 [M+H⁺] |
| 193 | 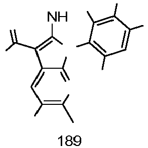 | 9.78 mg |
| | | ¹H NMR: (400 MHz, DMSO-*d*₆) δ9.64 (s, 1H), 8.27 (s, 1H), 7.15 (d, J = 5.7 Hz, 1H), 7.12 (s, 2H), 6.94 (s, 2H), 2.45 (s, 3H). |
| | | MS (ESI) m/z: 403.3 [M+H⁺] |
| 194 | 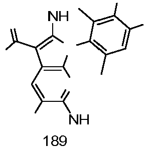 | 69.19 mg |
| | | ¹H NMR: (400 MHz, DMSO-*d*₆) δ 9.65 (s, 1H), 8.27 (s, 1H), 7.15 - 7.12 (m, 3H), 6.93 (s, 2H), 1.76 (s, 3H), 1.72 (s, 3H). |
| | | MS(ESI) m/z: 400.36 [M+H⁺] |
| 194.1 | 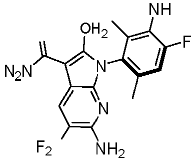 | 21.12 mg |
| | | SFC Rt = 1.746 min |
| | | [a]_{D}²⁰ = +44.1° (c = 0.08728, MeOH) |
| | | ¹H NMR: (400 MHz, DMSO-*d*₆) δ 9.63 (s, 1H), 8.26 (s, 1H), 7.19 - 7.03 (m, 3H), 6.92 (s, 2H), 1.77 (s, 3H), 1.72 (s, 3H). |
| | | MS (ESI) m/z: 400.4 [M+H⁺] |
| 194.2 | 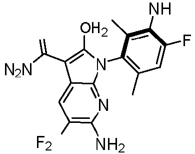 | 21.66 mg |
| | | SFC Rt = 2.436 min |
| | | [a]_{D}²⁰ = -31.3° (c = 0.09132, MeOH) |
| | | ¹H NMR: (400 MHz, DMSO-*d*₆) δ 9.63 (d, *J =* 1.4 Hz, 1H), 8.26 (s, 1H), 7.17 - 7.07 (m, 3H), 6.92 (s, 2H), 1.77 (s, 3H), 1.72 (s, 3H). |
| | | MS (ESI) m/z: 400.4 [M+H⁺] |
| The chiral separation method of Example 194.1 and Example 194.2 is as follows: | | |
| SFC method-instrument: SFC-150 mgm (waters), chiral column: Daicel OZ (25 * 250 mm, 10 µm), temperature: 30°C, mobile phase: CO₂/MeOH [0.2% NH₃ (7M MeOH)] = 65/35, flow rate: 100 mL/min, back pressure: 100 bar, detection wavelength: 214 nm, cycle time: 10 min. | | |
| 195 | 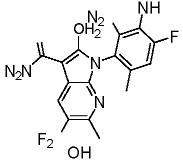 | 2.02 mg |
| | | ¹H NMR: (400 MHz, DMSO-*d*₆) δ 9.63 (s, 1H), 8.27 (s, 1H), 7.23 - 7.03 (m, 3H), 6.93 (s, 2H), 2.45 (d, J = 1.6Hz, 3H), 1.77 (s, 3H). |
| | | MS (ESI) m/z: 400.3 [M+H⁺] |
| 195.1 | 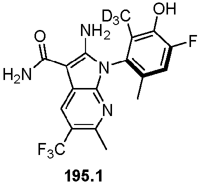 | 101.75 mg |
| | | SFC Rt = 1.099 min |
| | | [a]_{D}²⁰ = +30.8° (c = 0.09719, MeOH) |
| | | ¹H NMR: (400 MHz, DMSO-*d*₆) δ 9.64 (d, J = 1.2 Hz, 1H), 8.27 (s, 1H), 7.22 - 7.04 (m, 3H), 6.94 (s, 2H), 2.45 (d, J = 1.6 Hz, 3H), 1.77 (s, 3H). |
| | | MS (ESI) m/z: 400.4 [M+H⁺] |
| 195.2 | 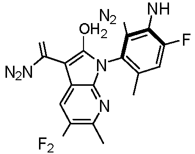 | 108.35 mg |
| | | SFC Rt = 1.455 min |
| | | [a]_{D}²⁰ = -31.8° (c = 0.09426, MeOH) |
| | | ¹H NMR: (400 MHz, DMSO-*d*₆) δ 9.64 (s, 1H), 8.27 (s, 1H), 7.19 - 7.07 (m, 3H), 6.94 (s, 2H), 2.45 (d, J = 1.6 Hz, 3H), 1.77 (s, 3H). |
| | | MS (ESI) m/z: 400.4 [M+H⁺] |
| The chiral separation method of Example 195.1 and Example 195.2 is as follows: | | |
| SFC method-instrument: SFC-150mgm (waters), chiral column: Daicel OZ (25 * 250 mm, 10 µm), temperature: 30°C, mobile phase: CO₂/IPA [0.5% NH₃ (7M MeOH)] = 55/45, flow rate: 100 mL/min, back pressure: 100 bar, detection wavelength: 214 nm, cycle time: 7 min. | | |
| 196 | 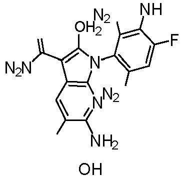 | 55.60 mg |
| | | ¹H NMR (400 MHz, DMSO-*d*₆) δ 9.59 (s, 1H), 8.24 (s, 1H), 7.11 (d, *J =* 11.4 Hz, 1H), 7.06 (s, 2H), 6.79 (s, 2H). |
| | | MS (ESI) m/z: 415.8 [M+H⁺] |
| 202 | 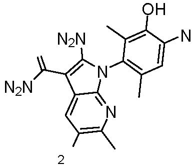 | 8.4 mg |
| | | ¹H NMR (400 MHz, DMSO-*d*₆) δ 9.54 (d, J = 1.3 Hz, 1H), 7.82 (s, 1H), 7.10 (d, J = 11.4 Hz, 1H), 6.79 (d, J = 11.3 Hz, 2H), 6.64 (s, 2H), 2.24 (s, 3H), 1.76 (s, 3H), 1.70 (s, 3H) |
| | | MS (ESI) m/z: 346.2 [M+H⁺] |
| 203 | 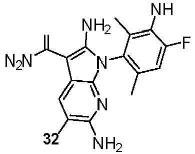 | 346.1 mg |
| | | ¹H NMR: (400 MHz, DMSO-*d*₆) δ 9.64 (s, 1H), 8.25 (s, 1H), 7.12 - 7.02 (m, 3H), 6.80 (s, 2H), 1.75 (s, 3H), 1.70 (s, 3H) |
| | | MS(ESI) m/z: 410.26 [M+H⁺] |
| 203.1 | 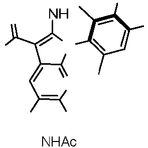 | 85.03 mg |
| | | SFC Rt = 2.280 min |
| | | [a]_{D}²⁰ = -30.6° (c = 0.09239, MeOH) |
| | | ¹H NMR (400 MHz, DMSO-*d*₆) δ 9.61 (s, 1H), 8.24 (s, 1H), 7.26 - 6.96 (m, 3H), 6.79 (s, 2H), 1.76 (s, 3H), 1.71 (s, 3H). |
| | | MS (ESI) m/z: 410.2 [M+H⁺] |
| 203.2 | 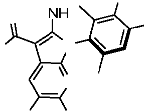 | 108.22 mg |
| | | SFC Rt = 1.508 min |
| | | [a]_{D}²⁰ = +60.1° (c = 0.08717, MeOH) |
| | | ¹H NMR (400 MHz, DMSO-*d*₆) δ 9.59 (d, *J =* 1.4 Hz, 1H), 8.24 (s, 1H), 7.11 (d, *J =* 11.6 Hz, 1H), 7.06 (s, 2H), 6.79 (s, 2H), 1.76 (s, 3H), 1.71 (s, 3H). |
| | | MS (ESI) m/z: 410.0 [M+H⁺] |
| Chiral separation method of Example 203.1 and Example 203.2: | | |
| SFC method-instrument: SFC-150mgm (waters), chiral column: Daicel OZ (25 * 250 mm, 10 µm), temperature: 30°C, mobile phase: CO₂/EtOH [0.5% NH₃ (7M MeOH)] = 50/50, flow rate: 100 mL/min, back pressure: 100 bar, detection wavelength: 214 nm, cycle time: 5.82 min. | | |
| 204 | 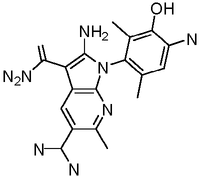 | 2.2 mg |
| | | H¹NMR: (400 MHz, DMSO-*d*₆) ¹H NMR (400 MHz, DMSO-*d*₆) δ 9.60 (s, 1H), 8.16 (s, 1H), 7.31 - 6.96 (m, 4H), 6.81 (s, 2H), 2.41 (s, 3H), 1.77 (s, 3H), 1.71 (s, 3H) |
| | | MS(ESI) m/z: 379.3 [M+H⁺] |
| 205 | 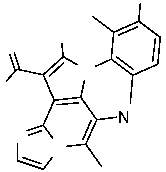 | 7.18 mg |
| | | ¹H NMR (400 MHz, DMSO-*d*₆) δ 10.60 (d, *J =* 2.6 Hz, 1H), 9.60 (s, 1H), 7.79 (d, *J =* 1.5 Hz, 1H), 7.59 (d, *J* = 1.5 Hz, 1H), 7.12 - 7.01 (m, 2H), 6.92 (d, *J =* 8.3 Hz, 1H), 6.68 (s, 2H), 2.45 (d, *J* = 3.0 Hz, 3H), 1.84 (s, 3H), 1.76 (s, 3H). |
| | | MS(ESI) m/z: 368.5 [M+H⁺] |
| 206 | 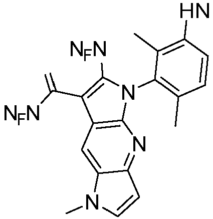 | 10.4 mg |
| | | ¹H NMR (400 MHz, DMSO-*d*₆) δ 9.48 (s, 1H), 8.10 (s, 1H), 7.31 (d, *J* = 3.2 Hz, 1H), 7.06 (d, *J =* 8.3 Hz, 1H), 6.95 - 6.83 (m, 3H), 6.75 (s, 2H), 6.27 (d, *J =* 3.1 Hz, 1H), 3.83 (s, 3H), 1.76 (s, 3H), 1.67 (s, 3H). |
| | | MS (ESI) m/z: 349.8 [M+H⁺]. |
| 207 | 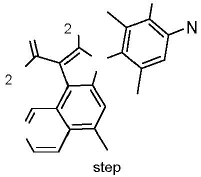 | 112.42 mg |
| | | H¹NMR: (400 MHz, DMSO-*d*₆) δ 9.87 (s, 1H), 8.24 (d, *J* = 5.8 Hz, 1H), 8.17 (d, *J* = 16.0 Hz, 2H), 7.77 (d, *J =* 5.9 Hz, 1H), 7.30 (d, *J* = 11.4 Hz, 1H), 6.94 (s, 2H), 6.64 (s, 2H), 2.67 (s, 3H), 1.79 (s, 3H), 1.74 (s, 3H). |
| | | MS(ESI) m/z:379.3 [M+H⁺] |
| 208 | 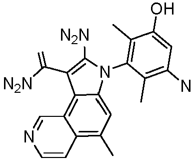 | 43.47 mg |
| | | H¹NMR: (400 MHz, DMSO-*d*₆) δ 10.28 (s, 1H), 8.25 (d, *J* = 6.4 Hz, 1H), 8.20 (s, 2H), 7.78 (d, *J =* 5.8 Hz, 1H), 7.05 - 6.88 (m, 3H), 6.67 (s, 2H), 2.68 (s, 3H), 1.68 (s, 3H), 1.65 (s, 3H). |
| | | MS(ESI) m/z: 379.39 [M+H⁺]. |
| 209-210 | 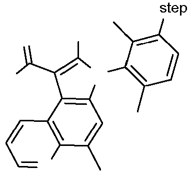 | 310 mg |
| | | ¹H NMR (400 MHz, DMSO-*d*₆) δ 9.31 (dd, *J* = 8.6, 1.7 Hz, 1H), 8.75 (dd, *J =* 4.1, 1.6 Hz, 1H), 8.24 (s, 1H), 7.44 (dd, *J* = 8.6, 4.1 Hz, 1H), 7.14 (d, *J =* 8.3 Hz, 1H), 7.08 (s, 2H), 6.98 (d, *J* = 8.3 Hz, 1H), 6.79 (s, 1H), 5.87 (s, 2H), 2.61 (s, 3H), 1.78 (s, 3H), 1.69 (s, 3H). |
| | | MS (ESI) m/z: 361.1 [M+H⁺]. |

### Biological activity test method:

Biological test example 1. Enzyme inhibition activity of compounds of the present disclosure

The enzyme activity of some of the example compounds was verified using the ADP-GLO method.

### Experimental materials:

PKMYT1 enzyme protein was purchased from Thermo Scientific (Cat. No.: A33387), and the ADP-GLO kit was purchased from Promega (Cat. No.: V9103).

PKMYT1 enzyme activity reaction buffer: 70 mM HEPES, 3 mM MgCl₂, 3 mM MnCl₂, 50 µg mL-1PEG20000, 3 µM sodium orthovanadate, 1.2 mM DTT.

### Experimental steps:

Using an ultra-micro pipette, the test compound (concentration: 3.33 mM, dissolved in DMSO) was diluted with 10 µM as the highest starting concentration, and then sequentially diluted in 10 gradients at a ratio of 1:3 (10000 nM, 3333 nM, 1111 nM, 370 nM, 123 nM, 41 nM, 13.7 nM, 4.6 nM, 1.5 nM, 0.5 nM) in a 384-well reaction plate, with two duplicate wells for each concentration; 16 wells for positive and negative controls were set, with the positive control being the duplicate wells of 1 µM positive compound and the negative control being the duplicate wells of DMSO. The reaction plate with the added compounds was centrifuged at 2500 rpm for 1 minute.

The prepared enzyme solution was added to the reaction plate, 5 µL per well, and the final concentration of enzyme was 10 nM. The reaction plate was centrifuged at 1000 rpm for 1 minute. The reaction plate was placed in an incubator at 37°C for 15 minutes.

The prepared ATP substrate solution was added to the reaction plate, 5 µL per well, and the final concentration of ATP was 5 µM. The reaction plate was centrifuged at 1000 rpm for 1 minute. After sealing the plate with aluminum foil, the plate was placed in an incubator and the reaction was carried out at 37°C for 90 minutes.

10 µL of ADP-glo^{™} reagent was added to stop the kinase reaction and consume unconsumed ATP, leaving only ADP and a very low ATP background. The reaction plate was centrifuged at 1000 rpm for 1 minute. After sealing the plate with aluminum foil, the reaction was carried out at room temperature for 60 minutes.

ADP was converted to ATP by adding 20 µL of kinase detection reagent, and luciferase and luciferin were introduced to detect ATP. The reaction plate was centrifuged at 1000 rpm for 1 minute. After sealing the plate with aluminum foil, the reaction was carried out at room temperature for 60 minutes.

The ADP-GLO luminescence signal values were read by the microplate reader PHERAstar, and the readings were analyzed. The average value of the inhibition rate of the positive control duplicate wells was set as the relative inhibition rate of 100%; the average value of the negative control duplicate wells was set as the relative inhibition rate of 0%. The ADP-GLO readings were converted to relative inhibition rates, and the compound IC₅₀ was fitted using a 4-parameter model.

### Experimental results:

The compounds have good enzymatic inhibitory activity. For specific results, see Table 1.

**Table 1. PKMYT1 enzyme activity test results**

| **Example number** | ADP-GLO IC₅₀ (nM) | **Example number** | ADP-GLO IC₅₀ (nM) | **Example number** | ADP-GLO IC50 (nM) |
|---|---|---|---|---|---|
| **1** | 8.62 | **2** | 2.17 | **3** | 377 |
| **4** | 25.35 | **5** | 8.01 | **6** | 10.1 |
| **7** | 9.41 | **8** | 10.5 | **9** | 9.05 |
| **10** | 13.01 | **11** | 15.71 | **12** | 6.58 |
| **13** | 12.21 | **14** | 24.3 | **15** | 167.7 |
| **16** | 11.7 | **17** | 71.5 | **18** | 18.32 |
| **19** | 4.5 | **20** | 15.27 | **21** | 4.13 |
| **22** | 85.5 | **23** | 8.58 | **24** | 368 |
| **25** | 4.26 | **26** | 11.3 | **27** | 7.15 |
| **28** | 13.8 | **29** | 12.4 | **30** | 13.7 |
| **31** | 11.1 | **32** | 263 | **33** | 11.3 |
| **34** | 25.7 | **35** | 37.8 | **36** | 17.4 |
| **37** | 3.09 | **38** | 14.95 | **39** | / |
| **40** | 23.6 | **41** | 11.18 | **42** | 6.62 |
| **43** | 26.4 | **44** | 11.8 | **45** | 10.8 |
| **46** | 43.6 | **47** | 12.9 | **48** | 36.8 |
| **49** | 318 | **50** | 220.2 | **51** | 22.1 |
| **52** | 73.86 | **53** | 194 | **54** | 17.5 |
| **55** | 5.72 | **56** | / | **57** | 3.04 |
| **58** | 3.57 | **58.1** | 3.08 | **58.2** | / |
| **59** | 8.67 | **59.1** | 4.49 | **59.2** | / |
| **60** | 4.31 | **60.1** | / | **60.2** | 1.88 |
| **42.1** | 11.5 | **42.2** | 996 | **19.1** | / |
| **19.2** | 9.55 | **61** | 38.6 | **62** | 16.9 |
| **62.1** | / | **62.2** | 14.4 | **63** | 5.14 |
| **63.1** | 5.96 | **63.2** | 251 | **64** | 15.7 |
| **65** | 23.8 | **66** | 22.5 | **66.1** | 16.5 |
| **66.2** | / | **67** | 54.6 | **68** | 299 |
| **69** | 15.6 | **70** | 34 | **71** | 7.99 |
| **71.1** | 4.29 | **71.2** | / | **72** | 9.72 |
| **73** | 13.4 | **74** | 7.11 | **75** | 5.5 |
| **76** | 4.02 | **77** | 20.2 | **78** | 8.23 |
| **79** | 4.74 | **80** | 3.51 | **81** | 9.19 |
| **82** | 5.43 | **83** | 41.8 | **84** | 5.28 |
| **85** | 6.7 | **86** | 5.47 | **87** | 3.35 |
| **57.1** | 15.1 | **57.2** | 313 | **88** | 16 |
| **89** | 3 | **90** | 90.7 | **91** | 41.7 |
| **92** | 12.6 | **94** | 408 | **95** | 9.47 |
| **96** | 9.4 | **97** | 187 | **98** | 8.11 |
| **99** | 9.68 | **100** | 3.02 | **100.1** | 1.78 |
| **100.2** | 184 | **101** | 6.8 | **102** | 27.3 |
| **103** | 1.94 | **104** | 3.21 | **104.1** | 356 |
| **104.2** | 2.36 | **105** | 2.43 | **106** | 10.2 |
| **107** | 14.1 | **108** | 31.4 | **110** | 5.77 |
| **111** | 3.57 | **111.1** | 6.68 | **111.2** | 293.6 |
| **112** | 33.8 | **113** | 8.33 | **113.1** | 93.1 |
| **113.2** | 4.1 | **114** | 6.63 | **114.1** | 4.21 |
| **115** | 26.4 | **116** | 25.9 | **117** | 192 |
| **118** | 4.1 | **118.1** | 626 | **118.2** | 4.25 |
| **119** | 15.2 | **119.1** | 4.66 | **119.2** | / |
| **120** | 9.09 | **120.1** | 2.3 | **120.2** | 292 |
| **121** | 5.11 | **122** | 8.71 | **123** | 6.48 |
| **124** | 10.4 | **125** | 3.71 | **126** | 7.37 |
| **127** | 9.17 | **128** | 16.9 | **129** | 10.5 |
| **130** | 4.09 | **131** | 11.2 | **132** | 8.92 |
| **132.1** | 6.23 | **133** | 6.62 | **133.1** | 2.43 |
| **134** | 33.6 | **135** | 12.8 | **136** | 9.9 |
| **137** | 36.2 | **138** | 27 | **139** | 143 |
| **140** | 7.73 | **141** | 5.8 | **141.1** | 2.56 |
| **141.2** | / | **142** | 20.7 | **142.1** | 10.1 |
| **143** | 18.2 | **143.1** | 20 | **144** | 5.74 |
| **144.1** | 2.08 | **144.2** | / | **145** | 24.3 |
| **146** | 35.2 | **147** | 13.6 | **148** | 7.32 |
| **149** | 12.6 | **150** | 27.8 | **151** | 20.1 |
| **152** | 47.9 | **153** | 172 | **154** | 13.9 |
| **154.1** | / | **154.2** | 5.1 | ***155*** | 23.2 |
| **156** | 26.4 | **157** | 60.6 | **158** | 8.18 |
| **158.1** | 7.5 | **158.2** | / | **159** | 15.1 |
| **159.1** | 4.42 | **159.2** | / | **160** | 7.85 |
| **162** | 14.5 | **162.1** | 9.1 | **162.2** | / |
| **163** | 23.39 | **163.1** | 5.7 | **164** | 7.06 |
| **165** | 33.4 | **166** | 8.4 | **166.1** | / |
| **166.2** | 3.78 | **167** | 22 | **168** | 4.92 |
| **169** | 26.2 | **169.1** | 6.31 | **169.2** | / |
| **170** | 18.1 | **170.1** | / | **170.2** | 10 |
| **171** | 26.8 | **172** | 16.4 | **172.1** | 4.6 |
| **172.2** | / | **173** | 7.68 | **173.1** | / |
| **173.2** | 4.5 | **174** | 4.7 | **175** | 4.8 |
| **176** | 9.81 | **176.1** | / | **176.2** | 10.2 |
| **177** | 34.7 | **177.1** | / | **177.2** | 8.15 |
| **178** | 15.5 | **179** | 3.13 | **180** | 30.5 |
| **181** | 34.8 | **182** | 85.7 | **183** | 33.8 |
| **184** | 88.8 | **185** | 214 | **186** | 7.41 |
| **186.1** | / | **186.2** | 3.91 | **187** | 43.5 |
| **188** | 10.5 | **188.1** | 3.6 | **188.2** | 337 |
| **189** | 5 | **190** | 4.93 | **190.1** | / |
| **190.2** | 2.22 | **191** | 2.85 | **192** | 12.5 |
| **193** | 7.94 | **194** | 6.36 | **194.1** | 5.3 |
| **194.2** | / | **195** | 4.6 | **195.1** | 2.8 |
| **195.2** | / | **196** | 4.7 | **202** | 4.42 |
| **203** | 2.86 | **203.1** | / | **203.2** | 1.8 |
| **189.1** | 115 | **189.2** | 3.62 | **204** | 18.9 |
| **205** | 7.81 | **206** | 6.58 | **207** | 4.08 |
| **208** | 4.52 | **209** | 28.7 | **210** | 9.71 |

| | | | | | |
|---|---|---|---|---|---|
| "/" indicates not tested or poor activity. | | | | | |

### Biological test example 2. Cytostatic activity of compounds of the present disclosure

CTG assay (HCC1569) was used to verify the biological activity of some of the example compounds of the present disclosure.

### Experimental materials:

HCC1569 cells were purchased from ATCC (Cat. No.: CRL-2330) and cultured in a 37°C, 5% CO₂ cell culture incubator. HCC1569 complete medium: RPMI-1640 liquid medium (Cat. No.: Gibico 11875-093), 20% FBS (Cat. No.: Gibico 10099-141), 1% Pen Strep (Cat. No.: Gibico 15070-063).

Test compound: Example compounds of the present disclosure.

### Experimental steps:

HCC1569 cells in a 75 cm² culture flask were digested with 2 mL of trypsin for 2-3 min, and then neutralized with 2 mL of 1640 complete medium. The mixture was centrifuged at 1200 rpm for 5 minutes. Cells were resuspended using 4 mL of 1640 complete medium. 500 µL of cell suspension was taken for cell counting using Vi-CELL-XR cell counter.

Using a Multidrop instrument, the HCC1569 cells were seeded in a 384-well plate at a density of 1000 cells per well (50 µL 1640 Growth Media) and administrated 24 hours later.

Using an ultra-micro pipette, the test compound (concentration: 3.33 mM, dissolved in DMSO) was diluted with 10 µM as the highest starting concentration, and then sequentially diluted in 10 gradients at a ratio of 1:3 (10000 nM, 3333 nM, 1111 nM, 370 nM, 123 nM, 41 nM, 13.7 nM, 4.6 nM, 1.5 nM, 0.5 nM) for administration treatment, with two duplicate wells for each concentration; 14 wells for positive and negative controls were set, with the positive control being the duplicate wells of 10 µM positive compound and the negative control being the duplicate wells of DMSO.

After the administration was completed, the cells were placed in a 37°C incubator for further culture for 5 days. After 5 days, 25 µL of CTG buffer was added to each well for CTG assay detection, and analyzed using a microplate reader. CTG readings were analyzed. The average value of the inhibition rate of the positive control duplicate wells was set as the relative inhibition rate of 100%; the average value of the negative control duplicate wells was set as the relative inhibition rate of 0%. The CTG readings were converted into relative inhibition rates, and the inhibition rates (inhibition%) of the test compounds at various concentrations on the cells were calculated according to the following formula: Inhibition% = (b-x)/(b-a)*100%;

a = CTG value (highest concentration), b = CTG value (blank well), x = CTG value(test well).

### IC₅₀ was calculated using GraphPad PRISM 8.

(1) The concentrations and inhibition rates corresponding to 10000 nM, 3333 nM, 1111 nM, 370 nM, 123 nM, 41 nM, 13.7 nM, 4.6 nM, 1.5 nM, and 0.5 nM were statistically analyzed. Log10 (A compound concentration) was used for statistical calculation. (2) The data were input into GraphPad PRISM 8, and Analysis was selected. (3) Nonlinear regression (curve fit) was selected. (4) Log (inhibitor) vs. response-Variable slope was selected. (5) The calculation formula was selected, and the calculation was carried out according to the following formula: Y = Bottom + (Top-Bottom)/(1+10^((LogIC₅₀-X)*HillSlope)), X: logarithm of dose or concentration, Y: response value, Top and Bottom: peak and bottom values. (6) The data were fitted to obtain IC₅₀ values. (7) The fitting conditions were adjusted according to the specific data. Appropriate Constraint conditions were adjusted for Bottom, Top, and HillSlope. The curve that best fits the actual situation has been reached.

The experimental results show that the compound has good cytostatic activity. For specific results, see Table 2.

**Table 2. CTG test results**

| **Example number** | IC₅₀ (nM) | **Example number** | IC₅₀ (nM) |
|---|---|---|---|
| **20** | 40 | **119.1** | 18 |
| **21** | 20 | **120** | 14 |
| **58** | 70 | **120.1** | 12 |
| **58.1** | 83 | **121** | 21 |
| **59.1** | 56 | **122** | 28 |
| **62.2** | 60 | **123** | 58 |
| **63.1** | 43 | **125** | 24 |
| **80** | 16 | **126** | 19 |
| **81** | 66 | **127** | 14 |
| **99** | 40 | **128** | 69 |
| **100** | 23 | **129** | 38 |
| **100.1** | 15 | **130** | 81 |
| **101** | 94 | **131** | 23 |
| **102** | 82 | **132** | 30 |
| **103** | 42 | **132.1** | 11 |
| **104** | 15 | **133** | 18 |
| **104.2** | 8 | **133.1** | 12 |
| **105** | 54 | **134** | 56 |
| **106** | 22 | **136** | 21 |
| **110** | 15 | **141** | 76 |
| **111** | 13 | **141.1** | 61 |
| **111.1** | 6 | **142** | 88 |
| **112** | 18 | **159** | 56.2 |
| **113** | 18 | **159.1** | 31 |
| **113.2** | 3 | **169.1** | 78 |
| **114** | 54 | **170.2** | 80 |
| **114.1** | 69 | **173.2** | 65 |
| **115** | 65 | **176.2** | 68 |
| **116** | 28 | **179** | 22 |
| **118** | 65 | **181** | 63 |
| **118.2** | 38 | **191** | 16 |
| **119** | 44 | **189.2** | 33 |

The IC₅₀ of the example compounds of the present disclosure for cells is preferably no more than 1 µM, preferably no more than 0.5 µM, preferably no more than 0.1 µM, preferably no more than 0.05 µM, and more preferably no more than 0.02 µM.

Biological test example 3: Study on the metabolic stability of compounds in mouse liver microsomes

The concentration of the parent drug in the incubation system was detected by LC/MS/MS, and the intrinsic clearance rate of the test compound in the microsome system was calculated to evaluate its stability. The test concentration of the test compound and the positive control compound was 1 µM.
1. 25 µL of NADPH (10 mM) or phosphate buffered saline (100 mM, pH = 7.4) was transferred to the liver microsome incubation system, and 2 µL of the test sample or verapamil at a concentration of 200 µM was added. Samples supplemented with NADPH were prepared in duplicate, and NADPH-negative samples were prepared in singlicate.
2. 30 µL of the suspension was taken at 0.5, 5, 15, 30, and 60 minutes, respectively. The reaction was stopped by adding 180 µL of acetonitrile containing internal standard and vortexed for 10 min.
3. After centrifuging at 3220 g for 20 minutes, protein precipitation was carried out. The plate was placed in a 4°C refrigerator for 30 minutes and then centrifuged again at 3220 g for 20 minutes. 100 µL of the supernatant was transferred to the sample injection plate, and 100 µL of pure water was added and mixed well for UPLC-MS/MS analysis.
4. All data calculations were performed using Microsoft Excel software. Peak areas were detected by extracting ion chromatograms. The *in vitro* half-life (t_{1/2}) of the parent drug was detected by linear fitting the natural logarithm of the parent drug elimination percentage with time.

The *in vitro* half-life (t_{1/2}) was calculated by the slope: *in vitro* t_{1/2} = 0.693/k

The *in vitro* clearance rate Clᵢₙₜ (unit: µL/min/mg) was calculated: *in vitro* CLᵢₙₜ = kV/N; V = incubation volume per well (400 µL); V = incubation volume per well (400 µL). The specific results are shown in Table 3.

Positive control: RP-6306, whose structure is as follows:

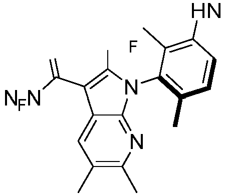

**Table 3. Results of metabolic stability test of mouse liver microsomes**

| Example number | T_{1/2} (min) | Clᵢₙₜ ([µL/min/mg) |
|---|---|---|
| RP-6306 | 22.6 | 61.3 |
| Example 58.1 | 69.7 | 19.92 |
| Example 59.1 | 185 | < 7.50 |

| | | |
|---|---|---|
| Conclusion: Compared with RP-6306, the compound of the present disclosure has a significantly longer half-life, a slower clearance rate, and better metabolic stability. | | |

### Biological test example 4. Tissue distribution of compounds of the present disclosure

OVCAR3 tumor cells (ATCC, Cat. No.: HTB-161) were cultured in RPMI 1640 medium containing inactivated 20% fetal bovine serum, supplemented with 0.01 mg/mL Insulin and 1% penicillin-streptomycin. The cells were cultured in an incubator at 37°C and 5% CO₂. The medium was changed every other day, and the cells were subcultured every 3 to 4 days after the cells had become confluent. Under sterile conditions, the *in vitro* cultured OVCAR3 cell suspension was taken, centrifuged, and added to adjust the cell concentration to 1 × 10⁸ cells/mL, and an equal volume of Matrigel was added, and inoculated subcutaneously on the back of the right hind forelimb of mice (BALB/c nude mice, female, Beijing Vital River Laboratory Animal Technology Co., Ltd.) (0.1 mL/mouse). After 31 days of inoculation, the average tumor volume was in the range of 100-150 mm³. The mice were randomly divided into groups according to tumor size and weight, and the administration of the test compound was started.

The experiment was divided into a solvent control group, a positive control group, and a test group, with 5-6 mice in each group. The specific experimental scheme, the dose, and the administration frequency of each group are shown in the table below. For the test compound administration group, the test compound was mixed in 0.5% methylcellulose and administered by oral gavage twice a day. The experiment was terminated 28 days after administration, and PK plasma (EDTA-K2 anticoagulated) samples were collected (0.5 h, 1 h, 2 h, and 6 h after administration), and the mice were euthanized and tumor samples were collected 2 h and 6 h after the last administration.

### Tumor concentration test method:

All tumor samples were added with pure water at a ratio of 1:3 (3 mL solvent per g tumor), and homogenized using a cryogenic homogenizer to obtain tumor sample solutions.

### Standard curve preparation method:

The powder of the test compound was diluted in a gradient manner with 50% acetonitrile aqueous solution to obtain a series of working solutions. 3 µL of working solution (10, 20, 40, 100, 200, 1000, 2000, 10000, and 20000 ng/mL) was added to 57 µL of blank Balb/c nude mouse tumor sample solution to obtain calibration standard solutions of 0.5 to 1000 ng at concentrations of 0.5, 1, 2, 5, 10, 50, 100, 500, and 1000 ng/mL, respectively, in a total volume of 60 µL.

Quality control samples were prepared in the same manner as the calibration standard solutions: 3 µL of working solution (30, 60, 120, 1000, 8000, and 16000 ng/mL) was added to 57 µL of blank Balb/c nude mouse tumor sample solution to obtain 6 quality control samples with concentrations of 1.5 ng/mL, 3 ng/mL, 6 ng/mL, 50 ng/mL, 400 ng/mL, and 800 ng/mL, respectively, with a total volume of 60 µL.

30 µL of the sample (including standard solution, quality control sample, and test sample) was taken, and 200 µL of protein precipitant containing acetonitrile was added to precipitate the protein, vortexed for 30 seconds, then centrifuged at 4°C and 3900 rpm for 15 minutes, and then the supernatant was aspirated and diluted 3 times with water. 5 µL of the diluted supernatant was loaded on the LC/MS/MS system for quantitative analysis.

### Dose and administration frequency of each group

| **Group** | **Administration group** | **Dose** | **Administration mode** | **Administration time** |
|---|---|---|---|---|
| 1 | Blank control (0.5% MC) | - | p.o. | BIDx28D |
| 2 | RP-6306 | 10 mpk | p.o. | BIDx28D |
| 3 | Example 59.1 | 10 mpk | p.o. | BIDx28D |

The experimental results are shown in Table 4:

**Table 4. Test results of tumor tissue concentration for compounds of the present disclosure**

| Test compound | Tumor tissue concentration (ng/g) | |
|---|---|---|
| | 2 h after administration | 6 h after administration |
| RP-6306 | 172 | 18 |
| Example 59.1 | 633 | 231 |

The above experimental results show that the tumor tissue concentration of the compounds of the present disclosure after 2 h and 6 h of administration is significantly better than that of the positive drug, indicating that the compounds of the present disclosure have potential better anti-tumor activity.

Biological test example 5. Metabolic stability of human hepatocytes of compounds of the present disclosure

Several 96-well sample precipitation plates were prepared, named T0, T15, T30, T60, T90, T120, T240, T0-MC, T240-MC, and blank matrix, respectively. The recovery medium and incubation medium were taken out in advance and placed in a 37°C water bath for preheating. The frozen hepatocytes were removed from the liquid nitrogen tank and immediately immersed into a 37°C water bath (about 90 seconds). After the frozen part was thawed and loosened, the hepatocytes were poured into centrifuge tubes containing 40 mL of recovery medium respectively, and the cells were allowed to resuspend in the recovery medium by gently inverting. The cells were centrifuged at 100 × g for 5 minutes at room temperature, then the supernatant was removed, and the hepatocytes were resuspended in an appropriate volume of incubation medium. The cell viability was calculated by trypan blue staining. 198 µL of hepatocyte suspension (0.51 × 10⁶ cells/mL) was added to the preheated incubation plate. For the medium control group, 198 µL of incubation medium without hepatocytes was added to the T0-MC and T120-MC incubation plates. All incubation plates were preincubated in a 37°C incubator for 10 minutes.

2 µL of the test sample and control compound working solution were added, mixed well, and the incubation plate was immediately put into the shaker in the incubator, and the timer was started to start the reaction. Two replicate samples were prepared for each compound at each time point. The incubation conditions were 37°C, saturated humidity, and 5% CO₂.

In the test system, the final concentration of the test sample was 1 µM, the final concentration of the control sample was 3 µM, the final concentration of hepatocytes was 0.5 × 10⁶ cells/mL, the final concentration of total organic solvents was 0.96%, and the final concentration of DMSO was 0.1%. At the end of the incubation at the corresponding time point, the incubation plate was taken out, and 25 µL of the mixture of the compound and cells and 25 µL of the mixture of the control compound and cells were taken out and added to the sample plate containing 125 µL of the stop solution (acetonitrile solution containing 200 ng/mL tolbutamide and labetalol). For the Blank sample plate, 25 µL of the incubation medium without hepatocytes was directly added. All sample plates were sealed and shaken on a plate shaker at 600 rpm for 10 minutes, followed by centrifugation at 3220 × g for 20 minutes. The supernatants of the test and control samples were diluted with ultrapure water at a ratio of 1:3. All samples were mixed and analyzed by LC/MS/MS.

The experimental results are shown in Table 5:

**Table 5. Human HMS metabolic stability of compounds of the present disclosure**

| **HMS** | **Gender** | **t_{1/2} (min)** | | ***In vitro* Clᵢₙₜ (µL/min/10⁶ cells)** | |
|---|---|---|---|---|---|
| | | RP-6306 | Example 59.1 | RP-6306 | Example 59.1 |
| Human | Male | 15 | 198 | 94 | 7.0 |
| | Female | 270 | 138 | 5.0 | 10 |

The experimental results of the present disclosure show that there is a significant gender difference in the metabolism of RP-6306 in human *in vitro* hepatocytes, and that the metabolic stability of male hepatocytes is significantly lower than that of females. However, the compounds of the present disclosure do not have gender differences in the *in vitro* human hepatocyte metabolic stability and all have good stability, which reduces the risk of gender factors affecting drug efficacy and safety and has more convenient clinical application.

### Experimental example 1. TS-FeSSIF & TS-FeSSGF solubility test

### 1. Preparation of FeSSIF

Prepare buffer B: 4.040 g of sodium hydroxide, 8.650 g of glacial acetic acid, and 11.874 g of sodium chloride were dissolved in about 900 mL of ultrapure water, and the pH of the solution was adjusted to 5.0 with 1 mol/L sodium hydroxide or 1 mol/L hydrochloric acid. The solution was then diluted to 1000 molar with ultrapure water at room temperature.

Powder addition: 11.200 g of FaSSIF, FeSSIF, and FaSSGF powder were added to about 500 mL of buffer B. The mixture was stirred until the powder was completely dissolved. The solution was then diluted to 1000 mL with buffer B at room temperature.

Ready to use: The solution was used within 48 hours at room temperature and within 24 hours at 37°C.

### 2. Preparation of FeSSGF

Prepare buffer D: 1.220 g of sodium acetate, 0.514 g of glacial acetic acid, and 6.926 g of sodium chloride were dissolved in about 500 mL of ultrapure water.

Mix milk: The solution was mixed with milk in equal volumes (1:1), and the pH of the solution was adjusted to 5.0 with 1 mol/L hydrochloric acid.

Ready to use: The solution was used within 48 hours at room temperature and within 24 hours at 37°C.

### 3. Solubility assay:

Stock solutions of test and control compounds were prepared at a concentration of 10 mmol/L in DMSO (Solarbio S&T Co., LTD).

First, 50 µL of the stock solution (10 mmol/L) of each sample was added to the vials of the uncapped solubility sample plate. The assay was performed in duplicate. The DMSO was then evaporated using a centrifugal vacuum evaporator. 500 µL of buffer was added to dissolve each sample. A stirring bar was placed on each vial and sealed with a PTFE/silicone stopper. The sample plate was then transferred to an Eppendorf Thermomixer Comfort plate shaker and shaken at 1100 rpm at 25°C for 24 hours. At the end, the stir bar was removed using a large magnet and the sample was transferred from the solubility sample plate to a filter plate. The mixture was centrifuged at 4000 rpm, 25°C for 30 minutes to obtain the supernatant. A 350 µL of aliquot was transferred from the supernatant. The pipette tip was immersed in acetonitrile for 5 seconds and then in water for 5 seconds. The first 25 µL was discarded, and 300 µL was dispensed into another 96-vial glass insert plate and centrifuged again (4000 rpm, 25°C, 30 minutes). A 5 µL of aliquot from the supernatant and 5 µL of DMSO were taken out, and 490 µL of a mixture of water and acetonitrile containing internal standard (1:1) was added. Depending on the peak shape, the diluent was diluted with a certain proportion of ultrapure water. The dilution factor was varied according to the solubility value and LC-MS signal response.

50 µL of the stock solution of each sample (10 mmol/L) was added to the vials of the uncapped solubility sample plate. The assay was performed in duplicate. The DMSO was then evaporated using a centrifugal vacuum evaporator. 500 µL of DMSO was added to dissolve the samples. A stirring bar was placed on each vial and sealed with a molded PTFE/silicone stopper. The solubility sample plate was then transferred to an Eppendorf Thermomixer Comfort plate shaker and shaken at 1100 rpm at 25°C for 2 hours. After 2 hours, each compound was expected to be fully dissolved. A 10 µL of aliquot was taken, and 990 µL of a mixture of water/acetonitrile with internal standard (1:1) was added to the 10 µL of aliquot. The concentration of the standard sample may vary depending on the LC/MS signal response.

All calculations were performed using Microsoft Excel. Samples were analyzed and quantified using LC/MS/MS based on standards of known concentration. The solubility of the test compound was calculated according to the following formula: [Sample] = (Area ratio_{Sample} × DF_{Sample} × [STD])/Area ratio_{STD}, where DF represents the dilution factor.

The experimental results are shown in Table 6:

**Table 6. Solubility test results of compounds of the present disclosure**

| Test system | Solubility (µg/mL) | |
|---|---|---|
| | RP-6306 | Example 59.1 |
| TS-FeSSIF | 31.6 | 351.8 |
| TS-FeSSGF | 33.9 | 253.2 |

The compound of the present disclosure has good solubility, which is beneficial for *in vivo* absorption and later preparation development.

## Claims

1. A compound represented by general formula (I), a pharmaceutically acceptable salt thereof, or an isomer thereof, 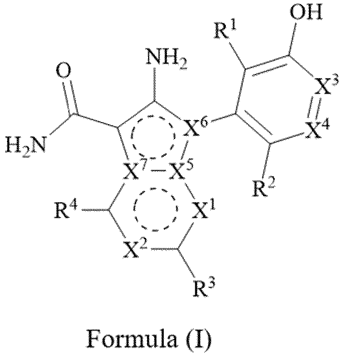 wherein
X¹ is N or CR⁵;
X² is N or CR⁶;
X³ is N or CR⁷;
X⁴ is N or CR⁸;
X⁵ is N or C;
X⁶ is N or C;
X⁷ is N or C;
provided that when X² is CR⁶, X⁵ is C, X⁶ is N, and X⁷ is C, then X³ and X⁴ are not simultaneously CH;
R¹ and R² are each independently halogen, C₁₋₄ alkyl, C₁₋₄ alkoxy, hydroxy-C₁₋₄ alkyl, or deuterated C₁₋₄ alkyl;
R³ is -H, -CN, -OH, -N(R^{a})(R^{b}), halogen, C₁₋₄ alkyl, halo-C₁₋₄ alkyl, C₁₋₄ alkoxy, C₂₋₄ alkenyl, C₂₋₄ alkynyl, -L-R^{c}, phenyl, C₃₋₆ cycloalkyl, C₃₋₆ cycloalkenyl, 3- to 6-membered heterocyclyl, 3- to 6-membered heterocycloalkenyl, deuterated C₁₋₄ alkyl, or 5- to 6-membered heteroaryl; wherein the phenyl, C₃₋₆ cycloalkyl, C₃₋₆ cycloalkenyl, 3- to 6-membered heterocyclyl, 3- to 6-membered heterocycloalkenyl, and 5- to 6-membered heteroaryl are optionally substituted by one or more R^{3a}, and the R^{3a} is halogen, C₁₋₄ alkyl, or C₁₋₄ alkoxy;
L is C₂₋₄ alkynylene, C₁₋₄ alkylene, or C₂₋₄ alkenylene;
R^{c} is C₁₋₄ alkyl, C₃₋₆ cycloalkyl, or 4- to 6-membered heterocyclyl, and the C₁₋₄ alkyl, C₃₋₆ cycloalkyl, and 4- to 6-membered heterocyclyl are optionally substituted by one or more R^{ca};
the R^{ca} is halogen or -OH;
R^{a} and R^{b} are each independently -H, C₁₋₄ alkyl, phenyl, p-methoxybenzyl, C₁₋₄ alkyl-C(O)-, C₃₋₆ cycloalkyl, or halo-C₁₋₄ alkyl, or R^{a} and R^{b} together with the nitrogen atom to which they are attached form a 4- to 6-membered heterocyclyl ring, and the 4- to 6-membered heterocyclyl is optionally substituted by halogen;
R⁴ is -H, halogen, C₁₋₄ alkyl, C₃₋₆ cycloalkyl, C₁₋₄ alkoxy, or halo-C₁₋₄ alkyl;
R⁵, R⁷, and R⁸ are each independently -H, -OH, -CN, -N(R^{d})(R^{e}), halogen, C₁₋₄ alkyl, C₁₋₄ alkoxy, or halo-C₁₋₄ alkyl;
R⁶ is -H, -OH, -CN, -N(R^{d})(R^{e}), halogen, C₁₋₄ alkyl, C₁₋₄ alkoxy, -COOH, halo-C₁₋₄ alkyl, halo-C₁₋₄ alkoxy, C₂₋₄ alkenyl, C₂₋₄ alkynyl, C₁₋₄ alkyl-S(O)₂-, hydroxy-C₁₋₄ alkyl, C₃₋₆ cycloalkyl, C₅₋₆ cycloalkenyl, C₃₋₆ cycloalkyl-C₁₋₄ alkyl, C₃₋₆ cycloalkyl-O-, 3- to 6-membered heterocyclyl, 3- to 6-membered heterocycloalkenyl, phenyl, 5- to 6-membered heteroaryl, or - CD₃; wherein the C₁₋₄ alkyl, C₃₋₆ cycloalkyl, C₅₋₆ cycloalkenyl, C₃₋₆ cycloalkyl-C₁₋₄ alkyl, C₃₋₆ cycloalkyl-O-, 3- to 6-membered heterocyclyl, 3- to 6-membered heterocycloalkenyl, phenyl, and 5- to 6-membered heteroaryl are optionally substituted by one or more R^{6a}, and the R^{6a} is halogen, C₁₋₄ alkyl, halo-C₁₋₄ alkyl, or C₁₋₄ alkoxy;
R^{d} and R^{e} are each independently -H, C₁₋₄ alkyl, -S(O)₂-N(R^{f})(R^{g}), -C(O)-aryl, -C(O)-NR^{f}-aryl, or -(C=S)-NR^{f}-aryl;
R^{f} and R^{g} are each independently -H or C₁₋₄ alkyl;
or, R² and R⁸ together with the atom to which they are attached form a C₅₋₆ cycloalkenyl ring;
and/or, R³ and R⁶ together with the atom to which they are attached form a phenyl, 5- to 6-membered heterocycloalkenyl, C₅₋₆ cycloalkenyl, or 5- to 6-membered heteroaryl ring;
and/or, R⁴ and R⁶ together with the atom to which they are attached form a phenyl, 5- to 6-membered heterocycloalkenyl, C₅₋₆ cycloalkenyl, or 5- to 6-membered heteroaryl ring;
the phenyl, 5- to 6-membered heteroaryl, 5- to 6-membered heterocycloalkenyl, and C₅₋₆ cycloalkenyl are optionally further substituted by one or more substituents selected from halogen, CN, C₁₋₆ alkyl, halo-C₁₋₆ alkyl, hydroxy-C₁₋₆ alkyl, C₁₋₆ alkoxy, and C₁₋₆ alkyl-C(O)-.

2. The compound, the pharmaceutically acceptable salt thereof, or the isomer thereof according to claim 1, wherein X¹ is CR⁵ X² is N, X⁵ is C, X⁶ is N, and X⁷ is C.

3. The compound, the pharmaceutically acceptable salt thereof, or the isomer thereof according to claim 1, wherein X¹ and X² are both N, X⁵ is C, X⁶ is N, and X⁷ is C.

4. The compound, the pharmaceutically acceptable salt thereof, or the isomer thereof according to any one of claims 1-3, wherein R¹ and R² are each independently -CH₃, -CH₂CH₃, -CH(CH₃)₂, -Cl, -F, -Br, -OCH₃, -CH₂OH, or -CD₃;
preferably, R¹ and R² are both -CH₃ or -CD₃.

5. The compound, the pharmaceutically acceptable salt thereof, or the isomer thereof according to any one of claims 1-4, wherein R³ is -H, -CN, -NH₂, -F, -Br, -Cl, -CH₃, -CH₂CH₃, -CH₂CH(CH₃)₂, -CF₃, -OCH₃, -OCH₂CH₃, -CH=CH₂, -N(CH₃)₂, -N(CH₂CH₃)₂, -NHPMB, phenyl, cyclopropyl, cyclobutyl, cyclopentyl, cyclohexyl, 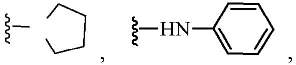 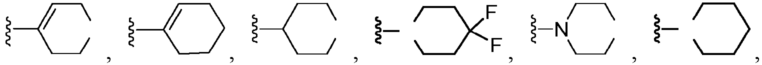 ethynyl, -C≡C-R^{c}, -OH, -CH(CH₃)₂, -CH₂CH₂CH₃, -CH₂CH(CH₃)₂, -OCH₃, -CH₂F, -CHF₂, -CH=CH₂, - CH=C(CH₃)₂, -CH=CHCH₃, -C(CH₃)=CH₂, -NHCH₃, -NHCH₂CH₃, -NHC(O)CH₃, 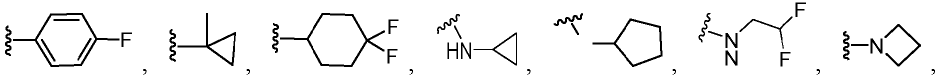 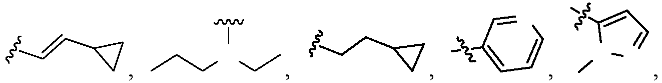 or -CD₃; R^{c} is the following group optionally substituted by one or more R^{ca}: methyl, ethyl, cyclopropyl, cyclobutyl, isopropyl, oxetanyl, or azetidinyl; the R^{ca} is -F, -Cl, or -OH.

6. The compound, the pharmaceutically acceptable salt thereof, or the isomer thereof according to claim 5, wherein -C≡C-R^{c} is -C≡C-CH₃,

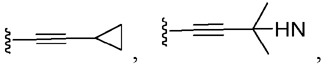

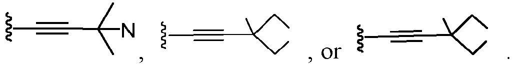

7. The compound, the pharmaceutically acceptable salt thereof, or the isomer thereof according to any one of claims 1-6, wherein R⁴ is -H, -Cl, -F, -Br, -CH₃, -CH₂CH₃, cyclopropyl, methoxy, or -CH₂CF₃;
preferably, R⁴ is H.

8. The compound, the pharmaceutically acceptable salt thereof, or the isomer thereof according to any one of claims 1-2 and 4-7, wherein
R⁵ is -H, -Cl, -F, -CH₃, -CN, -CF₃, or -OCH₃.

9. The compound, the pharmaceutically acceptable salt thereof, or the isomer thereof according to any one of claims 1-8, wherein R⁷ and R⁸ are each independently -H, -OH, -CN, -NH₂, -F, -Br, -Cl, -CH₃, -CH₂CH₃, -OCH₃, or -OCH₂CH₃;
preferably, R⁷ and R⁸ are each independently H or F.

10. The compound, the pharmaceutically acceptable salt thereof, or the isomer thereof according to any one of claims 1-9, selected from the structure represented by the following general formula: 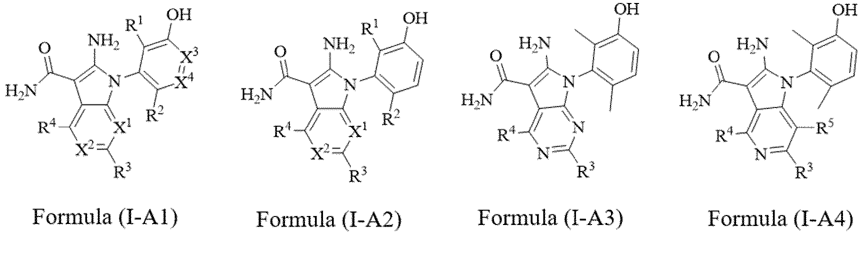 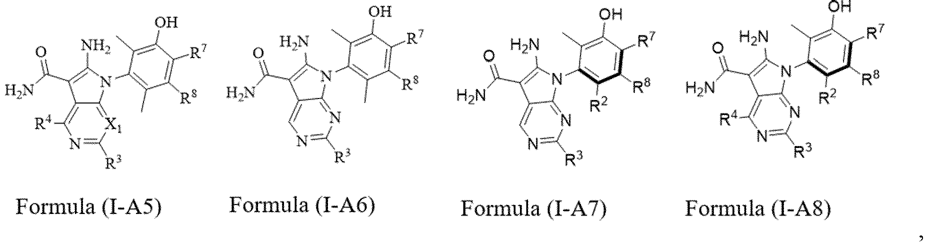 X¹, X², R¹, R², R³, R⁴, R⁵, R⁷, and R⁸ are as defined in any one of claims 1-9.

11. The compound, the pharmaceutically acceptable salt thereof, or the isomer thereof according to any one of claims 1 and 4-9, wherein X¹ is N; X² is CR⁶; X⁵ is C, X⁶ is N, X⁷ is C, X³ is N or CR⁷, and X⁴ is N or CR⁸; X³ and X⁴ are not simultaneously CH.

12. The compound, the pharmaceutically acceptable salt thereof, or the isomer thereof according to claim 11, wherein R¹ and R² are each independently -CH₃, -CH₂CH₃, -CH(CH₃)₂, -Cl, -F, -Br, -OCH₃, -CH₂OH, or -CD₃; preferably, R¹ and R² are both -CH₃ or -CD₃;
R³ is -CH₃, -NH₂, -OH, -F, -Cl, -Br, -CH(CH₃)₂, -CH₂CH₃, -CH₂CH₂CH₃, -CH₂CH(CH₃)₂, -OCH₃, -CH₂F, -CHF₂, -CF₃, -CH=CH₂, -CH=C(CH₃)₂, -CH=CHCH₃, -C(CH₃)=CH₂, -NHCH₃, -NHCH₂CH₃, -NHC(O)CH₃, 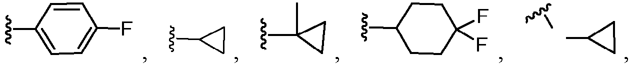 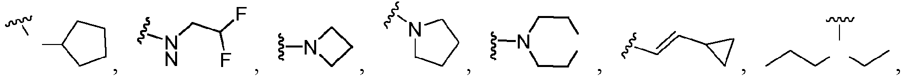 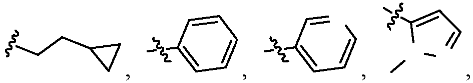 or -CD₃;
R⁴ is -H, -Cl, -F, -Br, -CH₃, -CH₂CH₃, cyclopropyl, or -CH₂CF₃;
R⁶ is -CH₃, -H, -OH, -Cl, -F, -Br, -COOH, -CN, -CH₂CH₃, -CH(CH₃)₂, -CH₂CH(CH₃)₂, - CF₃, -CHF₂, -CH₂F, -CF₂CH₃, -CF(CH₃)₂, -OCH₃, -OCH(CH₃)₂, -OCF₃, -OCH₂CF₃, -N(CH₃)₂, -CH=CH₂, -C(CH₃)=CH₂, -C≡CH, -S(O)₂CH₃, 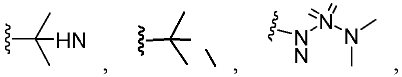 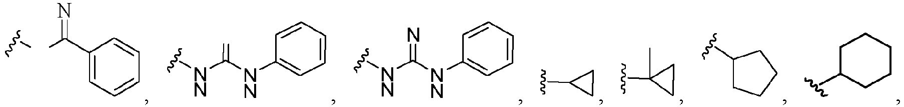 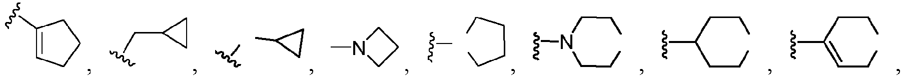 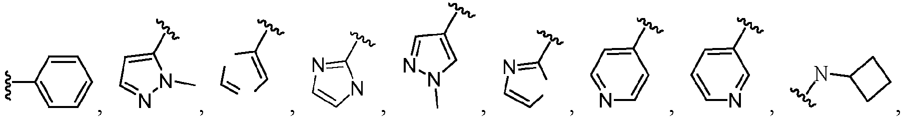 -CH₂CH₂CH=CH₂, -CH₂OH, -CH₂CF₃, or -CD₃;
X³ is N or CR⁷; X⁴ is N or CR⁸;
R⁷ and R⁸ are each independently -H, -OH, -CN, -NH₂, -CH₃, -CH₂CH₃, -Cl, -F, -Br, - OCH₃, or -OCH₂CH₃; R⁷ and R⁸ are not simultaneously H;
or, R² and R⁸ together with the atom to which they are attached form a C₅₋₆ cycloalkenyl ring.

13. The compound, the pharmaceutically acceptable salt thereof, or the isomer thereof according to claim 11, wherein
X³ is CR⁷, and X⁴ is CR⁸;
R¹ and R² are C₁₋₄ alkyl or deuterated C₁₋₄ alkyl; preferably, R¹ and R² are -CH₃ or -CD₃;
R³ is C₁₋₄ alkyl; preferably, R³ is -CH₃ or -CH₂CH₃;
R⁴ is -H;
R⁶ is halogen or halo-C₁₋₄ alkyl; preferably, R⁶ is -Cl, -Br, or -CF₃;
R⁷ and R⁸ are each independently -H or halogen, and R⁷ and R⁸ are not simultaneously H; preferably, R⁷ and R⁸ are each independently -H or -F, and R⁷ and R⁸ are not simultaneously H.

14. The compound, the pharmaceutically acceptable salt thereof, or the isomer thereof according to claim 11, wherein R¹ and R² are each independently -CH₃, -CH₂CH₃, -CH(CH₃)₂, -Cl, -F, -Br, -OCH₃, -CH₂OH, or -CD₃; preferably, R¹ and R² are both -CH₃ or -CD₃;
R³ and R⁶ together with the atom to which they are attached form the following group: 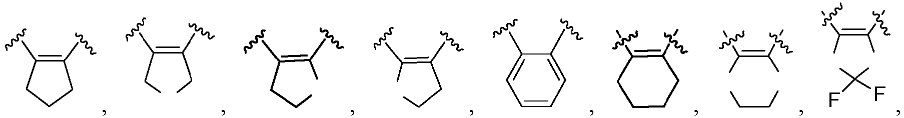 or 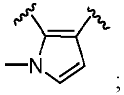
R⁴ is -H, -Cl, -F, -Br, -CH₃, -CH₂CH₃, or cyclopropyl;
X³ is N or CR⁷; X⁴ is N or CR⁸;
R⁷ and R⁸ are each independently -H, -OH, -CN, -NH₂, -CH₃, -CH₂CH₃, -Cl, -F, -Br, - OCH₃, or -OCH₂CH₃.

15. The compound, the pharmaceutically acceptable salt thereof, or the isomer thereof according to claim 11, wherein R¹ and R² are each independently -CH₃, -CH₂CH₃, -CH(CH₃)₂, -Cl, -F, -Br, -OCH₃, -CH₂OH, or -CD₃; preferably, R¹ and R² are both -CH₃ or -CD₃;
R³ is -H, -CH₃, or -CH₂CH₃;
R⁴ and R⁶ together with the atom to which they are attached form the following group: 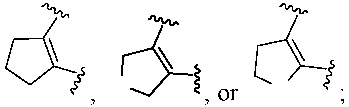
X³ is N or CR⁷; X⁴ is N or CR⁸;
R⁷ and R⁸ are each independently -H, -OH, -CN, -NH₂, -CH₃, -CH₂CH₃, -Cl, -F, -Br, - OCH₃, or -OCH₂CH₃.

16. The compound, the pharmaceutically acceptable salt thereof, or the isomer thereof according to any one of claims 1 and 11-15, having any one of the following structures: 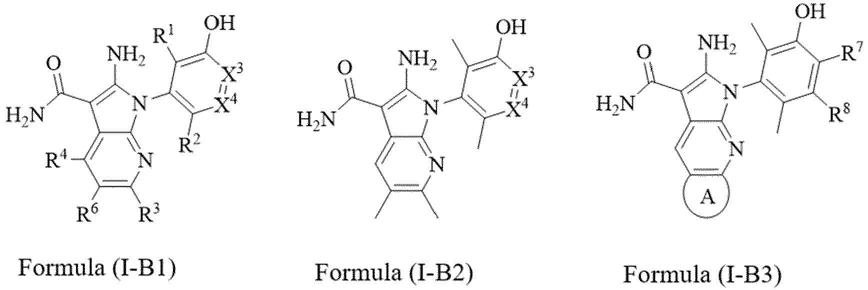 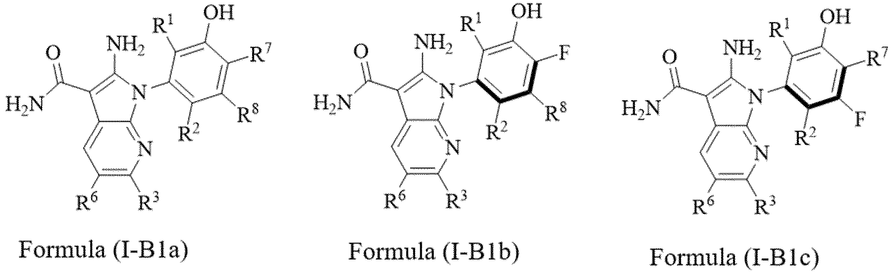 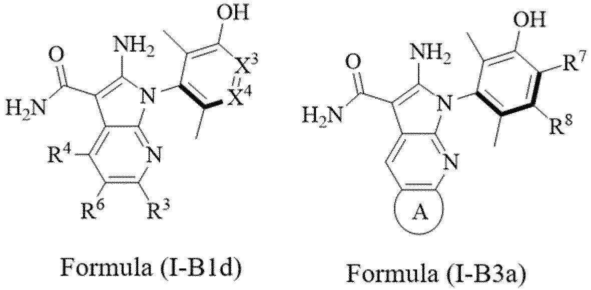
ring A is phenyl, 5- to 6-membered heterocycloalkenyl, C₅₋₆ cycloalkenyl, or 5- to 6-membered heteroaryl, and the phenyl, 5- to 6-membered heterocycloalkenyl, C₅₋₆ cycloalkenyl, and 5- to 6-membered heteroaryl are optionally further substituted by one or more substituents selected from halogen, CN, C₁₋₆ alkyl, halo-C₁₋₆ alkyl, hydroxy-C₁₋₆ alkyl, C₁₋₆ alkoxy, and C₁₋₆ alkyl-C(O)-;
wherein X³, X⁴, R¹, R², R³, R⁴, R⁶, X³, X⁴, R⁷, and R⁸ are as defined in any one of claims 1 and 11-15.

17. A compound represented by general formula (II), a pharmaceutically acceptable salt thereof, or an isomer thereof, 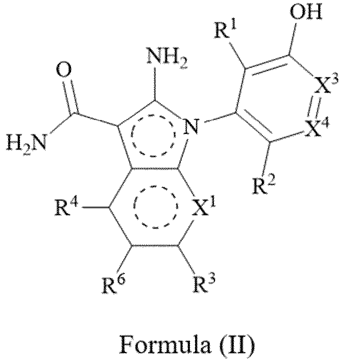
X¹ is N or CR⁵;
X³ is N or CR⁷;
X⁴ is N or CR⁸;
R¹ and R² are each independently halogen, C₁₋₄ alkyl, C₁₋₄ alkoxy, hydroxy-C₁₋₄ alkyl, or deuterated C₁₋₄ alkyl;
R³ is -CN, -OH, -N(R^{a})(R^{b}), halogen, C₁₋₄ alkyl, halo-C₁₋₄ alkyl, C₁₋₄ alkoxy, C₂₋₄ alkenyl, C₂₋₄ alkynyl, -L-R^{c}, phenyl, C₃₋₆ cycloalkyl, C₃₋₆ cycloalkenyl, 3- to 6-membered heterocyclyl, 3- to 6-membered heterocycloalkenyl, deuterated C₁₋₄ alkyl, or 5- to 6-membered heteroaryl; wherein the phenyl, C₃₋₆ cycloalkyl, C₃₋₆ cycloalkenyl, 3- to 6-membered heterocyclyl, 3- to 6-membered heterocycloalkenyl, and 5- to 6-membered heteroaryl are optionally substituted by one or more R^{3a}, and the R^{3a} is halogen, C₁₋₄ alkyl, or C₁₋₄ alkoxy;
L is C₂₋₄ alkynylene, C₁₋₄ alkylene, or C₂₋₄ alkenylene;
R^{c} is C₁₋₄ alkyl, C₃₋₆ cycloalkyl, or 4- to 6-membered heterocyclyl, and the C₁₋₄ alkyl, C₃₋₆ cycloalkyl, and 4- to 6-membered heterocyclyl are optionally substituted by one or more R^{ca}; the R^{ca} is halogen or -OH;
R^{a} and R^{b} are each independently -H, C₁₋₄ alkyl, phenyl, p-methoxybenzyl, C₁₋₄ alkyl-C(O)-, C₃₋₆ cycloalkyl, or halo-C₁₋₄ alkyl, or R^{a} and R^{b} together with the nitrogen atom to which they are attached form a 4- to 6-membered heterocyclyl ring, and the 4- to 6-membered heterocyclyl is optionally substituted by halogen;
R⁴ and R⁶ together with the atom to which they are attached form a phenyl, 5- to 6-membered heterocycloalkenyl, C₅₋₆ cycloalkenyl, or 5- to 6-membered heteroaryl ring; the phenyl, 5- to 6-membered heteroaryl, 5- to 6-membered heterocycloalkenyl, and C₅₋₆ cycloalkenyl are optionally further substituted by one or more substituents selected from halogen, CN, C₁₋₆ alkyl, halo-C₁₋₆ alkyl, hydroxy-C₁₋₆ alkyl, C₁₋₆ alkoxy, and C₁₋₆ alkyl-C(O)-;
R⁵, R⁷, and R⁸ are each independently -H, -OH, -CN, -N(R^{d})(R^{e}), halogen, C₁₋₄ alkyl, C₁₋₄ alkoxy, or halo-C₁₋₄ alkyl.

18. The compound, the pharmaceutically acceptable salt thereof, or the isomer thereof according to claim 17, wherein
R¹ and R² are each independently -CH₃, -CH₂CH₃, -CH(CH₃)₂, -Cl, -F, -Br, -OCH₃, - CH₂OH, or -CD₃;
R³ is -CH₃ or -CH₂CH₃;
R⁴ and R⁶ together with the atom to which they are attached form the following group: 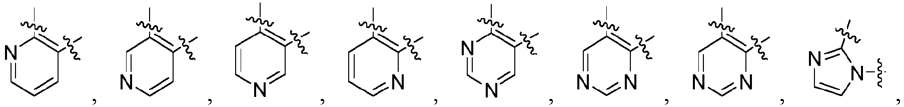 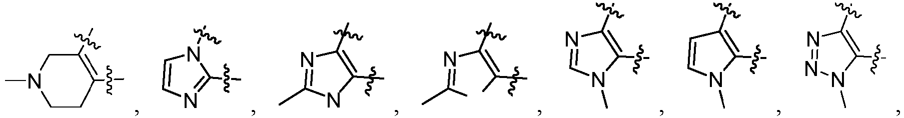
or 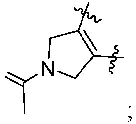
R⁵ is -H, -CN, halo-C₁₋₃ alkyl, or halogen;
R⁷ and R⁸ are each independently -H, -OH, -CN, -NH₂, -CH₃, -CH₂CH₃, -Cl, -F, -Br, - OCH₃, or -OCH₂CH₃.

19. The compound, the pharmaceutically acceptable salt thereof, or the isomer thereof according to claim 17 or 18, wherein
X¹ is CR ⁵; X³ is CR⁷; X⁴ is CR⁸;
R¹ and R² are each independently C₁₋₄ alkyl, preferably -CH₃;
R³ is C₁₋₄ alkyl, preferably -CH₃;
R⁴ and R⁶ together with the atom to which they are attached form a 5- to 6-membered heteroaryl ring, preferably a pyridyl ring, more preferably 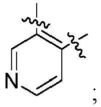
R⁵ is -H or halogen, preferably -H or -F, more preferably -H;
R⁷ and R⁸ are each independently -H.

20. The compound, the pharmaceutically acceptable salt thereof, or the isomer thereof according to any one of claims 17-19, having any one of the following structures: 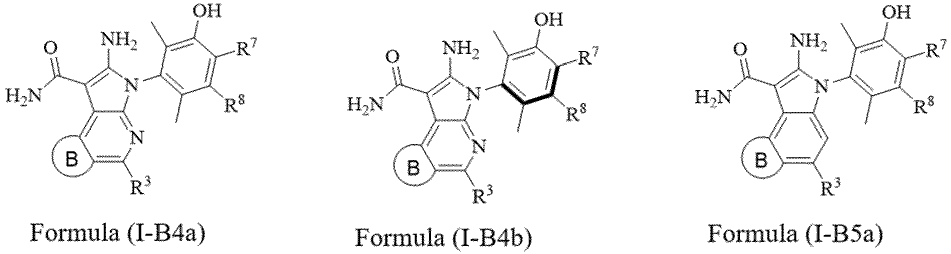 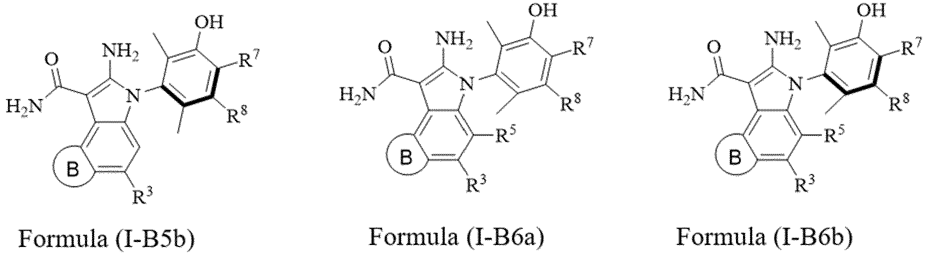
each ring B is independently phenyl, 5- to 6-membered heterocycloalkenyl, C₅₋₆ cycloalkenyl, or 5- to 6-membered heteroaryl, and the phenyl, 5- to 6-membered heterocycloalkenyl, C₅₋₆ cycloalkenyl, and 5- to 6-membered heteroaryl are optionally further substituted by one or more substituents selected from halogen, CN, C₁₋₆ alkyl, halo-C₁₋₆ alkyl, hydroxy-C₁₋₆ alkyl, and C₁₋₆ alkoxy;
wherein R³, R⁴, R⁵, R⁶, X³, X⁴, R⁷, and R⁸ are as defined in any one of claims 17-19.

21. The compound, the pharmaceutically acceptable salt thereof, or the isomer thereof according to any one of claims 1 and 4-9, wherein X¹ is N, X⁵ is N, X² is CR⁶, X⁶ is C, and X⁷ is C.

22. The compound, the pharmaceutically acceptable salt thereof, or the isomer thereof according to any one of claims 1 and 4-9, wherein X¹ is N, X² is CR⁶, X⁵ is C, X⁶ is C, and X⁷ is N.

23. The compound, the pharmaceutically acceptable salt thereof, or the isomer thereof according to claim 21 or 22, wherein R¹ and R² are each independently -CH₃, -CH₂CH₃, or - CH(CH₃)₂; preferably, R¹ and R² are both -CH₃;
R³ is -CH₃;
R⁴ is -H;
R⁶ is -CH₃;
X³ is N or CR⁷; X⁴ is N or CR⁸;
R⁷ and R⁸ are each independently -H, -OH, -CN, -NH₂, -CH₃, -CH₂CH₃, -Cl, -F, -OCH₃, or -OCH₂CH₃;
or, R² and R⁸ together with the atom to which they are attached form a C₅₋₆ cycloalkenyl ring.

24. The compound, the pharmaceutically acceptable salt thereof, or the isomer thereof according to any one of claims 21-23, having any one of the following structures: 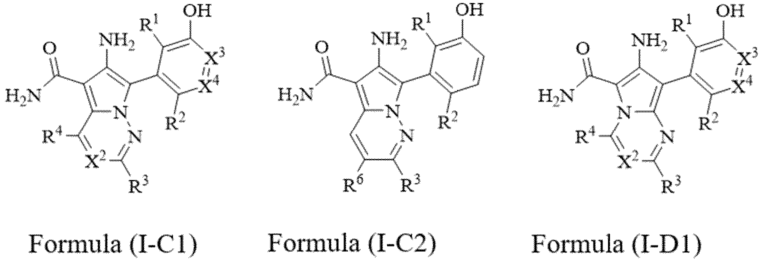 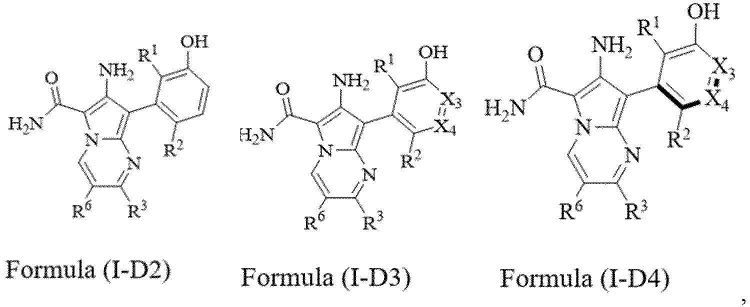 wherein R¹, R², R³, R⁴, R⁶, X³, and X⁴ are as defined in any one of claims 21-23.

25. A compound as described below, a pharmaceutically acceptable salt thereof, or an isomer thereof, wherein the compound is selected from any one of the following structures:

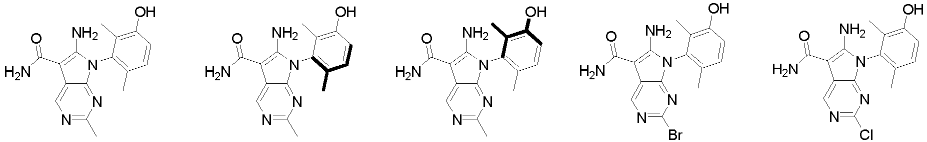

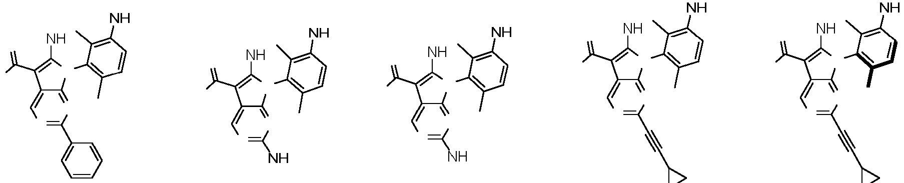

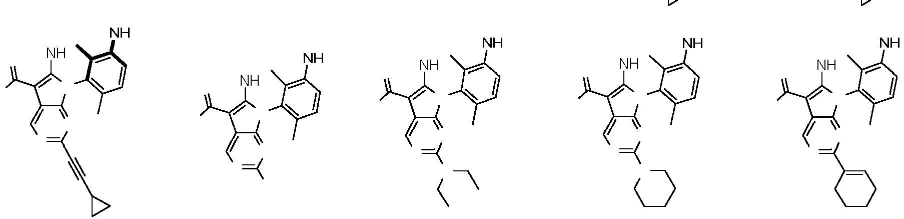

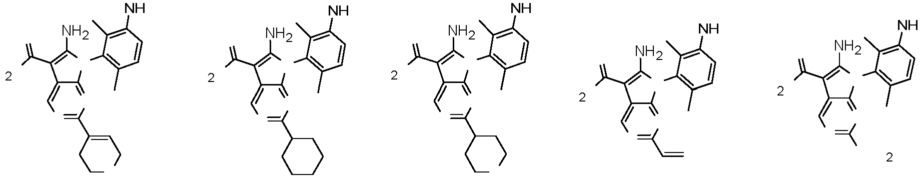

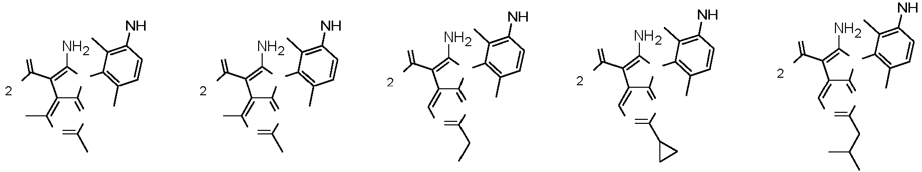

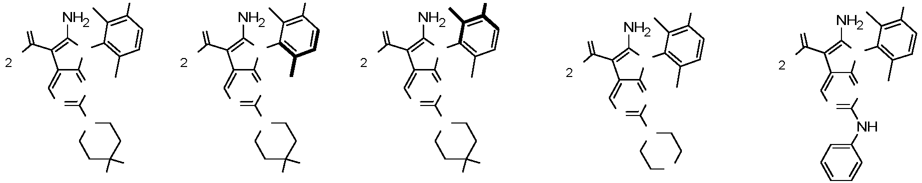

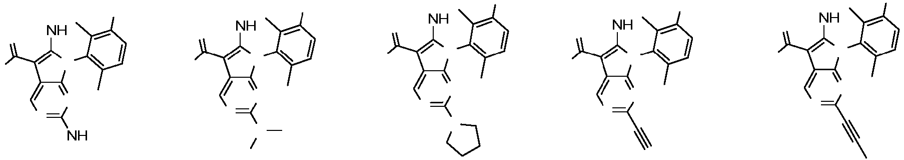

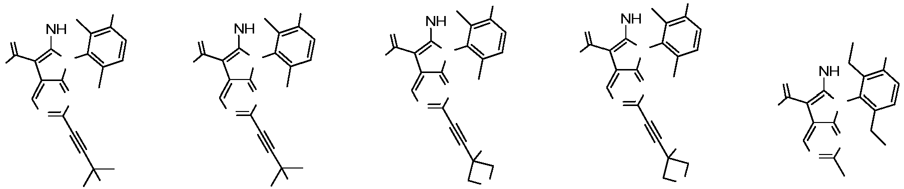

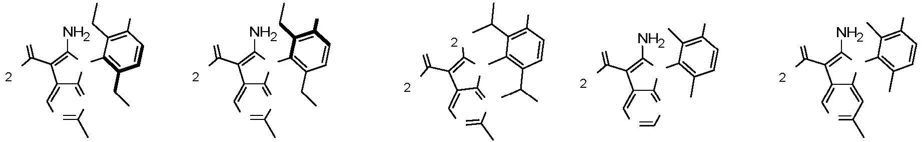

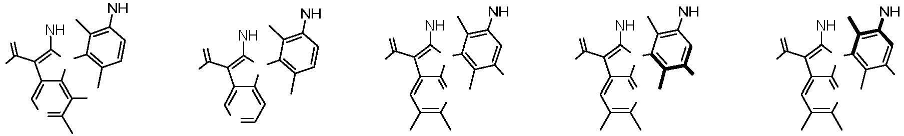

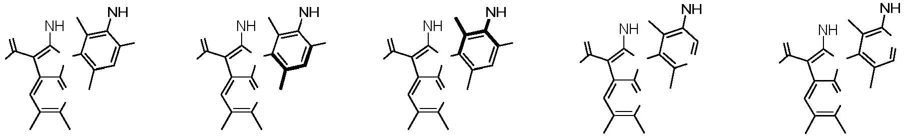

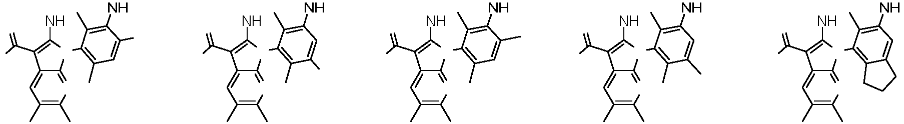

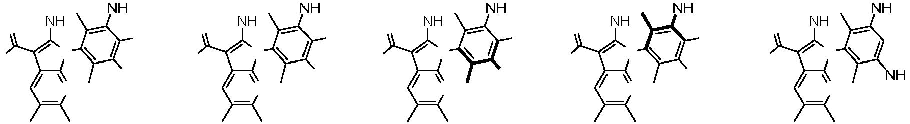

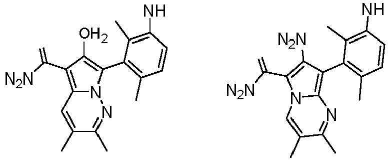

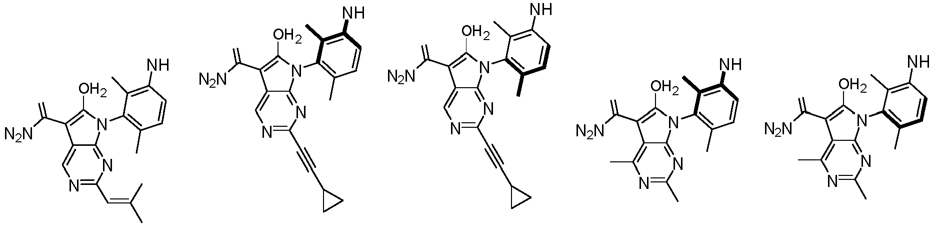

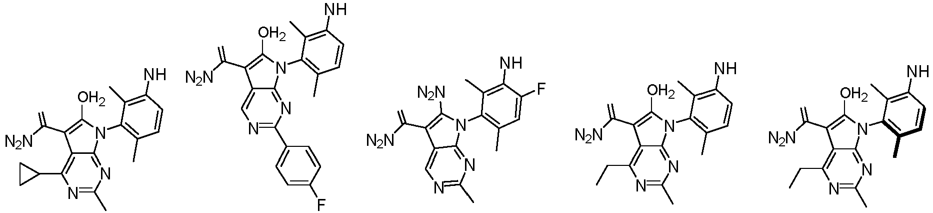

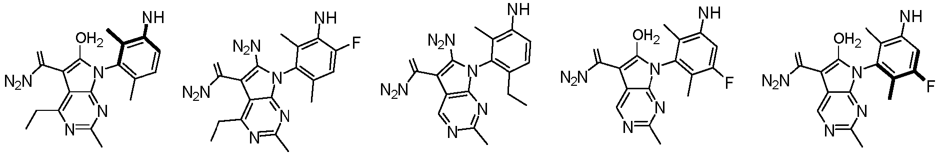

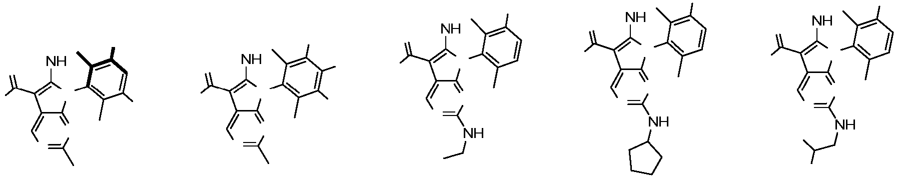

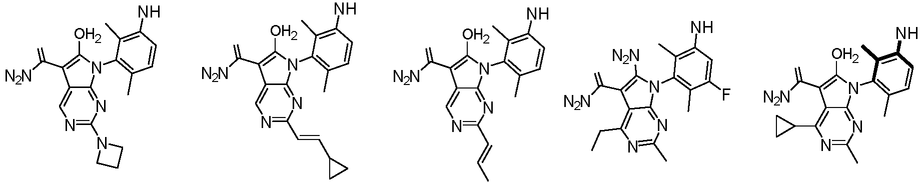

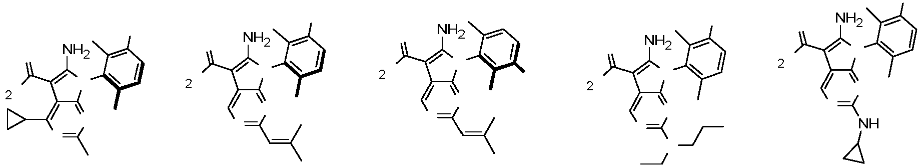

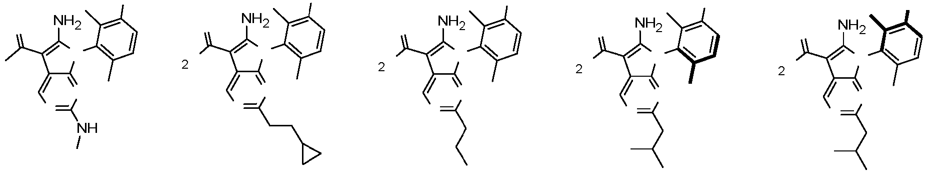

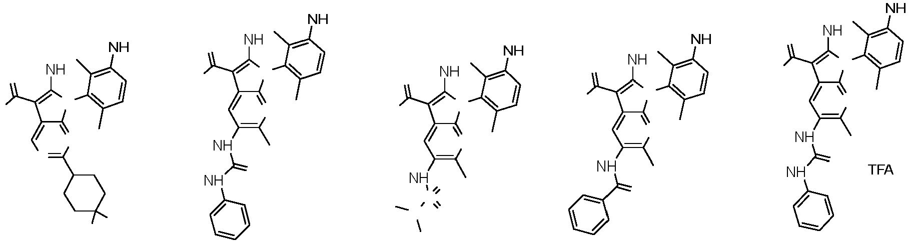

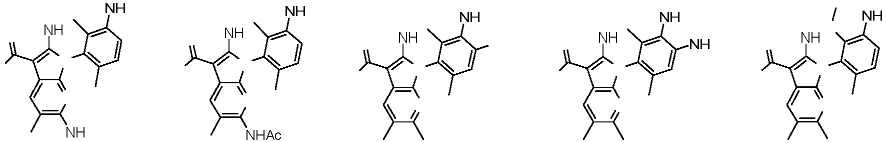

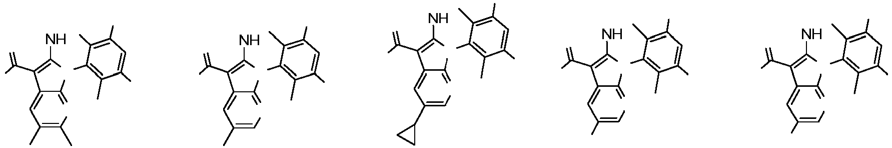

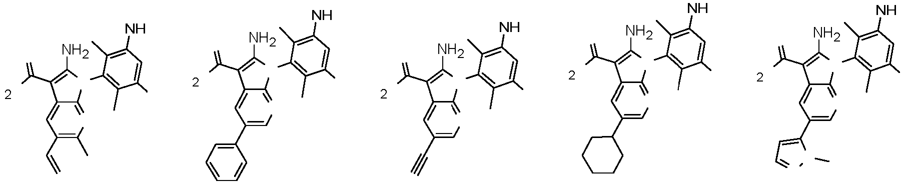

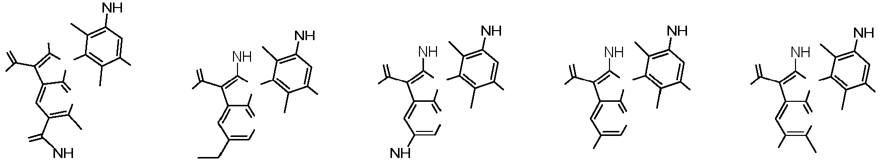

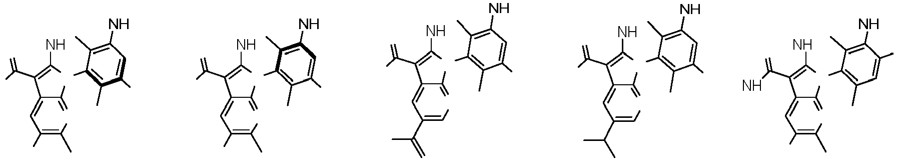

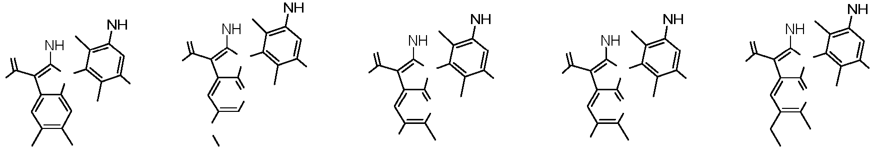

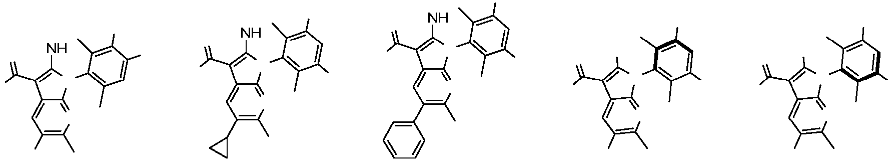

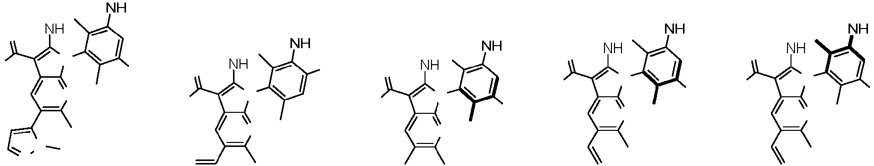

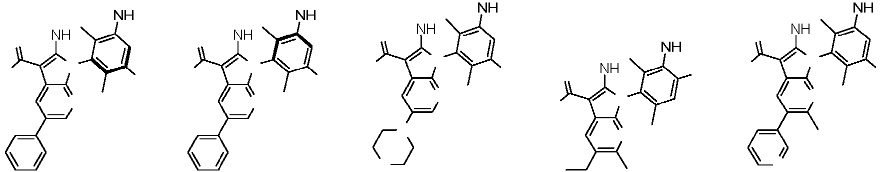

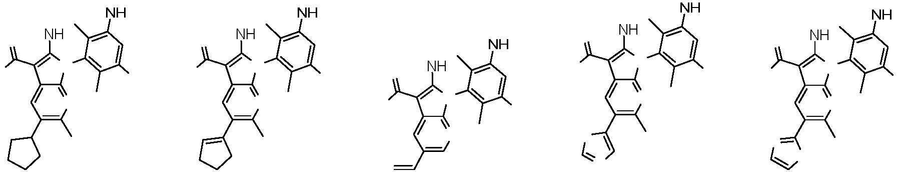

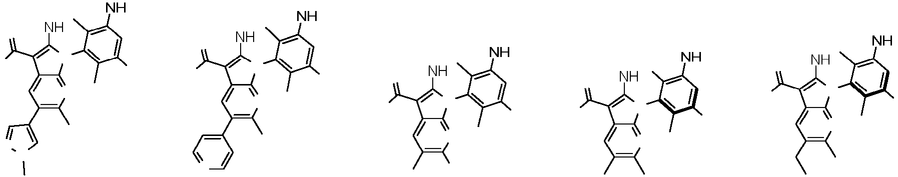

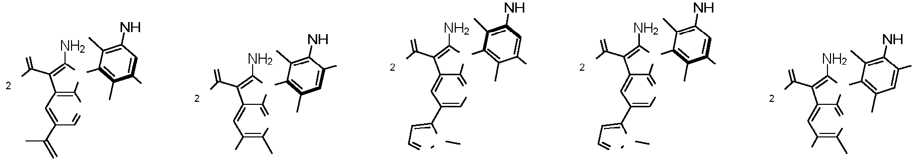

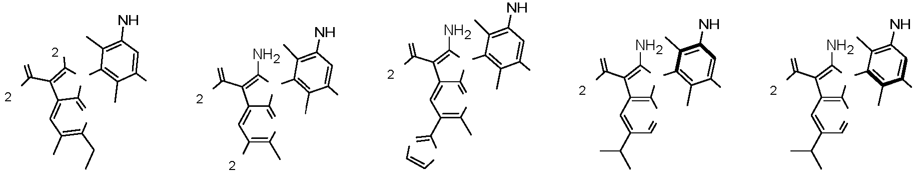

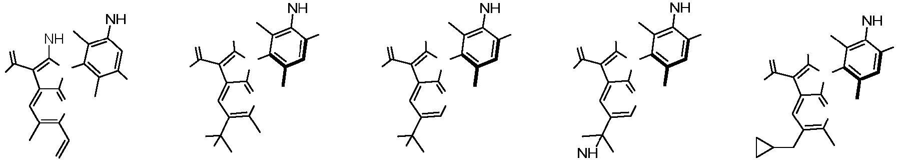

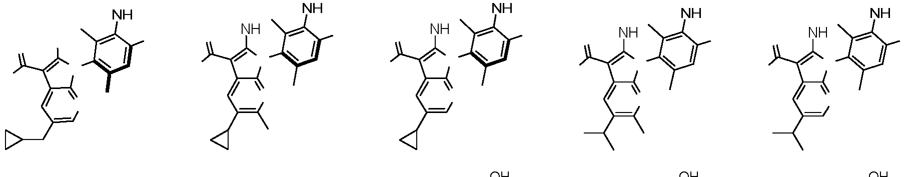

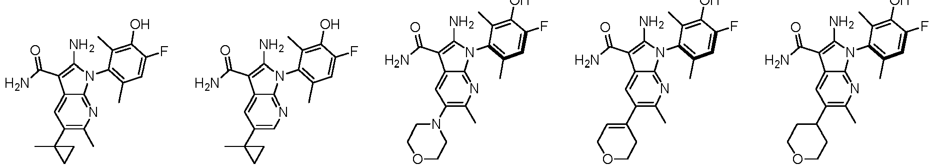

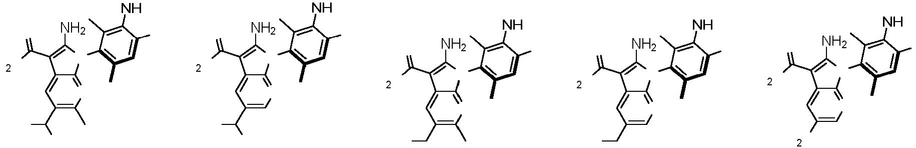

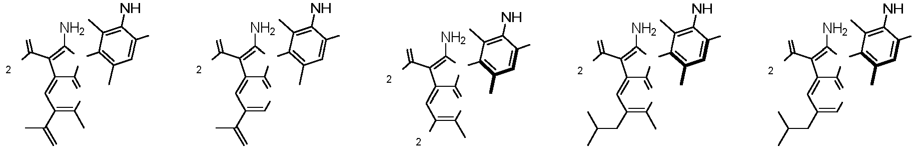

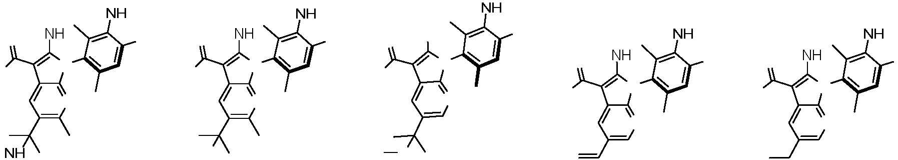

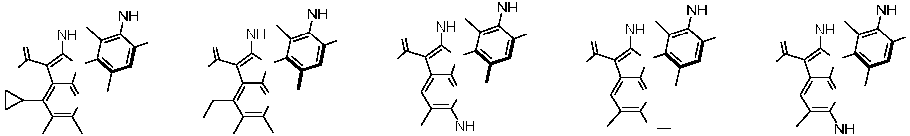

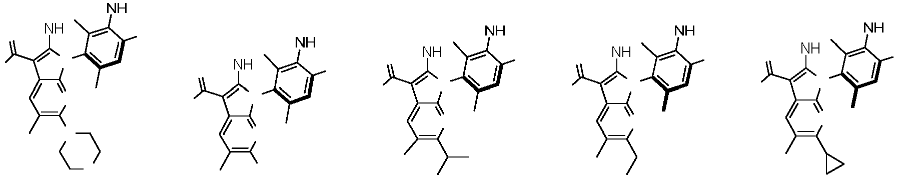

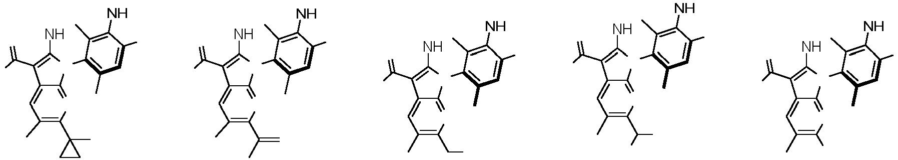

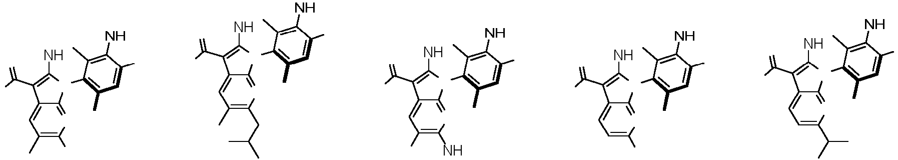

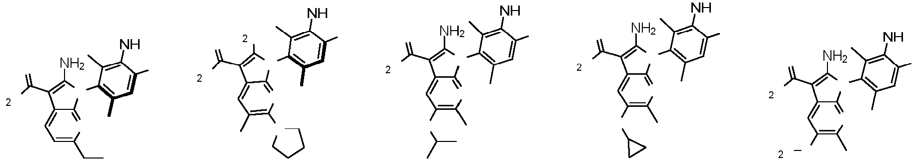

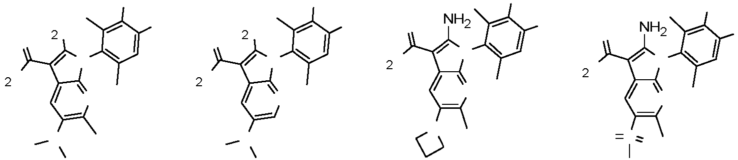

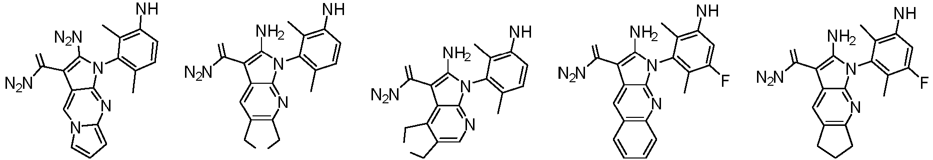

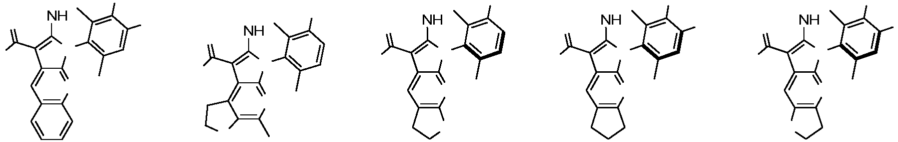

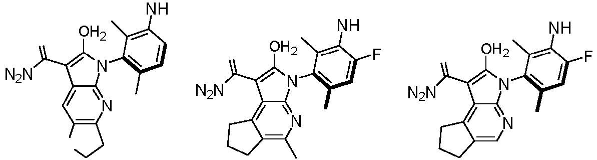

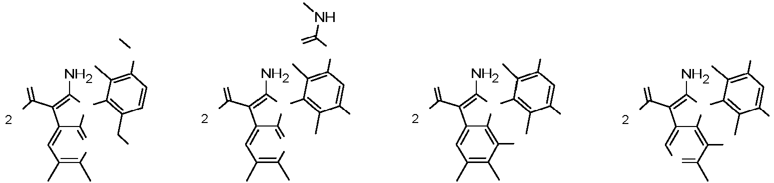

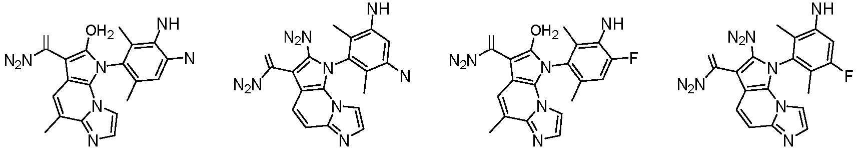

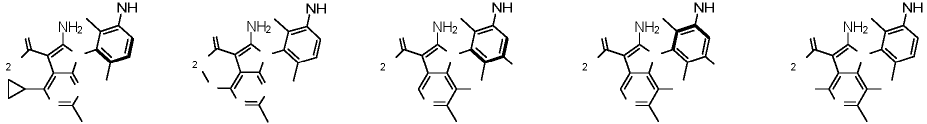

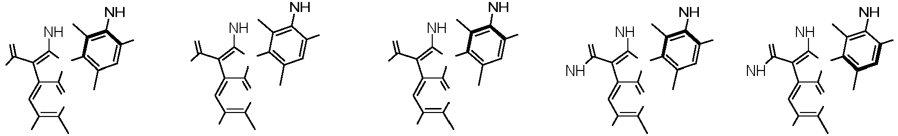

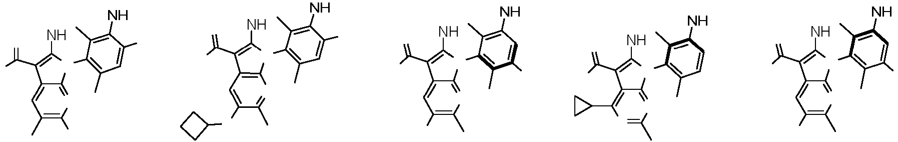

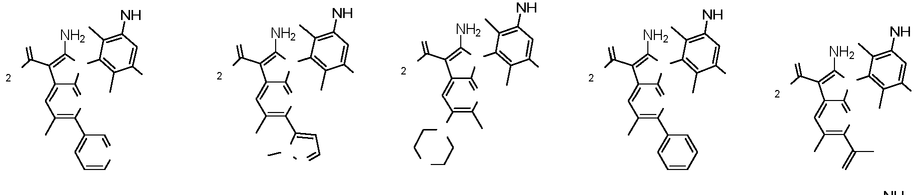

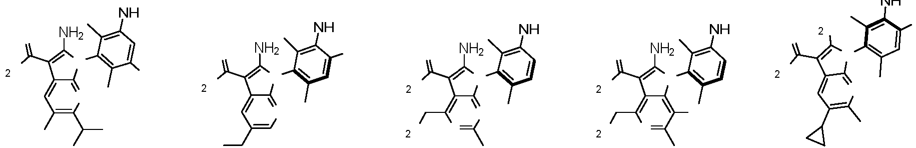

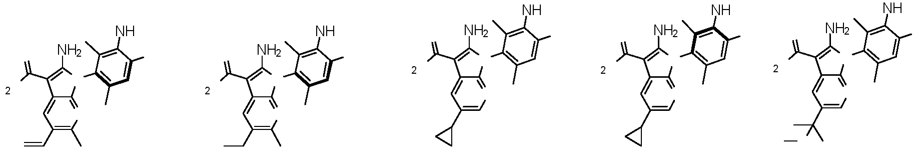

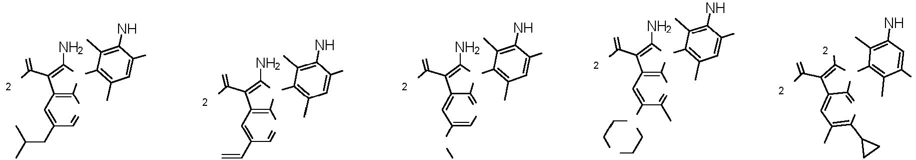

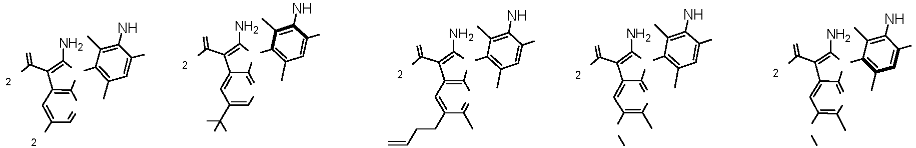

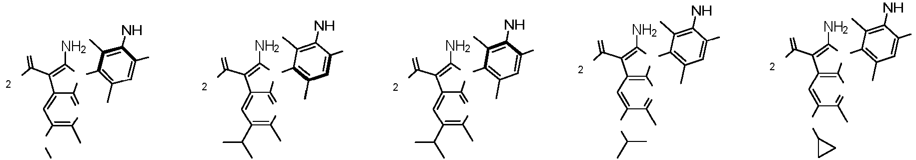

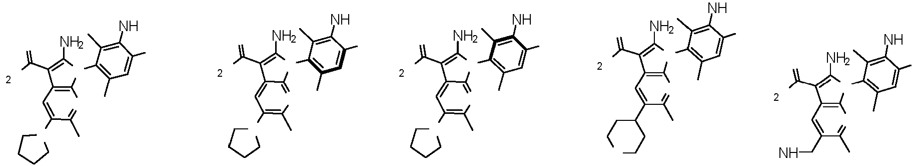

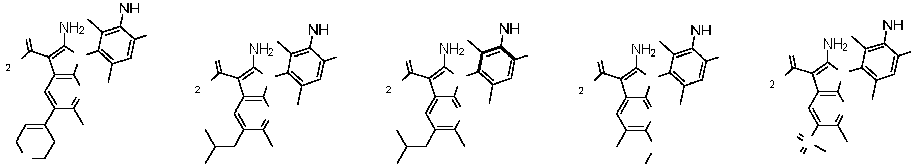

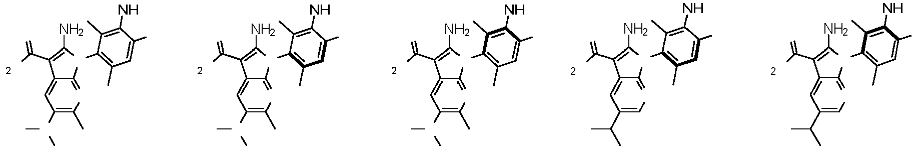

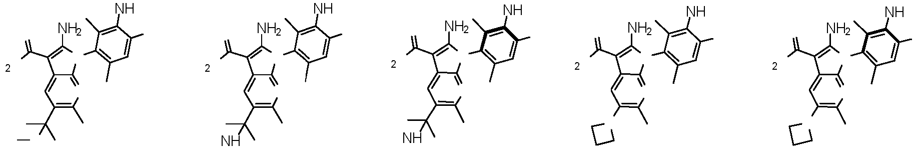

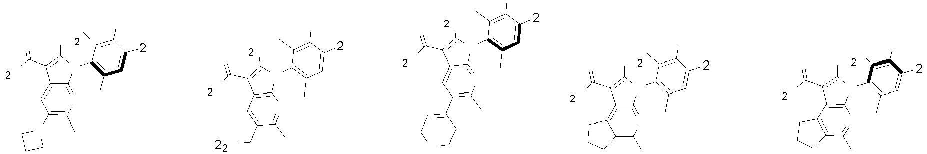

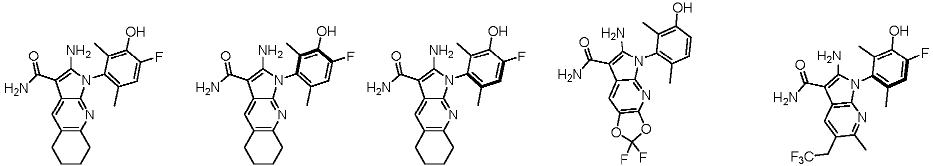

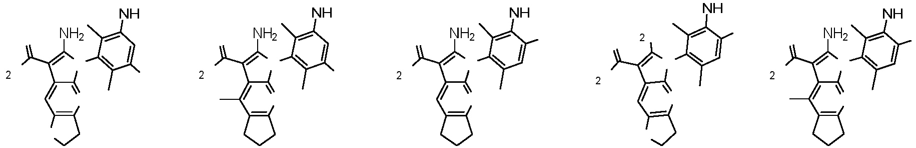

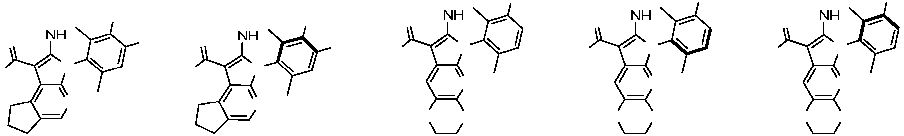

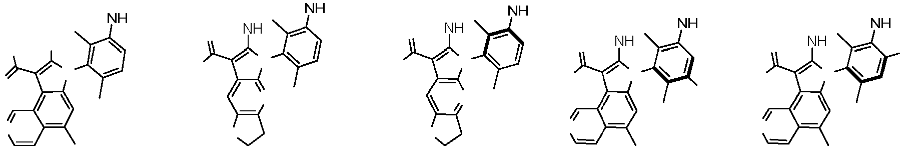

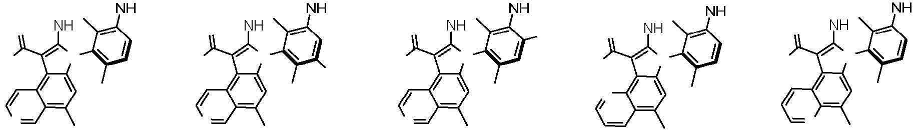

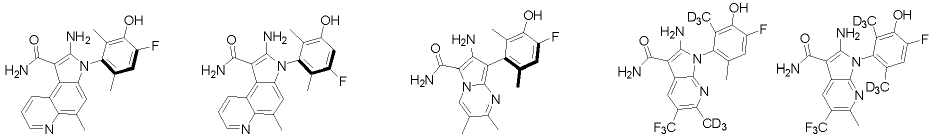

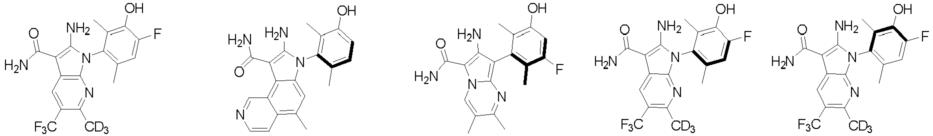

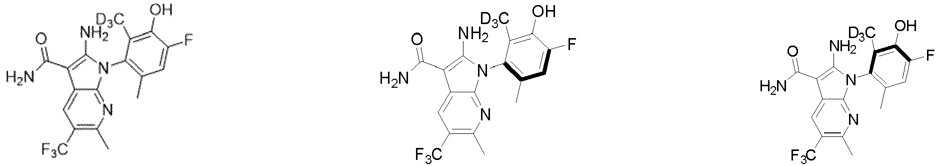

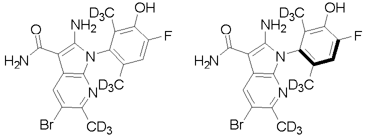

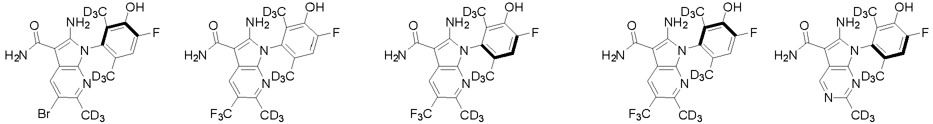

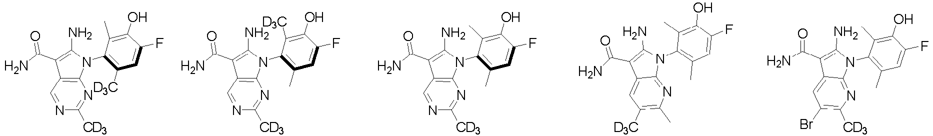

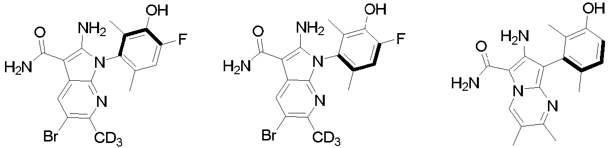

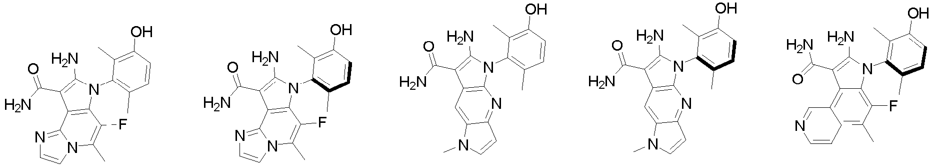

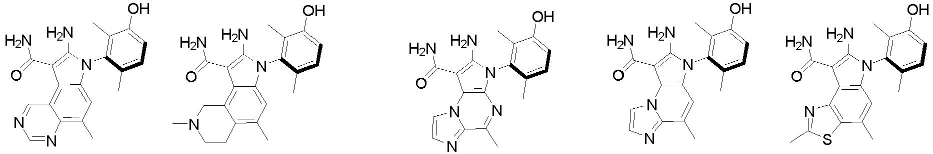

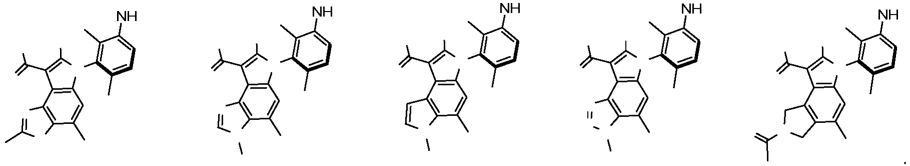

26. A pharmaceutical composition, comprising the compound, the pharmaceutically acceptable salt thereof, or the isomer thereof according to any one of claims 1 to 25, and one or more pharmaceutically acceptable carriers.

27. A use of the compound, the pharmaceutically acceptable salt thereof, or the isomer thereof according to any one of claims 1 to 25 in the manufacture of a medicament for the prevention and/or treatment of a tumor disease mediated by PKMYT1; the tumor disease mediated by PKMYT1 is preferably a tumor disease mediated by PKMYT1 caused by CCNE1 overexpression and/or FBXW7 inactivating mutation.

28. A method for treating a tumor disease mediated by PKMYT1, comprising administering to a subject a therapeutically or preventively effective amount of the compound, the pharmaceutically acceptable salt thereof, or the isomer thereof according to any one of claims 1 to 25; the tumor disease mediated by PKMYT1 is preferably a tumor disease mediated by PKMYT1 caused by CCNE1 overexpression and/or FBXW7 inactivating mutation.

29. The use according to claim 27 or the method according to claim 28, wherein the tumor disease is selected from one or more of ovarian cancer, breast cancer, gastric cancer, esophageal cancer, lung cancer, endometrial cancer, and colorectal cancer.
